# EUROPEAN PATENT APPLICATION

(11) **EP 4 775 220 A2**
(43) Date of publication of application: **15.07.2026**
(21) Application number: 26181174.9
(22) Date of filing: 27.05.2021
(51) Int. Cl.: A61K 39/00

(54) **NUCLEIC ACID BASED COMBINATION VACCINES**

(30) Priority: 29.05.2020 WO PCT/EP2020/065094; 03.02.2021 WO PCT/EP2021/052458
(62) Divisional of application: 21729297.8
(71) Applicant: CureVac SE, 72076 Tübingen (DE)
(72) Inventor: OOSTVOGELS, Cornelia, 72076 Tübingen (DE); PETSCH, Benjamin, 72076 Tübingen (DE); RAUCH, Susanne, 72076 Tübingen (DE); SCHWENDT, Kim Ellen, 72076 Tübingen (DE)
(74) Representative: Graf von Stosch Patentanwaltsgesellschaft mbH

(57) **Abstract**

The present invention is *inter alia* directed to pharmaceutical compositions comprising at least one nucleic acid encoding at least one antigenic peptide or protein from a Coronavirus, *preferably* a pandemic Coronavirus, and at least one nucleic acid encoding at least one antigenic peptide or protein from a further virus, e.g. an Influenza virus or an RSV virus. Pharmaceutical compositions provided herein are suitable for use in treatment or prophylaxis of an infection with at least one Coronavirus and at least one further virus infection, and may therefore be comprised in a combination vaccine. The nucleic acid sequences of the pharmaceutical compositions and combination vaccines are preferably in association with a polymeric carrier, a polycationic protein or peptide, or a lipid nanoparticle (LNP). The invention is also directed to first and second and further medical uses of the pharmaceutical compositions and combination vaccines, and to methods of treating or preventing a Coronavirus infection and a further virus infection.

## Description

### Introduction:

The present invention is *inter alia* directed to pharmaceutical compositions comprising at least one nucleic acid encoding at least one antigenic peptide or protein from a Coronavirus, *preferably* a pandemic Coronavirus, and at least one nucleic acid encoding at least one antigenic peptide or protein from a further virus, e.g. an Influenza virus or an RSV virus. Pharmaceutical compositions provided herein are suitable for use in treatment or prophylaxis of an infection with at least one Coronavirus and at least one further virus infection, and may therefore be comprised in a combination vaccine. The nucleic acid sequences of the pharmaceutical compositions and combination vaccines are preferably in association with a polymeric carrier, a polycationic protein or peptide, or a lipid nanoparticle (LNP). The invention is also directed to first and second and further medical uses of the pharmaceutical compositions and combination vaccines, and to methods of treating or preventing a Coronavirus infection and a further virus infection.

Coronaviruses are highly contagious, enveloped, positive single stranded RNA viruses of the Coronaviridae family. Coronaviruses (CoV) are genetically highly variable, and individual virus species can also infect several host species by overcoming the species barrier. Such transfers have resulted in infections in humans with the SARS-associated coronavirus (SARS-CoV-1), with the Middle East respiratory syndrome coronavirus (MERS-CoV), and with SARS-CoV-2 (causing COVID-19 disease).

SARS-CoV-1, which causes severe acute respiratory syndrome (SARS), infected 8422 humans and resulted in 916 deaths in 37 countries between 2002 and 2003. MERS-CoV was first identified in the Middle East in 2012. A report confirmed 1791 MERS-CoV infection cases, including at least 640 deaths in 27 countries, as of July 2016.

The Coronavirus pandemic that (most likely) started in the Chinese city of Wuhan at the turn of 2019/2020 has been attributed to a previously unknown coronavirus (SARS-CoV-2) which causes a severe respiratory disease (COVID-19). By end of Mai 2020, there were almost 6,000,000 confirmed cases of a SARS-CoV-2 infection, spreading across almost every country in the world, with more than 350,000 COVID-19 associated deaths.

These current Coronavirus outbreaks show the substantial risk of a severe global pandemic that can be caused by Coronaviruses. It would be fundamentally important for the global health to provide a vaccine that provides protection against (a pandemic) Coronavirus. To prevent re-occurrence of such a Coronavirus outbreak, it would also be advantageous to provide a combination vaccine (or multipathogen vaccine) that protects against a Coronavirus infection and at least one further virus infection, preferably a further infection with a virus that also causes respiratory diseases. Further, as the elderly population is strongly affected by such viruses, it would be advantageous to provide a combination vaccine that is efficient in the elderly population.

An example of such a suitable further virus is an Influenza virus. Influenza viruses are enveloped RNA viruses. Three genera of this family, influenza virus A, B and C, cause influenza in humans. They differ with respect to host range, variability of the surface glycoproteins, genome organization and morphology. Influenza virus A and B are further classified, based on the viral surface proteins hemagglutinin (HA) and neuraminidase (NA). Usually, two to three different strains of influenza circulate concurrently during an influenza season. Typical influenza epidemics cause increases in incidence of pneumonia and lower respiratory disease by increased rates of hospitalization or mortality. The elderly or those with underlying chronic diseases are most likely to experience such complications, but young infants also may suffer severe disease. It is estimated that more than
one billion cases of influenza occur globally every year, which results in 3 to 5 million cases of severe illness and 300,000 to 500,000 deaths. In addition, there are large losses both in productivity and quality of life for people who overcome mild cases of the disease in just a few days or weeks.

Besides Coronaviruses and Influenza viruses, viruses of the Pneumoviridae virus family also cause severe respiratory diseases. Members of the Pneumoviridae virus family include e.g. Respiratory syncytial virus (RSV), and Metapneumovirus (hMPV). RSV is the most common cause of bronchiolitis and pneumonia among children in their first year of life. RSV also causes repeated infections including severe lower respiratory tract disease, which may occur at any age, especially among the elderly or those with compromised cardiac, pulmonary, or immune systems. hPMV is an important cause of viral lower respiratory tract illness in young children. The seasonal epidemiology of hMPV appears to be similar to that of RSV, but the incidence of infection and illness appears to be substantially lower. However, hMPV is nearly as common and as severe as influenza in older adults. hMPV is associated with more severe disease in people with asthma and adults with chronic obstructive pulmonary disease (COPD). Numerous outbreaks of hMPV have been reported in long-term care facilities for children and adults, causing fatalities.

Besides Coronaviruses, Influenza viruses, and Pneumoviridae, viruses of the Paramyxoviridae family can cause severe respiratory diseases. Members of the Paramyxoviridae virus family include Parainfluenza virus (PIV) and Henipavirus virus.

Parainfluenza virus, e.g. PIV type 3 (PIV3) is a major cause of ubiquitous acute respiratory infections of infancy and early childhood. Its incidence peaks around 4-12 months of age, and the virus is responsible for 3-10% of hospitalizations, mainly for bronchiolitis and pneumonia. PIV3 can be fatal, and in some instances is associated with neurologic diseases, such as febrile seizures. It can also result in airway remodelling, a significant cause of morbidity. In developing regions of the world, infants and young children are at the highest risk of mortality, either from primary PIV3 viral infection or a secondary consequences, such as bacterial infections.

Henipavirus, including Hendra virus and Nipah virus, is a source of recently emerging potentially pandemic diseases. Hendra virus was first recognized in 1994 after an outbreak of respiratory illness among twenty horses and two humans in Hendra, Queensland, Australia. In 1995, a second unrelated outbreak was identified that had occurred in August 1994 in Mackay, Queensland, in which two horses died and one human became. The fatality rate has been reported at more than 70% in horses and 50% in humans. The Nipah virus was initially isolated in 1999 upon examining samples from an outbreak of encephalitis and respiratory illness among adult men in Malaysia and Singapore. The host for Nipah virus is still unknown, but flying foxes are suspected to be the natural host. Infection with Nipah virus in humans has been associated with encephalitis characterized by fever and drowsiness and more serious central nerve system disease, such as coma, seizures and inability to maintain breathing. Some patients have had a respiratory illness during the early part of their infections. During a Nipah virus disease outbreak in 1998-1999, about 40% of the patients with serious nerve disease who entered hospitals died.

As outlined above, an object of the present invention is the provision of a nucleic-acid based combination vaccine that provides effective protection against at least one Coronavirus, in particular against at least one pandemic Coronavirus, and additionally against at least one further virus, e.g. selected from at least one Influenza virus, at least one Pneumoviridae virus, and/or at least one Paramyxoviridae virus.

It is a big challenge to develop combination vaccines as the different components of such a vaccine are often not compatible with each other. Further, different vaccine components may required different vaccination intervals to be effective, which impedes the development of a combination vaccine. In addition to that, it is important that the all components of a combination vaccine induce efficient immune responses and that single components are not immuno dominant, or that the components do not show immune-interference. In particular, a vaccine is needed to protect the elderly population where high mortality rates in the case of SARS-CoV-2 have been observed.

Nucleic acid based vaccination, including DNA or RNA, represents a promising technique for novel vaccines against emerging viruses and for the provision of combination vaccines. Nucleic acids can be genetically engineered and administered to a human subject. Transfected cells directly produce the encoded antigen (e.g. provided by a DNA or an RNA, in particular an mRNA), which results in protective immunological responses.

A pivotal role for virus-specific memory T-cells in broad and long-term protection against SARS-CoV infection has been elucidated (see e.g. Channappanavar, Rudragouda, et al. "Virus-specific memory CD8 T cells provide substantial protection from lethal severe acute respiratory syndrome coronavirus infection. " Joumal of virology 88.19 (2014): 11034-11044). Virus-specific CD8 T cells are e.g. required for pathogen clearance and for mediating protection after viral challenge. An effective combination vaccine, e.g. comprising a SARS-CoV-2 vaccine, should therefore not only induce strong functional humoral immune responses against the respective pathogen, but also induce pathogen specific CD8+ T-cell and CD4+ T-cell responses, e.g. SARS-CoV-2 specific CD8+ T-cell and CD4+ T-cell responses.

Therefore, it is the object of the underlying invention to provide a nucleic acid based combination vaccine or multipathogen vaccine that provides protection against Coronavirus infections and protection against at least one further virus.

### Definitions

For the sake of clarity and readability the following definitions are provided. Any technical feature mentioned for these definitions may be read on each and every embodiment of the invention. Additional definitions and explanations may be specifically provided in the context of these embodiments.

Percentages in the context of numbers should be understood as relative to the total number of the respective items. In other cases, and unless the context dictates otherwise, percentages should be understood as percentages by weight (wt.⁻%). About: The term "about" is used when determinants or values do not need to be identical, i.e. 100% the same. Accordingly, "about" means, that a determinant or values may diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, 20%. The skilled person will know that e.g. certain parameters or determinants may slightly vary based on the method how the parameter was determined. For example, if a certain determinants or value is defined herein to have e.g. a length of "about 1000 nucleotides", the length may diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, 20%. Accordingly, the skilled person will know that in that specific example, the length may diverge by 1 to 200 nucleotides, preferably by 1 to 200 nucleotides; in particular, by 5, 10, 20, 30, 40, 50, 60, 70, 80, 90, 100, 110, 120, 130, 140, 150, 160, 170, 180, 190, 200 nucleotides.

Adaptive immune response: The term "adaptive immune response" as used herein will be recognized and understood by the person of ordinary skill in the art, and is e.g. intended to refer to an antigen-specific response of the immune system (the adaptive immune system). Antigen specificity allows for the generation of responses that are tailored to specific pathogens or pathogen-infected cells. The ability to mount these tailored responses is usually maintained in the body by "memory cells" (B-cells). In the context of the invention, the antigen is provided by the nucleic acid (e.g. an RNA or a DNA) encoding at least one antigenic peptide or protein derived from coronavirus (component A), or the further virus (component B).

Antigen: The term "antigen" as used herein will be recognized and understood by the person of ordinary skill in the art, and is e.g. intended to refer to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. Also fragments, variants and derivatives of peptides or proteins comprising at least one epitope are understood as antigens in the context of the invention. In the context of the present invention, an antigen may be the product of translation of a provided nucleic acid as specified herein.

Antigenic peptide or protein: The term "antigenic peptide or protein" or "immunogenic peptide or protein" will be recognized and understood by the person of ordinary skill in the art, and is e.g. intended to refer to a peptide, protein derived from a (antigenic or immunogenic) protein which stimulates the body's adaptive immune system to provide an adaptive immune response. Therefore an antigenic/immunogenic peptide or protein comprises at least one epitope (as defined herein) or antigen (as defined herein) of the protein it is derived from (e.g., spike protein (S) of coronavirus (preferably from SARS-CoV-2), HA of influenza virus, F protein of RSV virus ect.).

Cationic: Unless a different meaning is clear from the specific context, the term "cationic" means that the respective structure bears a positive charge, either permanently or not permanently, but in response to certain conditions such as pH. Thus, the term "cationic" covers both "permanently cationic" and "cationisable".

Cationisable: The term "cationisable" as used herein means that a compound, or group or atom, is positively charged at a lower pH and uncharged at a higher pH of its environment. Also in non-aqueous environments where no pH value can be determined, a cationisable compound, group or atom is positively charged at a high hydrogen ion concentration and uncharged at a low concentration or activity of hydrogen ions. It depends on the individual properties of the cationisable or polycationisable compound, in particular the pKa of the respective cationisable group or atom, at which pH or hydrogen ion concentration it is charged or uncharged. In diluted aqueous environments, the fraction of cationisable compounds, groups or atoms bearing a positive charge may be estimated using the so-called Henderson-Hasselbalch equation which is well-known to a person skilled in the art. E.g., in some embodiments, if a compound or moiety is cationisable, it is preferred that it is positively charged at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4, i.e. under physiological conditions, particularly under physiological salt conditions of the cell in vivo. In other embodiments, it is preferred that the cationisable compound or moiety is predominantly neutral at physiological pH values, e.g. about 7.0-7.4, but becomes positively charged at lower pH values. In some embodiments, the preferred range of pKa for the cationisable compound or moiety is about 5 to about 7.

Coding sequence/coding region: The terms "coding sequence" or "coding region" and the corresponding abbreviation "cds" as used herein will be recognized and understood by the person of ordinary skill in the art, and are e.g. intended to refer to a sequence of several nucleotide triplets, which may be translated into a peptide or protein. A coding sequence in the context of the present invention may be a DNA sequence, preferably an RNA sequence, consisting of a number of nucleotides that may be divided by three, which starts with a start codon and which preferably terminates with a stop codon.

Derived from: The term "derived from" as used throughout the present specification in the context of a nucleic acid, i.e. for a nucleic acid "derived from" (another) nucleic acid, means that the nucleic acid, which is derived from (another) nucleic acid, shares e.g. at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% sequence identity with the nucleic acid from which it is derived. The skilled person is aware that sequence identity is typically calculated for the same types of nucleic acids, i.e. for DNA sequences or for RNA sequences. Thus, it is understood, if a DNA is "derived from" an RNA or if an RNA is "derived from" a DNA, in a first step the RNA sequence is converted into the corresponding DNA sequence (in particular by replacing the uracils (U) by thymidines (T) throughout the sequence) or, vice versa, the DNA sequence is converted into the corresponding RNA sequence (in particular by replacing the T by U throughout the sequence). Thereafter, the sequence identity of the DNA sequences or the sequence identity of the RNA sequences is determined. Preferably, a nucleic acid "derived from" a nucleic acid also refers to nucleic acid, which is modified in comparison to the nucleic acid from which it is derived, e.g. in order to increase RNA stability even further and/or to prolong and/or increase protein production. In the context of amino acid sequences (e.g. antigenic peptides or proteins) the term "derived from" means that the amino acid sequence, which is derived from (another) amino acid sequence, shares e.g. at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% sequence identity with the amino acid sequence from which it is derived.

Epitope: The term "epitope" (also called "antigen determinant" in the art) as used herein will be recognized and understood by the person of ordinary skill in the art, and is e.g. intended to refer to T cell epitopes and B cell epitopes. T cell epitopes or parts of the antigenic peptides or proteins and may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 to about 20 or even more amino acids. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens, preferably having 5 to 15 amino acids, more preferably having 5 to 12 amino acids, even more preferably having 6 to 9 amino acids, which may be recognized by antibodies, i.e. in their native form. Such epitopes of proteins or peptides may furthermore be selected from any of the herein mentioned variants of such proteins or peptides. In this context epitopes can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment; The term "fragment" as used throughout the present specification in the context of a nucleic acid sequence (e.g. RNA or a DNA) or an amino acid sequence may typically be a shorter portion of a full-length sequence of e.g. a nucleic acid sequence or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 40%, 50%, 60%, 70%, 80%, 90%, 95% of the total (i.e. full-length) molecule from which the fragment is derived (e.g. spike protein (S) of coronavirus, HA/NA of an influenza virus, ect.). The term "fragment" as used throughout the present specification in the context of proteins or peptides may, typically, comprise a sequence of a protein or peptide as defined herein, which is, with regard to its amino acid sequence, N-terminally and/or C-terminally truncated compared to the amino acid sequence of the original protein. Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide. Fragments of proteins or peptides may comprise at least one epitope of those proteins or peptides.

Heterologous: The terms "heterologous" or "heterologous sequence" as used throughout the present specification in the context of a nucleic acid sequence or an amino acid sequence refers to a sequence (e.g. RNA, DNA, amino acid) has to be understood as a sequence that is derived from another gene, another allele, or e.g. another species or virus. Two sequences are typically understood to be "heterologous" if they are not derivable from the same gene or from the same allele. I.e., although heterologous sequences may be derivable from the same organism or virus, in nature, they do not occur in the same nucleic acid or protein.

Humoral immune response: The terms "humoral immunity" or "humoral immune response" will be recognized and understood by the person of ordinary skill in the art, and are e.g. intended to refer to B-cell mediated antibody production and optionally to accessory processes accompanying antibody production. A humoral immune response may be typically characterized, e.g. by Th2 activation and cytokine production, germinal center formation and isotype switching, affinity maturation and memory cell generation. Humoral immunity may also refer to the effector functions of antibodies, which include pathogen and toxin neutralization, classical complement activation, and opsonin promotion of phagocytosis and pathogen elimination.

Identity (of a sequence): The term "identity" as used throughout the present specification in the context of a nucleic acid sequence or an amino acid sequence will be recognized and understood by the person of ordinary skill in the art, and is e.g. intended to refer to the percentage to which two sequences are identical. To determine the percentage to which two sequences are identical, e.g. nucleic acid sequences or amino acid (aa) sequences as defined herein, preferably the aa sequences encoded by the nucleic acid sequence as defined herein or the aa sequences themselves, the sequences can be aligned in order to be subsequently compared to one another. Therefore, e.g. a position of a first sequence may be compared with the corresponding position of the second sequence. If a position in the first sequence is occupied by the same residue as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using an algorithm, e.g. an algorithm integrated in the BLAST program.

immunogen, immunogenic: The terms "immunogen" or "immunogenic" will be recognized and understood by the person of ordinary skill in the art, and are e.g. intended to refer to a compound that is able to stimulate/induce an immune response. Preferably, an immunogen is a peptide, polypeptide, or protein. An immunogen in the sense of the present invention is the product of translation of a provided nucleic acid (component A and B), comprising at least one coding sequence encoding at least one antigenic peptide, protein derived from e.g. a coronavirus spike protein (S) (preferably from SARS-CoV-2) as defined herein. Typically, an immunogen elicits an adaptive immune response.

Immune response: The term "immune response" will be recognized and understood by the person of ordinary skill in the art, and is e.g. intended to refer to a specific reaction of the adaptive immune system to a particular antigen (so called specific or adaptive immune response) or an unspecific reaction of the innate immune system (so called unspecific or innate immune response), or a combination thereof.

Immune system: The term "immune system" will be recognized and understood by the person of ordinary skill in the art, and is e.g. intended to refer to a system of the organism that may protect the organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Innate immune system: The term "innate immune system" (also known as non-specific or unspecific immune system) will be recognized and understood by the person of ordinary skill in the art, and is e.g. intended to refer to a system typically comprising the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be activated by ligands of pattern recognition receptor e.g. Toll-like receptors, NOD-like receptors, or RIGI like receptors etc..

Lipidoid compound: A lipidoid compound, also simply referred to as lipidoid, is a lipid-like compound, i.e. an amphiphilic compound with lipid-like physical properties. In the context of the present invention, the term lipid is considered to encompass lipidoid compounds.

Nucleic acid, nucleic acid molecule: The terms "nucleic acid" or "nucleic acid molecule" as used herein, in particular as used herein in the context of component A and component B, will be recognized and understood by the person of ordinary skill in the art. The terms "nucleic acid" or "nucleic acid molecule" preferably refers to DNA (molecules) or RNA (molecules). The term is used synonymously with the term polynucleotide. Preferably, a nucleic acid or a nucleic acid molecule is a polymer comprising or consisting of nucleotide monomers that are covalently linked to each other by phosphodiester-bonds of a sugar/phosphate-backbone. The terms "nucleic acid" or "nucleic acid molecule" also encompasses modified nucleic acid (molecules), such as base-modified, sugar-modified or backbone-modified DNA or RNA (molecules) as defined herein. Accordingly, the nucleic acid of component A and component B may be a DNA or an RNA.

Nucleic acid sequence, DNA sequence, RNA sequence: The terms "nucleic acid sequence", "DNA sequence", "RNA sequence" will be recognized and understood by the person of ordinary skill in the art, and e.g. refer to a particular and individual order of the succession of its nucleotides.

Multivalent vaccine or combination vaccine: Both terms have to be understood interchangeably herein. A multivalent vaccine or combination vaccine of the invention provides more than one valence (e.g. an antigen) derived from more than one virus (e.g. at least one Coronavirus as defined herein and at least one further virus as defined herein). The nucleic acid based combination vaccine may also be considered as a "multipathogen vaccine".

Permanently cationic: The term "permanently cationic" as used herein will be recognized and understood by the person of ordinary skill in the art, and means, e.g., that the respective compound, or group, or atom, is positively charged at any pH value or hydrogen ion activity of its environment. Typically, the positive charge results from the presence of a quaternary nitrogen atom. Where a compound carries a plurality of such positive charges, it may be referred to as permanently polycationic.

Stabilized RNA: The term "stabilized RNA" refer to an RNA that is modified such, that it is more stable to disintegration or degradation, e.g., by environmental factors or enzymatic digest, such as by exo- or endonuclease degradation, compared to an RNA without such modification. Preferably, a stabilized RNA in the context of the present invention is stabilized in a cell, such as a prokaryotic or eukaryotic cell, preferably in a mammalian cell, such as a human cell. The stabilization effect may also be exerted outside of cells, e.g. in a buffer solution etc., e.g., for storage of a composition comprising the stabilized RNA.

T-cell responses: The terms "cellular immunity" or "cellular immune response" or "cellular T-cell responses" as used herein will be recognized and understood by the person of ordinary skill in the art, and are for example intended to refer to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface.

Variant (of a sequence): The term "variant" as used throughout the present specification in the context of a nucleic acid sequence will be recognized and understood by the person of ordinary skill in the art, and is e.g. intended to refer to a variant of a nucleic acid sequence derived from another nucleic acid sequence. E.g., a variant of a nucleic acid sequence may exhibit one or more nucleotide deletions, insertions, additions and/or substitutions compared to the nucleic acid sequence from which the variant is derived. A variant of a nucleic acid sequence may at least 50%, 60%, 70%, 80%, 90%, or 95% identical to the nucleic acid sequence the variant is derived from. The variant is a functional variant in the sense that the variant has retained at least 50%, 60%, 70%, 80%, 90%, or 95% or more of the function of the sequence where it is derived from. A "variant" of a nucleic acid sequence may have at least 70%, 75%, 80%, 85%, 90%, 95%, 98% or 99% nucleotide identity over a stretch of at least 10, 20, 30, 50, 75 or 100 nucleotide of such nucleic acid sequence.

The term "variant" as used throughout the present specification in the context of proteins or peptides is e.g. intended to refer to a proteins or peptide variant having an amino acid sequence which differs from the original sequence in one or more mutation(s)/substitution(s), such as one or more substituted, inserted and/or deleted amino acid(s). Preferably, these fragments and/or variants have the same, or a comparable specific antigenic property (immunogenic variants, antigenic variants). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra). A "variant" of a protein or peptide may have at least 70%, 75%, 80%, 85%, 90%, 95%, 98% or 99% amino acid identity over a stretch of at least 10, 20, 30, 50, 75 or 100 amino acids of such protein or peptide. Preferably, a variant of a protein comprises a functional variant of the protein, which means, in the context of the invention, that the variant exerts essentially the same, or at least 40%, 50%, 60%, 70%, 80%, 90% of the immunogenicity as the protein it is derived from.

### Short description of the invention

As further defined in the claims and the underlying description, these objects of the invention are *inter alia* solved by providing a pharmaceutical composition comprising or consisting of a nucleic acid, e.g. an RNA or a DNA, comprising at least one coding sequence encoding at least one antigenic peptide or protein from a Coronavirus ("component A"), and a nucleic acid, e.g. an RNA or a DNA, comprising at least one coding sequence encoding at least one antigenic peptide or protein from at least one further virus ("component B)".

Suitably, the pharmaceutical composition or the combination vaccine of the invention has at least some of the following advantageous features:
- Translation of the nucleic acid of component A and component B at the site of injection/vaccination (e.g. muscle);
- Induction of antigen-specific immune responses against all the encoded proteins (provided by component A and component B), suitably at a very low dosage and dosing regimen;
- Suitability for vaccination of infants and/or newborns or the elderly, in particular the elderly;
- Suitability of the composition/vaccine for intramuscular administration;
- Induction of specific and functional humoral immune response against coronavirus, preferably SARS-CoV-2 (component A) and against the at least one further virus (component B);
- Induction of broad, functional cellular T-cell responses against coronavirus, preferably SARS-CoV-2 (component A) and against the at least one further virus (component B);
- Induction of specific B-cell memory against coronavirus, preferably SARS-CoV-2 (component A) and the at least one further virus (component B);
- Induction of functional antibodies that can effectively neutralize the Coronavirus, e.g. SARS-CoV-2, and the at least one further virus;
- Induction of functional antibodies that can effectively neutralize emerging variants of a Coronavirus, e.g. SARS-CoV-2;
- Eliciting of mucosal IgA immunity by inducing of mucosal IgA antibodies;
- Induction of a well-balanced B cell and T cell responses;
- Induction of protective immunity against coronavirus infection, e.g. against SARS-CoV-2 or emerging variants thereof;
- Fast onset of immune protection against coronavirus, preferably SARS-CoV-2 (component A) and the at least one further virus (component B);
- Longevity of the induced immune responses against coronavirus, preferably SARS-CoV-2 (component A) and the at least one further virus (component B);
- No enhancement of a virus infection (e.g. SARS-CoV-2 infection) due to vaccination or immunopathological effects;
- No antibody dependent enhancement (ADE) caused by the nucleic acid based combination vaccine;
- No excessive induction of systemic cytokine or chemokine response after application of the vaccine, which could lead to an undesired high reactogenicity upon vaccination;
- Well tolerability, no side-effects, non-toxicity of the combination vaccine;
- Advantageous stability characteristics of the nucleic acid-based combination vaccine;
- Speed, adaptability, simplicity and scalability of combination vaccine production;
- Advantageous vaccination regimen that only requires one or two vaccination for sufficient protection;
- Advantageous vaccination regimen that only requires a low dose of the composition/vaccine for sufficient protection.

The present invention is based on the inventor's surprising finding that a pharmaceutical composition comprising at least one nucleic acid encoding at least one peptide or protein from a Coronavirus ("component A") and at least one nucleic acid encoding at least one peptide or protein from a further virus ("component B") can efficiently be co-expressed in human cells. Even more surprising and unexpected, the administration of such a pharmaceutical compositions comprising said components induces antigen-specific immune responses against the encoded coronavirus antigen and against the at least one further virus antigen. Those findings are the basis for a nucleic acid based combination vaccine of the invention that provides protection against at least one Coronavirus, e.g. a pandemic Coronavirus, and at least one further virus, e.g. an influenza virus and/or an RSV virus (see Example section).

In a first aspect, the present invention provides pharmaceutical compositions comprising or consisting of at least one component A comprising at least one nucleic acid comprising at least one coding sequence encoding at least one antigenic peptide or protein selected or derived from at least one Coronavirus, or an immunogenic fragment or immunogenic variant thereof, and at least one component B comprising at least one nucleic acid comprising at least one coding sequence encoding at least one antigenic peptide or protein selected or derived from at least one further virus, or an immunogenic fragment or immunogenic variant thereof.

The at least one Coronavirus of component A may suitably be derived or selected from a pandemic Coronavirus, e.g. SARS-CoV-1, SARS-CoV-2, MERS-CoV. The at least one further virus of component B may suitably be derived or selected from at least one further virus, e.g. at least one different Coronavirus, at least one Influenza virus, at least one Pneumoviridae virus (e.g. Respiratory syncytial virus, and/or at least one Metapneumovirus), and/or at least one Paramyxoviridae virus (e.g. Parainfluenza virus, and/or at least one Henipavirus).

In a second aspect, the present invention provides combination vaccines, wherein the combination vaccines comprise the pharmaceutical compositions (including component A and component B) as defined in the first aspect. Accordingly, the second aspect relates to multipathogen vaccines.

In a third aspect, the present invention provides kits or kits of parts comprising at least one pharmaceutical composition of first aspect (including component A and component B), and/or at least one combination vaccine of the second aspect.

Further aspects of the invention concern methods of treating or preventing multiple virus infections in a subject, and first and second medical uses of the pharmaceutical compositions, the combination vaccines, or the kits. Also provided are methods of manufacturing the pharmaceutical compositions, or the combination vaccines.

### Detailed Description of the invention

The present application is filed together with a sequence listing in electronic format, which is part of the description (WIPO standard ST.25). The information contained in the sequence listing is incorporated herein by reference in its entirety. Where reference is made herein to a "SEQ ID NO", the corresponding nucleic acid sequence or amino acid (aa) sequence in the sequence listing having the respective identifier is referred to. For many sequences, the sequence listing also provides additional detailed information, e.g. regarding certain structural features, sequence optimizations, GenBank (NCBI) or GISAID (epi) identifiers, or additional detailed information regarding its coding capacity. In particular, such information is provided under numeric identifier <223> in the WIPO standard ST.25 sequence listing. Accordingly, information provided under said numeric identifier <223> is explicitly included herein in its entirety and has to be understood as integral part of the description of the underlying invention. Where reference is made to "SEQ ID NOs" of other published patent applications or patents, said sequences, e.g. amino acid sequences or nucleic acid sequences, are explicitly incorporated herein by reference. Accordingly, these sequences constitute an integral part of the underlying description. For "SEQ **ID** NOs" that are included by reference, information provided under numeric identifier <223> (of the respective sequence protocol) is also explicitly included herein in its entirety, and has to be understood as integral part of the description of the underlying invention. Accordingly, "SEQ ID NOs" provided in WO2014160463, WO2015024668, WO2017070626, WO2017070622, WO2017172890, WO2018081318, WO2018115527, WO2018170245, WO2018078053, WO2018115507, WO2019092153, WO2019202035 are herewith incorporated by reference, and are therefore part of the description.

### Pharmaceutical composition:

In a **first aspect,** the invention relates to a pharmaceutical composition *suitable for* a combination vaccine or a multipathogen vaccine.

It has to be noted that specific features and embodiments that are described in the context of the first aspect of the invention, that is the pharmaceutical composition of the invention, are likewise applicable to the second aspect (combination vaccine of the invention), the third aspect (kit or kit of parts of the invention), or further aspects including e.g. medical uses (first and second medical uses) and e.g. method of treatments.

In a preferred embodiment, the pharmaceutical composition of the first aspect comprises or consists of
- at least one component A comprising at least one nucleic acid comprising at least one coding sequence encoding at least one antigenic peptide or protein selected or derived from at least one Coronavirus, or an immunogenic fragment or immunogenic variant thereof, and
- at least one component B comprising at least one nucleic acid comprising at least one coding sequence encoding at least one antigenic peptide or protein selected or derived from at least one further virus, or an immunogenic fragment or immunogenic variant thereof.

A nucleic acid according to the invention, e.g. the DNA or the RNA, forms the basis for a nucleic acid based pharmaceutical composition or a nucleic acid based vaccine.

Such nucleic acid based pharmaceutical composition (first aspect) or nucleic acid based vaccines (second aspect) as provided herein have advantages over classical vaccine approaches: In general, protein-based vaccines, or live attenuated vaccines are suboptimal for use in developing countries due to their high production costs. In addition, protein-based vaccines, or live attenuated vaccines require long development times and are not suitable for rapid responses of pandemic virus outbreaks such as e.g. the Coronavirus SARS-CoV-2 outbreak in 2019/2020. Furthermore, using classical approaches it remains to be a challenge to provide a combination vaccine that is effective against a Coronavirus and a further virus (e.g. problems in regard of compatibility of the individual components).

In contrast, the nucleic acid-based pharmaceutical compositions and vaccines according to the present invention allow very fast and cost-effective manufacturing. Therefore, in comparison with known vaccines, vaccine based on the inventive nucleic acid can be produced and manufactured significantly cheaper and faster, which is very advantageous particularly for use in developing countries or in the context of a global pandemic.

One further advantage of a pharmaceutical compositions or vaccines based on nucleic acid of component A and component B is that the components are temperature-stable in comparison to protein or peptide-based vaccines. Moreover, the inventors found that respective nucleic acid sequences encoding antigens selected or derived from a Coronavirus can be combined with e.g. nucleic acid sequences encoding antigens selected or derived from a further virus (e.g. a different Coronavirus, a Influenza viruses, RSV viruses, PIV, hMPV, Hendra, Nipah) without impeding the efficacy (e.g. induction of cellular and humoral immune responses) of the individual components (see Example section).

### Component A:

In various preferred embodiments, the at least one component A of the pharmaceutical composition comprises at least one nucleic acid comprising at least one coding sequence encoding at least one antigenic peptide or protein selected or derived from at least one Coronavirus, or an immunogenic fragment or immunogenic variant thereof.

Coronaviruses can be classified into the genus Alphacoronavirus, Betacoronavirus, Deltacoronavirus, Gammacoronavirus, and unclassified Coronaviruses. Coronaviruses are genetically highly variable, and individual virus species can also infect several host species by overcoming the species barrier, to potentially become pandemic.

In preferred embodiments the at least one Coronavirus of component A is selected or derived from at least one pandemic Coronavirus.

In various embodiments the at least one Coronavirus of component A, or the at least one pandemic Coronavirus of component A, is selected from at least one Alphacoronavirus, at least one Betacoronavirus, at least one Gammacoronavirus, and/or at least one Deltacoronavirus, preferably a pandemic Alphacoronavirus, Betacoronavirus, Gammacoronavirus, Deltacoronavirus.

In various embodiments, the at least one Coronavirus of component A, or the at least one pandemic Coronavirus of component A, is a Betacoronavirus. Suitably, the Betacoronavirus is selected from at least one Sarbecovirus, at least one Merbecovirus, at least one Embecovirus, at least one Nobecovirus, and/or at least one Hibecovirus.

In a preferred embodiments, the at least one Coronavirus of component A, or the at least one pandemic Coronavirus of component A, is a Betacoronavirus, preferably a Sarbecovirus. In the context of the invention, a preferred Sarbecovirus may be selected from a SARS-associated Coronavirus. Preferred SARS-associated Coronaviruses can be selected from SARS-CoV-1 and/or SARS-CoV-2.

In preferred embodiments, the at least one Coronavirus of component A, or the at least one pandemic Coronavirus of component A, is a Betacoronavirus, preferably a Merbecovirus. In the context of the invention, a preferred Merbecovirus may be selected from a MERS-associated coronavirus. Preferred MERS-associated Coronaviruses can be selected from MERS-CoV.

The term "antigenic peptide or protein of a Coronavirus" relates to any peptide or protein that is selected or is derived from the respective Coronavirus as defined herein, but also to fragments, variants or derivatives thereof, preferably to immunogenic fragments or immunogenic variants thereof.

The term "immunogenic fragment" or "immunogenic variant" has to be understood as any fragment/variant of the corresponding Coronavirus antigen that is capable of raising an immune response in a subject.

In the context of the invention, any protein selected or derived from a Coronavirus, preferably a pandemic Coronavirus, may be used in the context of the invention and may be suitably encoded by the coding sequence or the nucleic acid of component A. It is further in the scope of the underlying invention, that the at least one antigenic peptide or protein may comprise or consist of a synthetically engineered or an artificial Coronavirus peptide or protein. The term "synthetically engineered" Coronavirus peptide or protein, or the term "artificial Coronavirus peptide or protein" relates to a protein that does not occur in nature. Accordingly, an "artificial Coronavirus peptide or protein" or a "synthetically engineered Coronavirus peptide or protein" may for example differ in at least one amino acid compared to the naturally existing Coronavirus peptide or protein, and/or may comprise an additional peptide or protein element (e.g. a heterologous element), and/or may be N-terminally or C-terminally extended or truncated.

In preferred embodiments, the nucleic acid of component A comprises at least one coding sequence encoding at least one antigenic peptide or protein selected or derived from a Coronavirus, preferably a pandemic Coronavirus, or an immunogenic fragment or immunogenic variant thereof, wherein the at least one antigenic peptide or protein comprises at least one peptide or protein that is selected or is derived from a structural protein, an accessory protein, or a replicase protein or an immunogenic fragment or immunogenic variant of any of these.

Suitably, the structural protein is selected or derived from a Coronavirus spike protein (S), a Coronavirus envelope protein (E), a Coronavirus membrane protein (M) or a Coronavirus nucleocapsid protein (N), or an immunogenic fragment or variant of any of these.

In particularly preferred embodiments, the encoded at least one antigenic peptide or protein of component A comprises or consists at least one peptide or protein selected or derived from a Coronavirus spike protein (S, S1, S2, or S1 and S2), or an immunogenic fragment or immunogenic variant of any of these.

The term spike protein (S) as used herein refers to a Coronavirus protein. Spike protein (S) is a typical type I viral fusion protein that exists as trimer on the viral surface with each monomer consisting of a Head (S1) and stem (S2). Individual precursor S polypeptides form a homotrimer and undergo glycosylation within the Golgi apparatus as well as processing to remove the signal peptide, and cleavage by a cellular protease to generate separate S1 and S2 polypeptide chains, which remain associated as S1/S2 protomers within the homotrimer and is therefore a trimer of heterodimers. The S1 domain of the spike glycoprotein includes the receptor binding domain (RBD) that engages (most likely) with the angiotensin-converting enzyme 2 receptors and mediates viral fusion into the host cell, an N-terminal domain that may make initial contact with target cells, and 2 subdomains, all of which are susceptible to neutralizing antibodies. S2 domain consists of a six helix bundle fusion core involved in membrane fusion with the host endosomal membrane and is also a target for neutralization. The S2 subunit further comprises two heptad-repeat sequences (HR1 and HR2) and a central helix typical of fusion glycoproteins, a transmembrane domain, and the cytosolic tail domain.

In preferred embodiments, a fragment of a spike protein (S) is encoded by the nucleic acid of component A, wherein said fragment may be N-terminally truncated, lacking e.g. the N-terminal amino acids 1 to up to 100 of the full length Coronavirus S protein. In embodiments, a fragment of a spike protein (S) may be encoded by the nucleic acid of component A, wherein said fragment may be C-terminally truncated, lacking e.g. the C-terminal amino acids 1 to up to 200 of the full length Coronavirus S protein. Such "fragment of a spike protein (S)" may comprise amino acid substitutions and, optionally, at least one heterologous peptide or protein element (as described below for specific Coronaviruses below). In preferred embodiments, a fragment of Coronavirus spike protein (S) may be C-terminally truncated, thereby lacking the C-terminal transmembrane domain.

In preferred embodiments, the encoded at least one antigenic peptide or protein of component A comprises or consists of a Coronavirus spike protein (S), wherein the spike protein (S) lacks the transmembrane domain (TM or TMflex). Without wishing to being bound to theory, a Coronavirus spike protein (S) lacking the transmembrane domain (TM or TMflex) as defined herein could be suitable for a vaccine as such a protein would be soluble and not anchored in the cell membrane. A soluble protein may therefore be produced (that is translated) in higher concentrations upon administration to a subject or a cell, thereby leading to improved immune responses.

Without wishing to being bound to theory, RBD and CND domains may be crucial for immunogenicity of the Coronavirus spike protein (S). Both regions are located at the S1 fragment of the Coronavirus spike protein. Accordingly, it may be suitable in the context of the invention that the antigenic peptide or protein comprises or consists of an S1 fragment of the spike protein of a Coronavirus or an immunogenic fragment or immunogenic variant thereof. Suitably, such an S1 fragment may comprise at least an RBD and/or a CND domain as defined above.

In particularly preferred embodiments, the encoded at least one antigenic peptide or protein of component A comprises or consists of a Coronavirus spike protein (S), wherein spike protein (S) comprises or consists of a spike protein fragment S1, or an immunogenic fragment or immunogenic variant thereof.

In preferred embodiments, the encoded at least one antigenic peptide or protein comprises an Coronavirus spike protein fragment S1, and lacks at least 70%, 80%, 90%, preferably 100% of spike protein fragment S2. Such embodiments may be beneficial as the S1 fragment comprises neutralizing epitopes without potential problems of full length protein comprising S1 and S2.

Without wishing to being bound to theory, it may be suitable that the antigenic peptide or protein of component A comprises or consists of Coronavirus spike protein fragment S1 and (at least a fragment of) Coronavirus spike protein fragment S2, because the formation of an immunogenic Coronavirus spike protein may be promoted.

Accordingly, in particularly preferred embodiments, the encoded at least one antigenic peptide or protein of component A comprises or consists of a Coronavirus spike protein (S), wherein the Coronavirus spike protein (S) comprises or consists of a Coronavirus spike protein fragment S1 or an immunogenic fragment or immunogenic variant thereof, and Coronavirus spike protein fragment S2 or an immunogenic fragment or immunogenic variant thereof.

In particularly preferred embodiments, the encoded at least one antigenic peptide or protein of component A comprises or consists of a full length Coronavirus spike protein or an immunogenic fragment or immunogenic variant of any of these. The term "full length Coronavirus spike protein" has to be understood as a Coronavirus spike protein, preferably derived from a pandemic Coronavirus, having an amino acid sequence corresponding to essentially the full spike protein.

In particularly preferred embodiments, the Coronavirus spike protein (S) that is provided by the nucleic acid of component A is designed or adapted to stabilize the S antigen in pre-fusion conformation. A pre-fusion conformation is particularly advantageous in the context of an efficient vaccine, as several potential epitopes for neutralizing antibodies may merely be accessible in said pre-fusion protein conformation. Furthermore, remaining of the S protein in the pre-fusion conformation is aimed to avoid immunopathological effects, like e.g. enhanced disease and/or antibody dependent enhancement (ADE).

Accordingly, in preferred embodiments, the nucleic acid of component A comprises at least one coding sequence encoding at least one antigenic peptide or protein that is selected or derived from an Coronavirus, preferably a pandemic Coronavirus, wherein the at least one antigenic peptide or protein is selected or derived or from a spike protein (S), wherein the spike protein (S) is a pre-fusion stabilized spike protein (S_stab). Suitably, said pre-fusion stabilized spike protein comprises at least one pre-fusion stabilizing mutation.

The term "pre-fusion conformation" as used herein relates to a structural conformation adopted by the ectodomain of the coronavirus S protein following processing into a mature coronavirus S protein in the secretory system, and prior to triggering of the fusogenic event that leads to transition of coronavirus S to the postfusion conformation.

A "pre-fusion stabilized spike protein (S_stab)" as described herein comprises one or more amino acid substitutions, deletions, or insertions compared to a native coronavirus S sequence that provide for increased retention of the prefusion conformation compared to coronavirus S ectodomain trimers formed from a corresponding native coronavirus S sequence. The "stabilization" of the prefusion conformation by the one or more amino acid substitutions, deletions, or insertions can be, for example, energetic stabilization (for example, reducing the energy of the prefusion conformation relative to the post-fusion open conformation) and/or kinetic stabilization (for example, reducing the rate of transition from the prefusion conformation to the postfusion conformation). Additionally, stabilization of the coronavirus S ectodomain trimer in the prefusion conformation can include an increase in resistance to denaturation compared to a corresponding native coronavirus S sequence.

Accordingly, in preferred embodiments, the Coronavirus spike protein includes one or more amino acid substitutions that stabilize the S protein in the pre-fusion conformation, for example, substitutions that stabilize the membrane distal portion of the S protein (including the N-terminal region) in the pre-fusion conformation.

In embodiments, the pre-fusion stabilizing mutation comprises an amino acid substitution at position at amino acids of the distal portion of the S protein (including the N-terminal region), wherein said amino acids are substituted with one selected from A, I, L, M, F, V, G, or P (amino acid positions according to reference), preferably wherein said amino acids are substituted with P. Suitably, stabilization of the Coronavirus spike protein may be obtained by substituting two consecutive amino acids with amino acids that stabilize the spike protein in a perfusion conformation.

In preferred embodiments, the at least one pre-fusion stabilizing mutation comprises a cavity filling mutation that further stabilizes the pre-fusion state of the Coronavirus S protein. The term "cavity filling mutation" or "cavity filling amino acid substitution" relates to an amino acid substitution that fills a cavity within the protein core of a protein, such as a Coronavirus S protein ectodomain. Cavities are essentially voids within a folded protein where amino acids or amino acid side chains are not present. In several embodiments, a cavity-filling amino acid substitution is introduced to fill a cavity present in the prefusion conformation of a Coronavirus S ectodomain core that collapses (e.g., has reduced volume) after transition to the postfusion conformation.

In preferred embodiments, the at least one pre-fusion stabilizing mutation comprises a mutated protonation site that further stabilizes the pre-fusion state.

In preferred embodiments, the at least one pre-fusion stabilizing mutation comprises an artificial intramolecular disulfide bond. Such an artificial intramolecular disulfide bond can be introduced to further stabilize the membrane distal portion of the S protein (including the N-terminal region) in the pre-fusion conformation; that is, in a conformation that specifically binds to one or more pre-fusion specification antibodies, and/or presents a suitable antigenic site that is present on the pre-fusion conformation but not in the post fusion conformation of the S protein. In embodiments, the at least one pre-fusion stabilizing mutation comprises 2, 3, 4, 5, 6, 7, or 8 different artificial intramolecular disulfide bonds.

It has to be emphasized that in the context of the invention any Coronavirus S protein, preferably any pandemic Coronavirus S protein may be mutated as described above to stabilize the spike protein in the pre-fusion conformation. Accordingly, a spike protein may be selected from any Coronavirus, preferably from any Alphacoronavirus, Betacoronavirus, Gammacoronavirus, Deltacoronavirus, more preferably Betacoronavirus.

According to various preferred embodiments, the nucleic acid of component A encodes at least one antigenic peptide or protein from Coronavirus as defined herein, preferably of a pandemic Coronavirus, and, additionally, at least one heterologous peptide or protein element.

Suitably, the at least one heterologous peptide or protein element may promote or improve secretion of the encoded Coronavirus antigenic peptide or protein (e.g. via secretory signal sequences), promote or improve anchoring of the encoded antigenic peptide or protein of the invention in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component **A** may additionally encode peptide linker elements, self-cleaving peptides, immunologic adjuvant sequences or dendritic cell targeting sequences.

Suitable multimerization domains may be selected from the list of amino acid sequences according to SEQ ID NOs: 1116-1167 of WO2017081082, or fragments or variants of these sequences. Suitable transmembrane elements may be selected from the list of amino acid sequences according to SEQ ID NOs: 1228-1343 of WO2017081082, or fragments or variants of these sequences. Suitable VLP forming sequences may be selected from the list of amino acid sequences according to SEQ ID NOs: 1168-1227 of the patent application WO2017081082, or fragments or variants of these sequences. Suitable peptide linkers may be selected from the list of amino acid sequences according to SEQ ID NOs: 1509-1565 of the patent application WO2017081082, or fragments or variants of these sequences. Suitable self-cleaving peptides may be selected from the list of amino acid sequences according to SEQ ID NOs: 1434-1508 of the patent application WO2017081082, or fragments or variants of these sequences. Suitable immunologic adjuvant sequences may be selected from the list of amino acid sequences according to SEQ ID NOs: 1360-1421 of the patent application WO2017081082, or fragments or variants of these sequences. Suitable dendritic cell (DCs) targeting sequences may be selected from the list of amino acid sequences according to SEQ ID NOs: 1344-1359 of the patent application WO2017081082, or fragments or variants of these sequences. Suitable secretory signal peptides may be selected from the list of amino acid sequences according to SEQ ID NOs: 1-1115 and SEQ ID NO: 1728 of published PCT patent application WO2017081082, or fragments or variants of these sequences In preferred embodiments, the at least one coding sequence additionally encodes one or more heterologous peptide or protein elements selected from a signal peptide, a linker peptide, a helper epitope, an antigen clustering element, a trimerization or multimerization element, a transmembrane element, or a VLP forming sequence.

In preferred embodiments, the nucleic acid of component A encoding at least one antigenic protein derived from a Coronavirus, additionally encodes at least one heterologous trimerization element, an antigen clustering element, or a VLP forming sequence.

In preferred embodiments, the antigen clustering elements may be selected from a ferritin element, or a lumazine synthase element, surface antigen of Hepatitis B virus (HBsAg), or encapsulin. Expressing a stably clustered Coronavirus spike protein, preferably in in its prefusion conformation, may increases the magnitude and breadth of neutralizing activity against the encoded Coronavirus antigen.

Lumazine synthase (Lumazine, LS, LumSynth) is an enzyme with particle-forming properties, present in a broad variety of organisms, and involved in riboflavin biosynthesis. In particularly preferred embodiments, lumazine synthase is used to promote antigen clustering and may therefore promote or enhance immune responses of the encoded Coronavirus antigen of the invention.

In particularly preferred embodiments, the antigen clustering element (multimerization element) is or is derived from lumazine synthase, wherein the amino acid sequences of said antigen clustering domain is preferably identical or at least 70%, 80%, 85%, 86%, 87%, 88%, 89%, 90%, 91%, 92%, 93%, 94%, 95%, 96%, 97%, 98%, or 99% identical to any one of amino acid sequences **SEQ ID NO: 112,** a fragment or variant thereof.

Ferritin is a protein whose main function is intracellular iron storage. Almost all living organisms produce ferritin which is made of 24 subunits, each composed of a four-alpha-helix bundle, that self-assemble in a quaternary structure with octahedral symmetry. Its properties to self-assemble into nanoparticles are well-suited to carry and expose antigens.

In particularly preferred embodiments, ferritin is used to promote the antigen clustering and may therefore promote immune responses of the encoded Coronavirus antigen, preferably spike protein.

In particularly preferred embodiments, the antigen clustering element (multimerization element) is selected or derived from ferritin wherein the amino acid sequences of said antigen clustering domain is preferably identical or at least 70%, 80%, 85%, 86%, 87%, 88%, 89%, 90%, 91%, 92%, 93%, 94%, 95%, 96%, 97%, 98%, or 99% identical to any one of amino acid sequence **SEQ ID NO: 113,** a fragment or variant thereof.

In some embodiments, the antigen clustering domain is a Hepatitis B surface antigen (HBsAg). HBsAg forms spherical particles. The addition of a fragment of the surface antigen of Hepatitis B virus (HBsAg) sequence may be particularly effective in enhancing the immune response of the nucleic-acid-based vaccine against Coronavirus.

In particularly preferred embodiments, HBsAg is used to promote the antigen clustering and may therefore promote immune responses of the encoded coronavirus antigen, preferably a spike protein as defined herein.

In some embodiments, the antigen clustering element is an encapsulin element. The addition of an encapsulin sequence may be particularly effective in enhancing the immune response of the nucleic-acid-based vaccine against Coronavirus. In particularly preferred embodiments, encapsulin is used to promote the antigen clustering and may therefore promote immune responses of the encoded coronavirus antigen, preferably a Coronavirus spike protein as defined herein.

Encapsulin is a protein isolated from thermophile Thermotoga maritima and may be used as an element to allow self-assembly of antigens to form antigen (nanoparticles. Encapsulin is assembled from 60 copies of identical 31kDa monomers having a thin and icosahedral T = 1 symmetric cage structure with interior and exterior diameters of 20nm and 24nm, respectively.

In embodiments where the coding sequence of the nucleic acid of component A additionally encodes heterologous antigen clustering element, it is particularly preferred and suitable to generate a fusion protein comprising an antigen clustering element and an antigenic peptide or protein derived from a Coronavirus. Suitably, said antigenic peptide or protein, preferably the spike protein, is lacking the C-terminal transmembrane domain (TM) or is lacking a part of the C-terminal transmembrane domain (TM).

In other embodiments, where the coding sequence of the nucleic acid of component A additionally encodes heterologous antigen clustering element as defined above, it is particularly preferred and suitable to generate a fusion protein comprising an antigen clustering element and an antigenic peptide or protein derived from a Coronavirus spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152).**

Further suitable multimerization elements may be selected from the list of amino acid sequences according to SEQ ID NOs: 1116-1167 of WO2017081082, or fragments or variants of these sequences. SEQ ID NOs: 1116-1167 of WO2017081082 are herewith incorporated by reference.

In preferred embodiments, the trimerization element may be selected from a foldon element. In preferred embodiments, the foldon element is a fibritin foldon element. Expressing a stable trimeric spike protein, preferably in its prefusion conformation, may increases the magnitude and breadth of neutralizing activity against a Coronavirus.

In particularly preferred embodiments, a fibritin foldon element is used to promote the antigen trimerization and may therefore promote immune responses of the encoded coronavirus antigen, preferably spike protein. Preferably, the foldon element is or is derived from a bacteriophage, preferably from bacteriophage T4, most preferably from fibritin of bacteriophage T4.

In particularly preferred embodiments, the trimerization element is selected or derived from foldon wherein the amino acid sequences of said trimerization element is preferably identical or at least 70%, 80%, 85%, 86%, 87%, 88%, 89%, 90%, 91%, 92%, 93%, 94%, 95%, 96%, 97%, 98%, or 99% identical to any one of amino acid sequence SEQ ID NO: 114, a fragment or variant of any of these.

In embodiments where the coding sequence of the nucleic acid of component A additionally encodes heterologous trimerization element, it is particularly preferred and suitable to generate a fusion protein comprising a trimerization element and an antigenic peptide or protein derived from a Coronavirus. Suitably, said antigenic peptide or protein, preferably the spike protein derived from Coronavirus that is lacking the C-terminal transmembrane domain, or is lacking a part of the C-terminal transmembrane domain (TMflex).

In other embodiments, where the coding sequence of the nucleic acid of component A additionally encodes heterologous trimerization element as defined above, it is particularly preferred and suitable to generate a fusion protein comprising an trimerization element and an antigenic peptide or protein derived from Coronavirus spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152).**

Further suitable trimerization elements may be selected from the list of amino acid sequences according to SEQ ID NOs: 1116-1167 of WO2017081082, or fragments or variants of these sequences. SEQ ID NOs: 1116-1167 of WO2017081082 are herewith incorporated by reference.

In preferred embodiments, the VLP forming sequence may be selected and fused to the Coronavirus antigen as defined herein. Expressing a stably clustered spike protein in VLP form may increases the magnitude and breadth of neutralizing activity against Coronavirus. VLPs structurally mimic infectious viruses and they can induce potent cellular and humoral immune responses.

Suitable VLP forming sequences may be selected from elements derived from Hepatitis B virus core antigen, HIV-1 Gag protein, or Woodchuck hepatitis core antigen element (WhcAg).

In particularly preferred embodiments, the at least one VLP-forming sequence is a Woodchuck hepatitis core antigen element (WhcAg). The WhcAg element is used to promote VLP formation and may therefore promote immune responses of the encoded coronavirus antigen, preferably spike protein.

In particularly preferred embodiments, the VLP forming sequence is selected or derived from foldon wherein the amino acid sequences of said VLP forming sequences is preferably identical or at least 70%, 80%, 85%, 86%, 87%, 88%, 89%, 90%, 91%, 92%, 93%, 94%, 95%, 96%, 97%, 98%, or 99% identical to any one of amino acid sequence **SEQ ID NO: 13171,** a fragment or variant of any of these.

In embodiments where the coding sequence of the nucleic acid of component A additionally encodes heterologous VLP forming sequence, it is particularly preferred and suitable to generate a fusion protein comprising a VLP forming sequence and an antigenic peptide or protein derived from Coronavirus. Suitably, said antigenic peptide or protein, preferably the spike protein derived from a Coronavirus that is lacking the C-terminal transmembrane domain, or is lacking a part of the C-terminal transmembrane domain.

In other embodiments, where the coding sequence of the nucleic acid of component A additionally encodes heterologous VLP-forming sequence as defined above, it is particularly preferred and suitable to generate a fusion protein comprising a VLP-forming sequence and an antigenic peptide or protein derived from a Coronavirus spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152).**

Further suitable VLP forming sequences in that context may be selected from the list of amino acid sequences according to SEQ ID NOs: 1168-1227 of the patent application WO2017081082, or fragments or variants of these sequences. SEQ ID NOs: 1168-1227 of WO2017081082 are herewith incorporated by reference.

In embodiments, the antigenic peptide or protein comprises a heterologous signal peptide. A heterologous signal peptide may be used to improve the secretion of the encoded Coronavirus antigen.

Suitable secretory signal peptides may be selected from the list of amino acid sequences according to SEQ ID NOs: 1-1115 and SEQ ID NO: 1728 of published PCT patent application WO2017081082, or fragments or variants of these sequences. 1-1115 and SEQ ID NO: 1728 of WO2017081082 are herewith incorporated by reference.

In embodiments where the coding sequence of the nucleic acid of component A additionally encodes heterologous secretory signal peptide, it is particularly preferred and suitable to generate a fusion protein comprising a heterologous secretory signal peptide and an antigenic peptide or protein derived from a Coronavirus. Suitably, said antigenic peptide or protein, preferably the spike protein derived from Coronavirus is lacking the N-terminal endogenous secretory signal peptide (lacking aa 1 to aa 15).

According to preferred embodiments, the nucleic acid of component A comprises at least one coding sequence encoding at least one antigenic peptide or protein selected or derived from a Coronavirus as defined herein, or fragments and variants thereof. In preferred embodiments, the nucleic acid of component A comprises at least one coding sequence encoding at least one antigenic peptide or protein selected or derived from at least one Coronavirus as defined herein, or fragments and variants thereof, wherein said at least one Coronavirus is selected from at least one (pandemic) Alphacoronavirus, at least one (pandemic) Betacoronavirus, at least one (pandemic) Gammacoronavirus, and/or at least one (pandemic) Deltacoronavirus.

In that context, any coding sequence encoding at least one antigenic protein of a Coronavirus as defined herein, or fragments and variants thereof may be understood as suitable coding sequence and may therefore be comprised in the nucleic acid of component A.

Suitable features and embodiments that apply to nucleic acids of component A are provided in paragraph *"Nucleic acid features and embodiments"* below.

Suitably, the nucleic acids of component A is formulated and/or complexed. Suitable features and embodiments that apply to nucleic acids complexation or formulation of component A are provided in paragraph *"Formulation and Complexation"* below.

In the context of the invention, the term "nucleic acid species" is not restricted to mean "one single nucleic acid molecule" but is understood to comprise an ensemble of essentially identical nucleic acid molecules. Accordingly, it may relate to a plurality of essentially identical nucleic acid molecules, e.g. DNA or RNA molecules.

In embodiments, component A may comprise at least 2, 3, 4, 5, 6, 7, 8, 9, 10 or even more different nucleic acid species, e.g. DNA or RNA, as defined herein, each encoding at least one antigenic peptide or protein derived from the same Coronavirus, or a fragment or variant thereof. Particularly, said (genetically) same Coronavirus expresses (essentially) the same repertoire of proteins or peptides, wherein all proteins or peptides have (essentially) the same amino acid sequence. Particularly, said (genetically) same Coronavirus expresses essentially the same proteins, peptides or polyproteins, wherein these protein, peptide or polyproteins preferably do not differ in their amino acid sequence(s).

In embodiments, the component A comprises at least 2, 3, 4, 5, 6, 7, 8, 9, 10 or even more different nucleic acid species, e.g. DNA or RNA, as defined herein, each encoding at least one peptide or protein derived from a genetically distinct Coronavirus (e.g. a distinct Coronavirus isolate, a distinct Betacoronavirus, a distinct pandemic Coronavirus), or a fragment or variant thereof. The terms "distinct" or "distinct Coronavirus" as used throughout the present specification have to be understood as the difference between at least two respective Coronaviruses (e.g. a distinct Coronavirus isolate, a distinct Betacoronavirus, a distinct pandemic Coronavirus, ect.), wherein the difference is manifested on the genome of the respective distinct Coronavirus. Particularly, said (genetically) distinct Coronavirus may express at least one distinct protein, peptide or polyprotein, wherein the at least one distinct protein, peptide or polyprotein differs in at least one amino acid.

In preferred embodiments, the component A comprises at least one nucleic acid encoding at least one antigenic peptide or protein that is selected or derived from at least one Coronavirus, or an immunogenic fragment or immunogenic variant thereof, wherein said component A is to be, preferably, administered intramuscularly or intradermal.

Preferably, intramuscular or intradermal administration of said component A results in expression of the encoded antigen construct in a subject. In embodiments where the nucleic acid is an RNA, administration of component A results in translation of the RNA and to a production of the encoded Coronavirus antigen in a subject. In embodiments where the nucleic acid is a DNA (e.g. plasmid DNA, adenovirus DNA), administration of said composition results in transcription of the DNA into RNA, and to a subsequent translation of the RNA into the encoded Coronavirus antigen in a subject.

In embodiments, administration of the pharmaceutical composition comprising component A to a subject elicits neutralizing antibodies and does not elicit disease enhancing antibodies. In particular, administration of a pharmaceutical composition comprising component A encoding Coronavirus pre-fusion stabilized spike protein to a subject does not elicit immunopathological effects, like e.g. enhanced disease and/or antibody dependent enhancement (ADE).

Preferably, the component A is suitable for a vaccine, in particular, suitable for a Coronavirus vaccine, preferably a combination vaccine of the invention.

Embodiments relating to specific Coronaviruses in the context of the invention are provided below (Component A-1, Component A-2, Component A-3).

### Component A-1: SARS-CoV-2

In a particularly preferred embodiment, the at least one Coronavirus of component A, is a SARS-CoV-2 virus (also referred to as component A-1).

Accordingly, in preferred embodiments of the first aspect, the nucleic acid of component A comprises at least one coding sequence encoding at least one antigenic peptide or protein selected or derived from at least one SARS-CoV-2, or an immunogenic fragment or immunogenic variant thereof.

It has to be understood that generic embodiments and features that have been described in paragraph *"Component A"* may also apply to a nucleic acid encoding a SARS-CoV-2 antigenic peptide or protein.

As used herein, the terms "Human coronavirus 2019", "Wuhan Human coronavirus" (WHCV), "nCoV-2019 coronavirus", "nCoV-2019", "Wuhan seafood market pneumonia virus", 'Wuhan coronavirus", "WHCV coronavirus", "HCoV-19", "SARS2", "COVID-19 virus", "hCoV-19", "SARS-CoV-2", or "coronavirus SARS-CoV-2" may be used interchangeable throughout the present invention, relating to a new pandemic coronavirus that has been emerged in the Chinese city of Wuhan at the turn of 2019/2020, causing the disease COVID-19. According to the WHO (February 2020), the virus is officially termed "SARS-CoV-2", and the associated disease is officially termed "COVID-19".

SARS-CoV-2 belongs to the Coronaviridae, in particular to Orthocoronaviruses, more specifically to the genus Betacoronavirus. Exemplary SARS-CoV-2 coronaviruses are isolates including but not limited to those provided in **List A** and **List B** below.

### List A: Exemplary SARS-CoV-2 coronavirus isolates (EPI/GISAID):

EPI_ISL_402119, EPI_ISL_402120, EPI_ISL_402121, EPI_ISL_402123, EPI_ISL_402124 (hCoV-19/Wuhan/WIV04/2019), EPI_ISL_402125, EPI_ISL_402127, EPI_ISL_402128(BetaCoV_Wuhan_WIV05_2019_EPI_ISL_402128), EPI_ISL_402129, EPI_ISL_402130, EPI_ISL_402131, EPI_ISL_402132, EPI_ISL_403928, EPI_ISL_403929, EPI_ISL_403930, EPI_ISL_403931, EPI_ISL_403932, EPI_ISL_403933, EPI_ISL_403934, EPI_ISL_403935, EPI_ISL_403936, EPI_ISL_403937, EPI_ISL_403962, EPI_ISL_403963, EPI_ISL_404227, EPI_ISL_404228, EPI_ISL_404253, EPI_ISL_404895, EPI_ISL_405839, EPI_ISL_406030, EPI_ISL_406031, EPI_ISL_406034, EPI_ISL_406036, EPI_ISL_406223, EPI_ISL_406531, EPI_ISL_406533, ERI_ISL_406534, EPI_ISL_406535, EPI_ISL_406536, EPI_ISL_406538, EPI_ISL_406592, EPI_ISL_406593, EPI_ISL_406594, EPI_ISL_406595, EPI_ISL_406596, EPI_ISL_406597, EPI_ISL_406798, EPI_ISL_406800, EPI_ISL_406801, EPI_ISL_406844, EPI_ISL_406862, EPI_ISL_406716, EPI_ISL_406717, EPI_ISL_406970, EPI_ISL_406973, EPI_ISL_407071, EPI_ISL_407073, EPI_ISL_407079, EPI_ISL_407084, EPI_ISL_407193, EPI_ISL_407214, EP1_ISL_407215, EPI_ISL_407313, EPI_ISL_407893, EPI_ISL_407894, EPI_ISL_407896, EPI_ISL_407976, EPI_ISL_407987, EPI_ISL_407988, EPI_ISL_408008, EPI_ISL_408009, EPI_ISL_408010, EPI_ISL_408430, EPI_ISL_408431, EPI_ISL_408478, EPI_ISL_408479, EPI_ISL_408480, EPI_ISL_408481, EPI_ISL_408482, EPI_ISL_408484, EPI_ISL_408486, EPI_ISL_408488, EPI_ISL_408489, EPI_ISL_408514, EPI_ISL_408515, EPI_ISL_408665, EPI_ISL_408666, EPI_ISL_408667, EPI_ISL_408668, EPI_ISL_408669, EPI_ISL_408670, EP1_ISL_408976, EPI_ISL_408977, EPI_ISL_409067, EPI_ISL_410044, EPI_ISL_410045, EPI_ISL_410218, EPI_ISL_410301, EPI_ISL_410486, EPI_ISL_410531, EPI_ISL_410532, EPI_ISL_410535, EPI_ISL_410536, EPI_ISL_410537, EPI_ISL_410538, EPI_ISL_410539, EPI_ISL_410540, EPI_ISL_410541, EPI_ISL_410542, EPI_ISL_410713, EPI_ISL_410714, EPI_ISL_410715, EPI_ISL_410716, EPI_ISL_410717, EPI_ISL_410718, EPI_ISL_410719, EPI_ISL_410720, EPI_ISL_410984, EPI_ISL_411060, EPI_ISL_411066, EPI_ISL_411218, EPI_ISL_411219, EPI_ISL_411220, EPI_ISL_411902, EPI_ISL_411915, EPI_ISL_411926, EPI_ISL_411927, EPI_ISL_411929, EPI_ISL_411950, EPI_ISL_411951, EPI_ISL_411952, EPI_ISL_411953, EPI_ISL_411954, EPI_ISL_411955, EPI_ISL_411956, EPI_ISL_411957, EPI_ISL_412026, EPI_ISL_412028, EPI_ISL_412029, EPI_ISL_412030, EPI_ISL_412459, EPI_ISL_412862, EPI_ISL_412869, EPI_ISL_412870, EPI_ISL_412871, EPI_ISL_412872, EPI_ISL_412873, EPI_ISL_412898, EPI_ISL_412899, EPI_ISL_412912, EPI_ISL_412966, EPI_ISL_412967, EPI_ISL_412968, EPI_ISL_412969, EPI_ISL_412970, EPI_ISL_412972, EPI_ISL_412973, EPI_ISL_412974, EPI_ISL_412975, EPI_ISL_412978, EPI_ISL_412979, EPI_ISL_412980, EPI_ISL_412981, EPI_ISL_412982, EPI_ISL_412983, EPI_ISL_413014, EPI_ISL_413015, EPI_ISL_413016, EPI_ISL_413017, EPI_ISL_413018, EPI_ISL_413021, EPI_ISL_413022, EPI_ISL_413023, EPI_ISL_413024, EPI_ISL_413213, EPI_ISL_413214, EPI_ISL_413455, EPI_ISL_413456, EPI_ISL_413457, EPI_ISL_413458, EPI_ISL_413459, EPI_ISL_413485, EPI_ISL_413486, EPI_ISL_413488, EPI_ISL_413489, EPI_ISL_413490, EPI_ISL_413513, EPI_ISL_413514, EPI_ISL_413515, EPI_ISL_413516, EPI_ISL_413518, EPI_ISL_413519, EPI_ISL_413520, EPI_ISL_413521, EPI_ISL_413522, EPI_ISL_413523, EPI_ISL_413555, EPI_ISL_413557, EPI_ISL_413558, EPI_ISL_413559, EPI_ISL_413560, EPI_ISL_413562, EPI_ISL_413563, EPI_ISL_413566, EPI_ISL_413572, EPI_ISL_413573, EPI_ISL_413577, EPI_ISL_413579, EPI_ISL_413580, EPI_ISL_413581, EPI_ISL_413582, EPI_ISL_413583, EPI_ISL_413584, EPI_ISL_413587, EPI_ISL_413589, EPI_ISL_413590, EPI_ISL_413591, EPI_ISL_413592, EPI_ISL_413593, EPI_ISL_413594, EPI_ISL_413595, EPI_ISL_413596, EPI_ISL_413597, EPI_ISL_413598, EPI_ISL_413599, EPI_ISL_413600, EPI_ISL_413602, EPI_ISL_413603, EPI_ISL_413604, EPI_ISL_413606, EPI_ISL_413607, EPI_ISL_413608, EPI_ISL_413609, EPI_ISL_413610, EPI_ISL_413611, EPI_ISL_413612, EPI_ISL_413613, EPI_ISL_413614, EPI_ISI_413615, EPI_ISL_413616, EPI_ISL_413617, EPI_ISL_413618, EPI_ISL_413619, EPI_ISL_413620, EPI_ISL_413621, EPI_ISL_413622, EPI_ISL_413647, EPI_ISL_413648, EPI_ISL_413691, EPI_ISL_413692, EPI_ISL_413693, EPI_ISL_413694, EPI_ISL_413697, EPI_ISL_413711, EPI_ISL_413729, EPI_ISL_413746, EPI_ISL_413748, EPI_ISL_413749, EPI_ISL_413750, EPI_ISL_413751, EPI_ISL_413761, EPI_ISL_413791, EPI_ISL_413809, EPI_ISL_413852, EPI_ISL_413853, EEPI_ISL_413854, EPI_ISL_413856, EPI_ISL_413857, EPI_ISL_413858, EPI_ISL_413860, EPI_ISL_413861, EPI_ISL_413862, EPI_ISL 413863, EPI_ISL_413928, EPI_ISL_413931, EPI_ISL_413996, EPI_ISL_413997, EPI_ISL_413999, EPI_ISL_414005, EPI_ISL_414006, EPI_ISL_414007, EPI_ISL_414008, EPI_ISL_414009, EPI_ISL_414011, EPI_ISL_414012, EPI_ISL_414019, EPI_ISL_414020, EPI_ISL_414021, EPI_ISL_414022, EPI_ISL_414023, EPI_ISL_414027, EPI_ISL_414040, EPI_ISL_414041, EPI_ISL_414042, EPI_ISL_414043, EPI_ISL_414044, EPI_ISL_414045, EPI_ISL_414363, EPI_ISL_414366, EPI_ISL_414367, EPI_ISL_414368, EPI_ISL_414369, EPI_ISL_414414, EPI_ISL_414423, EPI_ISL_414428, EPI_ISL_414429, EPI_ISL_414433, EPI_ISL_414435, EPI_ISL_414439, EPI_ISL_414443, EPI_ISL_414445, EPI_ISL_414446, EPI_ISL_414451, EPI_ISL_414457, EPI_ISL_414468, EPI_ISL_414470, EPI_ISL_414476, EPI_ISL_414477, EPI_ISL_414479, EPI_ISL_414480, EPI_ISL_414481, EPI_ISL_414482, EPI_ISL_414483, EPI_ISL_414484, EPI_ISL_414485, EPI_ISL_414487, EPI_ISL_414500, EPI_ISL_414505, EPI_ISL_414509, EPI_ISL_414510, EPI_ISL_414511, EPI_ISL_414517, EPI_ISL_414519, EPI_ISL_414520, EPI_ISL_414521, EPI_ISL_414522, EPI_ISL_414523, EPI_ISL_414524, EPI_ISL_414525, EPI_ISL_414526, EPI_ISL_414527, EPI_ISL_414528, EPI_ISL_414529, EPI_ISL_414530, EPI_ISL_414531, EPI_ISL_414532, EPI_ISL_414534, EPI_ISL_414535, EPI_ISL_414545, EPI_ISL_414546, EPI_ISL_414547, EPI_ISL_414548, EPI_ISL_414549, EPI_ISL_414552, EPI_ISL_414554, EPI_ISL_414555, EPI_ISL_414556, EPI_ISL_414557, EPI_ISL_414558, EPI_ISL_414559, EPI_ISL_414560, EPI_ISL_414561, EPI_ISL_414562, EPI_ISL_414564, EPI_ISL_414565, EPI_ISL_414566, EPI_ISL_414569, EPI_ISL_414571, EPI_ISL_414574, EPI_ISL_414577, EPI_ISL_414578, EPI_ISL_414579, EPI_ISL_414580, EPI_ISL_414586, EPI_ISL_414587, EPI_ISL_414588, EPI_ISL_414589, EPI_ISL_414590, EPI_ISL_414591, EPI_ISL_414592, EPI_ISL_414593, EPI_ISL_414594, EPI_ISL_414595, EPI_ISL_414596, EPI_ISL_414597, EPI ISL 414600, EPI_ISL_414601, EPI_ISL_414616, EPI_ISL_414617, EPI_ISL_414618, EPI_ISL_414619, EPI_ISL_414620, EPI_ISL_414621, EPI_ISL_414622, EPI_ISL_414623, EPI_ISL_414624, EPI_ISL_414625, EPI_ISL_414626, EPI_ISL_414627, EPI_ISL_414628, EPI_ISL_414629, EPI_ISL_414630, EPI_ISL_414631, EPI_ISL_414632, EPI_ISL_414633, EPI_ISL_414635, EPI_ISL_414637, EPI_ISL_414638, EPI_ISL_414641, EPI_ISL_414642, EPI_ISL_414643, EPI_ISL_414646, EPI_ISL_414648, EPI_ISL_414663, EPI_ISL_414684, EPI_ISL_414685, EPI_ISL_414686, EPI_ISL_414687, EPI_ISL_414688, EPI_ISL_414689, EPI_ISL_414690, EPI_ISL_414691, EPI_ISL_414692, EPI_ISL_414936, EPI_ISL_414937, EPI_ISL_414938, EPI_ISL_414940, EPI_ISL_494941, EPI_ISL_415105, EPI_ISL_415128, EPI_ISL_415129, EPI_ISL_415136, EPI_ISL_415141, EPI_ISL_415142, EPI_ISL_415147, EPI_ISL_415150, EPI_ISL_415151, EPI_ISL_415152, EPI_ISL_415153, EPI_ISL_415154, EPI_ISL_415155, EPI_ISL_415156, EPI_ISL_415157, EPI_ISL_415158, EPI_ISL_415159, EPI_ISL_415710, EPI_ISL_416426, EPI_ISL_416457, EPI_ISL_416481, EPI_ISL_416489, EPI_ISL_416491, EPI_ISL_416492, EPI_ISL_416514, EPI_ISL_416515, EPI_ISL_416516, EPI_ISL_416517, EPI_ISL_416518, EPI_ISL_416538, EPI_ISL_416539, EPI_ISL_416683, EPI_ISL_416685, EPI_ISL_416704, EPI_ISL_416711, EPI_ISL_416713, EPI_ISL_416715, EPI_ISL_416717, EPI_ISL_416744, EPI_ISL_416830, EPI_ISL_416831, EPI_ISL_416832, EPI_ISL_417020, EPI_ISL_417021, EPI_ISL_417022, EPI_ISL_417023, EPI_ISL_417024, EPI_ISL_417025, EPI_ISL_417026, EPI_ISL_417027, EPI_ISL_417028, EPI_ISL_417034, EPI_ISL_417200, EPI_ISL_417201, EPI_ISL_417202, EPI_ISL_417203, EPI_ISL_417204, EPI_ISL_417374, EPI_ISL_417375, EPI_ISL_417376, EPI_ISL_417377, EPI_ISL_417379, EPI_ISL_417382, EPI_ISL_417408, EPI_ISL_417409, EPI_ISL_417410, EPI_ISL_417411, EPI_ISL_417412, EPI_ISL_417413, EPI_ISL_417420, EPI_ISL_417435, EPI_ISL_417436, EPI_ISL_417437, EPI_ISL_417438, EPI_ISL_417439, EPI_ISL_417440, EPI_ISL_417441, EPI_ISL_417442, EPI_ISL_417467, EPI_ISL_417468, EPI_ISL_417504, EPI_ISL_417505, EPI_ISL_417506, EPI_ISL_417507, EPI_ISL_417508, EPI_ISL_417509, EPI_ISL_417510, EPI_ISL_417512, EPI_ISL_417513, EPI_ISL_417514, EPI_ISL_417515, EPI_ISL_417516, EPI_ISL_417517, EPI_ISL_417526, EPI_ISL_417527, EPI_ISL_417528, EPI_ISL_417529, EPI_ISL_417530, EPI_ISL_417531, EPI_ISL_417532, EPI_ISL_417533, EPI_ISL_417534, EPI_ISL_417536, EPI_ISL_417537, EPI_ISL_417538, EPI_ISL_417539, EPI_ISL_417540, EPI_ISL_417541, EPI_ISL 417542, EPI_ISL_417543, EPI_ISL_417544, EPI_ISL_417545, EPI_ISL_417546, EPI_ISL_417547, EPI_ISL_417548, EPI_ISL_417550, EPI_ISL_417551, EPI_ISL_417552, EPI_ISL_417553, EPI_ISL_417554, EPI_ISL_417555, EPI_ISL_417556, EPI_ISL_417557, EPI_ISL_417558, EPI_ISL_417559, EPI_ISL_417560, EPI_ISL_417561, EPI_ISL_417562, EPI_ISL_417563, EPI_ISL_417564, EPI_ISL_417565, EPI_ISL_417566, EPI_ISL_417567, EPI_ISL_417568, EPI_ISL_417569, EPI_ISL_417570, EPI_ISL_417571, EPI_ISL_417572, EPI_ISL_417573, EPI_ISL_417574, EPI_ISL_417575, EPI_ISL_417576, EPI_ISL_417577, EPI_ISL_417578, EPI_ISL_417579, EPI_ISL_417580, EPI_ISL_417581, EPI_ISL_417582, EPI_ISL_417583, EPI_ISL_417584, EPI_ISL_417585, EPI_ISL_417586, EPI_ISL_417587, EPI_ISL_417588, EPI_ISL_417589, EPI_ISL_417590, EPI_ISL_417591, EPI_ISL_417592, EPI_ISL_417593, EPI_ISL_417594, EPI_ISL_417595, EPI_ISL_417596, EPI_ISL_417597, EPI_ISL_417598, EPI_ISL_417599, EPI_ISL_417600, EPI_ISL_417601, EPI_ISL_417602, EPI_ISL_417603, EPI_ISL_417604, EPI_ISL_417605, EPI_ISL_417606, EPI_ISL_417607, EPI_ISL_417608, EPI_ISL_417609, EPI_ISL_417610, EPI_ISL_417611, EPI_ISL_417612, EPI_ISL_417613, EPI_ISL_417614, EPI_ISL_417615, EPI_ISL_417616, EPI_ISL_417617, EPI_ISL_417618, EPI_ISL_417619, EPI_ISL_417620, EPI_ISL_417621, EPI_ISL_417622, EPI_ISL_417623, EPI_ISL_417624, EPI_ISL_417625, EPI_ISL_417626, EPI_ISL_417627, EPI_ISL_417628, EPI_ISL_417629, EPI_ISL_417630, EPI_ISL_417631, EPI_ISL_417632, EPI_ISL_417633, EPI_ISL_417634, EPI_ISL_417635, EPI_ISL_417636, EPI_ISL_417637, EPI_ISL_417638, EPI_ISL_417639, EPI_ISL_417640, EPI_ISL_417641, EPI_ISL_417642, EPI_ISL_417643, EPI_ISL_417644, EPI_ISL_417645, EPI_ISL_417646, EPI_ISL_417647, EPI_ISL_417648, EPI_ISL_417649, EPI_ISL_417650, EPI_ISL_417651, EPI_ISL_417652, EPI_ISL_417653, EPI_ISL_417654, EPI_ISL_417666, EPI_ISL_417667, EPI_ISL_417668, EPI_ISL_417669, EPI_ISL_417670, EPI_ISL_417671, EPI_ISL_417672, EPI_ISL_417676, EPI_ISL_417678, EPI_ISL_417680, EPI_ISL_417685, EPI_ISL_417699, EPI_ISL_417700, EPI_ISL_417703, EPI_ISL_417706, EPI_ISL_417709, EPI_ISL_417712, EPI_ISL_417716, EPI_ISL_417717, EPI_ISL_417724, EPI_ISL_417733, EPI_ISL_417737, EPI_ISL_417740, EPI_ISL_417742, EPI_ISL_417743, EPI_ISL_417746, EPI_ISL_417750, EPI_ISL_417752, EPI_ISL_417753, EPI_ISL_417754, EPI_ISL_417762, EPI_ISL_417763, EPI_ISL_417764, EPI_ISL_417766, EPI_ISL_417774, EPI_ISL_417808, EPI_ISL_417809, EPI_ISL_417813, EPI_ISL_417814, EPI_ISL_417815, EPI_ISL_417816, EPI_ISL_417818, EPI_ISL_417819, EPI_ISL_417820, EPI_ISL_417821, EPI_ISL_417822, EPI_ISL_417823, EPI_ISL_417824, EPI_ISL_417825, EPI_ISL_417826, EPI_ISL_417827, EPI_ISL_417829, EPI_ISL_417830, EPI_ISL_417831, EPI_ISL_417832, EPI_ISL_417833, EPI_ISL_417834, EPI_ISL_417835, EPI_ISL_417836, EPI_ISL_417837, EPI_ISL_417838, EPI_ISL_417839, EPI_ISL_417864, EPI_ISL_417917, EPI_ISL_417918, EPI_ISL_417920, EPI_ISL_417925, EPI_ISL_417926, EPI_ISL_417931, EPI_ISL_417932, EPI_ISL_417933, EPI_ISL_417935, EPI_ISL_417936, EPI_ISL_417937, EPI_ISL_417938, EPI_ISL_417939, EPI_ISL_417940, EPI_ISL_417941, EPI_ISL_417942, EPI_ISL_417943, EPI_ISL_417944, EPI_ISL_417945, EPI_ISL_417946, EPI_ISL_417947, EPI_ISL_417948, EPI_ISL_417949, EPI_ISL_417950, EPI_ISL_417951, EPI_ISL_417953, EPI_ISL_417955, EPI_ISL_417958, EPI_ISL_417959, EPI_ISL_417960, EPI_ISL_417962, EPI_ISL_417964, EPI_ISL_417965, EPI_ISL_417966, EPI_ISL_417968, EPI_ISL_417970, EPI_ISL_417971, EPI_ISL_417973, EPI_ISL_417974, EPI_ISL_417976, EPI_ISL_417977, EPI_ISL_417982, EPI_ISL_417983, EPI_ISL_417984, EPI_ISL_417985, EPI_ISL_418009, EPI_ISL_418017, EPI_ISL_418018, EPI_ISL_418019, EPI_ISL_418020, EPI_ISL_418021, EPI_ISL_418022, EPI_ISL_418023, EPI_ISL_418024, EPI_ISL_418025, EPI_ISL_418026, EPI_ISL_418027, EPI_ISL_418029, EPI_ISL_418030, EPI_ISL_418031, EPI_ISL_418032, EPI_ISL_418033, EPI_ISL_418034, EPI_ISL_418037, EPI_ISL_418038, EPI_ISL_418040, EPI_ISL_418046, EPI_ISL_418047, EPI_ISL_418048, EPI_ISL_418050, EPI_ISL_418052, EPI_ISL_418053, EPI_ISL_418054, EPI_ISL_418063, EPI_ISL_418064, EPI_ISL_418067, EPI_ISL_418071, EPI_ISL_418072, EPI_ISL_418073, EPI_ISL_418074, EPI_ISL_418075, EPI_ISL_418076, EPI_ISL_418077, EPI_ISL_418078, EPI_ISL_418079, EPI_ISL_418080, EPI_ISL_418081, EPI_ISL_418082, EPI_ISL_418101, EPI_ISL_418102, EPI_ISL_418103, EPI_ISL_418104, EPI_ISL_418105, EPI_ISL_418126, EPI_ISL_418127, EPI_ISL_418128, EPI_ISL_418129, EPI_ISL_418130, EPI_ISL_418131, EPI_ISL_418132, EPI_ISL_418133, EPI_ISL_418134, EPI_ISL_418135, EPI_ISL_418136, EPI_ISL_418137, EPI_ISL_418138, EPI_ISL_418139, EPI_ISL_418140, EPI_ISL_418148, EPI_ISL_418149, EPI_ISL_418150, EPI_ISL_418151, EPI_ISL_418152, EPI_ISL_418153, EPI_ISL_418154, EPI_ISL_418155, EPI_ISL_418156, EPI_ISL_418157, EPI_ISL_418158, EPI_ISL_418159, EPI_ISL_418160, EPI_ISL_418161, EPI_ISL_418162, EPI_ISL_418163, EPI_ISL_418164, EPI_ISL_418165, EPI_ISL_418183, EPI_ISL_418184, EPI_ISL_418185, EPI_ISL_418186, EPI_ISL_418187, EPI_ISL_418188, EPI_ISL_418189, EPI_ISL_418190, EPI_ISL_418191, EPI_ISL_418192, EPI_ISL_418193, EPI_ISL_418194, EPI_ISL_418195, EPI_ISL_418197, EPI_ISL_418198, EPI_ISL_418199, EPI_ISL_418200, EPI_ISL_418201, EPI_ISL_418202, EPI_ISL_418203, EPI_ISL_418204, EPI_ISL_418231, EPI_ISL_418232, EPI_ISL_418233, EPI_ISL_418235, EPI_ISL_418236, EPI_ISL_418237, EPI_ISL_418238, EPI_ISL_418239, EPI_ISL_418240, EPI_ISL_418257, EPI_ISL_418260, EPI_ISL_418263, EPI_ISL_418264 or EPI_ISL_418265 or EPI_ISL_616802 (hCoV-19/Denmark/DCGC-3024/2020).

Exemplary SARS-CoV-2 coronaviruses can also be defined or identified by genetic information provided by GenBank Accession Numbers as provided in **List** B below.

### List B: GenBank Accession Numbers of different SARS-CoV-2 isolates:

NC_045512, LC528232, LC528233, LC529905, MN908947, MN938384, MN938385, MN938386, MN938387, MN938388, MN938389, MN938390, MN970003, MN970004, MN975262, MN975263, MN975264, MN975265, MN975266, MN975267, MN975268, MN985325, MN988668, MN988669, MN994467, MN994468, MN996527, MN996528, MN996529, MN996530, MN996531, MN997409, MT007544, MT012098, MT019529, MT019530, MT019531, MT019532, MT019533, MT020880, MT020881, MT027062, MT027063, MT027064, MT039873, MT039887, MT039888, MT039890, MT044257, MT044258, MT049951, MT050493, MT066156, MT066175, MT066176, MT072688, MT093571, MT093631, MT106052, MT106053, MT106054, MT118835, MT121215, MT123290, MT123291, MT123292, MT123293, MT126808, MT135041, MT135042, MT135043, MT135044, MT152824, MT159705, MT159706, MT159707, MT159708, MT159709, MT159710, MT159711, MT159712, MT159713, MT159714, MT159715, MT159716, MT159717, MT159718, MT159719, MT159720, MT159721, MT159722, MT163716, MT163717, MT163718, MT163719, MT163720, MT163721, MT184907, MT184908, MT184909, MT184910, MT184911, MT184912, MT184913, MT188339, MT188340, MT188341, MT192759, MT192765, MT192772 or MT192773.

SARS-CoV-2 coronavirus has been attributed the NCBI Taxonomy ID (NCBI:txid or taxlD): 2697049.

In the context of the invention, any protein selected or derived from a SARS-CoV-2 may be used in the context of the invention and may be suitably encoded by the coding sequence or the nucleic acid of component A. It is further in the scope of the underlying invention, that the at least one antigenic peptide or protein may comprise or consist of a synthetically engineered or an artificial SARS-CoV-2 peptide or protein. The term "synthetically engineered" SARS-CoV-2 peptide or protein, or the term "artificial SARS-CoV-2 peptide or protein" relates to a protein that does not occur in nature. Accordingly, an "artificial SARS-CoV-2 peptide or protein" or a "synthetically engineered SARS-CoV-2 peptide or protein" may for example differ in at least one amino acid compared to the natural SARS-CoV-2 peptide or protein, and/or may comprise an additional heterologous peptide or protein element, and/or may be N-terminally or C-terminally extended or truncated.

In preferred embodiments, the nucleic acid of component A (in particular component A-1) comprises at least one coding sequence encoding at least one antigenic peptide or protein selected or derived from SARS-CoV-2, or an immunogenic fragment or immunogenic variant thereof, wherein the at least one antigenic peptide or protein comprises at least one peptide or protein that is selected or is derived from a structural protein, an accessory protein, or a replicase protein or an immunogenic fragment or immunogenic variant of any of these.

Suitably, the structural protein is selected or derived from a spike protein (S), an envelope protein (E), a membrane protein (M) or a nucleocapsid protein (N), or an immunogenic fragment or variant of any of these.

In particularly preferred embodiments of the pharmaceutical composition, the encoded at least one antigenic peptide or protein of component A (in particular component A-1) comprises or consists at least one peptide or protein selected or derived from a SARS-CoV-2 spike protein (S, S1, S2, or S1 and S2), or an immunogenic fragment or immunogenic variant of any of these.

Suitable antigenic peptide or protein sequences that are provided by the nucleic acid of **component A** are disclosed in **Table 1,** rows 1 to 41, Column A and B. In addition, further information regarding said suitable antigenic peptide or protein sequences selected or derived from SARS-CoV-2 are provided under <223> identifier of the ST.25 sequence listing.

In the following, preferred antigenic peptide or protein sequences selected or derived from SARS-CoV-2 that are provided by the nucleic acid of **component A** (in particular component A-1) are described in detail.

It has to be noted that where reference is made to amino acid (aa) residues and their position in a spike protein (S) of SARS-CoV-2, any numbering used herein - unless stated otherwise - relates to the position of the respective amino acid residue in a corresponding spike protein (S) of SARS-CoV-2 coronavirus isolate EPI_ISL_402128 (BetaCoV_Wuhan_WIV05_2019_EPI_ISL_402128) according to **SEQ ID NO: 1.** Respective amino acid positions are, if referring to SARS-CoV-2 Spike protein, exemplarily indicated for spike protein (S) of SARS-CoV-2 coronavirus isolate EPl_ISL_402128 **(SEQ ID NO: 1**). The person skilled in the art will of course be able to adapt the teaching provided in the present specification exemplified for SARS-CoV-2 EPl_ISL_402128 **(SEQ ID NO: 1**) to other antigenic peptides or proteins in other SARS-CoV-2 coronavirus isolates, e.g. to isolates including but not limited to EPI_ISL_404227, EPI_ISL_403963, EPI_ISL_403962, EPI_ISL_403931, EPI_ISL_403930, EPI_ISL_403929, EPI_ISL_402130, EPI_ISL_402129, EPI_ISL_402128, EPI_ISL_402126, EPI_ISL_402125, EPI_ISL_402124, EPI_ISL_402123, EPI_ISL_402120, EPI_ISL_402119 (further SARS-CoV-2 isolates are provided in List A and/or List B)

Protein annotation for SARS-CoV-2 spike protein (S) was performed using **SEQ ID NO: 1** as a reference protein. The full length S of SARS-CoV-2 reference protein has 1273 amino acid residues, and comprises the following elements:

| | | |
|---|---|---|
| - | secretory signal peptide: | amino acid position aa 1 to aa 15 **(see SEQ ID NO: 28)** |
| - | spike protein fragment S1: | amino acid position aa 1 to aa 681 **(see SEQ ID NO: 27)** |
| - | receptor binding domain (RBD): | amino acid position aa 319 to aa 541 **(see SEQ ID NO: 13243)** |
| - | critical neutralisation domain (CND): | amino acid position aa 329 to aa 529 **(see SEQ ID NO: 13310)** |
| - | spike protein fragment S2: | amino acid position aa 682 to aa 1273 **(see SEQ ID NO: 30)** |
| - | transmembrane domain (TM): | amino acid position aa 1212 to aa 1273 **(see SEQ ID NO: 49)** |
| - | transmembrane domain (TMflex): | amino acid position aa 1148 to aa 1273 **(see SEQ ID NO: 13176)** |

It has to be noted that variation on amino acid level naturally occurs between spike proteins derived from different SARS-CoV-2 isolates (exemplary SARS-CoV-2 isolates are provided in **List A** and **List B).** In the context of the invention, such amino acid variations can be applied to each antigenic peptide or protein derived from a SARS-CoV-2 spike protein as described herein.

Accordingly, each spike protein of SARS-CoV-2 provided herein and contemplated as suitable antigen in the context of the invention may have one ore more of the following amino acid variations/substitutions (amino acid positions according to reference **SEQ ID NO: 1**):
D614G or G614D; H49Y or Y49H; V367F or F367V; P1263L or L1263P; V483A or A483V; S939F or F939S; S943P or P943S; L5F or F5L; L8V or V8L; S940F or F940S; C1254F or F1254C; Q239K or K239Q; M153T or T153M; V1040F or F1040V; A845S or S845A; Y145H or H145Y; A831V or V831A; M1229I or I1229M; H69 or H69del/aa deleted; V70 or H70del/aa deleted; H69_V70 or H69del and H70del/aa deleted; A222V or V222A; Y453F or F453Y; S477N or N477S; I692V or V692I; R403K or K403R; K417N or N417K; N437S or S437N; N439K or K439N; V445A or A445V; V445I or I445V; V445F or F445V; G446V or V446G; G446S or S446G; G446A or A446G; L455F or F455L; F456L or L456F; K458N or N458K; A475V or V475A; G476S or S476G; G476A or A476G; S477I or I477S; S477R or R477S; S477G or G477S; S477T or T477S; T478I or 1478T; T478K or K478T; T478R or R478T; T478A or A478T; E484Q or Q484E; E484K or K484E; E484A or A484E; E484D or D484E; G485R or R485G; G485S or S485G, F486L or L486F; N487I or I487N; Y489H or H489Y; F490S or S490F; F490L or L490F; Q493L or L493Q; Q493K or K493Q ; S494P or P494S; S494L or L494S; P499L or L499P; T500I or I500T; N501Y or Y501 N; N501T or T501N; N501S or S501N; V503F or F503V; V503I or I503V; G504D or D504G; Y505W or W505Y; Q506K or K506Q; Q506H or H506Q; H69 or H69del/aa deleted; V70 or H70del/aa deleted; H69_V70 or H69del and H70del/aa deleted; A222V or V222A; Y453F or F453Y; S477N or N477S; I692V or V692I; R403K or K403R; K417N or N417K; N437S or S437N; N439K or K439N; V445A or A445V; V445I or I445V; V445F or F445V; G446V or V446G; G446S or S446G; G446A or A446G; L455F or F455L; F456L or L456F; K458N or N458K; A475V or V475A; G476S or S476G; G476A or A476G; S477I or I477S; S477R or R477S; S477G or G477S; S477T or T477S; T478I or I478T; T478K or K478T; T478R or R478T; T478A or A478T; E484Q or Q484E; E484K or K484E; E484A or A484E; E484D or D484E; G485R or R485G; G485S or S485G; F486L or L486F; N487I or I487N; Y489H or H489Y; F490S or S490F; F490L or L490F; Q493L or L493Q; Q493K or K493Q; S494P or P494S; S494L or L494S; P499L or L499P; T500I or I500T; N501Y or Y501N; N501T or T501N; N501S or S501N; V503F or F503V; V503I or I503V; G504D or D504G; Y505W or W505Y; Q506K or K506Q; Q506H or H506Q; Y144 or Y144del/aa del; A570D or D570A; P681H or H681P; T716I or I716T; S982A or A982S; D1118H or H1118D; L18F or F18L; D80A or A80D; D215G or G215D; L242 or L242del/aa deleted; A243 or A243del/aa deleted; L244 or L244del/aa deleted; L242_A243_L244 or L242del and A243del and L244del/aa deleted; R246I or I246R; A701V or V701A; T20N or N20T; P26S or S26P; D138Y or Y138D; R190S or S190R; H655Y or Y655H; T1027I or I1027T; S13I or I13S; W152C or C152W; L452R or R452L; R346T or T346R; P384L or L384P; L452M or M452L; F456A or A456F; F456K or K456F; F456V or V456F; E484P or P484E; K417T or T417K; G447V or V447G; L452Q or Q452L; A475S or S475A; F486I or I486F; F490Y or Y490F; Q493R or R493Q; S494A or A494S; P499H or H499P; P499S or S499P; G502V or V502G; T748K or K748T; A522S or S522A; and/or V1176F or F1176V.

The following amino acid variations (amino acid positions according to reference **SEQ ID NO: 1**) are particularly preferred:
- H69del, V70del, Y144del, N501Y, A570D, D614G, P681H, T716I, S982A, and D1118H
- L18F, D80A, D215G, L242del, A243del, L244del, R246I, K417N, E484K, N501Y, D614G, and A701V
- K417N, E484K, N501Y, and D614G
- E484K and D614G
- L18F, T20N, P26S, D138Y, R190S, K417T, E484K, N501Y, D614G, H655Y, and T1027I
- S13I, W152C, L452R, and D614G
- delH69, delV70, Y453F, D614G, I692V, and M1229I
- E484K, E484P, or E484Q
- G446V
- G485R

In preferred embodiments, the SARS-CoV-2 spike proteins (S) comprises the following amino acid variations (amino acid positions according to reference **SEQ ID NO: 1**): L18F, D80A, D215G, delL242, delA243, delL244, R246I, K417N, E484K, N501Y, D614G, A701V.

Suitable SARS-CoV-2 spike proteins (S) may be selected or derived from emerging SARS-CoV-2 variants according to the following Table:

| **Variant** | **Amino acid changes in spike protein** |
|---|---|
| Mink Cluster 5 variant GISAID: EPI_1SL_616802 (hCoV-19/Denmark/DCGC-3024/2020) | delH69, delV70, Y453F, D614G, I692V, M1229I |
| B.1.1.7 (a.k.a., 20B/501Y.V1, 501Y.V1, Variant of Concern-202012/01, VOC-202012/01, VUI-202012/01, B117, "UK variant) | delH69, delV70, deIY144, N501Y, A570D, D614G, P681H, T716I, S982A, D1118H |
| B.1.351 (a.k.a., 20C/501Y.V2, 501Y.V2, N501Y.V2, "SA variant', "South Africa variant") | L18F, D80A, D215G, delL242, delA243, delL244, R2461, K417N, E484K, N501Y, D614G, A701V |
| P.1 (a.k.a., "Brazil variant" = "Japan variant") | L18F, T20N, P26S, D138Y, R190S, K417T, E484K, N501Y, D614G, H655Y, T1027I |
| CAL.20C (a.k.a., "California variant") | S13I, W152C, L452R, D614G |
| NCBI: QQN00429.1 (CAL.20.C example: SARS-CoV-2/human/USA/CA-LACPHL -AF00114/2021) | |

In a particularly preferred embodiment, the SARS-CoV-2 spike proteins (S) is selected or derived from B.1.351.

In some embodiments, a fragment of a SARS-CoV-2 spike protein (S) may be encoded by the nucleic acid of component A, wherein said fragment may be N-terminally truncated, lacking the N-terminal amino acids 1 to up to 100 of the full length SARS-CoV-2 coronavirus reference protein (**SEQ ID NO: 1**) and/or wherein said fragment may be C-terminally truncated, lacking the C-terminal amino acids (aa) 531 to up to aa 1273 of the full length SARS-CoV-2 coronavirus reference protein (**SEQ ID NO: 1**). Such "fragment of a spike protein (S)" may additionally comprise amino acid substitutions (as described below) and may additionally comprise at least one heterologous peptide or protein element (as described below). In preferred embodiments, a fragment of a SARS-CoV-2 spike protein (S) may be C-terminally truncated, thereby lacking the C-terminal transmembrane domain (that is, lacking aa 1212 to aa 1273 or lacking aa 1148 to aa 1273).

In some embodiments, the encoded at least one antigenic peptide or protein of component A comprises or consists of a SARS-CoV-2 spike protein (S), wherein the spike protein (S) lacks the transmembrane domain (TM) (amino acid position aa 1212 to aa 1273). In embodiments, the encoded at least one antigenic peptide or protein comprises or consists of a SARS-CoV-2 spike protein (S), wherein the spike protein (S) lacks an extended part of the transmembrane domain (TMflex) (amino acid position aa 1148 to aa 1273). Without wishing to being bound to theory, a SARS-CoV-2 spike protein (S) lacking the transmembrane domain (TM or TMflex) as defined herein could be suitable for a SARS-CoV-2 vaccine, as such a protein would be soluble and not anchored in the cell membrane. A soluble protein may therefore be produced (that is translated) in higher concentrations upon administration to a subject, leading to improved immune responses.

Without wishing to being bound to theory, RBD (aa 319 to aa 541) and CND (aa 29 to aa 529) domains may be crucial for immunogenicity of SARS-CoV-2 spike protein (S). Both regions are located at the S1 fragment of the spike protein. Accordingly, it may be suitable in the context of the invention that the antigenic peptide or protein comprises or consists of an S1 fragment of the spike protein or an immunogenic fragment or immunogenic variant thereof.

Suitably, a S1 fragment of SARS-CoV-2 may comprise at least an RBD and/or a CND domain as defined above.

In preferred embodiments, the encoded at least one antigenic peptide or protein comprises or consists of a receptor-binding domain (RBD; aa 319 to aa 541), wherein the RBD comprises or consists of a spike protein fragment, or an immunogenic fragment or immunogenic variant thereof.

In further preferred embodiments, the encoded at least one antigenic peptide or protein comprises or consists of a truncated receptor-binding domain (truncRBD; aa 334 to aa 528), wherein the RBD comprises or consists of a spike protein fragment, or an immunogenic fragment or immunogenic variant thereof.

Such "fragment of a spike protein (S)" (RBD; aa 319 to aa 541 or truncRBD, aa 334 to aa 528), may additionally comprise amino acid substitutions (as described below) and may additionally comprise at least one heterologous peptide or protein element (as described below).

In particularly preferred embodiments, the encoded at least one antigenic peptide or protein of component A comprises or consists of a SARS-CoV-2 spike protein (S), wherein the SARS-CoV-2 spike protein (S) comprises or consists of a spike protein fragment S1, or an immunogenic fragment or immunogenic variant thereof.

In preferred embodiments, the encoded at least one antigenic peptide or protein comprises a SARS-CoV-2 spike protein fragment S1, and lacks at least 70%, 80%, 90%, preferably 100% of spike protein fragment S2 (aa 682 to aa 1273). Such embodiments may be beneficial as the SARS-CoV-2 S1 fragment comprises neutralizing epitopes without potential problems of full length protein comprising S1 and S2.

Without wishing to being bound to theory, it may be suitable that the antigenic peptide or protein of component A comprises or consists of SARS-CoV-2 spike protein fragment S1 and (at least a fragment of) SARS-CoV-2 spike protein fragment S2, because the formation of an immunogenic SARS-CoV-2 spike protein may be promoted.

Accordingly, in particularly preferred embodiments, the encoded at least one antigenic peptide or protein of component A comprises or consists of a SARS-CoV-2 spike protein (S), wherein the SARS-CoV-2 spike protein (S) comprises or consists of a SARS-CoV-2 spike protein fragment S1 or an immunogenic fragment or immunogenic variant thereof, and SARS-CoV-2 spike protein fragment S2 or an immunogenic fragment or immunogenic variant thereof.

In particularly preferred embodiments, the encoded at least one antigenic peptide or protein of component A comprises or consists of a full length SARS-CoV-2 spike protein or an immunogenic fragment or immunogenic variant of any of these.

The term "full length SARS-CoV-2 spike protein" has to be understood as a SARS-CoV-2 spike protein, preferably derived from a SARS-CoV-2 coronavirus, having an amino acid sequence corresponding to essentially the full spike protein. Accordingly, a "full length spike protein" may comprise aa 1 to aa 1273 (reference protein: **SEQ ID NOs: 1).** Accordingly, a full length SARS-CoV-2 spike protein may typically comprise a secretory signal peptide, a spike protein fragment S1, a spike protein fragment S2, a receptor binding domain (RBD), and a critical neutralisation domain CND, and a transmembrane domain. Notably, also variants that comprise certain amino acid substitutions (e.g. for allowing pre-fusion stabilization of the S protein) or natural occurring amino acid deletions are encompassed by the term "full length SARS-CoV-2 spike protein".

In particularly preferred embodiments, the SARS-CoV-2 spike protein (S) that is provided by the nucleic acid of component A is designed or adapted to stabilize the antigen in pre-fusion conformation. A pre-fusion conformation is particularly advantageous in the context of an efficient SARS-CoV-2 vaccine, as several potential epitopes for neutralizing antibodies may merely be accessible in said pre-fusion protein conformation. Furthermore, remaining of the protein in the pre-fusion conformation is aimed to avoid immunopathological effects, like e.g. enhanced disease and/or antibody dependent enhancement (ADE).

In preferred embodiments, administration of a nucleic acid (or a composition or vaccine) encoding pre-fusion stabilized spike protein to a subject elicits spike protein neutralizing antibodies and does not elicit disease-enhancing antibodies. In particular, administration of a nucleic acid (or a composition or vaccine) encoding pre-fusion stabilized spike protein to a subject does not elicit immunopathological effects, like e.g. enhanced disease and/or antibody dependent enhancement (ADE).

Accordingly, in preferred embodiments, the nucleic acid of component A comprises at least one coding sequence encoding at least one antigenic peptide or protein that is selected or derived from an SARS-CoV-2 coronavirus, wherein the at least one antigenic peptide or protein is selected or derived or from a spike protein (S), wherein the spike protein (S) is a pre-fusion stabilized spike protein (S_stab). Suitably, said pre-fusion stabilized spike protein comprises at least one pre-fusion stabilizing mutation.

Stabilization of the SARS-CoV-2 spike protein may be obtained by substituting at least one amino acids at position K986 and/or V987 with amino acids that stabilize the spike protein in a perfusion conformation (amino acid positions according to reference **SEQ ID NO: 1**).

In embodiments, the pre-fusion stabilizing mutation of SARS-CoV-2 spike protein comprises an amino acid substitution at position K986, wherein the amino acids K986 is substituted with one selected from A, I, L, M, F, V, G, or P (amino acid positions according to reference SEQ **ID NO: 1**), preferably wherein the amino acids K986 is substituted with P. In embodiments, the pre-fusion stabilizing mutation comprises an amino acid substitution at position V987, wherein the amino acids V987 is substituted with one selected from A, I, L, M, F, V, G, or P (amino acid positions according to reference **SEQ ID NO: 1**), preferably wherein the amino acids V987 is substituted with P.

Suitably, stabilization of the SARS-CoV-2 spike protein may be obtained by substituting two consecutive amino acids at position K986 and V987 with amino acids that stabilize the spike protein in a perfusion conformation (Amino acid positions according to reference **SEQ ID NO: 1**).

In preferred embodiments, the pre-fusion stabilizing mutation of the SARS-CoV-2 spike protein comprises an amino acid substitution at position K986 and V987, wherein the amino acids K986 and/or V987 are substituted with one selected from A, I, L, M, F, V, G, or P (amino acid positions according to reference **SEQ ID NO: 1).**

Preferably, stabilization of the perfusion conformation is obtained by introducing two consecutive proline substitutions at residues K986 and V987 in the SARS-CoV-2 spike protein (Amino acid positions according to reference **SEQ ID NO: 1**).

Accordingly, in preferred embodiments, the pre-fusion stabilized spike protein (S_stab) of SARS-CoV-2 comprises at least one pre-fusion stabilizing mutation, wherein the at least one pre-fusion stabilizing mutation comprises the following amino acid substitutions: K986P and V987P (amino acid positions according to reference **SEQ ID NO: 1).**

Accordingly, any NCBI Protein Accession numbers provided above, or any protein selected from **SEQ ID NOs: 1-9**, **274-340, 22737, 22739, 22741, 22743, 22745, 22747, 22749, 22751, 22753, 22755, 22757, 22929-22946** or fragments or variants thereof can be chosen by the skilled person to introduce such amino acid changes into SARS-CoV-2 spike proteins, preferably amino acid substitutions: K986P and V987P (amino acid positions according to reference **SEQ ID NO: 1**).

In preferred embodiments, the at least one pre-fusion stabilizing mutation of SARS-CoV-2 spike protein comprises a cavity filling mutation that further stabilizes the pre-fusion state, wherein said mutation/amino acid substitution is selected from the list comprising T887W; A1020W; T887W and A1020W; or P1069F (amino acid positions according to reference **SEQ ID NO: 1).**

In some embodiments, at least one of the following amino acid substitutions T887W; A1020W; T887W and A1020W; or P1069F may be combined with a (K986P and V987P) substitution in the SARS-CoV-2 spike protein (amino acid positions according to reference **SEQ ID NO: 1**).

In particularly preferred embodiments, the SARS-CoV-2 spike protein comprises at least one of the following amino acid substitutions (amino acid positions according to reference **SEQ ID NO: 1):**
- T887W; K986P and V987P
- A1020W; K986P and V987P
- T887W and A1020W; K986P and V987P
- P1069F; K986P and V987P

Accordingly, any NCBI protein accession numbers of SARS-CoV-2 provided above, or any protein selected from **SEQ ID NOs: 1-9, 274-340, 22737, 22739, 22741, 22743, 22745, 22747, 22749, 22751, 22753, 22755, 22757, 22929-22946** or fragments or variants thereof, can be chosen by the skilled person to introduce such amino acid changes, suitably amino acid substitutions selected from T887W; A1020W; T887W and A1020W; or P1069F; or amino acid substitutions selected from (T887W; K986P and V987P); (A1020W; K986P and V987P); (T887W and A1020W; K986P and V987P); (P1069F; K986P and V987P) (amino acid positions according to reference **SEQ ID NO: 1).**

In embodiments, at least one of the following amino acid substitutions F817P, A892P, A899P and A942P may be combined with a (K986P and V987P) substitution (amino acid positions according to reference **SEQ ID NO: 1**).

In embodiments, the SARS-CoV-2 coronavirus spike protein comprises at least one of the following amino acid substitutions (Amino acid positions according to reference **SEQ ID NO: 1**):
- F817P; K986P and V987P
- A892P; K986P and V987P
- A899P; K986P and V987P
- A942P; K986P and V987P

In particularly preferred embodiments, the SARS-CoV-2 coronavirus spike protein comprises the following amino acid substitutions (Amino acid positions according to reference **SEQ ID NO: 1**):
- F817P, A892P, A899P, A942P, K986P and V987P (S_stab_PP_hex)

Accordingly, any NCBI protein accession numbers provided above, or any protein selected from **SEQ ID NOs: 1-9, 274-340, 22737, 22739, 22741, 22743, 22745, 22747, 22749, 22751, 22753, 22755, 22757, 22929-22946** or fragments or variants thereof can be chosen by the skilled person to introduce such amino acid changes, suitably amino acid substitutions selected from F817P, A892P, A899P, A942P; or amino acid substitutions selected from (F817P; K986P and V987P); (A892P; K986P and V987P); (A899P; K986P and V987P); (A942P; K986P and V987P); (F817P, A892P, A899P, A942P, K986P and V987P) (amino acid positions according to reference **SEQ ID NO: 1**).

In preferred embodiments, the at least one pre-fusion stabilizing mutation of SARS-CoV-2 spike protein comprises a mutated protonation site that further stabilizes the pre-fusion state, wherein said mutation/amino acid substitution is selected from H1048Q and H1064N; H1083N and H1101N; or H1048Q and H1064N and H1083N and H1101N (amino acid positions according to reference **SEQ ID NO: 1**).

In embodiments, at least one of the following amino acid substitutions H1048Q and H1064N; H1083N and H1101N; or H1048Q and H1064N and H1083N and H1101N may be combined with a (K986P and V987P) substitution (amino acid positions according to reference **SEQ ID NO: 1)** into a SARS-CoV-2 spike protein.

In particularly preferred embodiments, the SARS-CoV-2 spike protein comprises at least one of the following amino acid substitutions (Amino acid positions according to reference **SEQ ID NO: 1):**
- H1048Q and H1064N; K986P and V987P
- H1083N and H1101N; K986P and V987P
- H1048Q and H1064N and H1083N and H1101N; K986P and V987P

Accordingly, any SARS-CoV-2 NCBI protein accession numbers provided above, or any protein selected from **SEQ ID NOs: 1-9, 274-340, 22737, 22739, 22741, 22743, 22745, 22747, 22749, 22751, 22753, 22755, 22757, 22929-22946** or fragments or variants thereof can be chosen by the skilled person to introduce such amino acid changes into a SARS-CoV-2 spike protein, suitably amino acid substitutions selected from H1048Q and H1064N; H1083N and H1101N; or H1048Q and H1064N and H1083N and H1101N; or amino acid substitutions selected from (H1048Q and H1064N; K986P and V987P); (H1083N and H1101N; K986P and V987P); (H1048Q and H1064N and H1083N and H1101N; K986P and V987P); (amino acid positions according to reference **SEQ ID NO: 1).**

In preferred embodiments, the at least one pre-fusion stabilizing mutation of the SARS-CoV-2 spike protein comprises an artificial intramolecular disulfide bond. Such an artificial intramolecular disulfide bond can be introduced to further stabilize the membrane distal portion of the SARS-CoV-2 S protein (including the N-terminal region) in the pre-fusion conformation; that is, in a conformation that specifically binds to one or more pre-fusion specification antibodies, and/or presents a suitable antigenic site that is present on the pre-fusion conformation but not in the post fusion conformation of the SARS-CoV-2 S protein.

In preferred embodiments, the at least one pre-fusion stabilizing mutation of the SARS-CoV-2 spike protein comprises an artificial intramolecular disulfide bond, *preferably* wherein the at least one artificial intramolecular disulfide bond comprises at least two of the following amino acid substitutions selected from the list comprising I712C, I714C, P715C, T874C, G889C, A890C, I909C, N914C, Q965C, F970C, A972C, R995C, G999C, S1003C, L1034C, V1040C, Y1047C, S1055C, P1069C, T1077C, Y1110C, or S1123C (amino acid positions according to reference **SEQ ID NO: 1).**

In preferred embodiments, the at least one pre-fusion stabilizing mutation of the SARS-CoV-2 spike protein comprises an artificial intramolecular disulfide bond, wherein the at least one artificial intramolecular disulfide bond comprises at least one of the following amino acid substitutions: I712C and T1077C; I714C and Y1110C; P715C and P1069C; G889C and L1034C; I909C and Y1047C; Q965C and S1003C; F970C and G999C; A972C and R995C; A890C and V1040C; T874C and S1055C; or N914C and S1123C (amino acid positions according to reference **SEQ ID NO: 1**).

In embodiments, the at least one pre-fusion stabilizing mutation of the SARS-CoV-2 spike protein comprises 2, 3, 4, 5, 6, 7, or 8 different artificial intramolecular disulfide bonds, wherein each may be selected from the following amino acid substitutions: I712C and T1077C; I714C and Y1110C; P715C and P1069C; G889C and L1034C; I909C and Y1047C; Q965C and S1003C; F970C and G999C; A972C and R995C; A890C and V1040C; N914C and S1123C; T874C and S1055C; or N914C and S1123C (amino acid positions according to reference **SEQ ID NO: 1**).

In embodiments, at least one, preferably 2, 3, 4, 5 or more of the following amino acid substitutions I712C and T1077C; I714C and Y1110C; P715C and P1069C; G889C and L1034C; I909C and Y1047C; Q965C and S1003C; F970C and G999C; A972C and R995C; A890C and V1040C; T874C and S1055C; or N914C and S1123C may be combined with a (K986P and V987P) substitution. For example, a pre-fusion stabilized SARS-CoV-2 S protein may comprise two different artificial intramolecular disulfide bonds, e.g. I712C and T1077C; P715C and P1069C; and additionally a K986P and V987P substitution, etc. (amino acid positions according to reference **SEQ ID NO: 1**).

In particularly preferred embodiments, the SARS-CoV-2 spike protein comprises at least one of the following amino acid substitutions (amino acid positions according to reference **SEQ ID NO: 1**):
- I712C and T1077C; K986P and V987P
- I714C and Y1110C; K986P and V987P
- P715C and P1069C; K986P and V987P
- G889C and L1034C; K986P and V987P
- I909C and Y1047C; K986P and V987P
- Q965C and S1003C; K986P and V987P
- F970C and G999C; K986P and V987P
- A972C and R995C; K986P and V987P
- A890C and V1040C; K986P and V987P
- T874C and S1055C; K986P and V987P
- N914C and S1123C; K986P and V987P

Accordingly, any SASR-CoV-2 NCBI protein accession numbers provided above, or any protein selected from **SEQ ID NOs: 1-9, 274-340, 22737, 22739, 22741, 22743, 22745, 22747, 22749, 22751, 22753, 22755, 22757, 22929-22946** or fragments or variants thereof can be chosen by the skilled person to introduce such amino acid changes into a SARS-CoV-2 spike protein, suitably amino acid substitutions selected from I712C and T1077C; I714C and Y1110C; P715C and P1069C; G889C and L1034C; I909C and Y1047C; Q965C and S1003C; F970C and G999C; A972C and R995C; A890C and V1040C; T874C and S1055C; or N914C and S1123C; or amino acid substitutions selected from (I712C; T1077C; K986P; V987P) or (I714C; Y1110C; K986P; V987P) or (P715C; P1069C; K986P; V987P) or (G889C; L1034C; K986P; V987P) or (I909C; Y1047C; K986P; V987P) or (Q965C; S1003C; K986P; V987P) or (F970C; G999C; K986P; V987P) or (A972C; R995C; K986P; V987P) or (A890C and V1040C; K986P and V987P) or (T874C and S1055C; K986P and V987P) or (N914C and S1123C; K986P and V987P) (amino acid positions according to reference **SEQ ID NO: 1).**

It has to be emphasized that in the context of the invention any SARS-CoV-2 spike protein may be mutated or modified as described above (exemplified for reference protein **SEQ ID NO: 1**) to stabilize the spike protein in the pre-fusion conformation.

According to various preferred embodiments, the nucleic acid of component A encodes at least one antigenic peptide or protein selected or derived from SARS-CoV-2 as defined herein and, additionally, at least one heterologous peptide or protein element, preferably selected or derived from a signal peptide, a linker, a helper epitope, an antigen clustering element, a trimerization element, a transmembrane element, and/or a VLP-forming sequence.

Suitably, the at least one heterologous peptide or protein element may promote or improve secretion of the encoded antigenic peptide or protein of SARS-CoV-2 (e.g. via secretory signal sequences), promote or improve anchoring of the encoded antigenic peptide or protein of the invention in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component A may additionally encode peptide linker elements, self-cleaving peptides, immunologic adjuvant sequences or dendritic cell targeting sequences.

In preferred embodiments, the nucleic acid of component A encoding at least one antigenic protein selected or derived from SARS-CoV-2 as defined herein, additionally encodes at least one heterologous trimerization element, an antigen clustering element, or a VLP forming sequence.

In preferred embodiments, the antigen clustering elements may be selected from a ferritin element, or a lumazine synthase element, surface antigen of Hepatitis B virus (HBsAg), or encapsulin. Expressing a stably clustered SARS-CoV-2 spike protein, preferably in in its prefusion conformation may increases the magnitude and breadth of neutralizing activity against the encoded SARS-CoV-2 peptide/protein.

In preferred embodiments, lumazine synthase is used to promote antigen clustering of the SARS-CoV-2 protein and may therefore promote or enhance immune responses of the encoded SARS-CoV-2 antigen, preferably the SARS-CoV-2 spike protein. In preferred embodiments, ferritin is used to promote the antigen clustering of the SARS-CoV-2 protein and may therefore promote immune responses of the encoded SARS-CoV-2 antigen, preferably the SARS-CoV-2 spike protein. In preferred embodiments, HBsAg is used to promote the antigen clustering of the SARS-CoV-2 protein and may therefore promote immune responses of the encoded SARS-CoV-2 antigen, preferably the SARS-CoV-2 spike protein. In preferred embodiments, encapsulin is used to promote the antigen clustering of the SARS-CoV-2 protein and may therefore promote immune responses of the encoded SARS-CoV-2 antigen, preferably the SARS-CoV-2 spike protein.

In some embodiments where the coding sequence of component A additionally encodes heterologous antigen clustering element, it is particularly preferred and suitable to generate a fusion protein comprising an antigen clustering element and an antigenic peptide or protein derived from SARS-CoV-2. Suitably, said antigenic peptide or protein, preferably the spike protein, is lacking the C-terminal transmembrane domain (TM) (lacking aa 1212 to aa 1273) or is lacking a part of the C-terminal transmembrane domain (TMflex), e.g. lacking aa 1148 to aa 1273.

Accordingly, any amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-26, 274-1278, 13521-13587, 22732, 22737-22758, 22929-22964** can be modified to remove the endogenous transmembrane domain (TM) at position aa 1212 to aa 1273 and may therefore be used as "C-terminally truncated" SARS-CoV-2 proteins in the context of the invention (amino acid positions according to reference **SEQ ID NO: 1**). Furthermore, any amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-26, 274-1278, 13521-13587, 22732, 22737-22758, 22929-22964** can be modified to remove a part the endogenous transmembrane domain (TMflex) at position aa 1148 to aa 1273 and may therefore be used as "C-terminally truncated" SARS-CoV-2 proteins in the context of the invention (Amino acid positions according to reference **SEQ ID NO: 1**). Suitable spike proteins lacking the C-terminal transmembrane domain (TM or TMflex) may be selected from **SEQ ID NOs: 31-39, 1614-3623, 13377-13510.**

In other embodiments, where the coding sequence of component A additionally encodes heterologous antigen clustering element as defined above, it is particularly preferred and suitable to generate a fusion protein comprising an antigen clustering element and an antigenic peptide or protein selected or derived from SARS-CoV-2 spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152).**

In preferred embodiments, the trimerization element may be selected from a foldon element. In preferred embodiments, the foldon element is a fibritin foldon element. Expressing a stable trimeric spike protein, preferably in its prefusion conformation, may increases the magnitude and breadth of neutralizing activity against SARS-CoV-2.

In particularly preferred embodiments, a fibritin foldon element is used to promote the antigen trimerization and may therefore promote immune responses of the encoded SARS-CoV-2antigen, preferably SARS-CoV-2 spike protein. Preferably, the foldon element is or is derived from a bacteriophage, preferably from bacteriophage T4, most preferably from fibritin of bacteriophage T4.

In embodiments where the coding sequence of the nucleic acid of component A additionally encodes heterologous trimerization element, it is particularly preferred and suitable to generate a fusion protein comprising a trimerization element and an antigenic peptide or protein derived from SARS-CoV-2. Suitably, said antigenic peptide or protein, preferably the spike protein derived from SARS-CoV-2 that is lacking the C-terminal transmembrane domain (lacking aa 1212 to aa 1273), or is lacking a part of the C-terminal transmembrane domain (TMflex), e.g. lacking aa 1148 to aa 1273.

In other embodiments, where the coding sequence of the nucleic acid of component A additionally encodes heterologous trimerization element as defined above, it is particularly preferred and suitable to generate a fusion protein comprising an trimerization element and an antigenic peptide or protein derived from SARS-CoV-2 spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152**).

In preferred embodiments, a VLP forming sequence may be selected and fused to the SARS-CoV-2 antigen as defined herein. Expressing a stably clustered SARS-CoV-2spike protein in VLP form may increases the magnitude and breadth of neutralizing activity against SARS-CoV-2. VLPs structurally mimic infectious viruses and they can induce potent cellular and humoral immune responses.

Suitable VLP forming sequences may be selected from elements derived from Hepatitis B virus core antigen, HIV-1 Gag protein, or Woodchuck hepatitis core antigen element (WhcAg).

In particularly preferred embodiments, the at least one VLP-forming sequence is a Woodchuck hepatitis core antigen element (WhcAg). The WhcAg element is used to promote VLP formation and may therefore promote immune responses of the encoded coronavirus antigen, preferably spike protein.

In embodiments where the coding sequence of the nucleic acid of component A additionally encodes heterologous VLP forming sequence, it is particularly preferred and suitable to generate a fusion protein comprising a VLP forming sequence and an antigenic peptide or protein derived from SARS-CoV-2. Suitably, said antigenic peptide or protein, preferably the spike protein derived from SARS-CoV-2 that is lacking the C-terminal transmembrane domain (lacking aa 1212 to aa 1273), or is lacking a part of the C-terminal transmembrane domain (TMflex), e.g. lacking aa 1148 to aa 1273.

Accordingly, any amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-26, 274-1278, 13521-13587, 22732, 22737-22758, 22929-22964** can be modified to lack the endogenous transmembrane element at position aa 1212 to aa 1273 and may therefore be used as "C-terminally truncated" SARS-CoV-2 S proteins in the context of the invention. Furthermore, any amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-26, 274-1278, 13521-13587, 22732, 22737-22758, 22929-22964** can be modified to remove a part the endogenous transmembrane domain (TMflex) at position aa 1148 to aa 1273 and may therefore be used as "C-terminally truncated" SARS-CoV-2 S proteins in the context of the invention (amino acid positions according to reference **SEQ ID NO: 1**). Suitable SARS-CoV-2 spike proteins lacking the C-terminal transmembrane domain (TM or TMflex) may be selected from **SEQ ID NOs: 31-39, 1614-3623, 13377-13510.**

In other embodiments, where the coding sequence of the nucleic acid additionally encodes heterologous VLP-forming sequence as defined above, it is particularly preferred and suitable to generate a fusion protein comprising a VLP-forming sequence and an antigenic peptide or protein derived from SARS-CoV-2 spike protein fragment S1 (lacking S2 and/or TM and/or TMflex). Further, it may be suitable to use linker elements for separating the heterologous antigen clustering element from the antigenic peptide or protein (e.g. a linker according to **SEQ ID NO: 115, 13148, 13152).**

In embodiments, the antigenic peptide or protein comprises a heterologous signal peptide as defined above. A heterologous signal peptide may be used to improve the secretion of the encoded SARS-CoV-2 antigen.

In embodiments where the coding sequence of the nucleic acid of component A additionally encodes heterologous secretory signal peptide, it is particularly preferred and suitable to generate a fusion protein comprising a heterologous secretory signal peptide and an antigenic peptide or protein derived from SARS-CoV-2. Suitably, said antigenic peptide or protein, preferably the spike protein derived from SARS-CoV-2 is lacking the N-terminal endogenous secretory signal peptide (lacking aa 1 to aa 15). Accordingly, any amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-26, 274-1278, 13521-13587, 22732 or 22737-22758, 22929-22964** can be modified to lack the endogenous secretory signal peptide at position aa 1 to aa 15 and may therefore be used as "N-terminally truncated" SARS-CoV-2 proteins in the context of the invention.

In the following **List** 1, suitable SARS-CoV-2 coronavirus antigenic peptides and proteins as defined above are further specified in detail (e.g. nomenclature, protein elements, etc.).

### List 1: Exemplary suitable SARS-CoV-2 antigen designs:

- Full length spike protein (S) comprising aa 1 to aa 1273;
   ∘ see for example **SEQ ID NO: 1, 274.**
- Stabilized S protein comprising aa1-aa1273 and K986P, V987P substitutions (S_stab_PP);
   ∘ see for example **SEQ ID NO: 10, 341.**
- Stabilized S protein comprising aa1-aa1273 and K986P, V987P substitutions (S_stab_PP);
   ∘ see for example **SEQ ID NO: 22961.**
- Stabilized S protein comprising aa1-aa1273 and K986P, V987P substitutions (S_stab_PP);
   o see for example **SEQ ID NO: 22960.**
- Stabilized S protein comprising aa1-aa1273 and K986P, V987P, F817P, A892P, A899P, A942P proline substitutions; S_stab_PP_hex
   ∘ see for example **SEQ ID NO: 22732.**
- Stabilized S protein comprising aa1-aa1273 and K986P, V987P substitutions and a cavity filling mutation (T887W, A1020W), S_stab_PP_cav
   ∘ see for example **SEQ ID NO: 408.**
- Stabilized S protein comprising aa1-aa1273 and K986P, V987P substitutions and a cavity filling mutation (P1069F); S_stab_PP_cav
   ∘ see for example **SEQ ID NO: 475.**
- Stabilized S protein comprising aa1-aa1273 and K986P, V987P substitutions and a cavity filling mutation (H1048Q, H1064N, H1083N, H1101N); S_stab_PP_prot
   ∘ see for example **SEQ ID NO: 542.**
- Stabilized S protein comprising aa1-aa1273 and an artificial disulfide bond (S_stab_disul) I712C, T1077C;
   ∘ see for example **SEQ ID NO: 19, 609.**
- S without transmembrane domain comprising aa1-aa1211 (S_woTM);
   ∘ see for example **SEQ ID NO: 31, 1614.**
- S without transmembrane domain flex comprising aa1-aa1147 (S_woTMflex);
   ∘ see for example **SEQ ID NO: 2619.**
- S_woTM comprising K986P, V987P substitutions (S_stab_PP_woTM)
   ∘ see for example **SEQ ID NO: 40, 1681.**
- S_woTMflex comprising K986P, V987P substitutions (S_stab_PP_woTMflex)
   ∘ see for example **SEQ ID NO: 2686.**
- Spike protein fragment S1 comprising aa 1 to aa 681 (S1);
   ∘ see for example **SEQ ID NO: 27, 1279.**
- S_woTM comprising a lumazine synthase;
   ∘ see for example **SEQ ID NO: 58, 3624.**
- S_woTMflex comprising a lumazine synthase;
   ∘ see for example **SEQ ID NO: 7644.**
- S_stab_PP_woTM comprising a lumazine synthase;
   ∘ see for example **SEQ ID NO: 85, 3691.**
- S_stab_PP_ woTMflex comprising a lumazine synthase;
   ∘ see for example **SEQ ID NO: 7711.**
- S_woTM comprising a ferritin element;
   o see for example **SEQ ID NO: 67, 4629.**
- S_woTMflex comprising a ferritin element;
   ∘ see for example **SEQ ID NO: 8649.**
- S_stab_PP_woTM comprising a ferritin element;
   ∘ see for example **SEQ ID NO: 94, 4696.**
- S_stab_PP_woTMflex comprising a ferritin element;
   ∘ see for example **SEQ ID NO: 8716.**
- S_woTM comprising a foldon element;
   ∘ see for example **SEQ ID NO: 76, 5634.**
- S_woTMflex comprising a foldon element;
   ∘ see for example **SEQ ID NO: 9654.**
- S_stab_PP_woTM comprising a foldon element;
   ∘ see for example **SEQ ID NO: 103, 5701.**
- S_stab_PP_woTMflex comprising a foldon element;
   ∘ see for example **SEQ ID NO: 9721.**
- S_woTM comprising a VLP-sequence (WhcAg);
   ∘ see for example **SEQ ID NO: 6639.**
- S_woTMflex comprising a VLP-sequence (WhcAg);
   ∘ see for example **SEQ ID NO 10659.**
- S_stab_PP_woTM comprising a VLP-sequence (WhcAg);
   ∘ see for example **SEQ ID NO: 6706.**
- S_stab_PP_woTMflex comprising a VLP-sequence (WhcAg);
   ∘ see for example **SEQ ID NO: 10726.**
- truncRBD comprising a foldon element:
   ∘ see for example **SEQ ID NO: 22734.**
- truncRBD comprising a lumazine synthase (C-terminal)
   ∘ see for example **SEQ ID NO: 22735.**
- truncRBD comprising a lumazine synthase (N-terminal)
   ∘ see for example **SEQ ID NO: 22736**
- truncRBD comprising a ferritin element:
   ∘ see for example **SEQ ID NO: 22733.**

Amino acid positions provided in **List 1** are according to reference **SEQ ID NO: 1.**

Preferred antigenic peptide or proteins selected or derived from a SARS-CoV-2 coronavirus as defined above are provided in **Table 1** (rows 1 to 41). Therein, each row 1 to 41 corresponds to a suitable SARS-CoV-2 constructs. Column A of **Table 1** provides a short description of suitable SARS-CoV-2 antigen constructs. Column B of **Table 1** provides protein (amino acid) SEQ ID NOs of respective SARS-CoV-2 antigen constructs. Column C of **Table 1** provides SEQ ID NO of the corresponding wild type or reference nucleic acid coding sequences. Column D of **Table 1** provides SEQ ID NO of the corresponding G/C optimized nucleic acid coding sequences (opt1, gc). Column E of **Table 1** provides SEQ ID NO of the corresponding human codon usage adapted nucleic acid coding sequences (opt 3, human). Column F of **Table 1** provides SEQ ID NO of the corresponding G/C content modified nucleic acid coding sequences (opt10, gc mod).

Notably, the description of the invention explicitly includes the information provided under <223> identifier of the ST.25 sequence listing of the present application. Preferred nucleic acid constructs comprising coding sequences of **Table 1,** e.g. mRNA sequences comprising the coding sequences of **Table 1** are provided in **Table 3A and B.**

**Table 1: Preferred SARS-CoV-2 constructs (amino acid sequences and nucleic acid coding sequences):**

| **row** | **A** | **B** | **C** | **D** | **E** | **F** |
|---|---|---|---|---|---|---|
| 1 | Full-length spike protein; S | 1-9, 274-340, 22737, 22739, 22741, 22743, 22745, 22747, 22749, 22751, 22753, 22755, 22757, 22929-22946 | 116-131, 11664-11730 | 136, 11731-11797, 22764, 22766, 22768, 22770, 22772, 22774, 22776, 22778, 22780, 22782, 22784, 22969-23040 | 11967-12033 | 12034, 23041-23076 |
| 2 | Stabilized spike protein; S_stab_PP | 10-18, 341-407, 22738, 22740, 22742, 22744, 22746, 22748, 22750, 22752, 22754, 22756, 22758, 22947-22964 | | 137, 11798, 22765, 22767, 22769, 22771, 22773, 22775, 22777, 22779, 22781, 22783, 22785, 23077-23148 | 142 | 146, 12035, 23149-23184 |
| 3 | Stabilized spike protein; S_stab_PP_cav | 408-541 | | 11799, 11800 | | 12036, 12037 |
| 4 | Stabilized spike protein; S_stab_PP_prot | 542-608 | | 11801 | | 12038 |
| 5 | Stabilized spike protein; S_stab_disul | 19-26, 609-1278, 13521-13587 | | 11802-11811, 14124 | | 12039-12048, 14133 |
| 6 | Spike protein fragment S1 | 27, 1279-1345 | 132 | 138, 11812 | 143 | 147, 12049 |
| 7 | Spike protein fragment S2 | 30, 1346-1412 | 135 | 141, 11813 | | 12050 |
| 8 | Signal peptide of spike protein; SP | 28, 1413-1479 | 133 | 139, 11814 | 144 | 12051 |
| 9 | S1 without signal peptide; S1_woSP | 29, 1480-1546 | 134 | 140, 11815 | 145 | 12052 |
| 10 | Transmembrane domain of spike protein; TM/TMflex | 49-57, 1547-1613, 13176-13242 | | 11816, 13511 | | 12053, 13516 |
| 11 | S without transmembrane domain; S_woTM/woTMflex | 31-39, 1614-1680, 2619-2685 | | 11817, 11832 | | 12054, 12069 |
| 12 | Stabilized S without transmembrane domain; S_stab_PP_woTM/woTMflex | 40-48, 1681-1747, 2686-2752 | | 11818, 11833 | | 12055, 12070 |
| 13 | Stabilized S without transmembrane domain; S_stab_PP_cav_woTM/ woTMflex | 1748-1881, 2753-2886 | | 11819, 11820, 11834, 11835 | | 12056, 12057, 12071, 12072 |
| 14 | Stabilized S without transmembrane domain; S_stab_PP_prot_woTM/ woTMflex | 1882-1948, 2887-2953 | | 11821, 11836 | | 12058, 12073 |
| 15 | Stabilized S without transmembrane domain; S_stab_disul_woTM/ woTMflex | 1940-2618, 2954-3623, 13377-13510 | | 11822-11831, 11837-11846, 13514, 13515 | | 12059-12068, 12074-12083, 13519, 13520 |
| 16 | S_woTM/woTMflex comprising a lumazine synthase | 58-66, 3624-3690, 7644-7710 | | 11847, 11907 | | 12084, 12144 |
| 17 | S_stab_PP_woTM/woTMflex comprising a lumazine synthase | 85-93, 3691-3757, 7711-7777 | | 11848, 11908 | | 12085, 12145 |
| 18 | S_stab_PP_cav_woTM/ woTMflex comprising a lumazine synthase | 3758-3891, 7778-7911 | | 11849, 11850, 11909, 11910 | | 12086, 12087, 12146, 12147 |
| 19 | S_stab_PP_prot_woTM/ woTMflex comprising a lumazine synthase | 3892-3958, 7912-7978 | | 11851, 11911 | | 12088, 12148 |
| 20 | S_stab_disul_woTM/ woTMflex comprising a lumazine synthase | 3959-4628, 7979-8648, 13588-13654, 13856-13922 | | 11852-11861, 11912-11921, 14125, 14129 | | 12089-12098, 12149-12158, 14134, 14138 |
| 21 | S_woTM/woTMflex comprising a ferritin | 67-75, 4629-4695, 8649-8715 | | 11862, 11922 | | 12099, 12159 |
| 22 | S_stab_PP_woTM/woTMflex comprising a ferritin | 94-102, 4696-4762, 8716-8782 | | 11863, 11923 | | 12100, 12160 |
| 23 | S_stab_PP_cav_woTM/ woTMflex comprising a ferritin | 4763-4896, 8783-8916 | | 11864, 11865, 11924, 11925 | | 12101, 12102, 12161, 12162 |
| 24 | S_stab_PP_prot_woTM/ woTMflex comprising a ferritin | 4897-4963, 8917-8983 | | 11866, 11926 | | 12103, 12163 |
| 25 | S_stab_disul_woTM/ woTMflex comprising a ferritin | 4964-5633, 8984-9653, 13655-13721, 13923-13989 | | 11867-11876, 11927-11936, 14126, 14130 | | 12104-12113, 12164-12173, 14135, 14139 |
| 26 | S_woTM/woTMflex comprising a foldon | 76-84, 5634-5700, 9654-9720 | | 11877, 11937 | | 12114,12174 |
| 27 | S_stab_PP_woTM/ woTMflex comprising a foldon | 103-111, 5701-5767, 9721-9787 | | 11878, 11938 | | 12115, 12175 |
| 28 | S_stab_PP_cav_woTM/ woTMflex comprising a foldon | 5768-5901, 9788-9921 | | 11879, 11880, 11939, 11940 | | 12116,12117, 12176, 12177 |
| 29 | S_stab_PP_prot_woTM/ woTMflex comprising a foldon | 5902-5968, 9922-9988 | | 11881, 11941 | | 12118,12178 |
| 30 | S_stab_disul_woTM/ woTMflex comprising a foldon | 5969-6638, 9989-10658, 13722-13788, 13990-14056 | | 11882-11891, 11942-11951, 14127, 14131 | | 12119-12128, 12179-12188, 14136, 14140 |
| 31 | S_woTM/woTMflex comprising a WhcAg (VLP) | 6639-6705, 10659-10725 | | 11892, 11952 | | 12129,12189 |
| 32 | S_stab_PP_woTM/woTMflex comprising a WhcAg (VLP) | 6706-6772, 10726-10792 | | 11893, 11953 | | 12130, 12190 |
| 33 | S_stab_PP_cav_woTM/ woTMflex comprising a WhcAg (VLP) | 6773-6906, 10793-10926 | | 11894, 11895, 11954, 11955 | | 12131, 12132, 12191, 12192 |
| 34 | S_stab_PP_prot_woTM/ woTMflex comprising a WhcAg (VLP) | 6907-6973, 10927-10993 | | 11896, 11956 | | 12133, 12193 |
| 35 | S_stab_disul_woTM/ woTMflex comprising a WhcAg (VLP) | 6974-7643, 10994-11663, 13789-13855, 14057-14123 | | 11897-11906, 11957-11966, 14128, 14132 | | 12134-12143, 12194-12203, 14137, 14141 |
| 36 | Receptor binding domain; RBD | 13243-13309, 22917, 22923 | | 13512, 22914, 22918, 22919, 22924, 22925 | | 13517, 22915, 22916, 22920-22922, 22926-22928 |
| 37 | Critical neutralisation domain; CND | 13310-13376 | | 13513 | | 13518 |
| 38 | Stabilized spike protein; S_stab_PP_hex | 22732 | | 22759 | | |
| 39 | RBD comprising a lumazyne synthase | 22735, 22736 | | 22762, 22763, 22967, 22968 | | |
| 40 | RBD comprising a ferritin | 22733 | | 22760 | | |
| 41 | RBD comprising a foldon | 22734, 26938, 26939 | | 22761, 22966, 26940, 26941 | | |

In preferred embodiments, the at least one antigenic peptide or protein selected or derived from SARS-CoV-2 encoded by the at least one nucleic acid of component A (in particular component A-1) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 1-111, 274-11663, 13176-13510, 13521-14123, 22732-22758, 22917, 22923, 22929-22964, 26938, 26939** or an immunogenic fragment or immunogenic variant of any of these. Further information regarding said amino acid sequences is also provided in **Table 1** (see rows 1 to 41 of Column A and B), and under <223> identifier of the ST.25 sequence listing of respective sequence SEQ ID NOs.

In preferred embodiments, the at least one antigenic peptide or protein (pre-fusion stabilized spike protein (S_stab)) selected or derived from SARS-CoV-2 encoded by the at least one nucleic acid of component A (in particular component A-1) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 10-26, 40-48, 85-111, 341-1278, 1681-2618, 2686-3623, 3691-4628, 4696-5633, 5701-6638, 6706-7643, 7711-8648, 8716-9653, 9721-10658, 10726-11663, 13377-13510, 13521-14123, 22732, 22738, 22740, 22742, 22744, 22746, 22748, 22750, 22752, 22754, 22756, 22758, 22947-22964** or an immunogenic fragment or immunogenic variant of any of these. Further information regarding said amino acid sequences is also provided in **Table 1** (see rows 2 to 5, 12-15, 17-20, 22-25, 27-30, and 32-35 of Column A and B), and under <223> identifier of the ST.25 sequence listing of respective sequence SEQ ID NOs.

In particularly preferred embodiments, the at least one antigenic peptide or protein selected or derived from SARS-CoV-2 comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 10, 21, 22, 25, 27, 274, 341, 408, 475, 542, 743, 810, 1011, 1145, 1212, 1279, 8716, 10726, 22732-22758, 22929-22942, 22947-22964** or an immunogenic fragment or immunogenic variant of any of these.

In particularly preferred embodiments, the at least one antigenic peptide or protein selected or derived from SARS-CoV-2 comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 10-18, 341-407, 22947-22964** or an immunogenic fragment or immunogenic variant of any of these. Further information regarding said amino acid sequences is also provided in **Table 1** (see row 2 of Column A and B), and under <223> identifier of the ST.25 sequence listing of respective sequence SEQ ID NOs.

In a further, more preferred embodiment, the pre-fusion stabilized spike protein (S_stab) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 22960, 22961, 22963** or an immunogenic fragment or immunogenic variant of any of these.

In a particularly preferred embodiment, the pre-fusion stabilized spike protein (S_stab) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 22961,** or an immunogenic fragment or immunogenic variant of any of these.

In an even more preferred embodiment, the at least one antigenic peptide or protein selected or derived from SARS-CoV-2 comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 10 or 341** or an immunogenic fragment or immunogenic variant of any of these.

According to preferred embodiments, the nucleic acid of component A (in particular component A-1) comprises at least one coding sequence encoding at least one antigenic peptide or protein derived from SARS-CoV-2 as defined above, or fragments and variants thereof. In that context, any coding sequence encoding at least one SARS-CoV-2 antigenic protein as defined herein, or fragments and variants thereof may be understood as suitable coding sequence and may therefore be comprised in the nucleic acid of component A.

In preferred embodiments, the nucleic acid of component A (in particular component A-1) comprises or consists of at least one coding sequence encoding at least one antigenic peptide or protein selected or derived from a SARS-CoV-2 coronavirus as defined herein, preferably encoding any one of **SEQ ID NOs: 1-111, 274-11663, 13176-13510, 13521-14123, 22732-22758, 22917, 22923, 22929-22964, 26938, 26939** or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 1-111, 274-11663, 13176-13510, 13521-14123, 22732-22758, 22917, 22923, 22929-22964, 26938, 26939** or fragments or variants thereof, may be selected and may accordingly be understood as suitable coding sequence of the invention. Further information regarding said amino acid sequences is also provided in **Table 1** (see rows 1 to 41 of Column A and B), **Table 3A and B,** and under <223> identifier of the ST.25 sequence listing of respective sequence SEQ ID NOs.

In preferred embodiments, the nucleic acid of component A (in particular component A-1) comprises a coding sequence that comprises at least one of the nucleic acid sequences encoding a SARS-CoV-2 antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 116-132, 134-138, 140-143, 145-147, 148-175, 11664-11813, 11815, 11817-12050, 12052, 12054-13147, 13514, 13515, 13519, 13520, 14124-14177, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or a fragment or variant of any of these sequences. Further information regarding said nucleic acid sequences is also provided in **Table 1** (see rows 1 to 7, 9, 11-41 of Column C-F), **Table 3A and B,** and under <223> identifier of the ST.25 sequence listing of respective sequence SEQ ID NOs.

In preferred embodiments, the at least one coding sequence of the nucleic acid of component A is a codon modified coding sequence as defined herein, wherein the amino acid sequence, that is the SARS-CoV-2 peptide or protein, encoded by the at least one codon modified coding sequence is preferably not being modified compared to the amino acid sequence encoded by the corresponding wild type or reference coding sequence.

The term "reference coding sequence" relates to the coding sequence, which was the origin sequence to be modified and/or optimized.

In particularly preferred embodiments, the at least one coding sequence of the nucleic acid component A is a codon modified coding sequence, wherein the codon modified coding sequence is selected a G/C optimized coding sequence, a human codon usage adapted coding sequence, or a G/C modified coding sequence

In preferred embodiments, the nucleic acid of component A (in particular component A-1) comprises at least one coding sequence comprising or consisting a codon modified nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence selected from the group consisting of **SEQ ID NOs: 136-138, 140-143, 145-176, 11731-11813, 11815, 11817-12050, 12052, 12054-13147, 14142-14177, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or variant of any of these sequences. Additional information regarding each of these suitable nucleic acid sequences encoding may also be derived from the sequence listing, in particular from the details provided therein under identifier <223>. Suitable coding sequences of the first aspect are provided in **Table 1.** Further information regarding said nucleic acid sequences is also provided in Table 1 (see rows 1 to 7, 9, 11-41 of Column D-F), **Table 3A and B,** and under <223> identifier of the ST.25 sequence listing of respective sequence SEQ ID NOs.

In particularly preferred embodiments, the nucleic acid of component A (in particular component A-1) comprises at least one coding sequence comprising or consisting a G/C optimized coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence selected from the group consisting of **SEQ ID NOs: 136-138, 140, 141, 148, 149, 152, 155, 156, 159, 162, 163, 166, 169, 170, 173, 11731-11813, 11815, 11817-11966, 12271-12472, 12743-12944, 13514, 13515, 14124-14132, 14142-14150, 14160-14168, 22759, 22764-22786, 22791-22813, 22818-22839, 22969-23040, 23077-23148, 23189-23260, 23297-23368, 23409-23480, 23617-23688, 23629-23700, 23737-23808, 23849-23920, 23957-24028, 24069-24140, 24177-24248, 24289-24360, 24397-24468, 24509-24580, 24617-24688, 24729-24800, 24837-24908, 24949-25020, 25057-25128, 25169-25240, 25277-25348, 25389-25460, 25497-25568, 25609-25680, 25717-25788, 25829-25900, 25937-26008, 26049-26120, 26157-26228, 26269-26340, 26377-26448, 26489-26560, 26597-26668, 26709-26780, 26817-26888, 26925-26937** or a fragment or variant of any of these sequences. Additional information regarding each of these suitable nucleic acid sequences encoding may also be derived from the sequence listing, in particular from the details provided therein under identifier <223>. Suitable coding sequences of the first aspect are provided in **Table 1.** Further information regarding said nucleic acid sequences is also provided in **Table 1** (see rows 1 to 7, 9, 11-41 of Column D), **Table 3A and B,** and under <223> identifier of the ST.25 sequence listing of respective sequence SEQ ID NOs.

In particularly preferred embodiments, the nucleic acid of component A (in particular component A-1) comprises at least one coding sequence comprising or consisting a human codon usage adapted coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence selected from the group consisting of **SEQ ID NOs: 142, 143, 145, 150, 153, 157, 160, 164, 167, 171, 174, 11967-12033, 12473-12539, 12945-13011** or a fragment or variant of any of these sequences. Additional information regarding each of these suitable nucleic acid sequences encoding may also be derived from the sequence listing, in particular from the details provided therein under identifier <223>. Suitable coding sequences of the first aspect are provided in **Table 1.** Further information regarding said nucleic acid sequences is also provided in **Table 1** (see rows 1 to 7, 9, 11-41 of Column E), **Table 3A and B,** and under <223> identifier of the ST.25 sequence listing of respective sequence SEQ ID NOs.

In particularly preferred embodiments, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence selected from the group consisting of **SEQ ID NOs: 146, 147, 151, 154, 158, 161, 165, 168, 172, 175, 12034-12050, 12052, 12054-12203, 12540-12675, 13012-13147, 13519, 13520, 14133-14141, 14151-14159, 14169-14177, 23041-23076, 23149-23184, 23261-23296, 23369-23404, 23481-23516, 23589-23624, 23701-23736, 23809-23844, 23921-23966, 24029-24064, 24141-24176, 24249-24284, 24361-24396, 24469-24504, 24581-24616, 24689-24724, 24801-24836, 24909-24944, 25021-25056, 25129-25164, 25241-25276, 25349-25384, 25461-25496, 25569-25604, 25681-25716, 25789-25824, 25901-25936, 26009-26044, 26121-26156, 26229-26264, 26341-26376, 26449-26484, 26561-26596, 26669-26704, 26781-26816, 26889-26924** or a fragment or variant of any of these sequences. Additional information regarding each of these suitable nucleic acid sequences encoding may also be derived from the sequence listing, in particular from the details provided therein under identifier <223>. Suitable coding sequences of the first aspect are provided in **Table 1.** Further information regarding said nucleic acid sequences is also provided in **Table 1** (see rows 1 to 7, 9, 11-41 of Column F), **Table 3A and B,** and under <223> identifier of the ST.25 sequence listing of respective sequence SEQ ID NOs.

In even more preferred embodiments, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence selected from the group consisting of **SEQ ID NOs: 136-138, 142, 143, 146, 147, 11731, 11798, 11804, 11805, 11808, 11810, 11811, 11812, 12035, 12049, 22759-22785, 22965-22982, 23077-23094, 23149** or a fragment or variant of any of these sequences. Additional information regarding each of these suitable nucleic acid sequences encoding may also be derived from the sequence listing, in particular from the details provided therein under identifier <223>. Suitable coding sequences of the first aspect are provided in **Table 1.** Further information regarding said nucleic acid sequences is also provided in **Table 1** (see rows 1 to 7, 9, 11-41 of Column F), **Table 3A and B,** and under <223> identifier of the ST.25 sequence listing of respective sequence SEQ ID NOs.

In an particularly preferred embodiment, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence according to **SEQ ID NOs: 137** or a fragment or variant thereof.

In further particularly preferred embodiments, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence according to **SEQ ID NOs: 23090, 23091, 23093, 23094** or a fragment or variant thereof.

In further particularly preferred embodiments, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence according to **SEQ ID NOs: 23091,** or a fragment or variant thereof.

In further embodiments, the nucleic acid of component A (in particular component A-1) comprises at least one coding sequence comprising or consisting a G/C modified coding sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a codon modified nucleic acid sequence according to **SEQ ID NOs: 23113, 23167** or a fragment or variant thereof.

Preferred nucleic acid sequences of component A (in particular component A-1), including particularly preferred mRNA sequences, are provided in **Table 3A** (column C and D). Therein, each row represents a specific suitable SARS-CoV-2 construct of the invention (compare with **Table 1**), wherein the description of the SARS-CoV-2 construct is indicated in column A of **Table 3A** and the SEQ ID NOs of the amino acid sequence of the respective SARS-CoV-2 construct is provided in column B. The corresponding SEQ ID NOs of the coding sequences encoding the respective SARS-CoV-2 constructs are provided in **Table 1.** Further information is provided under <223> identifier of the respective SEQ ID NOs in the sequence listing.

The corresponding nucleic acid, preferably coding RNA sequences, in particular mRNA sequences comprising preferred coding sequences are provided in columns C and D, wherein column C provides nucleic acid sequences with an UTR combination "HSD17B4/PSMB3" as defined herein, wherein column D provides nucleic acid sequences with an "alpha-globin" 3' UTR as defined herein.

**Table 3A: Nucleic acid, preferably mRNA constructs encoding SARS-CoV-2 antigens**

| **row** | **A** | **S** | **C** | **D** |
|---|---|---|---|---|
| 1 | Full-length spike protein; S | 1-9, 274-340, 22737, 22739, 22741, 22743, 22745, 22747, 22749, 22751, 22753, 22755, 22757, 22929-22946 | 148, 155,12204-12337, 12473-12540, 22791, 22793, 22795, 22797, 22799, 22801, 22803, 22805, 22807, 22809, 22811, 23409-23516 | 162, 169, 12676-12809, 12945-13012, 22818, 22820, 22822, 22824, 22826, 22828, 22830, 22832, 22834, 22836, 22838, 23189-23296 |
| 2 | Stabilized spike protein; S_stab_PP_ | 10-18, 341-407, 22738, 22740, 22742, 22744, 22746, 22748, 22750, 22752, 22754, 22756, 22758, 22947-22964 | 149-151, 156-158, 12338, 12541, 22792, 22794, 22796, 22798, 22800, 22802, 22804, 22806, 22808, 22810, 22812, 23517-23624 | 163-165, 170-172, 12810, 13013, 22819, 22821, 22823, 22825, 22827, 22829, 22831, 22833, 22835, 22837, 22839, 23297-23404 |
| 3 | Stabilized spike protein; S_stab_PP_cav | 408-541 | 12339, 12340, 12542, 12543 | 12811, 12812, 13014, 13015 |
| 4 | Stabilized spike protein; S_stab_PP_prot | 542-608 | 12341, 12544 | 12813, 13016 |
| 5 | Stabilized spike protein; S_stab_disul | 19-26, 609-1278, 13521-13587 | 12342-12351, 12545-12554, 14142, 14151 | 12814-12823, 13017-13026, 14160, 14169 |
| 6 | Spike protein fragment S1 | 27, 1279-1345 | 152-154, 159-161, 12352, 12555 | 166-168, 173-175, 12824, 13027 |
| 7 | S_woTM comprising a lumazine synthase | 58-66, 3624-3690 | 12353, 12556 | 12825, 13028 |
| 8 | S_stab_PP_woTM comprising a lumazine synthase | 85-93, 3691-3757 | 12354, 12557 | 12826, 13029 |
| 9 | S_stab_PP_cav_woTM comprising a lumazine synthase | 3758-3891 | 12355, 12356, 12558, 12559 | 12827, 12828, 13030, 13031 |
| 10 | S_stab_PP_prot_woTM comprising a lumazine synthase | 3892-3958 | 12357, 12560 | 12829, 13032 |
| 11 | S_stab_disul_woTM comprising a lumazine synthase | 3959-4628, 13588-13654 | 12358-12367, 12561-12570, 14143, 14152 | 12830-12839, 13033-13042, 14161, 14170 |
| 12 | S_woTM comprising a ferritin | 67-75, 4629-4695 | 12368, 12571 | 12840, 13043 |
| 13 | S_stab_PP_woTM comprising a ferritin | 94-102, 4696-4762 | 12369, 12572 | 12841, 13044 |
| 14 | S_stab_PP_cav_woTM comprising a ferritin | 4763-4896 | 12370, 12371, 12573, 12574 | 12842, 12843, 13045, 13046 |
| 15 | S_stab_PP_prot_woTM comprising a ferritin | 4897-4963 | 12372, 12575 | 12844, 13047 |
| 16 | S_stab_disul_woTM comprising a ferritin | 4964-5633, 13655-13721 | 12373-12382, 12576-12585, 14144, 14153 | 12845-12854, 13048-13057, 14162,14171 |
| 17 | S_woTM comprising a foldon | 76-84, 5634-5700 | 12383, 12586 | 12855, 13058 |
| 18 | S_stab_PP_woTM comprising a foldon | 103-111, 5701-5767 | 12384, 12587 | 12856, 13059 |
| 19 | S_stab_PP_cav_woTM comprising a foldon | 5768-5901 | 12385, 12386, 12588, 12589 | 12857, 12858, 13060, 13061 |
| 20 | S_stab_PP_prot_woTM comprising a foldon | 5902-5968 | 12387, 12590 | 12859, 13062 |
| 21 | S_stab_disul_woTM comprising a foldon | 5969-6638, 13722-13788 | 12388-12397, 12591-12600, 14145, 14154 | 12860-12869, 13063-13072, 14163, 14172 |
| 22 | S_woTM comprising a WhcAg (VLP) | 6639-6705 | 12398, 12601 | 12870, 13073 |
| 23 | S_stab_PP_woTM comprising a WhcAg (VLP) | 6706-6772 | 12399, 12602 | 12871, 13074 |
| 24 | S_stab_PP_cav_woTM comprising a WhcAg (VLP) | 6773-6906 | 12400, 12401, 12603, 12604 | 12872, 12873, 13075, 13076 |
| 25 | S_stab_PP_prot_woTM comprising a WhcAg (VLP) | 6907-6973 | 12402, 12605 | 12874, 13077 |
| 26 | S_stab_disul_woTM comprising a WhcAg (VLP) | 6974-7643, 13789-13855 | 12403-12412, 12606-12615, 14146, 14155 | 12875-12884, 13078-13087, 14164, 14173 |
| 27 | S_woTMflex comprising a lumazine synthase | 7644-7710 | 12413, 12616 | 12885, 13088 |
| 28 | S_stab_PP_woTMflex comprising a lumazine synthase | 7711-7777 | 12414, 12617 | 12886, 13089 |
| 29 | S_stab_PP_cav_woTMflex comprising a lumazine synthase | 7778-7911 | 12415, 12416, 12618, 12619 | 12887, 12888, 13090, 13091 |
| 30 | S_stab_PP_prot_woTMflex comprising a lumazine synthase | 7912-7978 | 12417, 12620 | 12889, 13092 |
| 31 | S_stab_disul_woTMflex comprising a lumazine synthase | 7979-8648, 13856-13922 | 12418-12427, 12621-12630, 14147, 14156 | 12890-12899, 13093-13102, 14165, 14174 |
| 32 | S_woTMflex comprising a ferritin | 8649-8715 | 12428, 12631 | 12900, 13103 |
| 33 | S_stab_PP_woTMflex comprising a ferritin | 8716-8782 | 12429, 12632 | 12901, 13104 |
| 34 | S_stab_PP_cav_woTMflex comprising a ferritin | 8783-8916 | 12430, 12431, 12633, 12634 | 12902, 12903, 13105, 13106 |
| 35 | S_stab_PP_prot_woTMflex comprising a ferritin | 8917-8983 | 12432, 12635 | 12904, 13107 |
| 36 | S_stab_disul_woTMflex comprising a ferritin | 8984-9653, 13923-13989 | 12433-12442, 12636-12645, 14148, 14157 | 12905-12914, 13108-13117, 14166, 14175 |
| 37 | S_woTMflex comprising a foldon | 9654-9720 | 12443, 12646 | 12915, 13118 |
| 38 | S_stab_PP_woTMflex comprising a foldon | 9721-9787 | 12444, 12647 | 12916, 13119 |
| 39 | S_stab_PP_cav_woTMflex comprising a foldon | 9788-9921 | 12445, 12446, 12648, 12649 | 12917, 12918,13120, 13121 |
| 40 | S_stab_PP_prot_woTMflex comprising a foldon | 9922-9988 | 12447, 12650 | 12919, 13122 |
| 41 | S_stab_disul_woTMflex comprising a foldon | 9989-10658, 13990-14056 | 12448-12457, 12651-12660, 14149, 14158 | 12920-12929, 13123-13132, 14167,14176 |
| 42 | S_woTMflex comprising a WhcAg (VLP) | 10659-10725 | 12458, 12661 | 12930, 13133 |
| 43 | S_stab_PP_woTMflex comprising a WhcAg (VLP) | 10726-10792 | 12459, 12662 | 12931, 13134 |
| 44 | S_stab_PP _cav_woTMflex comprising a WhcAg (VLP) | 10793-10926 | 12460, 12461, 12663, 12664 | 12932, 12933, 13135, 13136 |
| 45 | S_stab_PP_prot_woTMflex comprising a WhcAg (VLP) | 10927-10993 | 12462, 12665 | 12934, 13137 |
| 46 | S_stab_disul_woTMflex comprising a WhcAg (VLP) | 10994-11663, 14057-14123 | 12463-12472, 12666-12675, 14150, 14159 | 12935-12944, 13138-13147, 14168, 14177 |
| 47 | Stabilized spike protein; S_stab_PP_hex | 22732 | 22786 | 22813 |
| 48 | RBD comprising a lumazyne synthase | 22735, 22736 | 22789, 22790 | 22816, 22817 |
| 49 | RBD comprising a ferritin | 22733 | 22787 | 22814 |
| 50 | RBD comprising a foldon | 22734 | 22788 | 22815 |

Further preferred nucleic acid sequences, preferably mRNA sequences of the invention are provided in **Table 3B.** Therein, each column represents a specific suitable SARS-CoV-2 (nCoV-2019) construct of the invention (compare with **Table 1** and **Table 3B),** wherein column B represents "Full-length spike protein; S",row 1 of Table 1 and Table 3A and column C "Stabilized spike protein; S_stab_PP", compare with row 2 of Table 1 and Table 3A.

The SEQ ID NOs of the amino acid sequence of the respective SARS-CoV-2 construct are provided in row 1. The corresponding SEQ ID NOs of the coding sequences encoding the respective SARS-CoV-2 constructs are provided in in **Table 1.** Further information is provided under <223> identifier of the respective SEQ ID NOs in the sequence listing.

The corresponding nucleic acid, preferably coding RNA sequences, in particular mRNA sequences comprising preferred coding sequences are provided in rows 2-16, wherein each row provides nucleic acid sequences with UTR combinations and suitable 3' ends.

**Table 3B: Nucleic acid preferably mRNA constructs suitable for a coronavirus vaccine**

| **row** | **A** | **B** | **C** |
|---|---|---|---|
| 1 | Protein | 1-9, 274-340, 22737, 22739, 22741, 22743, 22745, 22747, 22749, 22751, 22753, 22755, 22757, 22929-22946 | 10-18, 341-407, 22738, 22740, 22742, 22744, 22746, 22748, 22750, 22752, 22754, 22756, 22758, 22947-22964 |
| 2 | RNA i-3 (A64-N5-C30-hSL-N5) | 162, 169, 12676-12809, 12945-13012, 22818, 22820, 22822, 22824, 22826, 22828, 22830, 22832, 22834, 22836, 22838, 23189-23296 | 163-165, 170-172, 12810, 13013, 22819, 22821, 22823, 22825, 22827, 22829, 22831, 22833, 22835, 22837, 22839, 23297-23404 |
| 3 | RNA a-1 (hSL-A100) | 148, 155,12204-12337, 12473-12540, 22791, 22793, 22795, 22797, 22799, 22801, 22803, 22805, 22807, 22809, 22811, 23409-23516 | 149-151, 156-158, 12338, 12541, 22792, 22794, 22796, 22798, 22800, 22802, 22804, 22806, 22808, 22810, 22812, 23517-23624 |
| 4 | RNA a-3 (hSL-A100) | 23629-23736 | 23737-23844 |
| 5 | RNA e-2 (hSL-A100) | 23849-23956 | 23957-24064 |
| 6 | RNA i-3 (hSL-A100) | 24069-24176, 24289-24396, 24500-24616 | 24177-24284, 24397-24504, 24617-24724 |
| 7 | RNA a-1 (A100) | 24729-24836 | 24837-24944 |
| 8 | RNA a-3 (A100) | 24949-25056 | 25057-25164 |
| 9 | RNA e-2 (A100) | 25169-25276 | 25277-25384 |
| 10 | RNA i-3 (A100) | 25389-25496, 25609-25716, 25829-25936 | 25497-25604, 25717-25824, 25937-26044 |
| 13 | RNA x-1 (A100) | 26049-26156 | 26157-26264 |
| 14 | RNA x-2 (A100) | 26269-26376 | 26377-26484 |
| 15 | RNA x-1 (A100-N5) | 26489-26596 | 26597-26704 |
| 16 | RNA x-2 (A30-N10-A70) | 26709-26816 | 26817-26924 |

In preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected **SEQ ID NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or variant of any of these sequences. Further information regarding respective nucleic acid sequences is provided under <223> identifier of the respective SEQ ID NO in the sequence listing, and in **Table 3A** (see in particular Column C and D) and 3B (see in particular rows 2-16)..

In particularly preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 162-175, 12676-13147, 14160-14177, 22813-22839, 23189-23404** or a fragment or variant of any of these sequences. Further information regarding respective nucleic acid sequences is provided under <223> identifier of the respective SEQ ID NO in the sequence listing, and in **Table 3A** (see in particular Column D) and 3B (row 2).

In particularly preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 148-161, 12204-12675, 14142-14159, 22786-22812, 23409-23624, 24729-24944** or a fragment or variant of any of these sequences. Further information regarding respective nucleic acid sequences is provided under <223> identifier of the respective SEQ ID NO in the sequence listing, and in **Table 3A** (see in particular Column C) and **Table 3B** (see rows 3, 7).

In particularly preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 149-154, 156-161, 163-168, 170-175, 12338, 12352, 12541, 12555, 12810, 12824, 13013, 13027, 22786, 22792, 22794, 22796, 22798, 22800, 22802, 22804, 22806, 22808, 22810, 22812, 22813, 22819, 22821, 22823, 22825, 22827, 22829, 22831, 22833, 22836, 22837, 22839, 23517-23624, 23297-23404, 24837-24944** or a fragment or variant of any of these sequences. Further information regarding respective nucleic acid sequences is provided under <223> identifier of the respective SEQ ID NO in the sequence listing and in **Table 3A** (see Column C and D, rows 2 and 6) and Table 3B (see Column C).

In even more preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 149, 156, 12338, 150, 157, 151, 158, 12541, 163, 170, 12810, 164, 171, 165, 172, 13013, 12342-12351, 12545-12554, 12814-12823, 13017-13026, 14133** or a fragment or variant of any of these sequences. Further information regarding respective nucleic acid sequences is provided under <223> identifier of the respective SEQ ID NO in the sequence listing and in **Table 3 A** and **3B.**

In even more preferred embodiments, the nucleic acid of component A (in particular component A-1), preferably the RNA, comprises or consists of a nucleic acid sequence encoding a SARS-CoV-2 antigen selected from **SEQ ID NOs: 149, 150, 163, 164, 165, 24837, 23311, 23531, 24851, 23310, 23530, 24850, 23313, 23533, 24853, 23314, 23534, 24854** or a fragment or variant of any of these sequences. Further information regarding respective nucleic acid sequences is provided under <223> identifier of the respective SEQ ID NO in the sequence listing and in **Table** 3 (see Column C and D, row 2).

In a particularly preferred embodiment, the nucleic acid of component A (in particular component A-1), preferably the RNA, comprises or consists of a nucleic acid sequence encoding a SARS-CoV-2 antigen selected from **SEQ ID NOs: 163** or a fragment or variant of that sequence.

In a particularly preferred embodiment, the nucleic acid of component A (in particular component A-1), preferably the RNA, comprises or consists of a nucleic acid sequence encoding a SARS-CoV-2 antigen selected from **SEQ ID NOs: 149** or a fragment or variant of that sequence.

In a further particularly preferred embodiment, the nucleic acid of component A (in particular component A-1), preferably the RNA, comprises or consists of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence of **SEQ ID NO: 24837** or a fragment or variant of that sequence
In a further particularly preferred embodiment, the nucleic acid of component A (in particular component A-1), preferably the RNA, comprises or consists of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence of **SEQ ID NO: 23311, 23531, 24851** or a fragment or variant of these sequences.

In a further preferred embodiment, the nucleic acid of component A (in particular component A-1), preferably the RNA, comprises or consists of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence of **SEQ ID NO: 23310, 23530, 24850** or a fragment or variant of these sequences.

In a further preferred embodiment, the nucleic acid of component A (in particular component A-1), preferably the RNA, comprises or consists of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence of **SEQ ID NO: 23313, 23533, 24853, 23314, 23534, 24854** or a fragment or variant of these sequences.

In a further embodiment, the nucleic acid of component A (in particular component A-1), preferably the RNA, comprises or consists of a nudeic acid sequence which is identical or at least 70%, 80%, 85%, 86%, 87%, 88%, 89%, 90%, 91%, 92%, 93%, 94%, 95%, 96%, 97%, 98%, or 99% identical to a nudeic acid sequence of **SEQ ID NO: 26633 or 26907** or a fragment or variant of these sequences.

In further preferred embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** wherein said RNA sequences comprise a cap1 structure as defined herein. Further information regarding respective nucleic acid sequences is provided under <223> identifier of the respective SEQ ID NO in the sequence listing and in **Table 3A and 3B.**

In further embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** wherein at least one, preferably all uracil nucleotides in said RNA sequences are replaced by pseudouridine (ψ) nucleotides and/or N1-methylpseudouridine (m1ψ) nucleotides. Further information regarding respective nucleic acid sequences is provided under <223> identifier of the respective SEQ ID NO in the sequence listing and in **Table 3A and 3B.**

In further embodiments, the nucleic acid of component A (in particular component A-1), preferably the RNA, comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ **ID NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** wherein said RNA sequences comprise a cap1 structure as defined herein, and, wherein at least one, preferably all uracil nucleotides in said RNA sequences are replaced by pseudouridine (ψ) nucleotides and/or N1-methylpseudouridine (m1ψ) nucleotides. Further information regarding respective nucleic acid sequences is provided under <223> identifier of the respective SEQ ID NO in the sequence listing and in **Table 3A** and **3B.**

Suitable features and embodiments that apply to nucleic acids of component A-1 are provided in paragraph *"Nucleic acid features and embodiments"* below.

Suitably, the nucleic acids of component A-1 is formulated and/or complexed. Suitable features and embodiments that apply to nucleic acids complexation or formulation of component A are provided in paragraph *"Formulation and Complexation"* below.

In embodiments, the component A comprises a plurality or at least more than one of the nucleic acid species comprising at least one coding sequence encoding at least one antigenic peptide or protein selected or derived from at least one SARS-CoV-2, or an immunogenic fragment or immunogenic variant thereof, e.g. DNA or RNA species as defined herein. Preferably, component A (in particular component A-1) as defined herein comprises 2, 3, 4, 5, 6, 7, 8, 9, or 10 different nucleic acids each defined as defined herein.

In embodiments, component A may comprise at least 2, 3, 4, 5, 6, 7, 8, 9, 10 or even more different nucleic acid species, e.g. DNA or RNA, as defined herein, each encoding at least one antigenic peptide or protein derived from the same SARS-CoV-2, or a fragment or variant thereof. Particularly, said (genetically) same SARS-CoV-2expresses (essentially) the same repertoire of proteins or peptides, wherein all proteins or peptides have (essentially) the same amino acid sequence. Particularly, said (genetically) same SARS-CoV-2expresses essentially the same proteins, peptides or polyproteins, wherein these protein, peptide or polyproteins preferably do not differ in their amino acid sequence(s).

In embodiments, the component A comprises at least 2, 3, 4, 5, 6, 7, 8, 9, 10 or even more different nucleic acid species, e.g. DNA or RNA, as defined herein, each encoding at least one peptide or protein derived from a genetically distinct SARS-CoV-2 (e.g. a distinct SARS-CoV-2 isolate), or a fragment or variant thereof. The terms "distinct" or "distinct SARS-CoV-2" as used throughout the present specification have to be understood as the difference between at least two respective SARS-CoV-2 (e.g. a distinct SARS-CoV-2 isolate), wherein the difference is manifested on the genome of the respective distinct SARS-CoV-2. Particularly, said (genetically) distinct SARS-CoV-2 may express at least one distinct protein, peptide or polyprotein, wherein the at least one distinct protein, peptide or polyprotein differs in at least one amino acid.

In embodiments, the component A-1 comprises 2, 3, 4 or 5 nucleic acid species (e.g. DNA or RNA), preferably RNA species, wherein said nucleic acid species comprise or consist of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of **SEQ ID NOs: 116-132, 134-138, 140-143, 145-175, 11664-11813, 11815, 11817-12050, 12052, 12054-13147, 13514, 13515, 13519, 13520, 14124-14177, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** and, optionally, at least one pharmaceutically acceptable carrier or excipient, wherein each of the 2, 3, 4 or 5 nucleic acid species encode a different antigenic peptide or protein of a SARS-CoV-2 coronavirus.

Accordingly, in embodiments, the component A-1 comprises two nucleic acid species (e.g. DNA or RNA), preferably RNA species, wherein the nucleic acid species comprise or consist of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of **SEQ** ID **NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** and, optionally, at least one pharmaceutically acceptable carrier or excipient, wherein each of the two nucleic acid species encode a different antigenic peptide or protein of a SARS-CoV-2 coronavirus.

In embodiments, the component A-1 comprises three nucleic acid species (e.g. DNA or RNA), preferably RNA species, wherein the nucleic acid comprises or consists of a nucleic acid sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting **148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** and, optionally, at least one pharmaceutically acceptable carrier or excipient, wherein each of the 2, 3, 4 or 5 nucleic acid species encode a different antigenic peptide or protein of a SARS-CoV-2 coronavirus.

Preferably, the at least 2, 3, 4, 5, 6, 7, 8, 9, 10 or even more different nucleic acid species of component A-1 each encode a different prefusion stabilized spike protein (as defined in the first aspect). Preferably, stabilization of the perfusion conformation is obtained by introducing two consecutive proline substitutions at residues K986 and V987 in the spike protein (Amino acid positions according to reference **SEQ ID NO: 1**). Accordingly, in preferred embodiments, the at least 2, 3, 4, 5, 6, 7, 8, 9, 10 pre-fusion stabilized spike proteins (S_stab) each comprises at least one pre-fusion stabilizing mutation, wherein the at least one pre-fusion stabilizing mutation comprises the following amino acid substitutions: K986P and V987P (amino acid positions according to reference **SEQ ID NO: 1**).

Suitably, the different spike proteins or prefusion stabilized spike proteins are derived from at least B.1.1.7, B.1.351, P.1, or CAL.20C.

Suitably, the different spike proteins or prefusion stabilized spike proteins have amino acid changes in the S protein comprising:
(i) deIH69, delV70, Y453F, D614G, 1692V and M1229I;
(ii) delH69, delV70, delY144, N501Y, A570D, D614G, P681H, 17161, S982A and D1118H;
(iii) L18F, D80A, D215G, deIL242, delA243, delL244, R246I, K417N, E484K, N501Y, D614G and A701V;
(iv) L18F, T20N, P26S, D138Y, R190S, K417T, E484K, N501Y, D614G, H655Y and T10271; and/or
(v) S13I, W152C, L452R, and D614G.

Accordingly, the at least 2, 3, 4, 5, 6, 7, 8, 9, 10 or even more different nucleic acid species of component A-1 each encode a different prefusion stabilized spike protein, wherein the at least 2, 3, 4, 5, 6, 7, 8, 9, 10 or even more stabilized spike proteins are selected from amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 10-26, 341-407, 609-1278, 13521-13587, 22738, 22740, 22742, 22744, 22746, 22748, 22750, 22752, 22754, 22756, 22758, 22947-22964** or an immunogenic fragment or immunogenic variant of any of these.

In preferred embodiments, component A-1 comprises at 2, 3, 4, or 5 nucleic acid species comprising a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 10, 22961; 22960, 22963, 22941, 22964.**

In preferred embodiments, component A-1 comprises one nucleic acid species comprising a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 10,** wherein the multivalent composition additionally comprises at least 2, 3, 4 further RNA species selected from
i) one nucleic acid species comprises a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22961;** and/or
ii) one nucleic acid species comprises a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22960;** and/or
iii) one nucleic acid species comprises a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22963;** and/or
iv) one nucleic acid species comprises a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22941;** and/or
v) one nucleic acid species comprises a coding sequence encoding an amino acid sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22964.**

Preferably, the at least 2, 3, 4, 5, 6, 7, 8, 9, 10 or even more different nucleic acid species of component A comprise nucleic acid coding sequences each encoding a different prefusion stabilized spike protein, wherein the at least 2, 3, 4, 5, 6, 7, 8, 9, 10 or even more nucleic acid coding sequences are selected from nucleic acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 136-138, 140-143, 145-175, 11731-11813, 11815, 11817-12050, 12052, 12054-12203, 13514, 13515, 13519, 13520, 14124-14141, 22759, 22764-22785, 22969-23184** or fragments or variants of any of these.

In preferred embodiments, component A-1 comprises one nucleic acid species comprising a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 137,** wherein the multivalent composition additionally comprises at least 2, 3, 4 further RNA species selected from
i) one nucleic acid species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23091;** and/or
ii) one nucleic acid species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23090;** and/or
iii) one nucleic acid species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23093;** and/or
iv) one nucleic acid species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 22999;** and/or
v) one nucleic acid species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23094.**

Preferably, the at least 2, 3, 4, 5, 6, 7, 8, 9, 10 or even more different nucleic acid species of component A-1 composition comprise nucleic acid coding sequences each encoding a different prefusion stabilized spike protein,
wherein the at least 2, 3, 4, 5, 6, 7, 8, 9, 10 or even more nucleic acid coding sequences are selected from RNA sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 149-151, 163-165, 12338, 12541, 12810-12813, 12901, 12931, 13013, 22792, 22794, 22796, 22798, 22802, 22804, 22806, 22810, 22813, 22819, 22821, 22823, 22825, 22827, 22829, 22831, 22833, 22836, 22837, 22839, 23297-23314, 23369, 23517-23520, 23523-23525, 23527, 23529, 23530, 23589, 23737, 23957, 24397, 24837, 25057, 25277, 25717, 26925-26937** or fragments or variants of any of these.

In preferred embodiments, component A-1 comprises one RNA species comprising or consisting of an RNA sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 163,** wherein the multivalent composition additionally comprises at least 2, 3, 4 further RNA species selected from
i) one RNA species comprising or consisting of an RNA sequence being identical or at least 70%, 80%, 85%, 86%, 87%, 88%, 89%, 90%, 91%, 92%, 93%, 94%, 95%, 96%, 97%, 98%, or 99% identical to any one **of SEQ ID NOs: 23311;** and/or
ii) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23310;** and/or
iii) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23313;** and/or
iv) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one **of SEQ ID NOs: 23219;** and/or
v) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23314;**
wherein, preferably, each of the mRNA species comprise a Cap1 structure, and, optionally, each of the mRNA species do not comprise modified nucleotides.

In preferred embodiments, component A-1 comprises one RNA species comprising or consisting of an RNA sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 149 or 24837,** wherein the multivalent composition additionally comprises at least 2, 3, 4 further RNA species selected from
i) one RNA species comprising or consisting of an RNA sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23531 or 24851;** and/or
ii) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23530 or 24850;** and/or
iii) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23533 or 24853;** and/or
iv) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23439 or 24759;** and/or
v) one RNA species comprises a coding sequence being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 23534 or 24854;**
wherein, preferably, each of the mRNA species comprise a Cap1 structure, and, optionally, each of the mRNA species do not comprise modified nucleotides.

In further preferred embodiments, component A-1 comprises at least two RNA species being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 149 or 24837, 23531 or 24851, 23530 or 24850, 23533 or 24853, 23439 or 24759 or 23534** or **24854.**

In preferred embodiments, component A-1 comprises at least one nucleic acid encoding at least one antigenic peptide or protein that is selected or derived from at least one SARS-CoV-2, or an immunogenic fragment or immunogenic variant thereof, wherein said component A is to be, preferably, administered intramuscularly or intradermal.

Preferably, intramuscular or intradermal administration of said component A-1 results in expression of the encoded SARS-CoV-2 antigen construct in a subject. In embodiments where the nucleic acid is an RNA, administration of component A results in translation of the RNA and to a production of the encoded SARS-CoV-2 antigen in a subject In embodiments where the nucleic acid is a DNA (e.g. plasmid DNA, adenovirus DNA), administration of said composition results in transcription of the DNA into RNA, and to a subsequent translation of the RNA into the encoded SARS-CoV-2 antigen in a subject.

In embodiments, administration of the pharmaceutical composition comprising component A-1 to a subject elicits neutralizing antibodies against SARS-CoV-2 and does not elicit disease enhancing antibodies. In particular, administration of a pharmaceutical composition comprising component A-1 encoding SARS-CoV-2 pre-fusion stabilized spike protein to a subject does not elicit immunopathological effects, like e.g. enhanced disease and/or antibody dependent enhancement (ADE).

In preferred embodiments, administration of component A-1 elicits antigen-specific immune responses comprising T-cell responses and/or B-cell responses against the encoded SARS-CoV-2 antigens provided by the at least one nucleic acid of component A-1.

Preferably, the component A-1 is suitable for a vaccine, in particular, suitable for a SARS-CoV-2 vaccine, preferably a combination vaccine of the invention.

In embodiments, the nucleic acid as comprised in component A-1 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component A-1 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

In the following, preferred embodiments of component A1 are provided in form of an item list (see below).

**Item 1:** Component A-1 relates to a composition comprising an mRNA comprising:
(a) at least one coding sequence encoding a SARS-CoV-2 spike protein (S) at least 90% identical to **SEQ ID NOs: 10, 341, 22960, 22961, 22963,** preferably **SEQ ID NO: 10,** that is a pre-fusion stabilized spike protein (S_stab) comprising at least one pre-fusion stabilizing mutation;
(b) at least one heterologous untranslated region (UTR); and
(c) at least one pharmaceutically acceptable carrier,

wherein the mRNA is complexed or associated with lipid nanoparticles (LNP) and wherein the LNP comprises:
   (i) at least one cationic lipid;
   (ii) at least one neutral lipid;
   (iii) at least one steroid or steroid analogue; and
   (iv) at least one PEG-lipid,
wherein (i) to (iv) are in a molar ratio of about 20-60% cationic lipid, 5-25% neutral lipid, 25-55% sterol, and 0.5-15% PEG-lipid.

**Item 2:** Component A-1 of item 1, wherein the mRNA comprises at least one poly(A) sequence, preferably comprising 30 to 200 adenosine nucleotides and/or at least one poly(C) sequence, preferably comprising 10 to 40 cytosine nucleotides.

**Item 3:** Component A-1 of item 1 or 2, wherein the mRNA comprises a 5'-cap structure, preferably m7G, cap0, cap1, cap2, a modified cap0 or a modified cap1 structure.

**Item 4:** Component A-1 of any of the proceeding items, wherein the S protein comprises a pre-fusion stabilizing K986P and V987P mutation.

**Item 5:** Component A-1 of any of the proceeding items, wherein the at least one coding sequence is a codon modified coding sequence, wherein the at least one codon modified coding sequence is selected from C maximized coding sequence, CAI maximized coding sequence, human codon usage adapted coding sequence, G/C content modified coding sequence, and G/C optimized coding sequence, or any combination thereof.

**Item 6:** Component A-1 of any of the proceeding items, wherein the at least one coding sequence has a G/C content that is increased by at least 10% 20% or 30% compared to the G/C content of the coding sequence of the corresponding wild-type or reference nucleic acid sequence.

**Item 7:** Component A-1 of any of the proceeding items, wherein the at least one coding sequence has a G/C content of at least about 50%, 55%, or 60%, preferably of about 63.9%.

**Item 8:** Component A-1 of any of the proceeding items, wherein the mRNA comprises a sequence at least 90% identical to **SEQ ID NOs: 149, 163, 24837, 26633, 26907,** preferably **SEQ ID NO: 163.**

**Item 9:** Component A-1 of any of the proceeding items, wherein the at least one heterologous untranslated region is selected from at least one heterologous 5'-UTR and/or at least one heterologous 3'-UTR.

**Item 10:** Component A-1 of item 9, wherein the at least one heterologous 3'-UTR comprises or consists of a nucleic acid sequence derived from a 3'-UTR of a gene selected from PSMB3, ALB7, alpha-globin (referred to as "muag"), CASP1, COX6B1, GNAS, NDUFA1 and RPS9, or from a homolog, a fragment or a variant of any one of these genes.

**Item 11:** Component A-1 of item 9, wherein the at least one heterologous 5'-UTR comprises or consists of a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes.

**Item 12:** Component A-1 of any of the proceeding items, wherein the nucleic acid comprises at least one histone stem-loop.

**Item 13:** Component A-1 of any of the proceeding items, wherein the mRNA comprises a nucleotide analog.

**Item 14:** Component A-1 of any of the proceeding items, wherein the mRNA does not comprise a 1-methylpseudouridine substitution.

**Item 15:** Component A-1 of any of the proceeding items, wherein the mRNA has an RNA integrity of at least about 50%, preferably of at least about 60%, more preferably of at least about 70%, most preferably of at least about 80%.

**Item 16:** Component A-1 of any of the proceeding items, wherein the mRNA is a purified mRNA that has been purified by RP-HPLC and/or TFF.

**Item 17:** Component A-1 of any of the proceeding items, wherein the mRNA is a purified mRNA that has been purified by RP-HPLC and/or TFF and comprises about 5%, 10%, or 20% less double stranded RNA side products as an RNA that has not been purified with RP-HPLC and/or TFF.

**Item 18:** Component A-1 of any of the proceeding items, wherein the LNP comprises a cationic lipid according to formula III: 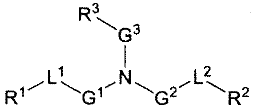 or a pharmaceutically acceptable salt, tautomer, prodrug or stereoisomer thereof, wherein:
L1 or L2 is each independently -O(C=O)-, -(C=O)O-, -C(=O)-, -O-, -S(O)x-, -S-S-, -C(=O)S-, -SC(=O)-, -NRaC(=O)-, -C(=O)NRa-, -NRaC(=O)NRa-, -OC(=O)NRa- or -NRaC(=O)O-, preferably L1 or L2 is -O(C=O)- or -{C=O)O-;
G1 and G2 are each independently unsubstituted C1-C12 alkylene or C1-C12 alkenylene;
G3 is C1-C24 alkylene, C1-C24 alkenylene, C3-C8 cycloalkylene, or C3-C8 cycloalkenylene;
Ra is H or C1-C12 alkyl;
R1 and R2 are each independently C6-C24 alkyl or C6-C24 alkenyl;
R3 is H, OR5, CN, -C(=O)OR4, -OC(=O)R4 or -NR5C(=O)R4;
R4 is C1-C12 alkyl;
R5 is H or C1-C6 alkyl; and
x is 0, 1 or 2.

**Item 19:** Component A-1 of any of the proceeding items, wherein the LNP comprises a cationic lipid according to formula III-3:

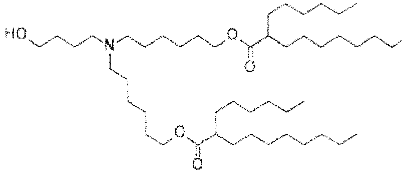

**item 20:** Component A-1 of any of the proceeding items, wherein the LNP comprises a PEG lipid according to formula IVa:

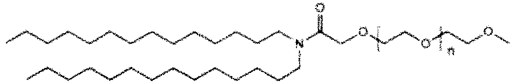

**Item 21:** Component A-1 of any of the proceeding items, wherein the LNP comprises:
(i) at least one cationic lipid according to formula (III-3);
(ii) at least one neutral lipid comprising 1,2-distearoyl-sn-glycero-3-phosphocholine (DSPC);
(iii) at least one sterol comprising cholesterol; and
(iv) at least one PEG-lipid according to formula (IVa),
wherein (i) to (iv) are in a molar ratio of about 20-60% cationic lipid, 5-25% neutral lipid, 25-55% sterol, and 0.5-15% PEG-lipid.

**Item 22:** Component A-1 of any of the proceeding items, wherein at least 70%, 75%, 80%, 85%, 90% or 95% of the mRNA is intact at least about two weeks after storage as a liquid at temperatures of about 5°C.

**Item 23:** Component A-1 of any of the proceeding items, wherein the composition comprises less than about 20% free RNA, preferably less than about 15% free RNA, more preferably less than about 10% free RNA.

**Item 24:** Component A-1 of any of the proceeding items, wherein the LNPs have a mean diameter of from about 30nm, 35nm, 40nm, 45nm, 50nm, 55nm, 60nm, 65nm, 70nm, 75nm, 80nm, 85nm, 90nm, 95nm, 100nm, 105nm, 110nm, 115nm, 120nm, 125nm, 130nm, 135nm, 140nm, 145nm, 150nm, 160nm, 170nm, 180nm, 190nm to 200nm.

**Item 25:** Component A-1 of any of the proceeding items, wherein component A-1 is suitable for treating or preventing COVID-19.

**Item 26:** Component A-1 of any of the proceeding items, wherein (upon administration) component A-1 induces neutralizing antibodies against SARS-CoV-2 in the subject.

**Item 27:** Component A-1 of any of the proceeding items, wherein (upon administration) component A-1 stimulates an antibody response that produces between about 10 and about 300; about 20 and about 300; about 20 and about 200; or about 30 and about 100 coronavirus spike protein-binding antibodies for every coronavirus neutralizing antibody.

**Item 27:** Component A-1 of any of the proceeding items, wherein (upon administration) component A-1 stimulates an immune response in the subject that protects the subject from COVID-19.

### Component A-2: SARS-associated virus

In a particularly preferred embodiment, the at least one Coronavirus of component A, is a SARS-associated virus, preferably a SARS-CoV-1 virus (also referred to as component A-2).

Accordingly, in embodiments of the first aspect, the nucleic acid of component A comprises at least one coding sequence encoding at least one antigenic peptide or protein selected or derived from at least one SARS-associated virus, preferably a SARS-CoV-1, or an immunogenic fragment or immunogenic variant thereof.

It has to be understood that generic embodiments and features that have been described in paragraph *"Component A", may* also apply to a nucleic acid encoding a SARS-CoV-1 antigenic peptide or protein.

SARS-CoV associated viruses belong to the Coronaviridae, in particular to Orthocoronaviruses, more specifically to the genus Betacoronavirus. SARS-CoV-1 (severe acute respiratory syndrome coronavirus, SARS-Coronavirus, SCV) causes a severe respiratory syndrome disease (SARS). An exemplary SARS-CoV-1 coronaviruses is identifiable by NCBI Taxonomy ID: 694009, NCBI Reference: DQ182595.1.

Further suitable SARS-associated viruses in the context of the invention are SARS-CoV/Tor2, HCoV/OC43, HCoV/HKU1/N5, HCoV/229E/BN1/GER/2015, HCoV/NL63/RPTEC/2004, Bat SARS-like CoV/WIV1, BatCoV/HKU9-1 BF_005I, PDCoV/Swine/Thailand/S5011/2015, PEDV/NPL-PEDv/2013/P10, PEDV/NPL-PEDv/2013/P10, or MHV/S.

In the context of the invention, any protein selected or derived from a SARS-associated virus, preferably a SARS-CoV-1 may be used in the context of the invention and may be suitably encoded by the coding sequence or the nucleic acid of component A (in particular, component A-2). It is further in the scope of the underlying invention, that the at least one antigenic peptide or protein may comprise or consist of a synthetically engineered or an artificial SARS-CoV-1 peptide or protein. The term "synthetically engineered" SARS-CoV-1 peptide or protein, or the term "artificial coronavirus peptide or protein" relates to a protein that does not occur in nature. Accordingly, an "artificial coronavirus peptide or protein" or a "synthetically engineered coronavirus peptide or protein" may for example differ in at least one amino acid compared to the natural SARS-CoV-1 peptide or protein, and/or may comprise an additional heterologous peptide or protein element, and/or may be N-terminally or C-terminally extended or truncated.

In preferred embodiments, the nucleic acid of component A comprises at least one coding sequence encoding at least one antigenic peptide or protein selected or derived from a SARS-associated virus, preferably a SARS-CoV-1, or an immunogenic fragment or immunogenic variant thereof, wherein the at least one antigenic peptide or protein comprises at least one peptide or protein that is selected or is derived from a structural protein, an accessory protein, or a replicase protein or an immunogenic fragment or immunogenic variant of any of these.

Suitably, the structural protein is selected or derived from a spike protein (S), an envelope protein (E), a membrane protein (M) or a nucleocapsid protein (N), or an immunogenic fragment or variant of any of these.

In particularly preferred embodiments of the pharmaceutical composition, the encoded at least one antigenic peptide or protein component A (in particular component A-2) comprises or consists at least one peptide or protein selected or derived from a SARS-associated virus, preferably a SARS-CoV-1 spike protein (S, S1, S2, or S1 and S2), or an immunogenic fragment or immunogenic variant of any of these.

Suitable antigenic peptide or protein sequences that are provided by the nucleic acid of component A (in particular, component A-2) are disclosed in **Table 4,** rows 1 to 45, Column A and B. In addition, further information regarding said suitable antigenic peptide or protein sequences selected or derived from SARS-associated virus, preferably a SARS-CoV-1 are provided under <223> identifier of the ST.25 sequence listing.

In the following, preferred antigenic peptide or protein sequences selected or derived from SARS-CoV-1 that are provided by the nucleic acid of component A are described in detail.

It has to be noted that variation on amino acid level naturally occurs between spike proteins derived from different SARS isolates. In the context of the invention, such amino acid variations can be applied to each antigenic peptide or protein derived from a SARS-associated virus or a SARS-CoV-1 spike protein as described herein.

In embodiments, a fragment of a SARS-associated virus, preferably a SARS-CoV-1 spike protein (S) may be encoded by the nucleic acid of component A, wherein said fragment may be N-terminally truncated, and/or wherein said fragment may be C-terminally truncated. Such "fragment of a spike protein (S)" may additionally comprise amino acid substitutions (as described below) and may additionally comprise at least one heterologous peptide or protein element (as described below).

In embodiments, the encoded at least one antigenic peptide or protein of component A comprises or consists of a SARS-associated virus, preferably a SARS-CoV-1 spike protein (S), wherein the spike protein (S) lacks the transmembrane domain (TM). Without wishing to being bound to theory, a SARS-CoV-1 spike protein (S) lacking the transmembrane domain (TM) as defined herein could be suitable for a combination vaccine, as such a protein would be soluble and not anchored in the cell membrane. A soluble protein may therefore be produced (that is translated) in higher concentrations upon administration to a subject, leading to improved immune responses.

Without wishing to being bound to theory, RBD and CND domains may be crucial for immunogenicity of SARS-associated virus, preferably a SARS-CoV-1 spike protein (S). Both regions are located at the S1 fragment of the spike protein. Accordingly, it may be suitable in the context of the invention that the antigenic peptide or protein comprises or consists of an S1 fragment of the spike protein or an immunogenic fragment or immunogenic variant thereof.

Suitably, a S1 fragment of SARS-associated virus, preferably a SARS-CoV-1 may comprise at least an RBD and/or a CND domain as defined above.

In particularly preferred embodiments, the encoded at least one antigenic peptide or protein of component A (in particular component A-2) comprises or consists of a SARS-associated virus, preferably a SARS-CoV-1 spike protein (S), wherein the spike protein (S) comprises or consists of a spike protein fragment S1, or an immunogenic fragment or immunogenic variant thereof.

In preferred embodiments, the encoded at least one antigenic peptide or protein comprises a SARS-associated virus, preferably a SARS-CoV-1 spike protein fragment S1, and lacks at least 70%, 80%, 90%, preferably 100% of spike protein fragment S2. Such embodiments may be beneficial as the SARS-CoV-1 S1 fragment comprises neutralizing epitopes without potential problems of full length protein comprising S1 and S2.

Without wishing to being bound to theory, it may be suitable that the antigenic peptide or protein of component A (in particular component A-2) comprises or consists of SARS-associated virus, preferably a SARS-CoV-1 spike protein fragment S1 and (at least a fragment of) SARS-CoV-1 spike protein fragment S2, because the formation of an immunogenic spike protein may be promoted.

Accordingly, in particularly preferred embodiments, the encoded at least one antigenic peptide or protein of component A (in particular component A-2) comprises or consists of a SARS-associated virus, preferably a SARS-CoV-1 spike protein (S), wherein the spike protein (S) comprises or consists of a spike protein fragment S1 or an immunogenic fragment or immunogenic variant thereof, and spike protein fragment S2 or an immunogenic fragment or immunogenic variant thereof.

In particularly preferred embodiments, the encoded at least one antigenic peptide or protein of component A (in particular component A-2) comprises or consists of a full length SARS-associated virus, preferably a SARS-CoV-1 spike protein or an immunogenic fragment or immunogenic variant of any of these.

In particularly preferred embodiments, the SARS-associated virus, preferably a SARS-CoV-1 spike protein (S) that is provided by the nucleic acid of component A (in particular component A-2) is designed or adapted to stabilize the antigen in pre-fusion conformation. A pre-fusion conformation is particularly advantageous in the context of an efficient SARS-CoV-1 vaccine, as several potential epitopes for neutralizing antibodies may merely be accessible in said pre-fusion protein conformation. Furthermore, remaining of the protein in the pre-fusion conformation is aimed to avoid immunopathological effects, like e.g. enhanced disease and/or antibody dependent enhancement (ADE).

Accordingly, in preferred embodiments, the nucleic acid of component A (in particular component A-2) comprises at least one coding sequence encoding at least one antigenic peptide or protein that is selected or derived from an SARS-associated virus, preferably a SARS-CoV-1, wherein the at least one antigenic peptide or protein is selected or derived or from a spike protein (S), wherein the spike protein (S) is a pre-fusion stabilized spike protein (S_stab). Suitably, said pre-fusion stabilized spike protein comprises at least one pre-fusion stabilizing mutation.

Stabilization of the SARS-CoV-1 spike protein may be obtained by substituting at least one amino acids at position K968 and/or V969 with amino acids that stabilize the spike protein in a perfusion conformation (amino acid positions according to reference **SEQ ID NO: 14906).**

In embodiments, the pre-fusion stabilizing mutation of SARS-CoV-1 spike protein comprises an amino acid substitution at position K968, wherein the amino acids K968 is substituted with one selected from A, I, L, M, F, V, G, or P (amino acid positions according to reference **SEQ ID NO: 14906),** preferably wherein the amino acids K968 is substituted with P. In embodiments, the pre-fusion stabilizing mutation comprises an amino acid substitution at position V969, wherein the amino acids V969 is substituted with one selected from A, I, L, M, F, V, G, or P (amino acid positions according to reference **SEQ ID NO: 14906),** preferably wherein the amino acids V969 is substituted with P.

Accordingly, in preferred embodiments, the pre-fusion stabilized spike protein (S_stab) of SARS-CoV-1 comprises at least one pre-fusion stabilizing mutation, wherein the at least one pre-fusion stabilizing mutation comprises the following amino acid substitutions: K968P and V969P (amino acid positions according to reference **SEQ ID NO: 14906**).

Any SARS-associated virus protein or fragments or variants thereof can be chosen by the skilled person to introduce such amino acid changes (e.g. such a double Proline mutation). Examples comprise HCoV/OC43 spike protein (1-13583)(A1070P_L1071P), HCoV/OC43/1783A_10 spike protein (1-1362)(A1079P_L1080P), HCoV/HKU1/N5 spike protein (1-1351)(N1067P_L1008P), HCoV/229E/BN1/GER/2015 spike protein (1-1171)(I869P_I870P), HCoV/NL63/RPTEC/2004 spike protein (1-1356)(S1052P_I1053P), Bat SARS-like CoV/WIV1 spike protein (1-1256)(K969P _V970P), BatCoV/HKU9-1 BF_005I spike protein (1-1274)(G983P_L984P), PDCoV/Swine/Thailand/S5011/2015 spike protein (1-1160)((E855P_V856P), PEDV/NPL-PEDv/2013/P10 spike protein (1-1386)(I1076P_L1077P), MHV/S spike protein (1-1361)(A1073P_L1074P).

In embodiments, the at least one pre-fusion stabilizing mutation of SARS-associated virus, preferably a SARS-CoV-1 spike protein comprises a cavity filling mutation.

In embodiments, the at least one pre-fusion stabilizing mutation of SARS-associated virus, preferably a SARS-CoV-1 spike protein comprises a mutated protonation site.

In embodiments, the at least one pre-fusion stabilizing mutation of the SARS-associated virus, preferably a SARS-CoV-1 spike protein comprises an artificial intramolecular disulfide bond. Such an artificial intramolecular disulfide bond can be introduced to further stabilize the membrane distal portion of the S protein (including the N-terminal region) in the pre-fusion conformation; that is, in a conformation that specifically binds to one or more pre-fusion specification antibodies, and/or presents a suitable antigenic site that is present on the pre-fusion conformation but not in the post fusion conformation of the S protein.

Any SARS-associated virus, preferably a SARS-associated virus, preferably a SARS-CoV-1 S protein or fragments or variants thereof can be chosen by the skilled person to introduce at least one cavity filling mutation, at least one mutated protonation site, and/or at least one artificial intramolecular disulfide bond.

According to various preferred embodiments, the nucleic acid of component A (in particular component A-2) encodes at least one antigenic peptide or protein selected or derived from SARS-associated virus, preferably a SARS-CoV-1 as defined herein and, additionally, at least one heterologous peptide or protein element, preferably selected or derived from a signal peptide, a linker, a helper epitope, an antigen clustering element, a trimerization element, a transmembrane element, and/or a VLP-forming sequence.

Suitably, the at least one heterologous peptide or protein element may promote or improve secretion of the encoded antigenic peptide or protein of SARS-associated virus, preferably a SARS-CoV-1 (e.g. via secretory signal sequences), promote or improve anchoring of the encoded antigenic peptide or protein in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component A (in particular component A-2) may additionally encode peptide linker elements, self-cleaving peptides, immunologic adjuvant sequences or dendritic cell targeting sequences.

In preferred embodiments, the nucleic acid of component A (in particular component A-2) encoding at least one antigenic protein selected or derived from SARS-associated virus, preferably a SARS-CoV-1 as defined herein, additionally encodes at least one heterologous trimerization element as defined herein, an antigen clustering element as defined herein, or a VLP forming sequence as defined herein.

The antigen clustering elements may be selected from a ferritin element as defined herein, or a lumazine synthase element as defined herein, surface antigen of Hepatitis B virus (HBsAg) as defined herein, or encapsulin as defined herein. In embodiments where the coding sequence of component A (in particular component A-2) additionally encodes heterologous antigen clustering element, it is particularly preferred and suitable to generate a fusion protein comprising an antigen clustering element and an antigenic peptide or protein derived from SARS-CoV-1. Suitably, said antigenic peptide or protein, preferably the SARS-CoV-1 spike protein, is lacking the C-terminal transmembrane domain (TM) or parts of the TM. Further, it may be suitable to use linker elements as defined for separating the heterologous antigen clustering element from the antigenic peptide or protein.

The trimerization element may be selected from a foldon element as defined herein. In preferred embodiments, the foldon element is a fibritin foldon element as defined herein. In embodiments where the coding sequence of the nucleic acid of component A (in particular component A-2) additionally encodes heterologous trimerization element, it is particularly preferred and suitable to generate a fusion protein comprising a trimerization element and an antigenic peptide or protein derived from SARS-associated virus, preferably a SARS-CoV-1. Suitably, said antigenic peptide or protein, preferably the spike protein derived from SARS-associated virus, preferably a SARS-CoV-1 that is lacking the C-terminal transmembrane domain, or parts of the TM. Further, it may be suitable to use linker elements as defined for separating the heterologous antigen clustering element from the antigenic peptide or protein.

The VLP forming sequence may be selected and fused to the SARS-associated virus, preferably a SARS-CoV-1 antigen as defined herein. Suitable VLP forming sequences may be selected from elements derived from Hepatitis B virus core antigen as defined herein, HIV-1 Gag protein as defined herein, or Woodchuck hepatitis core antigen element (WhcAg) as defined herein. In embodiments where the coding sequence of the nucleic acid of component A (in particular component A-2) additionally encodes heterologous VLP forming sequence, it is particularly preferred and suitable to generate a fusion protein comprising a VLP forming sequence and an antigenic peptide or protein derived from SARS-CoV-1. Suitably, said antigenic peptide or protein, preferably the spike protein derived from SARS-CoV-1 that is lacking the C-terminal transmembrane domain (TM), or parts of the TM. Further, it may be suitable to use linker elements as defined herein for separating the heterologous antigen clustering element from the antigenic peptide or protein.

In embodiments, the antigenic peptide or protein comprises a heterologous signal peptide as defined above. A heterologous signal peptide may be used to improve the secretion of the encoded SARS-associated virus, preferably a SARS-CoV-1 antigen. In embodiments where the coding sequence of the nucleic acid of component A (in particular component A-2) additionally encodes heterologous secretory signal peptide, it is particularly preferred and suitable to generate a fusion protein comprising a heterologous secretory signal peptide and an antigenic peptide or protein derived from SARS-associated virus, preferably a SARS-CoV-1. Suitably, said antigenic peptide or protein, preferably the spike protein derived from SARS-associated virus, preferably a SARS-CoV-1 is lacking the N-terminal endogenous secretory signal peptide.

Preferred antigenic peptide or proteins selected or derived from a SARS-associated virus, preferably a SARS-CoV-1 as defined above are provided in **Table 4** (rows 1 to 45). Therein, each row 1 **to 45** corresponds to a suitable SARS-CoV-1 constructs or a SARS-associated virus construct. Column A of **Table 4** provides a short description of suitable antigen constructs. Column B of **Table 4** provides protein (amino acid) SEQ ID NOs of respective antigen constructs. Column D of **Table 4** provides SEQ ID NO of the corresponding G/C optimized nucleic acid coding sequences (opt1, gc). Column E of **Table 4** provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of **Table 4,** e.g. mRNA sequences comprising the coding sequences of **Table 4** are provided in **Table 5.**

**Table 4: SARS-CoV-1 and SARS-associated constructs (amino acid sequences and nucleic acid coding sequences):**

| **row** | **A** | **B** | **D** | **E** |
|---|---|---|---|---|
| 1 | SARS-CoV spike protein (1-1255) | 14906 | 14951 | 14996 |
| 2 | SARS-CoV spike protein (1-1255)(K968P_V969P) | 14907 | 14952 | 14997 |
| 3 | SARS-CoV spike protein (1-17_747-1255) | 14908 | 14953 | 14998 |
| 4 | SARS-CoV/Tor2 spike protein (1-1255) | 14909 | 14954 | 14999 |
| 5 | SARS-CoV/Tor2 spike protein (1-1255)(K968P_V969P) | 14910 | 14955 | 15000 |
| 6 | SARS-CoV/Tor2 spike protein (variant) | 14911 | 14956 | 15001 |
| 7 | SARS-CoV/Tor2 spike protein (variant) | 14912 | 14957 | 15002 |
| 8 | HCoV/OC43 spike protein (1-1353) | 14913 | 14958 | 15003 |
| 9 | HCoV/OC43 spike protein (1-1353)(A1070P_L1071P) | 14914 | 14959 | 15004 |
| 10 | HCoV/OC43/1783A_10 spike protein (1-1362) | 14915 | 14960 | 15005 |
| 11 | HCoV/OC43/1783A-10 spike protein (1-1362)(A1079P_L1080P) | 14916 | 14961 | 15006 |
| 12 | HCoV/OC43/1783A_10 spike protein (variant) | 14917 | 14962 | 15007 |
| 13 | HCoV/OC43/1783A_10 spike protein (variant) | 14918 | 14963 | 15008 |
| 14 | HCoV/HKU1/N5 spike protein (1-1351) | 14919 | 14964 | 15009 |
| 15 | HCoV/HKU1/N5 spike protein (1-1351)(N1067P_L1068P) | 14920 | 14965 | 15010 |
| 16 | HCoV/HKU1/N5 spike protein (variant) | 14921 | 14966 | 15011 |
| 17 | HCoV/HKU1/N5 spike protein (variant) | 14922 | 14967 | 15012 |
| 18 | HCoV/229E/BN1/GER/2015 spike protein (1-1171) | 14923 | 14968 | 15013 |
| 19 | HCoV/229E/BN1/GER/2015 spike protein (1-1171)(I869P_I870P) | 14924 | 14969 | 15014 |
| 20 | HCoV/229E/BN1/GER/2015 spike protein (variant) | 14925 | 14970 | 15015 |
| 21 | HCoV/229E/BN1/GER/2015 spike protein (variant) | 14926 | 14971 | 15016 |
| 22 | HCoV/NL63/RPTEC/2004 spike protein (1-1356) | 14927 | 14972 | 15017 |
| 23 | HCoV/NL63/RPTEC/2004 spike protein (1-1356)(S1052P_I1053P) | 14928 | 14973 | 15018 |
| 24 | HCoV/NL63/RPTEC/2004 spike protein (variant) | 14929 | 14974 | 15019 |
| 25 | HCoV/NL63/RPTEC/2004 spike protein (variant) | 14930 | 14975 | 15020 |
| 26 | Bat SARS-like CoVNVIV1 spike protein (1-1256) | 14931 | 14976 | 15021 |
| 27 | Bat SARS-like CoV/WIV1 spike protein (1-1256)(K969P_V970P) | 14932 | 14977 | 15022 |
| 28 | Bat SARS-like CoV/WIV1 spike protein (variant) | 14933 | 14978 | 15023 |
| 29 | Bat SARS-like CoV/WIV1 spike protein (variant) | 14934 | 14979 | 15024 |
| 30 | BatCoV/HKU9-1 BF_005I spike protein (1-1274) | 14935 | 14980 | 15025 |
| 31 | BatCoV/HKU9-1 BF_005I spike protein (1-1274)(G983P_L984P) | 14936 | 14981 | 15026 |
| 32 | BatCoV/HKU9-1 BF_005I spike protein (variant) | 14937 | 14982 | 15027 |
| 33 | BatCoV/HKU9-1 BF_005I spike protein (variant) | 14938 | 14983 | 15028 |
| 34 | PDCoV/Swine/Thailand/S5011/2015 spike protein (1-1160) | 14939 | 14984 | 15029 |
| 35 | PDCoV/Swine/Thailand/S5011/2015 spike protein (1-1160)((E855P_V856P) | 14940 | 14985 | 15030 |
| 36 | PDCoV/Swine/Thailand/S5011/2015 spike protein (variant) | 14941 | 14986 | 15031 |
| 37 | PDCoV/Swine/Thailand/S5011/2015 spike protein (variant) | 14942 | 14987 | 15032 |
| 38 | PEDV/NPL-PEDv/2013/P10 spike protein (1-1386) | 14943 | 14988 | 15033 |
| 39 | PEDV/NPL-PEDv/2013/P10 spike protein (1-1386)(I1076P_L1077P) | 14944 | 14989 | 15034 |
| 40 | PEDV/NPL-PEDv/2013/P10 spike protein (variant) | 14945 | 14990 | 15035 |
| 41 | PEDV/NPL-PEDv/2013/P10 spike protein (variant) | 14946 | 14991 | 15036 |
| 42 | MHV/S spike protein (1-1361) | 14947 | 14992 | 15037 |
| 43 | MHV/S spike protein (1-1361)(A1073P_L1074P) | 14948 | 14993 | 15038 |
| 44 | MHV/S spike protein (variant) | 14949 | 14994 | 15039 |
| 45 | MHV/S spike protein (variant) | 14950 | 14995 | 15040 |

In preferred embodiments, the at least one antigenic peptide or protein selected or derived from a SARS associated virus, preferably SARS-CoV-1 encoded by the at least one nucleic acid of component A (in particular component A-2) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 14906-14950** or an immunogenic fragment or immunogenic variant of any of these. Further information regarding said amino acid sequences is also provided in **Table 4** (see rows 1 to 45 of Column A and B), and under <223> identifier of the ST.25 sequence listing of respective sequence SEQ ID NOs.

In further embodiments, the at least one antigenic peptide or protein selected or derived from SARS-CoV-1 encoded by the at least one nucleic acid of component A (in particular component A-2) comprises or consists of at least one of the amino acid sequences being identical or at least 50%, 80%, 70%, 80%, 85%, 86%, 87%, 88%, 89%, 90%, 91%, 92%, 93%, 94%, 95%, 96%, 97%, 98%, or 99% identical to any one of SEQ ID NOs: 29, 32 or 34 of published PCT patent application WO2017070626 or an immunogenic fragment or immunogenic variant of any of these. SEQ ID NOs: 29, 32 or 34 of WO2017070626, and the corresponding disclosure relating thereto are herewith incorporated by reference.

In further embodiments, the at least one antigenic peptide or protein selected or derived from SARS-CoV-1 encoded by the at least one nucleic acid of component A (in particular component A-2) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of SEQ ID NOs: 7 or 30 of published PCT patent application WO2018081318 or an immunogenic fragment or immunogenic variant of any of these. SEQ ID NOs: 7 or 30 of WO2018081318, and the corresponding disclosure relating thereto are herewith incorporated by reference.

Further suitable antigenic peptide or proteins selected or derived from SARS-CoV-1 can be selected or derived from Table 12 of WO2017070626. Accordingly, the full content of Table 12 of WO2017070626 herewith incorporated by reference.

In preferred embodiments, the at least one antigenic peptide or protein (pre-fusion stabilized spike protein (S_stab)) selected or derived from a SARS associated virus, preferably a SARS-CoV-1 encoded by the at least one nucleic acid of component A (in particular component A-2) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 14907, 14910, 14914, 14916, 14920, 14924, 14928, 14932, 14936, 14940, 14944, 14948** or an immunogenic fragment or immunogenic variant of any of these. Further information regarding said amino acid sequences is also provided in **Table 4,** and under <223> identifier of the ST.25 sequence listing of respective sequence SEQ ID NOs.

According to preferred embodiments, the nucleic acid of component A (in particular component A-2) comprises at least one coding sequence encoding at least one antigenic peptide or protein derived from SARS associated virus, preferably a SARS-CoV-1 as defined above, or fragments and variants thereof. In that context, any coding sequence encoding at least one SARS associated virus, preferably a SARS-CoV-1 antigenic protein as defined herein, or fragments and variants thereof may be understood as suitable coding sequence and may therefore be comprised in the nucleic acid of component A.

In preferred embodiments, the nucleic acid of component A (in particular component A-2) comprise or consist of at least one coding sequence encoding at least one antigenic peptide or protein selected or derived from a SARS-associated virus as defined herein, preferably encoding any one of **SEQ ID NOs: 14906-14950;** SEQ ID NOs: 1-152, 1448-1548 of WO2018115527; SEQ ID NOs: 29, 32 or 34 of WO2017070626; SEQ ID NOs: 7 or 30 of WO2018081318, or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14906-14950;** SEQ ID NOs: 1-152, 1448-1548 of WO2018115527; SEQ ID NOs: 29, 32 or 34 of WO2017070626; SEQ ID NOs: 7 or 30 of WO2018081318, or fragments or variants thereof, may be selected and may accordingly be understood as suitable coding sequence of the invention. Further information regarding said amino acid sequences is also provided in **Table 4** (see rows 1 to 45 of Column A and B), **Table 5,** and under <223> identifier of the ST.25 sequence listing of respective sequence SEQ ID NOs.

In preferred embodiments, the nucleic acid of component A (in particular component A-2) comprises a coding sequence that comprises at least one of the nucleic acid sequences encoding a SARS-associated virus antigen, preferably a SARS-CoV-1 antigen, being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14951-15220,** or a fragment or a fragment or variant of any of these sequences. Further information regarding said nucleic acid sequences is also provided in **Table 4** (see rows 1 to 45), **Table 5,** and under <223> identifier of the ST.25 sequence listing of respective sequence SEQ ID NOs.

In preferred embodiments, the at least one coding sequence of the nucleic acid of component A (in particular component A-2) is a codon modified coding sequence as defined herein, wherein the amino acid sequence, that is the SARS-associated virus peptide or protein, encoded by the at least one codon modified coding sequence, is preferably not being modified compared to the amino acid sequence encoded by the corresponding wild type or reference coding sequence.

In particularly preferred embodiments, the at least one coding sequence of the nucleic acid component A (in particular component A-2) is a codon modified coding sequence, wherein the codon modified coding sequence is selected a G/C optimized coding sequence, a human codon usage adapted coding sequence, or a G/C modified coding sequence.

Preferred nucleic acid sequences of component A (in particular component A-2), including particularly preferred mRNA sequences, are provided in **Table 5** (column C and D). Therein, each row represents a specific suitable SARS-associated virus construct of the invention (compare with **Table 4**)**,** wherein the description of the SARS-associated virus construct is indicated in column A of **Table 5** and the SEQ ID NOs of the amino acid sequence of the respective SARS-associated virus construct is provided in column B. The corresponding SEQ ID NOs of the coding sequences encoding the respective SARS-associated virus constructs are provided in **Table 4.** Further information is provided under <223> identifier of the respective SEQ ID NOs in the sequence listing.

**Table 5: Nucleic acid, preferably mRNA constructs encoding SARS-associated or SARS-CoV-1 antigens**

| **A** | **B** | **C** | **D** |
|---|---|---|---|
| SARS-CoV spike protein (1-1255) | 14906 | 15041, 15086 | 15131, 15176 |
| SARS-CoV spike protein (1-1255)(K968P_V969P) | 14907 | 15042, 15087 | 15132, 15177 |
| SARS-CoV spike protein (1-17_747-1255) | 14908 | 15043, 15088 | 15133, 15178 |
| SARS-CoV/Tor2 spike protein (1-1255) | 14909 | 15044, 15089 | 15134, 15179 |
| SARS-CoV/Tor2 spike protein (1-1255)(K968P_V969P) | 14910 | 15045, 15090 | 15135, 15180 |
| SARS-CoV/Tor2 spike protein (variant) | 14911 | 15046, 15091 | 15136, 15181 |
| SARS-CoV/Tor2 spike protein (variant) | 14912 | 15047, 15092 | 15137, 15182 |
| HCoV/OC43 spike protein (1-1353) | 14913 | 15048, 15093 | 15138, 15183 |
| HCoV/OC43 spike protein (1-1353)(A1070P_L1071P) | 14914 | 15049, 15004 | 15139, 15184 |
| HCoV/OC43/1783A_10 spike protein (1-1362) | 14915 | 15050, 15095 | 15140, 15185 |
| HCoV/OC43/1783A_10 spike protein (1-1362)(A1079P_L1080P) | 14916 | 15051, 15096 | 15141, 15186 |
| HCoV/OC43/1783A_10 spike protein (variant) | 14917 | 15052, 15097 | 15142, 15187 |
| HCoV/OC43/1783A_10 spike protein (variant) | 14918 | 15053, 15098 | 15143, 15188 |
| HCoV/HKU1/N5 spike protein (1-1351) | 14919 | 15054, 15099 | 15144, 15189 |
| HCoV/HKU1/N5 spike protein (1-1351)(N1067P_L1068P) | 14920 | 15055, 15100 | 15145, 15190 |
| HCoV/HKU1/N5 spike protein (variant) | 14921 | 15056, 15101 | 15146, 15191 |
| HCoV/HKU1/N5 spike protein (variant) | 14922 | 15057, 15102 | 15147, 15192 |
| HCoV/229E/BN1/GER/2015 spike protein (1-1171) | 14923 | 15058, 15103 | 15148, 15193 |
| HCoV/229E/BN1/GER/2015 spike protein (1-1171)(I869P_I870P) | 14924 | 15059, 15104 | 15149, 15194 |
| HCoV/229E/BN1/GER/2015 spike protein (variant) | 14925 | 15060, 15105 | 15150, 15195 |
| HCoV/229E/BN1/GER/2015 spike protein (variant) | 14926 | 15061, 15106 | 15151, 15196 |
| HCoV/NL63/RPTEC/2004 spike protein (1-1356) | 14927 | 15062, 15107 | 15152, 15197 |
| HCoV/NL63/RPTEC/2004 spike protein (1-1356)(S1052P_I1053P) | 14928 | 15063, 15108 | 15153, 15198 |
| HCoV/NL63/RPTEC/2004 spike protein (variant) | 14929 | 15064, 15109 | 15154, 15199 |
| HCoV/NL63/RPTEC/2004 spike protein (variant) | 14930 | 15065, 15110 | 15155, 15200 |
| Bat SARS-like CoV/WIV1 spike protein (1-1256) | 14931 | 15066, 15111 | 15156, 15201 |
| Bat SARS-like CoV/WIV1 spike protein (1-1256)(K969P_V970P) | 14932 | 15067, 15112 | 15157, 15202 |
| Bat SARS-like CoV/WIV1 spike protein (variant) | 14933 | 15068, 15113 | 15158, 15203 |
| Bat SARS-like CoV/WIV1 spike protein (variant) | 14934 | 15069, 15114 | 15159, 15204 |
| BatCoV/HKU9-1 BF_005I spike protein (1-1274) | 14935 | 15070, 15115 | 15160, 15205 |
| BatCoV/HKU9-1 BF_005I spike protein (1-1274)(G983P_L984P) | 14936 | 15071, 15116 | 15161, 15206 |
| BatCoV/HKU9-1 BF_005I spike protein (variant) | 14937 | 15072, 15117 | 15162, 15207 |
| BatCoV/HKU9-1 BF_005I spike protein (variant) | 14938 | 15073, 15118 | 15163, 15208 |
| PDCoV/Swine/Thailand/S5011/2015 spike protein (1-1160) | 14939 | 15074, 15119 | . 15164, 15209 |
| PDCoV/Swine/Thailand/S5011/2015 spike protein (1-1160)((E855P_V856P) | 14940 | 15075, 15120 | 15165, 15210 |
| PDCoV/Swine/Thailand/S5011/2015 spike protein (variant) | 14941 | 15076, 15121 | 15166, 15211 |
| PDCoV/Swine/Thailand/S5011/2015 spike protein (variant) | 14942 | 15077, 15122 | 15167, 15212 |
| PEDV/NPL-PEDv/2013/P10 spike protein (1-1386) | 14943 | 15078, 15123 | 15168, 15213 |
| PEDV/NPL-PEDv/2013/P10 spike protein (1-1386)(11076P_L1077P) | 14944 | 15079, 15124 | 15169, 15214 |
| PEDV/NPL-PEDv/2013/P10 spike protein (variant) | 14945 | 15080, 15125 | 15170, 15215 |
| PEDV/NPL-PEDv/2013/P10 spike protein (variant) | 14946 | 15081, 15126 | 15171, 15216 |
| MHV/S spike protein (1-1361) | 14947 | 15082, 15127 | 15172, 15217 |
| MHV/S spike protein (1-1361)(A1073P_L1074P) | 14948 | 15083, 15128 | 15173, 15218 |
| MHV/S spike protein (variant) | 14949 | 15084, 15129 | 15174, 15219 |
| MHV/S spike protein (variant) | 14950 | 15085, 15130 | 15175, 15220 |

In preferred embodiments, the nucleic acid of component A (in particular component A-2), preferably the RNA, comprises or consists of a nucleic acid sequence encoding a SARS-associated virus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 15041-15220** or a fragment or variant of any of these sequences. Further information regarding respective nucleic acid sequences is provided under <223> identifier of the respective SEQ ID NO in the sequence listing, and in **Table 5** (see in particular Column C and D). Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides.

Suitable features and embodiments that apply to nucleic acids of component A-2 are provided in paragraph *"Nucleic acid features and embodiments"* below.

Suitably, the nucleic acids of component A-2 is formulated and/or complexed. Suitable features and embodiments that apply to nucleic acids complexation or formulation of component A are provided in paragraph "Formulation *and Complexation"* below.

In embodiments, the component A (in particular component A-2) comprises a plurality or at least more than one of the nucleic acid species comprising at least one coding sequence encoding at least one antigenic peptide or protein selected or derived from at least one SARS-associated virus, preferably SARS-CoV-1, or an immunogenic fragment or immunogenic variant thereof, e.g. DNA or RNA species as defined herein. Preferably, component A (in particular component A-2) as defined herein comprises 2, 3, 4, 5, 6, 7, 8, 9, or 10 different nucleic acids each defined as defined herein.

In embodiments, component A (in particular component A-2) may comprise at least 2, 3, 4, 5, 6, 7, 8, 9, 10 or even more different nucleic acid species, e.g. DNA or RNA, as defined herein, each encoding at least one antigenic peptide or protein derived from the same SARS-associated virus, or a fragment or variant thereof. Particularly, said (genetically) same SARS-associated virus expresses (essentially) the same repertoire of proteins or peptides, wherein all proteins or peptides have (essentially) the same amino acid sequence. Particularly, said (genetically) same SARS-associated virus expresses essentially the same proteins, peptides or polyproteins, wherein these protein, peptide or polyproteins preferably do not differ in their amino acid sequence(s).

In embodiments, the component A (in particular component A-2) comprises at least 2, 3, 4, 5, 6, 7, 8, 9, 10 or even more different nudeic acid species, e.g. DNA or RNA, as defined herein, each encoding at least one peptide or protein derived from a genetically distinct SARS-associated virus (e.g. a distinct SARS-associated virus isolate), or a fragment or variant thereof. The terms "distinct" or "distinct SARS-associated virus" as used throughout the present specification have to be understood as the difference between at least two respective SARS-associated virus (e.g. a distinct SARS-associated virus isolate), wherein the difference is manifested on the genome of the respective distinct SARS-associated virus. Particularly, said (genetically) distinct SARS-associated virus may express at least one distinct protein, peptide or polyprotein, wherein the at least one distinct protein, peptide or polyprotein differs in at least one amino acid.

In preferred embodiments, the component A-2 comprises at least one nucleic acid encoding at least one antigenic peptide or protein that is selected or derived from at least one SARS-associated virus, preferably SARS-CoV-1, or an immunogenic fragment or immunogenic variant thereof, wherein said component A-2 is to be, preferably, administered intramuscularly or intradermal.

Preferably, intramuscular or intradermal administration of said component A-2 results in expression of the encoded SARS-associated virus antigen construct in a subject. In embodiments where the nucleic acid is an RNA, administration of component A-2 results in translation of the RNA and to a production of the encoded SARS-associated virus antigen in a subject. In embodiments where the nucleic acid is a DNA (e.g. plasmid DNA, adenovirus DNA), administration of said composition results in transcription of the DNA into RNA, and to a subsequent translation of the RNA into the encoded SARS-associated virus antigen in a subject.

In embodiments, administration of the pharmaceutical composition comprising component A-2 to a subject elicits neutralizing antibodies against SARS-associated virus and does not elicit disease enhancing antibodies. In particular, administration of a pharmaceutical composition comprising component A-2 encoding SARS-associated virus pre-fusion stabilized spike protein to a subject does not elicit immunopathological effects, like e.g. enhanced disease and/or antibody dependent enhancement (ADE).

In preferred embodiments, administration of component A-2 elicits antigen-specific immune responses comprising T-cell responses and/or B-cell responses against the encoded SARS-associated virus antigens provided by the at least one nucleic acid of component A-2.

Preferably, the component A-2 is suitable for a vaccine, in particular, suitable for a SARS-associated virus, preferably a SARS-CoV-1 vaccine, preferably a combination vaccine of the invention.

In embodiments, the nucleic acid as comprised in component A-2 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component A-2 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component A-3: MERS-CoV

In a particularly preferred embodiment, the at least one Coronavirus of component A, is a MERS-CoV virus (also referred to as component A-3).

Accordingly, in embodiments of the first aspect, the nucleic acid of component A comprises at least one coding sequence encoding at least one antigenic peptide or protein selected or derived from at least one MERS-CoV, or an immunogenic fragment or immunogenic variant thereof.

It has to be understood that generic embodiments and features that have been described in paragraph *"Component A",* may also apply to a nucleic acid encoding a MERS-CoV antigenic peptide or protein.

MERS-CoV belongs to the Coronaviridae, in particular to Orthocoronaviruses, more specifically to the genus Betacoronavirus. MERS-CoV (Middle East respiratory syndrome coronavirus, MERS-Coronavirus, EMC/2012 (HCoV-EMC/2012)) causes a severe respiratory syndrome disease. An exemplary MERS-CoV coronaviruses is identifiable by NCBI Taxonomy ID: 1335626, NCBI Reference: NC_038294.1. Suitable MERS-CoV strains / isolates may be selected from MERS-CoV/MERS-CoV-Jeddah-human-1, MERS-CoV/AI-Hasa_4_2013, MERS-CoV/Riyadh_14_2013, MERS-CoV/Riyadh_14_ 2013, MERS-CoV/Riyadh_14_2013 spike protein, MERS-CoV/England 1 spike protein, MERS-CoV/England 1 spike protein (variant).

In the context of the invention, any protein selected or derived from a MERS-CoV may be used in the context of the invention and may be suitably encoded by the coding sequence or the nucleic acid of component A (in particular, component A-3). It is further in the scope of the underlying invention, that the at least one antigenic peptide or protein may comprise or consist of a synthetically engineered or an artificial MERS-CoV peptide or protein. The term "synthetically engineered" MERS-CoV peptide or protein, or the term "artificial coronavirus peptide or protein" relates to a protein that does not occur in nature. Accordingly, an "artificial coronavirus peptide or protein" or a "synthetically engineered coronavirus peptide or protein" may for example differ in at least one amino acid compared to the natural MERS-CoV peptide or protein, and/or may comprise an additional heterologous peptide or protein element, and/or may be N-terminally or C-terminally extended or truncated.

In preferred embodiments, the nucleic acid of component A comprises at least one coding sequence encoding at least one antigenic peptide or protein selected or derived from a MERS-CoV, or an immunogenic fragment or immunogenic variant thereof, wherein the at least one antigenic peptide or protein comprises at least one peptide or protein that is selected or is derived from a structural protein, an accessory protein, or a replicase protein or an immunogenic fragment or immunogenic variant of any of these.

Suitably, the structural protein is selected or derived from a spike protein (S), an envelope protein (E), a membrane protein (M) or a nucleocapsid protein (N), or an immunogenic fragment or variant of any of these.

In particularly preferred embodiments of the pharmaceutical composition, the encoded at least one antigenic peptide or protein of component A (in particular component A-3) comprises or consists at least one peptide or protein selected or derived from a MERS-CoV spike protein (S, S1, S2, or S1 and S2), or an immunogenic fragment or immunogenic variant of any of these.

Suitable antigenic peptide or protein sequences that are provided by the nucleic acid of component A (in particular, component A-3) are disclosed in **Table 6,** rows 1 to 16, Column A and B. In addition, further information regarding said suitable antigenic peptide or protein sequences selected or derived from MERS-CoV are provided under <223> identifier of the ST.25 sequence listing.

In the following, preferred antigenic peptide or protein sequences selected or derived from MERS-CoV that are provided by the nucleic acid of component A are described in detail.

It has to be noted that variation on amino acid level naturally occurs between spike proteins derived from different MERS-CoV isolates. In the context of the invention, such amino acid variations can be applied to each antigenic peptide or protein derived from a MERS-CoV spike protein as described herein.

In embodiments, a fragment of a MERS-CoV spike protein (S) may be encoded by the nucleic acid of component A, wherein said fragment may be N-terminally truncated, and/or wherein said fragment may be C-terminally truncated. Such "fragment of a spike protein (S)" may additionally comprise amino acid substitutions (as described below) and may additionally comprise at least one heterologous peptide or protein element (as described below).

In embodiments, the encoded at least one antigenic peptide or protein of component A comprises or consists of a MERS-CoV spike protein (S), wherein the spike protein (S) lacks the transmembrane domain (TM). Without wishing to being bound to theory, a MERS-CoV spike protein (S) lacking the transmembrane domain (TM) as defined herein could be suitable for a combination vaccine, as such a protein would be soluble and not anchored in the cell membrane. A soluble protein may therefore be produced (that is translated) in higher concentrations upon administration to a subject, leading to improved immune responses.

Without wishing to being bound to theory, RBD and CND domains may be crucial for immunogenicity of MERS-CoV spike protein (S). Both regions are located at the S1 fragment of the spike protein. Accordingly, it may be suitable in the context of the invention that the antigenic peptide or protein comprises or consists of an S1 fragment of the spike protein or an immunogenic fragment or immunogenic variant thereof.

Suitably, a S1 fragment of MERS-CoV may comprise at least an RBD and/or a CND domain as defined above.

In particularly preferred embodiments, the encoded at least one antigenic peptide or protein of component A (in particular component A-3) comprises or consists of a MERS-CoV spike protein (S), wherein the MERS-CoV spike protein (S) comprises or consists of a spike protein fragment S1, or an immunogenic fragment or immunogenic variant thereof.

In preferred embodiments, the encoded at least one antigenic peptide or protein comprises a MERS-CoV spike protein fragment S1, and lacks at least 70%, 80%, 90%, preferably 100% of spike protein fragment S2. Such embodiments may be beneficial as the MERS-CoV S1 fragment comprises neutralizing epitopes without potential problems of full length protein comprising S1 and S2.

Without wishing to being bound to theory, it may be suitable that the antigenic peptide or protein of component A (in particular component A-3) comprises or consists of MERS-CoV spike protein fragment S1 and (at least a fragment of) MERS-CoV spike protein fragment S2, because the formation of an immunogenic MERS-CoV spike protein may be promoted.

Accordingly, in particularly preferred embodiments, the encoded at least one antigenic peptide or protein of component A (in particular component A-3) comprises or consists of a MERS-CoV spike protein (S), wherein the MERS-CoV spike protein (S) comprises or consists of a MERS-CoV spike protein fragment S1 or an immunogenic fragment or immunogenic variant thereof, and MERS-CoV spike protein fragment S2 or an immunogenic fragment or immunogenic variant thereof.

In particularly preferred embodiments, the encoded at least one antigenic peptide or protein of component A (in particular component A-3) comprises or consists of a full length MERS-CoV spike protein or an immunogenic fragment or immunogenic variant of any of these.

In particularly preferred embodiments, the MERS-CoV spike protein (S) that is provided by the nucleic acid of component A (in particular component A-3) is designed or adapted to stabilize the antigen in pre-fusion conformation. A pre-fusion conformation is particularly advantageous in the context of an efficient MERS-CoV vaccine, as several potential epitopes for neutralizing antibodies may merely be accessible in said pre-fusion protein conformation. Furthermore, remaining of the protein in the pre-fusion conformation is aimed to avoid immunopathological effects, like e.g. enhanced disease and/or antibody dependent enhancement (ADE).

Accordingly, in preferred embodiments, the nucleic acid of component A (in particular component A-3) comprises at least one coding sequence encoding at least one antigenic peptide or protein that is selected or derived from MERS-CoV, wherein the at least one antigenic peptide or protein is selected or derived or from a spike protein (S), wherein the spike protein (S) is a pre-fusion stabilized spike protein (S_stab). Suitably, said pre-fusion stabilized spike protein comprises at least one pre-fusion stabilizing mutation.

Stabilization of the MERS-CoV spike protein may be obtained by substituting at least one amino acids at position V1060 and/or L1061 with amino acids that stabilize the spike protein in a perfusion conformation (amino acid positions according to reference **SEQ ID NO: 14794).**

In embodiments, the pre-fusion stabilizing mutation of MERS-CoV spike protein comprises an amino acid substitution at position V1060, wherein the amino acids V1060 is substituted with one selected from A, I, L, M, F, V, G, or P (amino acid positions according to reference **SEQ ID NO: 14794),** preferably wherein the amino acids V1060 is substituted with P. In embodiments, the pre-fusion stabilizing mutation comprises an amino acid substitution at position L1061, wherein the amino acids L1061 is substituted with one selected from A, I, L, M, F, V, G, or P (amino acid positions according to reference **SEQ ID NO: 14794),** preferably wherein the amino acids L1061 is substituted with P.

Accordingly, in preferred embodiments, the pre-fusion stabilized spike protein (S_stab) of MERS-CoV comprises at least one pre-fusion stabilizing mutation, wherein the at least one pre-fusion stabilizing mutation comprises the following amino acid substitutions: V1060P and L1061P (amino acid positions according to reference **SEQ ID NO: 14794**).

Any MERS-CoV spike protein or fragments or variants thereof can be chosen by the skilled person to introduce such amino acid changes, preferably amino acid substitutions: V1060P and L1061P (amino acid positions according to reference **SEQ ID NO: 14794).**

Any MERS-CoV spike protein or fragments or variants thereof can be chosen by the skilled person to introduce such amino acid changes (e.g. such a double Proline mutation). Examples comprise MERS-CoV/MERS-CoV-Jeddah-human-1 spike protein (1-1353)(V1060P_L1061P), MERS-CoV/AI-Hasa_4_2013 spike protein (1-1353)(V1060P_L1061P), MERS-CoV/Riyadh_14_2013 spike protein (1-1353)(V1060P_L1061P), MERS-CoV/England 1 spike protein (1-1353)(V1060P_L1061P).

In embodiments, the at least one pre-fusion stabilizing mutation of MERS-CoV spike protein comprises a cavity filling mutation.

In some embodiments, the cavity filling substitutions to stabilize the MERS-CoV S ectodomain the prefusion conformation, may be selected from the following amino acid substitutions: N1072F and A1083I; N1072F and L1086F; N1072F and V1087I; N1072F and E1090I; T1076F and A1083I; T1076F and L1086F; T1076F and V1087I; T1076F and EI 0901; T1076I and A1083I; T1076I and L1086F; T1076I and V1087I; T1076I and E10901; A1018V; or A1018I.

In embodiments, the at least one pre-fusion stabilizing mutation of MERS-CoV spike protein comprises a mutated protonation site (R1020Q).

In embodiments, the at least one pre-fusion stabilizing mutation of MERS-CoV spike protein comprises a repacking substitution to stabilize the S ectodomain the prefusion conformation, such as one of E793M and K1102F; E793M, K1102F, and H1138F; D1068M and R1069W; A1083L; A1083L and V1087I; A1083L, V1087, and E1090L; A834L and Q1084M; Q1066M; S454F; R921W; S612F and G1052F; or P476V, T477A, and R1057W.

In embodiments, the at least one pre-fusion stabilizing mutation of the MERS-CoV spike protein comprises an artificial intramolecular disulfide bond. Such an artificial intramolecular disulfide bond can be introduced to further stabilize the membrane distal portion of the MERS-CoV spike protein (including the N-terminal region) in the pre-fusion conformation; that is, in a conformation that specifically binds to one or more pre-fusion specification antibodies, and/or presents a suitable antigenic site that is present on the pre-fusion conformation but not in the post fusion conformation of the MERS-CoV spike protein.

In some embodiments, the disulfide bond substitutions to stabilize the MERS-CoV S ectodomain the prefusion conformation, may be selected from the following amino acid substitutions: T63C and V631C; T63C and Q638C; Q733C and D940C; S676C and D910C; V1087C (which forms a disulfide bond with a cysteine present in the native sequence); A432C and L1058C; or A432C and D1059C to stabilize the S ectodomain the prefusion conformation.

Any MERS-CoV spike protein or fragments or variants thereof can be chosen by the skilled person to introduce at least one cavity filling mutation, at least one mutated protonation site, and/or at least one artificial intramolecular disulfide bond.

According to various preferred embodiments, the nucleic acid of component A (in particular component A-3) encodes at least one antigenic peptide or protein selected or derived from MERS-CoV as defined herein and, additionally, at least one heterologous peptide or protein element, preferably selected or derived from a signal peptide, a linker, a helper epitope, an antigen clustering element, a trimerization element, a transmembrane element, and/or a VLP-forming sequence.

Suitably, the at least one heterologous peptide or protein element may promote or improve secretion of the encoded antigenic peptide or protein of MERS-CoV (e.g. via secretory signal sequences), promote or improve anchoring of the encoded antigenic peptide or protein of MERS-CoV in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component A (in particular component A-3) may additionally encode peptide linker elements, self-cleaving peptides, immunologic adjuvant sequences or dendritic cell targeting sequences.

In preferred embodiments, the nucleic acid of component A (in particular component A-3) encoding at least one antigenic protein selected or derived from MERS-CoV as defined herein, additionally encodes at least one heterologous trimerization element as defined herein, an antigen clustering element as defined herein, or a VLP forming sequence as defined herein.

The antigen clustering elements may be selected from a ferritin element as defined herein, or a lumazine synthase element as defined herein, surface antigen of Hepatitis B virus (HBsAg) as defined herein, or encapsulin as defined herein. In embodiments where the coding sequence of component A (in particular component A-3) additionally encodes heterologous antigen clustering element, it is particularly preferred and suitable to generate a fusion protein comprising an antigen clustering element and an antigenic peptide or protein derived from MERS-CoV. Suitably, said antigenic peptide or protein, preferably the MERS-CoV spike protein, is lacking the C-terminal transmembrane domain (TM) or parts of the TM. Further, it may be suitable to use linker elements as defined for separating the heterologous antigen clustering element from the antigenic peptide or protein.

The trimerization element may be selected from a foldon element as defined herein. In preferred embodiments, the foldon element is a fibritin foldon element as defined herein. In embodiments where the coding sequence of the nucleic acid of component A (in particular component A-3) additionally encodes heterologous trimerization element, it is particularly preferred and suitable to generate a fusion protein comprising a trimerization element and an antigenic peptide or protein derived from MERS-CoV. Suitably, said antigenic peptide or protein, preferably the spike protein derived from MERS-CoV that is lacking the C-terminal transmembrane domain, or parts of the TM. Further, it may be suitable to use linker elements as defined for separating the heterologous antigen clustering element from the antigenic peptide or protein.

The VLP forming sequence may be selected and fused to the MERS-CoV antigen as defined herein. Suitable VLP forming sequences may be selected from elements derived from Hepatitis B virus core antigen as defined herein, HIV-1 Gag protein as defined herein, or Woodchuck hepatitis core antigen element (WhcAg) as defined herein. In embodiments where the coding sequence of the nucleic acid of component A (in particular component A-3) additionally encodes heterologous VLP forming sequence, it is particularly preferred and suitable to generate a fusion protein comprising a VLP forming sequence and an antigenic peptide or protein derived from MERS-CoV. Suitably, said antigenic peptide or protein, preferably the spike protein derived from MERS-CoV that is lacking the C-terminal transmembrane domain (TM), or parts of the TM. Further, it may be suitable to use linker elements as defined herein for separating the heterologous antigen clustering element from the antigenic peptide or protein.

In embodiments, the antigenic peptide or protein comprises a heterologous signal peptide as defined above. A heterologous signal peptide may be used to improve the secretion of the encoded MERS-CoV antigen. In embodiments where the coding sequence of the nucleic acid of component A (in particular component A-3) additionally encodes heterologous secretory signal peptide, it is particularly preferred and suitable to generate a fusion protein comprising a heterologous secretory signal peptide and an antigenic peptide or protein derived from MERS-CoV. Suitably, said antigenic peptide or protein, preferably the spike protein derived from MERS-CoV is lacking the N-terminal endogenous secretory signal peptide.

Preferred antigenic peptide or proteins selected or derived from a MERS-CoV as defined above are provided in **Table 6** (rows 1 to 16). Therein, each row **1 to 16** corresponds to a suitable MERS-CoV constructs. Column A of **Table 6** provides a short description of suitable MERS-CoV antigen constructs. Column B of **Table 6** provides protein (amino acid) SEQ ID NOs of respective MERS-CoV antigen constructs. Column D of **Table 6** provides SEQ ID NO of the corresponding G/C optimized nucleic acid coding sequences (opt1, gc). Column E of **Table 6** provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of **Table 6,** e.g. mRNA sequences comprising the coding sequences of **Table 6** are provided in **Table 7.**

**Table 6: Preferred MERS-CoV constructs (amino acid sequences and nucleic acid coding sequences):**

| **row** | **A** | **B** | **D** | **E** |
|---|---|---|---|---|
| 1 | MERS-CoV/MERS-CoV-Jeddah-human-1 spike protein (1-1353) | 14794 | 14810 | 14826 |
| 2 | MERS-CoV/MERS-CoV-Jeddah-human-1 spike protein (1-1353)(V1060P_L1061P) | 14795 | 14811 | 14827 |
| 3 | MERS-CoV/Al-Hasa_4_2013 spike protein (1-1353) | 14796 | 14812 | 14828 |
| 4 | MERS-CoV/Al-Hasa_4_2013 spike protein (1-1353)(V1060P_L1061P) | 14797 | 14813 | 14829 |
| 5 | MERS-CoV/Riyadh_14_2013 spike protein (1-1353) | 14798 | 14814 | 14830 |
| 6 | MERS-CoV/Riyadh_14_2013 spike protein (1-1353)(V1060P_L1061P) | 14799 | 14815 | 14831 |
| 7 | MERS-CoV/Riyadh_14_2013 spike protein (1-1353)(R1020Q) | 14800 | 14816 | 14832 |
| 8 | MERS-CoV/Riyadh_14_2013 spike protein (1-17_726-1296)(R1020Q) | 14801 | 14817 | 14833 |
| 9 | MERS-CoV/Riyadh_14_2013 spike protein (1-17_726-1296)(R1020Q)_LeucineZipper | 14802 | 14818 | 14834 |
| 10 | MERS-CoV/England 1 spike protein (1-1353) | 14803 | 14819 | 14835 |
| 11 | MERS-CoV/England 1 spike protein (1-1353)(V1060P_L1061P) | 14804 | 14820 | 14836 |
| 12 | MERS-CoV/England 1 spike protein (variant) | 14805 | 14821 | 14837 |
| 13 | MERS-CoV/England 1 spike protein (variant) | 14806 | 14822 | 14838 |
| 14 | MERS-CoV/England 1 spike protein (variant) | 14807 | 14823 | 14839 |
| 15 | MERS-CoV/England 1 spike protein (variant) | 14808 | 14824 | 14840 |
| 16 | MERS-CoV/England 1 spike protein (variant) | 14809 | 14825 | 14841 |

In preferred embodiments, the at least one antigenic peptide or protein selected or derived from MERS-CoV encoded by the at least one nucleic acid of component A (in particular component A-3) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 14794-14809** or an immunogenic fragment or immunogenic variant of any of these. Further information regarding said amino acid sequences is also provided in **Table** 6 (see rows 1 to 16 of Column A and B), and under <223> identifier of the ST.25 sequence listing of respective sequence SEQ ID NOs.

In further embodiments, the at least one antigenic peptide or protein selected or derived from at least one MERS-CoV encoded by the at least one nucleic acid of component A (in particular, component A-3) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-152 or 1448 to 1548 of published PCT application WO2018115527, or an immunogenic fragment or immunogenic variant of any of these. Accordingly, SEQ ID NOs: 1-152 or 1448 to 1548 of WO2018115527 and the corresponding disclosure relating thereto (e.g. information in the respective sequence listing, column 1 or 2 of Tables 1-4 and Table 7) are herewith incorporated by reference.

In further embodiments, the at least one antigenic peptide or protein selected or derived from at least one MERS-CoV encoded by the at least one nucleic acid of component A (in particular, component A-3) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 2-4, 28-29 of published PCT application WO2018081318, or an immunogenic fragment or immunogenic variant of any of these. Accordingly, SEQ ID NOs: 2-4, 28-29 of WO2018081318 and the corresponding disclosure relating thereto are herewith incorporated by reference.

In further embodiments, the at least one antigenic peptide or protein selected or derived from at least one MERS-CoV encoded by the at least one nucleic acid of component A (in particular, component A-3) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 24-28 or 33 of published PCT application WO2017070626, or an immunogenic fragment or immunogenic variant of any of these. Accordingly, SEQ ID NOs: 24-28 or 33 of WO2017070626 and the corresponding disclosure relating thereto are herewith incorporated by reference.

Further suitable antigenic peptide or proteins selected or derived from MERS-CoV can be selected or derived from Table 12 of WO2017070626. Accordingly, the full content of Table 12 of WO2017070626 herewith incorporated by reference.

In preferred embodiments, the at least one antigenic peptide or protein (pre-fusion stabilized spike protein (S_stab)) selected or derived from MERS-CoV encoded by the at least one nucleic acid of component A (in particular component A-3) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of **SEQ ID NOs: 14795, 14797, 14799-14802, 14804** or an immunogenic fragment or immunogenic variant of any of these. Further information regarding said amino acid sequences is also provided in **Table 6** (Column A and B), and under <223> identifier of the ST.25 sequence listing of respective sequence SEQ ID NOs.

According to preferred embodiments, the nucleic acid of component A (in particular component A-3) comprises at least one coding sequence encoding at least one antigenic peptide or protein derived from MERS-CoV as defined above, or fragments and variants thereof. In that context, any coding sequence encoding at least one MERS-CoV antigenic protein as defined herein, or fragments and variants thereof may be understood as suitable coding sequence and may therefore be comprised in the nucleic acid of component A.

In preferred embodiments, the nucleic acid of component A (in particular component A-3) comprise or consist of at least one coding sequence encoding at least one antigenic peptide or protein selected or derived from a MERS-CoV as defined herein, preferably encoding any one of **SEQ ID NOs: 14794-14809;** SEQ ID NOs: 1-152, 1448-1548 of WO2018115527; SEQ ID NOs: 2-4, 28-29 of WO2018081318; SEQ ID NOs: 24-28 or 33 of WO2017070626, or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14794-14809;** SEQ ID NOs: 1-152, 1448-1548 of WO2018115527; SEQ ID NOs: 2-4, 28-29 of WO2018081318; SEQ ID NOs: 24-28 or 33 of WO2017070626, or fragments or variants thereof, may be selected and may accordingly be understood as suitable coding sequence of the invention. Further information regarding said amino acid sequences is also provided in **Table 6, Table 7,** and under <223> identifier of the ST.25 sequence listing of respective sequence SEQ ID NOs.

In preferred embodiments, the nucleic acid of component A (in particular component A-3) comprises a coding sequence that comprises at least one of the nucleic acid sequences encoding a MERS-CoV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences **SEQ ID NOs: 14810-14905** or a fragment or a fragment or variant of any of these sequences. Further information regarding said nucleic acid sequences is also provided in **Table 6, Table 7,** and under <223> identifier of the ST.25 sequence listing of respective sequence SEQ ID NOs.

In further embodiments, the at least one coding sequence of the at least one nucleic acid of component A (in particular, component A-3) comprises or consists at least one nucleic acid sequence encoding a MERS-CoV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences SEQ ID NOs: 153-304 or 1549-1649, 305-1368, 1650-2356, 2365, 2366, 2373-2378 of published PCT application WO2018115527, or an immunogenic fragment or immunogenic variant of any of these. Notably, SEQ ID NOs: 153-304 or 1549-1649, 305-1368, 1650-2356, 2365, 2366, 2373-2378 of WO2018115527 and the corresponding disclosure relating thereto (e.g. information in the respective sequence listing, column 4 or 4 of Tables 1-4 and Table 7) are herewith incorporated by reference.

In further embodiments, the at least one coding sequence of the at least one nucleic acid of component A (in particular, component A-3) comprises or consists at least one nucleic acid sequence encoding a MERS-CoV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences SEQ ID NOs: 20-23, 65-68 of published PCT application WO2017070626, or an immunogenic fragment or immunogenic variant of any of these. Notably, SEQ ID NOs: 20-23, 65-68 of WO2017070626and the corresponding disclosure relating thereto (e.g. information in the respective sequence listing, Tables 1-4 and Table 7) are herewith incorporated by reference.

In preferred embodiments, the at least one coding sequence of the nucleic acid of component A (in particular component A-3) is a codon modified coding sequence as defined herein, wherein the amino acid sequence, that is the MERS-CoV peptide or protein, encoded by the at least one codon modified coding sequence, is preferably not being modified compared to the amino acid sequence encoded by the corresponding wild type or reference coding sequence.

In particularly preferred embodiments, the at least one coding sequence of the nucleic acid component A (in particular component A-3) is a codon modified coding sequence, wherein the codon modified coding sequence is selected a G/C optimized coding sequence, a human codon usage adapted coding sequence, or a G/C modified coding sequence.

Preferred nucleic acid sequences of component A (in particular component A-3), including particularly preferred mRNA sequences, are provided in **Table 7** (column C and D). Therein, each row represents a specific suitable MERS-CoV construct of the invention (compare with **Table 6),** wherein the description of the MERS-CoV construct is indicated in column A of **Table 7** and the SEQ ID NOs of the amino acid sequence of the respective MERS-CoV construct is provided in column B. The corresponding SEQ ID NOs of the coding sequences encoding the respective MERS-CoV constructs are provided in in **Table 6.** Further information is provided under <223> identifier of the respective SEQ ID NOs in the sequence listing.

**Table 7: Nucleic acid, preferably mRNA constructs encoding MERS-CoV antigens**

| **A** | **B** | **C** | **D** |
|---|---|---|---|
| MERS-CoV/MERS-CoV-Jeddah-human-1 spike protein (1-1353) | 14794 | 14842, 14858 | 14874, 14890 |
| MERS-CoV/MERS-CoV-Jeddah-human-1 spike protein (1-1353)(V1060P_L1061P) | 14795 | 14843, 14859 | 14875, 14891 |
| MERS-CoV/Al-Hasa_4_2013 spike protein (1-1353) | 14796 | 14844, 14860 | 14876, 14892 |
| MERS-CoV/Al-Hasa_4_2013 spike protein (1-1353)(V1060P_L1061P) | 14797 | 14845, 14861 | 14877, 14893 |
| MERS-CoV/Riyadh_14_2013 spike protein (1-1353) | 14798 | 14846, 14862 | 14878, 14894 |
| MERS-CoV/Riyadh_14_2013 spike protein (1-1353)(V1060P_L1061P) | 14799 | 14847, 14863 | 14879, 14895 |
| MERS-CoV/Riyadh_14_2013 spike protein (1-1353)(R1020Q) | 14800 | 14848, 14864 | 14880, 14896 |
| MERS-CoV/Riyadh_14_2013 spike protein (1-17_726-1296)(R1020Q) | 14801 | 14849, 14865 | 14881, 14897 |
| MERS-CoV/Riyadh_14_2013 spike protein (1-17_726-1296)(R1020Q)_LeucineZipper | 14802 | 14850, 14866 | 14882, 14898 |
| MERS-CoV/England 1 spike protein (1-1353) | 14803 | 14851, 14867 | 14883, 14899 |
| MERS-CoV/England 1 spike protein (1-1353)(V1060P _L1061P) | 14804 | 14852, 14868 | 14884, 14900 |
| MERS-CoV/England 1 spike protein (variant) | 14805 | 14853, 14869 | 14885, 14901 |
| MERS-CoV/England 1 spike protein (variant) | 14806 | 14854, 14870 | 14886, 14902 |
| MERS-CoV/England 1 spike protein (variant) | 14807 | 14855, 14871 | 14887, 14903 |
| MERS-CoV/England 1 spike protein (variant) | 14808 | 14856, 14872 | 14888, 14904 |
| MERS-CoV/England 1 spike protein (variant) | 14809 | 14857, 14873 | 14889, 14905 |

In preferred embodiments, the nucleic acid of component A (in particular component A-3), preferably the RNA, comprises or consists of a nucleic acid sequence encoding a MERS-CoV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence seceded from **SEQ ID NOs: 14842-14905** or a fragment or variant of any of these sequences. Further information regarding respective nucleic acid sequences is provided under <223> identifier of the respective SEQ ID NO in the sequence listing, and in **Table 7** (see in particular Column C and D). Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In further embodiments, the nucleic acid of component A (in particular component A-3), preferably the RNA, comprises or consists of a nucleic acid sequence encoding a MERS-CoV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NOs: 2373-2378 of WO2018115527 and SEQ ID NOs: 65-68 of WO2017070626, or a fragment or variant of any of these sequences. Further information regarding respective nucleic acid sequences is provided under <223> identifier of the respective SEQ ID NOs in the sequence listing of WO2018115527 or WO2017070626, and in Tables 1-4 and Table 7 of WO2018115527. Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides.

Suitable features and embodiments that apply to nucleic acids of component A-3 are provided in paragraph *"Nucleic acid features and embodiments"* below.

Suitably, the nucleic acids of component A-3 is formulated and/or complexed. Suitable features and embodiments that apply to nucleic acids complexation or formulation of component A are provided in paragraph *"Formulation and Complexation"* below.

In embodiments, the component A (in particular component A-3) comprises a plurality or at least more than one of the nucleic acid species comprising at least one coding sequence encoding at least one antigenic peptide or protein selected or derived from at least one MERS-CoV, or an immunogenic fragment or immunogenic variant thereof, e.g. DNA or RNA species as defined herein. Preferably, component A (in particular component A-3) as defined herein comprises 2, 3, 4, 5, 6, 7, 8, 9, or 10 different nucleic acids each defined as defined herein.

In embodiments, component A (in particular component A-3) may comprise at least 2, 3, 4, 5, 6, 7, 8, 9, 10 or even more different nucleic acid species, e.g. DNA or RNA, as defined herein, each encoding at least one antigenic peptide or protein derived from the same MERS-CoV, or a fragment or variant thereof. Particularly, said (genetically) same MERS-CoV expresses (essentially) the same repertoire of proteins or peptides, wherein all proteins or peptides have (essentially) the same amino acid sequence. Particularly, said (genetically) same MERS-CoV expresses essentially the same proteins, peptides or polyproteins, wherein these protein, peptide or polyproteins preferably do not differ in their amino acid sequence(s).

In embodiments, the component A (in particular component A-3) comprises at least 2, 3, 4, 5, 6, 7, 8, 9, 10 or even more different nucleic acid species, e.g. DNA or RNA, as defined herein, each encoding at least one peptide or protein derived from a genetically distinct MERS-CoV (e.g. a distinct MERS-CoV isolate), or a fragment or variant thereof. The terms "distinct" or "distinct MERS-CoV" as used throughout the present specification have to be understood as the difference between at least two respective MERS-CoV (e.g. a distinct MERS-CoV isolate), wherein the difference is manifested on the genome of the respective distinct MERS-CoV. Particularly, said (genetically) distinct MERS-CoV may express at least one distinct protein, peptide or polyprotein, wherein the at least one distinct protein, peptide or polyprotein differs in at least one amino acid.

In preferred embodiments, the component A-3 comprises at least one nudeic acid encoding at least one antigenic peptide or protein that is selected or derived from at least one MERS-CoV, or an immunogenic fragment or immunogenic variant thereof, wherein said component A-3 is to be, preferably, administered intramuscularly or intradermal.

Preferably, intramuscular or intradermal administration of said component A-3 results in expression of the encoded MERS-CoV antigen construct in a subject. In embodiments where the nucleic acid is an RNA, administration of component A-3 results in translation of the RNA and to a production of the encoded MERS-CoV antigen in a subject. In embodiments where the nucleic acid is a DNA (e.g. plasmid DNA, adenovirus DNA), administration of said composition results in transcription of the DNA into RNA, and to a subsequent translation of the RNA into the encoded MERS-CoV antigen in a subject.

In embodiments, administration of the pharmaceutical composition comprising component A-3 to a subject elicits neutralizing antibodies against MERS-CoV and does not elicit disease enhancing antibodies. In particular, administration of a pharmaceutical composition comprising component A-3 encoding MERS-CoV pre-fusion stabilized spike protein to a subject does not elicit immunopathological effects, like e.g. enhanced disease and/or antibody dependent enhancement (ADE).

In preferred embodiments, administration of component A-3 elicits antigen-specific immune responses comprising T-cell responses and/or B-cell responses against the encoded MERS-CoV antigens provided by the at least one nucleic acid of component A-3.

Preferably, the component A-3 is suitable for a vaccine, in particular, suitable for a MERS-CoV vaccine, preferably a combination vaccine of the invention.

In embodiments, the nudeic acid as comprised in component A-3 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component A-3 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component B:

In various preferred embodiments, the at least one component B of the pharmaceutical composition comprises at least one nucleic acid comprising at least one coding sequence encoding at least one antigenic peptide or protein selected or derived from at least one further virus, or an immunogenic fragment or immunogenic variant thereof.

In the context of the invention, the term "peptide or protein selected or derived from at least one further virus" in the broadest sense relates to peptides or proteins from a further virus, wherein that further virus is not selected for component A.

Accordingly, in embodiments where SARS-CoV-2 is selected as the at least one Coronavirus of component A, the at least one further virus of component B can be any virus excluding SARS-CoV-2.

In preferred embodiments the at least one further virus of component B is selected or derived from at least one different Coronavirus, at least one Influenza virus, at least one Pneumoviridae virus, and/or at least one Paramyxoviridae virus.

In preferred embodiments, the at least one Pneumoviridae virus is selected from at least one Respiratory syncytial virus, and/or at least one Metapneumovirus.

In preferred embodiments, the at least one Paramyxoviridae virus is selected from at least one Parainfluenza virus, and/or at least one Henipavirus.

In preferred embodiments, the at least one Paramyxoviridae virus is selected from at least one Parainfluenza virus, and/or at least one Henipavirus.

According to embodiments, the nucleic acid of component B comprises at least one coding sequence encoding at least one antigenic peptide or protein selected or derived from at least one further virus as defined herein, or fragments and variants thereof. In preferred embodiments, the nucleic acid of component B comprises at least one coding sequence encoding at least one antigenic peptide or protein selected or derived from at least one further virus as defined herein, or fragments and variants thereof, wherein said at least one further virus is selected from at least one different Coronavirus, at least one Influenza virus, at least one Pneumoviridae virus, and/or at least one Paramyxoviridae virus.

In that context, any coding sequence encoding at least one antigenic protein of a further virus as defined herein, or fragments and variants thereof may be understood as suitable coding sequence and may therefore be comprised in the nucleic acid of component B.

Suitable features and embodiments that apply to nucleic acids of component B are provided in paragraph *"Nucleic acid features and embodiments"* below.

Suitably, the nucleic acids of component B is formulated and/or complexed. Suitable features and embodiments that apply to nucleic acids complexation or formulation of component A are provided in paragraph *"Formulation and Complexation"* below.

In embodiments, the component B may comprise a plurality or at least more than one of the nucleic acid species comprising at least one coding sequence encoding at least one antigenic peptide or protein selected or derived from at least one further virus, or an immunogenic fragment or immunogenic variant thereof, e.g. DNA or RNA species as defined herein. Preferably, component B as defined herein may comprise 2, 3, 4, 5, 6, 7, 8, 9, or 10 different nucleic acids each defined as defined herein.

In embodiments, component B may comprise at least 2, 3, 4, 5, 6, 7, 8, 9, 10 or even more different nucleic acid species, e.g. DNA or RNA, as defined herein, e.g. encoding at least one antigenic peptide or protein derived from the same further virus, or a fragment or variant thereof. Particularly, said (genetically) same further virus expresses (essentially) the same repertoire of proteins or peptides, wherein all proteins or peptides have (essentially) the same amino acid sequence. Particularly, said (genetically) same further virus expresses essentially the same proteins, peptides or polyproteins, wherein these protein, peptide or polyproteins preferably do not differ in their amino acid sequence(s).

In embodiments, the component B comprises at least 2, 3, 4, 5, 6, 7, 8, 9, 10 or even more different nucleic acid species, e.g. DNA or RNA, as defined herein, each encoding at least one peptide or protein derived from a genetically distinct further virus (e.g. a distinct Influenza virus, a distinct RSV virus, a distinct PIV), or a fragment or variant thereof. The terms "distinct" or "distinct further virus" as used throughout the present specification have to be understood as the difference between at least two respective further viruses , wherein the difference is manifested on the genome of the respective distinct further virus. Particularly, said (genetically) distinct further viruses may express at least one distinct protein, peptide or polyprotein, wherein the at least one distinct protein, peptide or polyprotein differs in at least one amino acid.

In preferred embodiments, the component B comprises at least one nucleic acid encoding at least one antigenic peptide or protein that is selected or derived from at least one further virus, or an immunogenic fragment or immunogenic variant thereof, wherein said component B is to be, preferably, administered intramuscularly or intradermal.

Preferably, intramuscular or intradermal administration of said component B results in expression of the encoded further virus antigen construct in a subject. In embodiments where the nucleic acid is an RNA, administration of component B results in translation of the RNA and to a production of the encoded further virus antigen in a subject In embodiments where the nucleic acid is a DNA (e.g. plasmid DNA, adenovirus DNA), administration of said composition results in transcription of the DNA into RNA, and to a subsequent translation of the RNA into the encoded further virus antigen in a subject.

In preferred embodiments, administration of component B elicits antigen-specific immune responses comprising T-cell responses and/or B-cell responses against the encoded further virus antigens provided by the at least one nucleic acid of component B.

Preferably, component B is suitable for a vaccine, preferably a combination vaccine or combination vaccine of the invention.

Embodiments relating to specific further viruses in the context of the invention are provided below (Component B-1, Component B-2, Component B-3, Component B-4).

### Component B-1: Different Coronavirus

In preferred embodiments, the at least one further virus of component B is selected from at least one different Coronavirus (also referred to as component B-1), wherein said at least one different Coronavirus is not the Coronavirus of component A.

It has to be understood that generic embodiments and features that have been described in paragraph *"Component B"* may also apply to a nucleic acid encoding a different Coronavirus antigenic peptide or protein.

Features and embodiments provided in the context of component A (including component A-1, component A-2, and component A-3) may likewise apply to component B (in particular component B-1). Accordingly, any nucleic acid, e.g. DNA or RNA provided in the context of component A (including component A-1, component A-2, and component A-3) may likewise be selected for component B (in particular component B-1).

In embodiments where Component A comprises at least one nucleic acid comprising at least one coding sequence encoding at least one antigenic peptide or protein selected or derived from at least one SARS-CoV-2 as defined herein, or an immunogenic fragment or immunogenic variant thereof, the at least one different Coronavirus of component B may be selected from any Coronavirus, preferably any pandemic Coronavirus, wherein the at least one different Coronavirus is not SARS-CoV-2. Accordingly, any nucleic acid provided in the context of component A (including component A-1, component A-2, and component A-3) may likewise be selected for component B (in particular component B-1), wherein the at least one nucleic acid of component B is does not encode a SARS-CoV-2 antigen.

In embodiments where Component A comprises at least one nucleic acid comprising at least one coding sequence encoding at least one antigenic peptide or protein selected or derived from at least one SARS-CoV-1 as defined herein, or an immunogenic fragment or immunogenic variant thereof, the at least one different Coronavirus of component B may be selected from any Coronavirus, preferably any pandemic Coronavirus, wherein the at least one different Coronavirus is not SARS-CoV-1. Accordingly, any nucleic acid provided in the context of component A (including component A-1, component A-2, and component A-3) may likewise be selected for component B (in particular component B-1), wherein the at least one nucleic acid of component B is does not encode a SARS-CoV-1 antigen.

In embodiments where Component A comprises at least one nucleic acid comprising at least one coding sequence encoding at least one antigenic peptide or protein selected or derived from at least one MERS-CoV as defined herein, or an immunogenic fragment or immunogenic variant thereof, the at least one different Coronavirus of component B may be selected from any Coronavirus, preferably any pandemic Coronavirus, wherein the at least one different Coronavirus is not MERS-CoV. Accordingly, any nucleic acid provided in the context of component A (including component A-1, component A-2, and component A-3) may likewise be selected for component B (in particular component B-1), wherein the at least one nucleic acid of component B is does not encode a MERS-CoV antigen.

In preferred embodiments, the at least one antigenic peptide or protein selected or derived from at least one different Coronavirus encoded by the at least one nucleic acid of component B comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 1-111, 274-11663, 13176-13510, 13521-14123, 14794-14809, 14906-14950, 22732-22758, 22917, 22923, 22929-22964, 26938, 26939,** or an immunogenic fragment or immunogenic variant of any of these.

In preferred embodiments, the at least one coding sequence of the at least one nucleic acid of component B comprises or consists at least one nucleic acid sequence encoding at least one different Coronavirus antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences **SEQ ID NOs: 116-132, 134-138, 140-143, 146-147, 148-175, 11664-11813, 11815, 11817-12050, 12052, 12054-13147, 13514, 13515, 13519, 13520, 14124-14177, 14810-14905, 14951-15220, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937,** or a fragment or variant of any of these sequences.

In preferred embodiments, the at least one nucleic acid of component B comprises or consists of a nucleic acid sequence encoding at least one different Coronavirus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 148-175, 12204-13147, 14142-14177, 14842-14905, 15041-15220, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937,** or a fragment or variant of any of these sequences.

In embodiments, component B (in particular component B-1) may comprise a plurality or at least more than one of the nucleic acid species comprising at least one coding sequence encoding at least one antigenic peptide or protein selected or derived from at least one different Coronavirus, or an immunogenic fragment or immunogenic variant thereof, e.g. DNA or RNA species as defined herein. Preferably, component B as defined herein may comprise 2, 3, 4, 5, 6, 7, 8, 9, or 10 different nucleic acids each defined as defined herein.

In preferred embodiments, the component B-1 comprises at least one nucleic acid encoding at least one antigenic peptide or protein that is selected or derived from at least one different Coronavirus, or an immunogenic fragment or immunogenic variant thereof, wherein said component B-1 is to be, preferably, administered intramuscularly or intradermal.

Preferably, intramuscular or intradermal administration of said component B-1 results in expression of the encoded different Coronavirus antigen construct in a subject. In embodiments where the nucleic acid is an RNA, administration of component B-1 results in translation of the RNA and to a production of the encoded different Coronavirus antigen in a subject. In embodiments where the nucleic acid is a DNA (e.g. plasmid DNA, adenovirus DNA), administration of said composition results in transcription of the DNA into RNA, and to a subsequent translation of the RNA into the encoded different Coronavirus antigen in a subject.

In embodiments, administration of the pharmaceutical composition comprising component B-1 to a subject elicits neutralizing antibodies against the different Coronavirus and does not elicit disease enhancing antibodies. In particular, administration of a pharmaceutical composition comprising component B-1 encoding Coronavirus pre-fusion stabilized spike protein to a subject does not elicit immunopathological effects, like e.g. enhanced disease and/or antibody dependent enhancement (ADE).

In preferred embodiments, administration of component B-1 elicits antigen-specific immune responses comprising T-cell responses and/or B-cell responses against the encoded different Coronavirus antigens provided by the at least one nucleic acid of component B-1.

Preferably, the component B-1 is suitable for a vaccine, in particular, suitable for a different Coronavirus vaccine, preferably a combination vaccine of the invention.

In embodiments, the nucleic acid as comprised in component B-1 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-1 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In embodiments, the amount of nucleic acid for each nucleic acid species is essentially equal in mass. In other embodiments, the amount of nucleic acid for each nucleic acid species is selected to be equimolar.

### Component B-2: Influenza virus

In preferred embodiments, the at least one further virus of component B is selected from at least one Influenza virus (also referred to as component B-2). Influenza viruses belong to the Orthomyxoviridae family (NCBI Taxonomy ID: 11308), the Orthomyxoviridae family being sub-divided into Alphainfluenzavirus (the genus that includes Influenza A viruses), Betainflumzavirus (the genus that includes Influenza B viruses), Gammainfluenzavirus (the genus that includes Influenza C viruses), and Deltainfluenzavirus (the genus that Includes influenza D viruses)

It has to be understood that generic embodiments and features that have been described in paragraph *"Component B"* may also apply to a nucleic acid encoding a Influenza virus antigenic peptide or protein.

In the context of the invention, any Influenza virus, irrespective of a specific genotype, species, strain, isolate, or serotype may selected as the "at least one further virus" of component B.

Suitably, the at least one Influenza virus of component B (in particular component B-2) may be selected from at least one Influenza A virus (NCBI Taxonomy ID: 11320), and/or at least one Influenza B virus (NCBI Taxonomy ID: 11520), and/or at least one Influenza C virus (NCBI Taxonomy ID: 11552), and/or at least one Influenza D virus (NCBI Taxonomy ID: 1511084).

Accordingly, in embodiments, the at least one Influenza virus of component B (in particular component B-2) is selected from at least one Influenza A, and/or at least one Influenza B and/or at least one Influenza C. In preferred embodiments, the at least one Influenza virus is selected from at least one Influenza A virus and/or at least one Influenza B virus.

The at least one influenza A virus may be selected from influenza A viruses characterized by a hemagglutinin (HA) selected from the group consisting of H1, H2, H3, H4, H5, H6, H7, H8, H9, H10, H11, H12, H13, H14, H15, H16, H17 and H18. Preferably the influenza A virus is selected from an Influenza virus characterized by a hemagglutinin (HA) selected from the group consisting of H1, H3, H5, H7, H9, or H10.

Furthermore, particularly preferred are influenza A viruses characterized by a neuraminidase (NA) selected from the group consisting of N1, N2, N3, N4, N5, N6, N7, N8, N9, N10, and N11. Most preferably the influenza A virus is selected from an Influenza virus characterized by a neuraminidase (NA) selected from the group consisting of N1, N2, and N8.

In embodiments, the at least one Influenza virus is selected from at least one Influenza A virus selected from at least one of the list comprising H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N7 and H10N8, preferably from at least one of H1N1, H3N2, H5N1, and H5N8.

Suitable influenza virus A and B strains may be selected from FLUAV/H1N1/A/California/7/2009, FLUAV/H1N1/A/Michigan/45/2015, FLUAV/H1N1/A/Netherlands/602/2009, FLUAV/H3N2/A/Hong Kong/4801/2014, FLUBV/H0N0/B/Brisbane/60/2008, FLUBV/H0N0/B/Phuket/3073/2013, FLUAV/H1N1/A/Brisbane/02/2018, FLUAV/H1N1/A/Brisbane/02/2018, FLUAV/H1N1/A/Guangdong-Maonan/SWL1536/2019, FLUAV/H1N1/A/Guangdong-Maonan/SWL 1536/2019, FLUAV/H3N2/A/Singapore/INFIMH-16-0019/2016, FLUAV/H3N2/A/Singapore/INFIMH-16-0019/2016 NYMC-X-307, FLUAV/H3N2/A/Singapore/INFIMH-16-0019/2016-CDC-LV18A, FLUAV/H3N2/A/Kansas/14/2017, FLUAV/H3N2/A/Kansas/14/2017 CBER-22B CDC19A, FLUAV/H3N2/A/Switzerland/8060/2017, FLUAV/H3N2/A/Hong Kong/2671/2019, FLUAV/H3N2/A/Hong Kong/45/2019, FLUAV/H3N2/A/South Australia/34/2019, FLUAV/H7N9/A/chicken/Zhejiang/C483/2013(H7N9), FLUAV/H10N8/A/Jiangxi/IPB13/2013(H10N8), FLUAV/Vietnam/1203/2004 (H5N1)

In embodiments, the at least one antigenic peptide or protein encoded by the at least one nucleic acid of component B (in particular component B-2) comprises or consists at least one peptide or protein selected or derived from at least one Influenza virus hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, and/or polymerase basic protein 2 (PB2), or a fragment or variant thereof or from any synthetically engineered influenza virus peptide or protein.

In a preferred embodiment, the at least one antigenic peptide or protein encoded by the at least one nucleic acid of component B comprises or consists at least one peptide or protein selected or derived from at least one hemagglutinin (HA) or neuraminidase (NA) or an immunogenic fragment or immunogenic variant of any of these.

In particularly preferred embodiments the at least one coding region encodes at least one full-length protein of hemagglutinin (HA), and/or at least one full-length protein of neuraminidase (NA) of an influenza virus or a variant thereof.

According to various embodiments, the nucleic acid of component B (in particular component B-2) encodes at least one antigenic peptide or protein from an Influenza virus as defined herein, and, additionally, at least one heterologous peptide or protein element.

Suitably, the at least one heterologous peptide or protein element may promote or improve secretion of the encoded Influenza antigenic peptide or protein (e.g. via secretory signal sequences), promote or improve anchoring of the encoded antigenic peptide or protein of the invention in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component B may additionally encode peptide linker elements, self-cleaving peptides, immunologic adjuvant sequences or dendritic cell targeting sequences.

Suitable multimerization domains may be selected from the list of amino acid sequences according to SEQ ID NOs: 1116-1167 of WO2017081082, or fragments or variants of these sequences. Suitable transmembrane elements may be selected from the list of amino acid sequences according to SEQ ID NOs: 1228-1343 of WO2017081082, or fragments or variants of these sequences. Suitable VLP forming sequences may be selected from the list of amino acid sequences according to SEQ ID NOs: 1168-1227 of the patent application WO2017081082, or fragments or variants of these sequences. Suitable peptide linkers may be selected from the list of amino acid sequences according to SEQ ID NOs: 1509-1565 of the patent application WO2017081082, or fragments or variants of these sequences. Suitable self-cleaving peptides may be selected from the list of amino acid sequences according to SEQ ID NOs: 1434-1508 of the patent application WO2017081082, or fragments or variants of these sequences. Suitable immunologic adjuvant sequences may be selected from the list of amino acid sequences according to SEQ ID NOs: 1360-1421 of the patent application WO2017081082, or fragments or variants of these sequences. Suitable dendritic cell (DCs) targeting sequences may be selected from the list of amino acid sequences according to SEQ ID NOs: 1344-1359 of the patent application WO2017081082, or fragments or variants of these sequences. Suitable secretory signal peptides may be selected from the list of amino acid sequences according to SEQ ID NOs: 1-1115 and SEQ ID NO: 1728 of published PCT patent application WO2017081082, or fragments or variants of these sequences.

In preferred embodiments, the at least one coding sequence additionally encodes one or more heterologous peptide or protein elements selected from a signal peptide, a linker peptide, a helper epitope, an antigen clustering element, a trimerization or multimerization element, a transmembrane element, or a VLP forming sequence.

Preferred antigenic peptide or proteins selected or derived from an Influenza virus as defined above are provided in **Table 8** (rows 1 to 52). Therein, each row 1 to 52 corresponds to a suitable Influenza constructs. Column A of **Table 8** provides a short description of suitable Influenza antigen constructs. Column B of **Table 8** provides protein (amino acid) SEQ ID NOs of respective Influenza antigen constructs. Column D of **Table 8** provides SEQ ID NO of the corresponding G/C optimized nucleic acid coding sequences (opt1, gc). Column E of **Table 8** provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of **Table 8,** e.g. mRNA sequences comprising the coding sequences of **Table 8** are provided in **Table 9** and **Table 10.**

**Table 8: Preferred Influenza virus constructs (amino acid sequences and nucleic acid coding sequences):**

| **row** | **A** | **B** | **D** | **E** |
|---|---|---|---|---|
| 1 | FLUAV/H1N1/A/Califomia/7/2009 hemagglutinin (HA) | 14178 | 14230 | 14282 |
| 2 | FLUAV/H1N1/A/Michigan/45/2015 hemagglutinin (HA) | 14179 | 14231 | 14283 |
| 3 | FLUA VIH1 N1/A/Netherlands/602/2009 hemagglutinin (HA) | 14180 | 14232 | 14284 |
| 4 | FLUAV/H3N2/A/Hong Kong/4801/2014 hemagglutinin (HA) | 14181 | 14233, 26946 | 14285 |
| 5 | FLUBV/H0N0/B/Brisbane/60/2008 hemagglutinin (HA) | 14182 | 14234 | 14286 |
| 6 | FLUBV/H0N0/B/Phuket/3073/2013 hemagglutinin (HA) | 14183 | 14235 | 14287 |
| 7 | FLUAV/H1N1/A/Brisbane/02/2018 hemagglutinin (HA) | 14184 | 14236 | 14288 |
| 8 | FLUAV/H1N1/A/Brisbane/02/2018 hemagglutinin (HA) | 14185 | 14237 | 14289 |
| 9 | FLUAV/H1N1/A/Guangdong-Maonan/SWL1536/2019 hemagglutinin (HA) | 14186 | 14238 | 14290 |
| 10 | FLUAV/H1N1/A/Guangdong-Maonan/SWL1536/2019 hemagglutinin (HA) | 14187 | 14239 | 14291 |
| 11 | FLUAV/H3N2/A/Singapore/INFIMH-16-0019/2016 hemagglutinin (HA) | 14188 | 14240 | 14292 |
| 12 | FLUAV/H3N2/A/Singapore/INFIMH-16-00198/2016 hemagglutinin (HA) | 14189 | 14241 | 14293 |
| 13 | FLUAV/H3N2/A/Singapore/INFIMH-16-0019/2016 NYMC-X-307 hemagglutinin (HA) | 14190 | 14242 | 14294 |
| 14 | FLUAV/H3N2/A/Singapore/INFIMH-16-0019/2016-CDC-LV18A hemagglutinin (HA) | 14191 | 14243 | 14295 |
| 15 | FLUAV/H3N2/A/Kansas/14/2017 hemagglutinin (HA) | 14192 | 14244 | 14296 |
| 16 | FLUAV/H3N2/A/Kansas/14/2017 hemagglutinin (HA) | 14193 | 14245 | 14297 |
| 17 | FLUAV/H3N2/A/Kansas/14/2017 hemagglutinin (HA) | 14194 | 14246 | 14298 |
| 18 | FLUAV/H3N2/A/Kansas/14/2017 CBER-22B CDC19A hemagglutinin (HA) | 14195 | 14247 | 14299 |
| 19 | FLUAV/H3N2/A/Switzerland/8060/2017 hemagglutinin (HA) | 14196 | 14248 | 14300 |
| 20 | FLUAV/H3N2/A/Switzerland/8060/2017 hemagglutinin (HA) | 14197 | 14249 | 14301 |
| 21 | FLUAV/H3N2/A/Hong Kong/2671/2019 hemagglutinin (HA) | 14198 | 14250 | 14302 |
| 22 | FLUAV/H3N2/A/Hong Kong/2671/2019 hemagglutinin (HA) | 14199 | 14251 | 14303 |
| 23 | FLUAV/H3N2/A/Hong Kong/45/2019 hemagglutinin (HA) | 14200 | 14252 | 14304 |
| 24 | FLUAV/H3N2/A/South Australia/34/2019 hemagglutinin (HA) | 14201 | 14253 | 14305 |
| 25 | FLUAV/H3N2/A/South Australia/34/2019 hemagglutinin (HA) | 14202 | 14254 | 14306 |
| 26 | FLUAV/H7N9/A/chicken/Zhejiang/C483/2013(H7N9) hemagglutinin (HA) | 14203 | 14255 | 14307 |
| 27 | FLUAV/H10N8/A/Jiangxi/IPB13/2013(H10N8) hemagglutinin (HA) | 14204 | 14256 | 14308 |
| 28 | FLUBV/H0N0/B/Colorado/06/2017 hemagglutinin (HA) | 14205 | 14257 | 14309 |
| 29 | FLUBV/H0N0/B/Colorado/06/2017 hemagglutinin (HA) | 14206 | 14258 | 14310 |
| 30 | FLUBV/H0N0/B/Colorado/06/2017 BX-71 hemagglutinin (HA) | 14207 | 14259 | 14311 |
| 31 | FLUBV/H0N0/B/Washington/02/2019 hemagglutinin (HA) | 14208 | 14260 | 14312 |
| 32 | FLUBV/H0N0/B/Washington/02/2019 hemagglutinin (HA) | 14209 | 14261 | 14313 |
| 33 | FLUAV/H1N1/A/California/7/2009 neuraminidase (NA) | 14210 | 14262 | 14314 |
| 34 | FLUAV/H1N1/A/Michigan/45/2015 neuraminidase (NA) | 14211 | 14263 | 14315 |
| 35 | FLUAVIH1 N1/A/Netherlands/602/2009 neuraminidase (NA) | 14212 | 14264 | 14316 |
| 36 | FLUAV/H3N2/A/Hong Kong/4801/2014 neuraminidase (NA) | 14213 | 14265 | 14317 |
| 37 | FLUBV/H0N0/B/Brisbane/60/2008 neuraminidase (NA) | 14214 | 14266 | 14318 |
| 38 | FLUBV/H0N0/B/Phuket/3073/2013 neuraminidase (NA) | 14215 | 14267 | 14319 |
| 39 | FLUAV/H1N1/A/Brisbane/02/2018 neuraminidase (NA) | 14216 | 14268 | 14320 |
| 40 | FLUAV/H1N1/A/Guangdong-Maonan/SWL1536/2019 neuraminidase (NA) | 14217 | 14269 | 14321 |
| 41 | FLUAV/H3N2/A/Singapore/INFIMH-16-0019/2016 neuraminidase (NA) | 14218 | 14270 | 14322 |
| 42 | FLUAV/H3N2/A/Kansas/14/2017 neuraminidase (NA) | 14219 | 14271 | 14323 |
| 43 | FLUAV/H3N2/A/Kansas/14/2017-CDC-LV24A neuraminidase (NA) | 14220 | 14272 | 14324 |
| 44 | FLUAV/H3N2/A/Switzerland/8060/2017 neuraminidase (NA) | 14221 | 14273 | 14325 |
| 45 | FLUAV/H3N2/A/Switzerland/8060/2017(H3N2)-CDC-LV21A neuraminidase (NA) | 14222 | 14274 | 14326 |
| 46 | FLUAV/H3N2/A/Hong Kong/2671/2019 neuraminidase (NA) | 14223 | 14275 | 14327 |
| 47 | FLUAV/H3N2/A/Hong Kong/45/2019 neuraminidase (NA) | 14224 | 14276 | 14328 |
| 48 | FLUAV/H3N2/A/South Australia/34/2019 neuraminidase (NA) | 14225 | 14277 | 14329 |
| 49 | FLUAV/H7N9/A/chicken/Zhejiang/C483/2013(H7N9) neuraminidase (NA) | 14226 | 14278 | 14330 |
| 50 | FLUAV/H10N8/A/Jiangxi/IPB13/2013(H10N8) neuraminidase (NA) | 14227 | 14279 | 14331 |
| 51 | FLUBV/H0N0/B/Colorado/06/2017 neuraminidase (NA) | 14228 | 14280 | 14332 |
| 52 | FLUBV/H0N0/B/Washington/02/2019 neuraminidase (NA) | 14229 | 14281 | 14333 |

In preferred embodiments, the at least one antigenic peptide or protein selected or derived from at least one Influenza virus encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14178-14229,** or an immunogenic fragment or immunogenic variant of any of these. Further information regarding said amino acid sequences is also provided in **Table 8** (see rows 1 to 52 of Column A and B), and under <223> identifier of the ST.25 sequence listing of respective sequence SEQ ID NOs.

In preferred embodiments, the at least one antigenic peptide or protein selected or derived from at least one Influenza A virus (HA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14178-14181, 14184-14204** (HA of Influenza A), or an immunogenic fragment or immunogenic variant of any of these. Further information regarding said amino acid sequences is also provided in **Table 8** (Column A and B), and under <223> identifier of the ST.25 sequence listing of respective sequence SEQ ID NOs.

In preferred embodiments, the at least one antigenic peptide or protein selected or derived from at least one Influenza A virus (NA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one **of SEQ ID NOs: 14210-14213, 14216-14227** (NA of Influenza A), or an immunogenic fragment or immunogenic variant of any of these. Further information regarding said amino acid sequences is also provided in **Table 8** (Column A and B), and under <223> identifier of the ST.25 sequence listing of respective sequence SEQ ID NOs.

In preferred embodiments, the at least one antigenic peptide or protein selected or derived from at least one Influenza B virus (HA und NA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14182-14183, 14205-14209, 14214-14215, 14228-14229** or an immunogenic fragment or immunogenic variant of any of these. Further information regarding said amino acid sequences is also provided in **Table 8** (Column A and B), and under <223> identifier of the ST.25 sequence listing of respective sequence SEQ ID NOs.

In further embodiments, the at least one antigenic peptide or protein selected or derived from at least one Influenza virus encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NO: 1-444, 458, 460, 462-479, or 543-565 of published PCT application WO2018170245, or an immunogenic fragment or immunogenic variant of any of these. Notably, SEQ ID NO: 1-444, 458, 460, 462-479, or 543-565 of WO2018170245 and the corresponding disclosure relating thereto are herewith incorporated by reference.

In embodiments, the at least one antigenic peptide or protein selected or derived from at least one Influenza virus encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-32012, 224269, 224309 of published PCT application WO2018078053, or an immunogenic fragment or immunogenic variant of any of these. Accordingly, SEQ ID NOs: 1-32012, 224269, 224309, 224310 of WO2018078053 and the corresponding disclosure relating thereto are herewith incorporated by reference. In embodiments, the at least one antigenic peptide or protein selected or derived from at least one Influenza A virus (HA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-14031 of published PCT application WO2018078053, or an immunogenic fragment or immunogenic variant of any of these. Accordingly, SEQ ID NOs: 1-14031 of WO2018078053 and the corresponding disclosure relating thereto are herewith incorporated by reference. In embodiments, the at least one antigenic peptide or protein selected or derived from at least one Influenza A virus (NA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 14032-26397, 224309, 224310 of published PCT application WO2018078053, or an immunogenic fragment or immunogenic variant of any of these. Accordingly, SEQ ID NOs: 14032-26397, 224309, 224310 of WO2018078053 and the corresponding disclosure relating thereto are herewith incorporated by reference. In embodiments, the at least one antigenic peptide or protein selected or derived from at least one influenza B virus (HA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 26398-28576 of published PCT application WO2018078053, or an immunogenic fragment or immunogenic variant of any of these. Accordingly, SEQ ID NOs: 26398-28576 of WO2018078053 and the corresponding disclosure relating thereto are herewith incorporated by reference. In embodiments, the at least one antigenic peptide or protein selected or derived from at least one Influenza B virus (NA) encoded by the at least one nucleic acid of component B (in particular, component B-2) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 28577-30504 of published PCT application WO2018078053, or an immunogenic fragment or immunogenic variant of any of these. Accordingly, SEQ ID NOs: 28577-30504 of WO2018078053 and the corresponding disclosure relating thereto are herewith incorporated by reference.

According to preferred embodiments, the nucleic acid of component B (in particular, component B-2) comprises at least one coding sequence encoding at least one antigenic peptide or protein derived from at least one Influenza virus as defined above, or fragments and variants thereof. In that context, any coding sequence encoding at least one Influenza virus antigenic protein as defined herein, or fragments and variants thereof, may be understood as suitable coding sequence and may therefore be comprised in the nucleic acid of component B.

In embodiments, the nucleic acid of component B (in particular component B-2) comprises or consists of at least one coding sequence encoding at least one antigenic peptide or protein selected or derived from an Influenza virus as defined herein, preferably encoding any one of SEQ ID NO: 1-444, 458, 460, 462-479, or 543-565 of WO2018170245; SEQ ID NOs: 1-32012, 224269, 224309 of WO2018078053, or fragment or variants of any of these. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NO: 1-444, 458, 460, 462-479, or 543-565 of WO2018170245, SEQ ID NOs: 1-32012, 224269, 224309 of WO2018078053, or fragment or variants of any of these, may be selected and may accordingly be understood as suitable coding sequence of the invention.

In preferred embodiments, the nucleic acid of component B (in particular component B-2) comprises or consists of at least one coding sequence encoding at least one antigenic peptide or protein selected or derived from an Influenza virus as defined herein, preferably encoding any one of **SEQ ID NOs: 14178-14229** or fragments of variants of any of these. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14178-14229** or fragment or variants of any of these, may be selected and may accordingly be understood as suitable coding sequence of the invention.

In embodiments, the at least one coding sequence of the at least one nucleic acid of component B (in particular, component B-2) comprises or consists at least one nucleic acid sequence encoding a Influenza virus antigen being identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NO: 447-457, 459, 461, 491-503, 505-523, 524-542, 566-569, 570-573 of published PCT application WO2018170245, or an immunogenic fragment or immunogenic variant of any of these. Accordingly, SEQ ID NO: 447-457, 459, 461, 491-503, 505-523, 524-542, 566-569, 570-573 of WO2018170245 and the corresponding disclosure relating thereto are herewith incorporated by reference.

In embodiments, the at least one coding sequence of the at least one nucleic acid of component B (in particular, component B-2) comprises or consists at least one nucleic acid sequence encoding a Influenza virus antigen being identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NOs: 32013-46043, 64025-78055, 224085-224106, 96037-110067, 128049-142079, 160061-174091, 192073-206103, 58410-60588, 90422-92600, 224107-224112, 122434-124612, 154446-156624, 186458-188636, 218470-220648, 46044-58409, 224311, 224312, 78056-90421, 224113, 224313-224317, 110068-122433, 142080-154445, 174092-186457, 206104-218469, 60589-62516, 92601-94528, 124613-126540, 156625-158552, 188637-190564, 220649-222576, 224085-224284 of published PCT application WO2018078053, or an immunogenic fragment or immunogenic variant of any of these. Accordingly, SEQ ID NOs: 32013-46043, 64025-78055, 224085-224106, 96037-110067, 128049-142079, 160061-174091, 192073-206103, 58410-60588, 90422-92600, 224107-224112, 122434-124612, 154446-156624, 186458-188636, 218470-220648, 46044-58409, 224311, 224312, 78056-90421, 224113, 224313-224317, 110068-122433, 142080-154445, 174092-186457, 206104-218469, 60589-62516, 92601-94528, 124613-126540, 156625-158552, 188637-190564, 220649-222576, 224085-224284 of WO2018078053 and the corresponding disclosure relating thereto are herewith incorporated by reference.

In embodiments, the at least one coding sequence of the at least one nucleic acid of component B (in particular, component B-2) comprises or consists at least one nucleic acid sequence encoding a Influenza virus antigen being identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NOs: 26-14079, 14080-16264, 16265-28640, 28641 of published PCT application WO2019092153, or an immunogenic fragment or immunogenic variant of any of these. Accordingly, SEQ ID NO: 26 to 14079, 14080 to 16264, 16265 to 28640, 28641 of WO2019092153 and the corresponding disclosure relating thereto are herewith incorporated by reference.

In preferred embodiments, the at least one coding sequence of the at least one nucleic acid of component B (in particular component B-2) comprises or consists at least one nucleic acid sequence encoding a Influenza virus antigen, the nucleic acid being identical or at least 70%, 80%, 85%, 86%, 87%, 88%, 89%, 90%, 91%, 92%, 93%, 94%, 95%, 96%, 97%, 98%, or 99% identical to any one of the nucleic acid sequence selected from **SEQ ID NOs: 14230-14333, 14334-14541, 26946, 26947-26955,** or a fragment or variant of any of these sequences. Further information regarding said nucleic acid sequences is also provided in **Table 8** (Column C-F), **Table 9,** and under <223> identifier of the ST.25 sequence listing of respective sequence SEQ ID NOs.

In preferred embodiments, the at least one coding sequence of the at least one nucleic acid of component B (in particular component B-2) comprises or consists at least one nucleic acid sequence encoding an Influenza virus A antigen (HA) being identical or at least 70%, 80%, 85%, 86%, 87%, 88%, 89%, 90%, 91%, 92%, 93%, 94%, 95%, 96%, 97%, 98%, or 99% identical to any one of the nucleic acid sequence selected from **SEQ ID NOs: 14230-14233, 14236-14256, 14282-14285, 14288-14308, 14334-14337, 14340-14360, 14386-14389, 14392-14412, 14438-14441, 14444-14464, 14490-14493, 14496-14516, 26949, 26947, 26950, 26948,** or a fragment or variant of any of these sequences. Further information regarding said nucleic acid sequences is also provided in **Table 8** (Column C-F), **Table 9,** and under <223> identifier of the ST.25 sequence listing of respective sequence SEQ ID NOs.

In preferred embodiments, the at least one coding sequence of the at least one nucleic acid of component B (in particular component B-2) comprises or consists at least one nudeic acid sequence encoding a Influenza virus A (NA) antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequence selected from **SEQ ID NOs: 14366-14369, 14372-14383, 14418-14421, 14424-14435, 14470-14473, 14476-14487, 14522-14525, 14528-14539, 26953, 26954,** or a fragment or variant of any of these sequences. . Further information regarding said nucleic acid sequences is also provided in **Table 8** (Column C-F), **Table 9,** and under <223> identifier of the ST.25 sequence listing of respective sequence SEQ ID NOs.

In preferred embodiments, the at least one coding sequence of the at least one nucleic acid of component B (in particular component B-2) comprises or consists at least one nucleic acid sequence encoding a Influenza virus B antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequence selected from **SEQ ID NOs: 14234, 14235, 14257-14261, 14266, 14267, 14280, 14281, 14286, 14287, 14309-14313, 14318, 14319, 14332, 14333, 14338, 14339, 14361-14365, 14370, 14371, 14384, 14385, 14390, 14391, 14413-14417, 14422, 14423, 14436, 14437, 14442, 14443, 14465-14469, 14474, 14475, 14488, 14489, 14494, 14495, 14517-14521, 14526, 14527, 14540, 14541, 26951, 26952, 26955,** or a fragment or variant of any of these sequences. Further information regarding said nucleic acid sequences is also provided in **Table 8** (Column C-F), **Table 9,** and under <223> identifier of the ST.25 sequence listing of respective sequence SEQ ID NOs.

In preferred embodiments, the at least one coding sequence of the nucleic acid of component B (in particular of component B-2) is a codon modified coding sequence as defined herein, wherein the amino acid sequence, that is the Influenza virus peptide or protein, encoded by the at least one codon modified coding sequence is preferably not being modified compared to the amino acid sequence encoded by the corresponding wild type or reference coding sequence. In particularly preferred embodiments, the at least one coding sequence of the nucleic acid component B is a codon modified coding sequence, wherein the codon modified coding sequence is selected a G/C optimized coding sequence, a human codon usage adapted coding sequence, or a G/C modified coding sequence.

Preferred nucleic acid sequences of component B (in particular of component B-2) including particularly preferred mRNA sequences, are provided in **Table 9** (column C and D). Therein, each row represents a specific suitable Influenza virus construct of the invention (compare with **Table 8),** wherein the description of the Influenza virus construct is indicated in column A of **Table 9** and the SEQ ID NOs of the amino acid sequence of the respective Influenza virus construct is provided in column B. The corresponding SEQ ID NOs of the coding sequences encoding the respective Influenza virus constructs are provided in **Table 8.** Further information is provided under <223> identifier of the respective SEQ ID NOs in the sequence listing.

**Table 9: Nucleic acid, preferably mRNA constructs encoding Influenza virus antigens**

| **A** | **B** | **C** | **D** |
|---|---|---|---|
| FLUAVH1N1/A/Califomnia/7/2009 hemagglutinin (HA) | 14178 | 14334, 14386, 26949 | 14438, 14490 |
| FLUAV/H1N1/A/Michigan/45/2015 hemagglutinin (HA) | 14179 | 14335, 14387 | 14439, 14491 |
| FLUAV/H1N1/A/Netherlands/602/2009 hemagglutinin (HA) | 14180 | 14336, 14388 | 14440, 14492 |
| FLUAV/H3N2/A/Hong Kong/4801/2014 hemagglutinin (HA) | 14181 | 14337, 14389, 26947, 26950 | 14441, 14493, 26948 |
| FLUBV/H0N0/B/Brisbane/60/2008 hemagglutinin (HA) | 14182 | 14338, 14390, 26951 | 14442, 14494 |
| FLUBV/H0N0/B/Phuket/3073/2013 hemagglutinin (HA) | 14183 | 14339, 14391, 26952 | 14443, 14495 |
| FLUAV/H1N1/A/Brisbane/02/2018 hemagglutinin (HA) | 14184 | 14340, 14392 | 14444, 14496 |
| FLUAV/H1N1/A/Brisbane/02/2018 hemagglutinin (HA) | 14185 | 14341, 14393 | 14445, 14497 |
| FLUAV/H1N1/A/Guangdong-Maonan/SWL1536/2019 hemagglutinin (HA) | 14186 | 14342, 14394 | 14446, 14498 |
| FLUAV/H1N1/A/Guangdong-Maonan/SWL1536/2019 hemagglutinin (HA) | 14187 | 14343, 14395 | 14447, 14499 |
| FLUAV/H3N2/A/Singapore/INFIMH-16-0019/2016 hemagglutinin (HA) | 14188 | 14344, 14396 | 14448, 14500 |
| FLUAV/H3N2/A/Singapore/INFIMH-16-0019/2016 hemagglutinin (HA) | 14189 | 14345, 14397 | 14449, 14501 |
| FLUAV/H3N2/A/Singapore/lNFIMH-16-0019/2016 NYMC-X-307 hemagglutinin (HA) | 14190 | 14346, 14398 | 14450, 14502 |
| FLUAV/H3N2/A/Singapore/INFIMH-16-0019/2016-CDC-LV18A hemagglutinin (HA) | 14191 | 14347, 14399 | 14451, 14503 |
| FLUAV/H3N2/A/Kansas/14/2017 hemagglutinin (HA) | 14192 | 14348, 14400 | 14452, 14504 |
| FLUAV/H3N2/A/Kansas/14/2017 hemagglutinin (HA) | 14193 | 14349, 14401 | 14453, 14505 |
| FLUAV/MH3N2/A/Kansas/14/2017 hemagglutinin (HA) | 14194 | 14350, 14402 | 14454, 14506 |
| FLUAV/H3N2/A/Kansas/14/2017 CBER-22B CDC19A hemagglutinin (HA) | 14195 | 14351, 14403 | 14455, 14507 |
| FLUAV/H3N2/A/Switzerland/8060/2017 hemagglutinin (HA) | 14196 | 14352, 14404 | 14456, 14508 |
| FLUAV/H3N2/A/Switzerland/8060/2017 hemagglutinin (HA) | 14197 | 14353, 14405 | 14457, 14509 |
| FLUAV/H3N2/A/Hong Kong/2671/2019 hemagglutinin (HA) | 14198 | 14354, 14406 | 14458, 14510 |
| FLUAV/H3N2/A/Hong Kong/2671/2019 hemagglutinin (HA) | 14199 | 14355, 14407 | 14459, 14511 |
| FLUAV/H3N2/A/Hong Kong/45/2019 hemagglutinin (HA) | 14200 | 14356, 14408 | 14460, 14512 |
| FLUAV/H3N2/A/South Australia/34/2019 hemagglutinin (HA) | 14201 | 14357, 14409 | 14461, 14513 |
| FLUAV/H3N2/A/South Australia/34/2019 hemagglutinin (HA) | 14202 | 14358, 14410 | 14462, 14514 |
| FLUAV/H7N9/A/chicken/Zhejiang/C483/2013(H7N9) hemagglutinin (HA) | 14203 | 14359, 14411 | 14463, 14515 |
| FLUAV/H10N8/A/Jiangxi/IPB13/2013(H10N8) hemagglutinin (HA) | 14204 | 14360, 14412 | 14464, 14516 |
| FLUBV/H0N0/B/Colorado/06/2017 hemagglutinin (HA) | 14205 | 14361, 14413 | 14465, 14517 |
| FLUBV/H0N0/B/Colorado/06/2017 hemagglutinin (HA) | 14206 | 14362, 14414 | 14466, 14518 |
| FLUBV/H0N0/B/Colorado/06/2017 BX-71 hemagglutinin (HA) | 14207 | 14363, 14415 | 14467, 14519 |
| FLUBV/H0N0/B/Washington/02/2019 hemagglutinin (HA) | 14208 | 14364, 14416 | 14468, 14520 |
| FLUBV/H0N0/B/Washington/02/2019 hemagglutinin (HA) | 14209 | 14365, 14417 | 14469, 14521 |
| FLUAV/H1N1/A/California/7/2009 neuraminidase (NA) | 14210 | 14366, 14418, 26953 | 14470, 14522 |
| FLUAV/H1N1/A/Michigan/45/2015 neuraminidase (NA) | 14211 | 14367, 14419 | 14471, 14523 |
| FLUAV/H1N1/A/Netherlands/602/2009 neuraminidase (NA) | 14212 | 14368, 14420 | 14472, 14524 |
| FLUAV/H3N2/A/Hong Kong/4801/2014 neuraminidase (NA) | 14213 | 14369, 14421, 26954 | 14473, 14525 |
| FLUBV/H0N0/B/Brisbane/60/2008 neuraminidase (NA) | 14214 | 14370, 14422, 26955 | 14474, 14526 |
| FLUBV/H0N0/B/Phuket/3073/2013 neuraminidase (NA) | 14215 | 14371, 14423 | 14475, 14527 |
| FLUAV/H1N1/A/Brisbane/02/2018 neuraminidase (NA) | 14216 | 14372, 14424 | 14476, 14528 |
| FLUA VIH1 N1/A/Guangdong-Maonan/SWL1536/2019 neuraminidase (NA) | 14217 | 14373, 14425 | 14477, 14529 |
| FLUAV/H3N2/A/Singapore/INFIMH-16-0019/2016 neuraminidase (NA) | 14218 | 14374, 14426 | 14478, 14530 |
| FLUAV/H3N2/A/Kansas/14/2017 neuraminidase (NA) | 14219 | 14375, 14427 | 14479, 14531 |
| FLUAV/H3N2/A/Kansas/14/2017-CDC-LV24A neuraminidase (NA) | 14220 | 14376, 14428 | 14480, 14532 |
| FLUAV/H3N2/A/Switzedand/8060/2017 neuraminidase (NA) | 14221 | 14377, 14429 | 14481, 14533 |
| FLUAV/H3N2/A/Switzerland/8060/2017(H3N2)-CDC-LV21A neuraminidase (NA) | 14222 | 14378, 14430 | 14482, 14534 |
| FLUAV/H3N2/A/Hong Kong/2671/2019 neuraminidase (NA) | 14223 | 14379, 14431 | 14483, 14535 |
| FLUAV/H3N2/A/Hong Kong/45/2019 neuraminidase (NA) | 14224 | 14380, 14432 | 14484, 14536 |
| FLUAV/H3N2/A/South Australia/34/2019 neuraminidase (NA) | 14225 | 14381, 14433 | 14485, 14537 |
| FLUAV/H7N9/A/chicken/Zhejiang/C483/2013(H7N9) neuraminidase (NA) | 14226 | 14382, 14434 | 14486, 14538 |
| FLUAV/H10N8/A/Jiangxi/IPB13/2013(H10N8) neuraminidase (NA) | 14227 | 14383, 14435 | 14487, 14539 |
| FLUBV/H0N0/B/Colorado/06/2017 neuraminidase (NA) | 14228 | 14384, 14436 | 14488, 14540 |
| FLUBV/H0N0/B/Washington/02/2019 neuraminidase (NA) | 14229 | 14385, 14437 | 14489, 14541 |

In embodiments, the at least one coding sequence of the at least one nucleic acid of component B (in particular, component B-2) comprises or consists at least one nucleic acid sequence, preferably mRNA sequence, encoding a Influenza virus antigen being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 224085-224284 of published PCT application WO2018078053, or an immunogenic fragment or immunogenic variant of any of these. Optionally, wherein said nucleic acid sequences comprise a cap1 structure as defined herein, and/or wherein at least one, preferably all uracil nucleotides in said RNA sequences are replaced by pseudouridine (ψ) nucleotides and/or N1-methylpseudouridine (m1ψ) nucleotides.

In preferred embodiments, the nucleic acid of component B (in particular component B-2), preferably the RNA, comprises or consists of a nucleic acid sequence encoding an Influenza antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14334-14541, 26946-26955,** or a fragment or variant of any of these sequences. Optionally, wherein said nucleic acid sequences comprise a cap1 structure as defined herein, and/or wherein at least one, preferably all uracil nucleotides in said RNA sequences are replaced by pseudouridine (ψ) nucleotides and/or N1-methylpseudouridine (m1ψ) nucleotides. Further information regarding respective nucleic acid sequences is provided under <223> identifier of the respective SEQ ID NO in the sequence listing, and in **Table 9** (see in particular Column C and D).

In embodiments, the component B (in particular component B-2) comprises a plurality or at least more than one of the nucleic acid species comprising at least one coding sequence encoding at least one antigenic peptide or protein selected or derived from at least one Influenza virus, or an immunogenic fragment or immunogenic variant thereof, e.g. DNA or RNA species as defined herein. Preferably, component B (in particular component B-2) as defined herein comprises 2, 3, 4, 5, 6, 7, 8, 9, or 10 different nucleic acids each as defined herein.

In embodiments, component B (in particular component B-2) may comprise at least 2, 3, 4, 5, 6, 7, 8, 9, 10 or even more different nucleic acid species, e.g. DNA or RNA, as defined herein, each encoding at least one antigenic peptide or protein derived from the same Influenza virus, or a fragment or variant thereof. Particularly, said (genetically) same Influenza virus expresses (essentially) the same repertoire of proteins or peptides, wherein all proteins or peptides have (essentially) the same amino acid sequence. Particularly, said (genetically) same Influenza virus expresses essentially the same proteins, peptides or polyproteins, wherein these protein, peptide or polyproteins preferably do not differ in their amino acid sequence(s).

In embodiments, the component B (in particular component B-2) comprises at least 2, 3, 4, 5, 6, 7, 8, 9, 10 or even more different nucleic acid species, e.g. DNA or RNA, as defined herein, each encoding at least one peptide or protein derived from a genetically distinct Influenza virus (e.g. a distinct Influenza virus isolate/strain), or a fragment or variant thereof. The terms "distinct" or "distinct Influenza virus" as used throughout the present specification have to be understood as the difference between at least two respective Influenza virus (e.g. a distinct Influenza virus isolate), wherein the difference is manifested on the genome of the respective distinct Influenza virus. Particularly, said (genetically) distinct Influenza virus may express at least one distinct protein, peptide or polyprotein, wherein the at least one distinct protein, peptide or polyprotein differs in at least one amino acid.

In preferred embodiments, component B (in particular component B-2) comprises a plurality of nucleic acid sequences encoding an Influenza virus antigen, preferably 2, 3, 4, 5, 6, 7, 6, 9, 10, 11, 12, 13, 14, 15, 16, 17, 18, 19, 20, 25, 30, 35, 40, 45, 50, 60, 70, 80, or 100 of nucleic acid sequences encoding an Influenza virus antigen, *preferably* wherein each of the plurality of nucleic acid sequences is selected from nucleic acid sequences encoding Influenza antigens as defined herein.

In preferred embodiments, the plurality of nucleic acid sequences of component B (in particular component B-2) encodes at least one, preferably 2, 3, 4, 5, 6 antigenic peptide or protein selected or derived from HA as defined herein and at least one, preferably 2, 3, 4, 5, 6 antigenic peptide or protein selected or derived from NA as defined herein of at least one Influenza A virus as defined herein.

In preferred embodiments, the plurality of nucleic acid sequences of component B (in particular component B-2) encodes at least one, preferably 2, 3, 4, 5, 6 antigenic peptide or protein selected or derived from HA as defined herein and at least one, preferably 2, 3, 4, 5, 6 antigenic peptide or protein selected or derived from NA as defined herein of at least one Influenza B virus.

In this context it is particularly preferred that component B (in particular component B-2) of the pharmaceutical composition comprises at least one nucleic acid, in particular RNA sequence encoding at least one antigenic peptide or protein derived from a protein of influenza A virus H1, preferably hemagglutinin (HA) and/or neuraminidase (NA), at least one nucleic acid, in particular RNA sequence encoding at least one antigenic peptide or protein derived from a protein of influenza A virus H3, preferably hemagglutinin (HA) and/or neuraminidase (NA), at least one nucleic acid, in particular RNA sequence encoding at least one antigenic peptide or protein derived from a protein of influenza A virus H5, preferably hemagglutinin (HA) and/or neuraminidase (NA), and optionally at least one nucleic acid, in particular RNA sequence encoding at least one antigenic peptide or protein derived from a protein of influenza A virus H7, preferably hemagglutinin (HA) and/or neuraminidase (NA), and/or optionally at least one nucleic acid, in particular RNA sequence encoding at least one antigenic peptide or protein derived from a protein of influenza A virus H9, preferably hemagglutinin (HA) and/or neuraminidase (NA).

Preferably, component B (in particular component B-2) of the pharmaceutical composition comprises at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of influenza A virus H1, at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of influenza A virus H3, at least one nucleic acid, in particular RNA sequence encoding at least one antigenic peptide or protein derived from hemagglutinin (HA) and/or at least one nudeic acid, in particular RNA sequence encoding at least one antigenic peptide or protein derived from neuraminidase (NA) of influenza A virus H5, and optionally at least one nucleic acid, in particular RNA sequence encoding at least one antigenic peptide or protein derived from preferably hemagglutinin (HA) and/or at least one nucleic acid, in particular RNA sequence encoding at least one antigenic peptide or protein derived from neuraminidase (NA) of influenza A virus H7, and/or optionally at least one nucleic acid, in particular RNA sequence encoding at least one antigenic peptide or protein derived from, preferably hemagglutinin (HA) and/or at least one nucleic acid, in particular RNA sequence encoding at least one antigenic peptide or protein derived from neuraminidase (NA) of influenza A virus H9.

In a specific embodiment, component B (in particular component B-2) of the pharmaceutical composition comprises at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of influenza A virus H1N1, at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of influenza A virus H3N2, at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of influenza A virus H5N1.

Additionally, component B (in particular component B-2) of the pharmaceutical composition preferably further comprises at least one nucleic acid, in particular RNA sequence encoding at least one antigenic peptide or protein derived from hemagglutinin (HA) and/or at least one nucleic acid, in particular RNA sequence encoding at least one antigenic peptide or protein derived from neuraminidase (NA) of at least one influenza B virus.

Preferably in this context, the nucleic acid sequence(s) of component B comprises or consists of the following RNA sequences of **Table 10** ("preferred RNA sequences"):

**Table 10: preferred Influenza virus RNA sequences**

| **Strain / Organism** | **HA** | **NA** |
|---|---|---|
| FLUAV/H1N1/A/Califomia/7/2009 | 14334, 14386, 14438, 14490, 26949 | 14366, 14418, 14470, 14522, 26953 |
| FLUAV/H1N1/A/Michigan/45/2015 | 14335, 14387, 14439, 14491 | 14367, 14419, 14471, 14523 |
| FLUAV/H1N1/A/Netherlands/602/2009 | 14336, 14388, 14440, 14492 | 14368, 14420, 14472, 14524 |
| FLUAV/H3N2/A/Hong Kong/4801/2014 | 14337, 14389, 14441, 14493, 26947, 26950, 26948 | 14369, 14421, 14473, 14525, 26954 |
| FLUBV/H0N0/B/Brisbane/60/2008 | 14338, 14390, 14442, 14494, 26951 | 14370, 14422, 14474, 14526, 26955 |
| FLUBV/H0N0/B/Phuket/3073/2013 | 14339, 14391, 14443, 14495, 26952 | 14371, 14423, 14475, 14527 |

In preferred embodiments, component B (in particular component B-2) of the pharmaceutical composition is a tetravalent influenza cocktail, comprising nucleic acid, preferably RNA sequences as defined herein. Particularly preferred in this context is the combination of nucleic acids, preferably RNAs encoding the following protein sequences (e.g. suitable for a tetravalent influenza cocktail):
- HA protein of FLUAV/H3N2/A/Hong Kong/4801/2014
- HA protein of FLUAV/H1N1/A/CaliforniaR/2009
- HA protein of FLUBV/H0N0/B/Phuket/3073/2013
- HA protein of FLUBV/H0N0/B/Brisbane/60/2008

Suitable RNA sequences for said tetravalent influenza cocktail can be selected from **Table 10.**

In preferred embodiments, component B (in particular component B-2) of the pharmaceutical composition is a trivalent influenza cocktail, comprising nucleic acid, preferably RNA sequences as defined herein. Particularly preferred in this context is the combination of nucleic acids, preferably RNAs encoding the following protein sequences (e.g. suitable for a trivalent influenza cocktail):
- NA protein of FLUAV/H3N2/A/Hong Kong/4801/2014
- NA protein of FLUAV/H1N1/A/California/7/2009
- NA protein of FLUBV/H0N0/B/Brisbane/60/2008

Suitable RNA sequences for said trivalent influenza cocktail can be selected from **Table 10.**

In more preferred embodiments, component B (in particular component B-2) of the pharmaceutical composition is a tetravalent influenza cocktail, comprising nucleic acid, preferably RNA sequences as defined herein. Particularly preferred in this context is the combination of nucleic acids, preferably RNAs encoding the following protein sequences (e.g. suitable for a tetravalent influenza cocktail):
- HA protein of FLUAV/H1N1/A/Netherlands/602/2009
- HA protein of FLUAV/H3N2/A/Hong Kong/4801/2014
- HA protein of FLUBV/H0N0/B/Brisbane/60/2008
- HA protein of influenza A/Vietnam/1194/2004 (H5N1)

Suitable RNA sequences for said tetravalent influenza cocktail can be selected from **Table 10.**

In an also particularly preferred embodiment, component B (in particular component B-2) of the pharmaceutical composition is a septavalent (or septivalent) influenza cocktail, comprising nucleic acid, preferably RNA sequences as defined herein. Particularly preferred in this context is the combination of nudeic acids, preferably RNAs encoding the following protein sequences (e.g. suitable for a septavalent influenza cocktail):
- HA protein of FLUAV/H3N2/A/Hong Kong/4801/2014
- HA protein of FLUAV/H1N1/A/Califomia/7/2009
- HA protein of FLUBV/H0N0/B/Phuket/3073/2013
- HA protein of FLUBV/H0N0/B/Brisbane/60/2008
- NA protein of FLUAV/H3N2/A/Hong Kong/4801/2014
- NA protein of FLUAV/H1N1/A/Califomia/7/2009
- NA protein of FLUBV/H0N0/B/Brisbane/60/2008

Suitable RNA sequences for said septavalent influenza cocktail can be selected from **Table 10.**

Accordingly, in embodiments, component B (in particular B-2) comprises a plurality of nucleic acid sequences encoding an Influenza virus antigen, wherein the influenza virus is selected from the list comprising FLUAV/H1N1/A/California/7/2009, FLUAV/H1N1/A/Michigan/45/2015, FLUAV/H1N1/A/Netherlands/602/2009, FLUAV/H3N2/A/Hong Kong/4801/2014, FLUBV/H0N0/B/Brisbane/60/2008, FLUBV/H0N0/B/Phuket/3073/2013.

In preferred embodiments, component B (in particular B-2) comprises a plurality of nucleic acid sequences, preferably seven nucleic acid sequences, encoding a plurality of Influenza virus antigens, preferably seven Influenza virus antigens, wherein the influenza virus antigens are selected from
- HA protein of FLUAV/H3N2/A/Hong Kong/4801/2014,
- HA protein of FLUAV/H1N1/A/California/7/2009,
- HA protein of FLUBV/H0N0/B/Phuket/3073/2013,
- HA protein of FLUBV/H0N0/B/Brisbane/60/2008,
- NA protein of FLUAV/H3N2/A/Hong Kong/4801/2014,
- NA protein of FLUAV/H1N1/A/California/7/2009, and
- NA protein of FLUBV/H0N0/B/Brisbane/60/2008,

Preferably, wherein nucleic acid sequences, preferably RNA sequences, are selected from **Table 10.**

In preferred embodiments, the component B-2 comprises at least one nucleic acid encoding at least one antigenic peptide or protein that is selected or derived from at least one Influenza virus, or an immunogenic fragment or immunogenic variant thereof, wherein said component B-2 is to be, preferably, administered intramuscularly or intradermal.

Preferably, intramuscular or intradermal administration of said component B-2 results in expression of the encoded Influenza virus antigen construct in a subject. In embodiments where the nucleic acid is an RNA, administration of component B-2 results in translation of the RNA and to a production of the encoded Influenza virus antigen in a subject. In embodiments where the nucleic acid is a DNA (e.g. plasmid DNA, adenovirus DNA), administration of said composition results in transcription of the DNA into RNA, and to a subsequent translation of the RNA into the encoded Influenza virus antigen in a subject.

In preferred embodiments, administration of component B-2 elicits antigen-specific immune responses comprising T-cell responses and/or B-cell responses against the encoded Influenza virus antigens provided by the at least one nucleic acid of component B-2.

Preferably, the component B-2 is suitable for a vaccine, in particular, suitable for a Influenza virus vaccine, preferably a combination vaccine of the invention.

In embodiments, the nucleic acid as comprised in component B-2 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-2 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component B-3: Pneumoviridae virus

In preferred embodiments, the at least one further virus of component B is selected from at least one virus of the Pneumoviridae virus family.

It has to be understood that generic embodiments and features that have been described in paragraph *"Component B"* may also apply to a nucleic acid encoding a Pneumoviridae antigenic peptide or protein,

Pneumoviridae (NCBI Taxonomy ID: 11244) is a virus family in the order Mononegavirales. It was created in 2015 by elevating the now dissolved paramyxoviral subfamily Pneumoviridae. There are currently five species in the Pneumoviridae family, divided between 2 genera (Metapneumovirus and Orthopneumovirus). Orthopneumovirus (NCBI Taxonomy ID: 1868215) is a genus that includes Human respiratory syncytial viruses RSV (e.g. RSV A, RSV B). Metapneumovirus (NCBI Taxonomy ID: 162387) is a genus that includes Human metapneumovirus (hMPV).

The genome of Pneumoviridae viruses is composed of negative-sense single-stranded RNA that is non-segmented. It is about 15kb in size, and typically encodes 11 proteins. A unique feature of the genome is the M2 gene, which encodes proteins M2-1 and M2-2. Viruses in this family are often associated with respiratory infections, and are transmitted through respiratory secretions
In embodiments of the invention, any protein selected or derived from a Pneumoviridae may be used in the context of the invention and may be encoded by the coding sequence or the nucleic acid of component B (in particular, component B-4).

Suitable in the context of the invention are pathogenic Pneumoviridae viruses, including Respiratory syncytial virus, and/or at least one Metapneumovirus.

### Component B-3a: Respiratory syncytial virus (RSV):

In preferred embodiments, the at least one further virus of component B is selected from at least one Respiratory syncytial virus (RSV) of the Pneumoviridae virus family (also herein referred to as component B-3a).

As used herein, the term "Respiratory syncytial virus" or the corresponding abbreviation "RSV" is not limited to a particular virus strain, variant, serotype, or isolate, etc. comprising any Respiratory syncytial virus of any origin.

Suitable RSV viruses in the context of the invention may be selected from List 1 ("RSV virus strains") of published PCT patent application WO2019202035. Accordingly, List 1 of WO2019202035, and the disclosure relating thereto, is herewith incorporated by reference.

In preferred embodiments of the invention, the at least one antigenic peptide or protein is derived from a Respiratory syncytial virus isolate RSV Memphis-37 (strain Memphis-37) (NCBI Taxonomy ID: 12814) and/or a Human respiratory syncytial virus A2, Human respiratory syncytial virus (strain A2) (NCBI Taxonomy ID: 11259).

In embodiments, the at least one antigenic peptide or protein encoded by the at least one nucleic acid of component B (in particular component B-3) comprises or consists at least one peptide or protein selected or derived from at least one RSV fusion protein F, RSV matrix protein M, RSV nucleoprotein N, RSV M2-1 protein, and/or RSV phosphoprotein P, or an immunogenic fragment or immunogenic variant of any of these.

In preferred embodiments, the at least one antigenic peptide or protein encoded by the at least one nucleic acid of component B (in particular component B-3) comprises or consists at least one peptide or protein selected or derived from at least one RSV fusion protein F, or an immunogenic fragment or immunogenic variant thereof.

RSV F protein is initially expressed (after infection of a host cell) as a single polypeptide precursor, designated full-length fusion protein F (herein referred to as "F0"). F0 forms a trimer in the endoplasmic reticulum and is processed by a cellular/host furin-like protease at two conserved sites, generating, F1, F2, and Pep27 polypeptides. The Pep27 polypeptide is excised and does not form part of the mature F protein. The F2 polypeptide originates from the N-terminal portion of the F0 precursor and links to the F1 polypeptide via two disulfide bonds. The F1 polypeptide originates from the C-terminal portion of the F0 precursor and anchors the mature F protein in the membrane via a transmembrane domain, which is linked to a cytoplasmic tail. Three F2-F1 heterodimer units ("protomers") assemble to form a mature F protein. Initially, the mature F protein is in a metastable form (herein referred to as "pre-fusion conformation"). Upon triggering, it undergoes a dramatic and irreversible conformational change (herein referred to as "postfusion conformation") that fuses the viral and target-cell membranes.

In the context of the invention, a suitable RSV F protein may be selected or derived from RSV F proteins provided in List 2 of published PCT patent application WO2019202035. Accordingly, List 2 of WO2019202035, and the disclosure relating thereto, is herewith incorporated by reference.

In preferred embodiments, the at least one antigenic peptide or protein selected or derived from RSV F protein is a full-length F protein (F0) or an F protein with deleted C-terminus (F-del), or an immunogenic fragment or immunogenic variant thereof.

In the context of the present invention, "RSV F protein", "RSV fusion protein (F)", "RSV F", or "F" may be understood in its broadest sense and refers to F0 (F polypeptide precursor), F1, F2 and Pep27 polypeptides, F2-F1 heterodimer, or the mature F protein (comprising three F2-F1 heterodimers), or fragments and variants of any of these. Accordingly, the term "peptide or protein derived from a RSV fusion (F) protein" refers to a peptide, protein, fragment or variant derived from e.g. F0 (F protein polypeptide precursor), F1, F2 and Pep27 polypeptides, F2-F1 heterodimer, or the mature F protein. Additionally, the term "peptide or protein derived from a RSV fusion (F) protein" refers to peptide, protein, fragment or variant derived from "RSV F protein" or "RSV fusion protein (F)" as defined above which may be genetically engineered to e.g. to lack certain protein elements (e.g. the cytoplasmic tail, the furin cleavage site, Pep27) or e.g. comprise additional elements (e.g., linker elements, heterologous signal peptides etc.).

It has to be noted that where reference is made to amino acid (aa) residues and their position in an RSV F protein, any numbering used herein - unless stated otherwise - relates to the position of the respective aa residue in a corresponding F0 precursor protein of HRSV(A2) (SEQ ID NO: 68 of WO2019202035) or a corresponding F0 precursor protein of HRSV(Memphis-37) (SEQ ID NOs: 8937 or 11726 of WO2019202035) wherein position "1" corresponds to the first aa residue, i.e. the aa residue at the N-terminus of a HRSV(A2) F0 precursor protein or a HRSV(Memphis-37) F0 precursor protein.

In particularly preferred embodiments, the nucleic acid of component B (in particular, component B-3a) encodes least one antigenic peptide or protein derived from RSV F protein, wherein said RSV F protein is designed to stabilize the antigen in pre-fusion conformation. A pre-fusion conformation is particularly advantageous in the context of an efficient RSV vaccine, as several potential epitopes for neutralizing antibodies are merely accessible in said protein conformation.

Accordingly, in preferred embodiments, the RSV F protein is selected or derived from a pre-fusion stabilized F protein (F_stab) comprising at least one pre-fusion stabilizing mutation.

In several embodiments, the RSV F protein includes one or more amino acid substitutions that stabilize the F protein in the pre-fusion conformation, for example, substitutions that stabilize the membrane distal portion of the F protein (including the N-terminal region of the F1 polypeptide) in the pre-fusion conformation. For example, the amino acid substitution can introduce a non-natural disulfide bond or can be a cavity-filling amino acid substitution.

Accordingly, in several embodiments, a preferred RSV F protein includes S155C and S290C substitutions that form a non-natural disulfide bond that stabilizes the protein in a pre-fusion conformation; that is, in a conformation that specifically binds to one or more pre-fusion specification antibodies, and/or presents a suitable antigenic site that is present on the pre-fusion conformation but not in the postfusion conformation of RSV F protein. In further embodiments, the recombinant RSV F protein can additionally include F, L, W, Y, H, or M substitution at position 190, position 207, or positions 190 and 207.

Accordingly, in particularly preferred embodiments, the nucleic acid of component B encodes least one antigenic peptide or protein derived from RSV F protein, wherein the RSV F protein comprises a DSCav1 mutation (S155C, S290C, S190F, and V207L), or a fragment or a variant thereof.

In preferred embodiments, the at least one antigenic peptide or protein may be an engineered protein comprising the two subunits, F1 and F2 of mature F as a single polypeptide chain, wherein F2 and F1 are preferably connected via a linker (GS). F2-linker-F1 RSV F proteins lack aa104-144 (comprising the furine cleavage site and Pep27) and comprise a linker element between F2 polypeptide and F1 polypeptide (e.g. GS linker). F2-linker-F1 proteins may show superior properties in terms of stability and/or antigenicity.

Accordingly, in preferred embodiments, the RSV F protein comprises the two subunits F2 and F1 fused into a single polypeptide chain, wherein F2 and F1 are connected via a linker element, preferably a GS linker as specified herein, preferably generating a stable F2-linker-F1 proteins.

Preferably, said F2-linker-F1 fusion proteins, e.g. F(1-103)-GS-F(145-574) or F(1-103)-GS-F(145-553) additionally comprise a DScav1 mutation as outlined above (herein referred to as "mut0").

In particularly preferred embodiments, the RSV F protein may additionally comprise at least one further mutation selected from (S46G, A149C, S215P, Y458C, K465Q), (S46G, E92D, A149C, S215P, Y458C, K465Q), (S46G, N67I, E92D, A149C, S215P, Y458C, K465Q), (A149C, Y458C), (N183GC, N428C), (Q98C, Q361C, S46G, E92D, L95M, S215P, I217P, I221M, R429K, K465Q), (Q98C, Q361C, L95M, I221M, R429K), or (N183GC, N428C, S46G, N671, E92D, S215P, K465Q) or a fragment or a variant thereof.

In particularly preferred embodiments, F2-linker-F1 proteins (F(1-103)-GS-F(145-574) or F(1-103)-GS-F(145-553)) may additionally comprise, preferably in addition to the DSCav1 mutation, at least one mutation selected from S46G, A149C, S215P, Y458C, K465Q, herein referred to as "mut1"; S46G, E92D, A149C, S215P, Y458C, K465Q, herein referred to as "mut2"; S46G, N671, E92D, A149C, S215P, Y458C, K465Q, herein referred to as "mut3"; A149C, Y458C, herein referred to as "mut4"; N183GC, N428C, herein referred to as "mut5"; Q98C, Q361C, S46G, E92D, L95M, S215P, I217P, I221M, R429K, K465Q, herein referred to as "mut6"; Q98C, Q361C, L95M, I221M, R429K, herein referred to as "mut7"; N183GC, N428C, S46G, N671, E92D, S215P, K465Q, herein referred to as "mut8".

According to various embodiments, the nucleic acid of component B (in particular component B-3a) encodes at least one antigenic peptide or protein from an RSV as defined herein, and, additionally, at least one heterologous peptide or protein element.

Suitably, the at least one heterologous peptide or protein element may promote or improve secretion of the encoded RSV antigenic peptide or protein (e.g. via secretory signal sequences), promote or improve anchoring of the encoded RSV antigenic peptide or protein in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component B (in particular component B-3a) may additionally encode peptide linker elements, self-cleaving peptides, immunologic adjuvant sequences or dendritic cell targeting sequences.

Suitable multimerization domains may be selected from the list of amino acid sequences according to SEQ ID NOs: 1116-1167 of WO2017081082, or fragments or variants of these sequences. Suitable transmembrane elements may be selected from the list of amino acid sequences according to SEQ ID NOs: 1228-1343 of WO2017081082, or fragments or variants of these sequences. Suitable VLP forming sequences may be selected from the list of amino acid sequences according to SEQ ID NOs: 1168-1227 of the patent application WO2017081082, or fragments or variants of these sequences. Suitable peptide linkers may be selected from the list of amino acid sequences according to SEQ ID NOs: 1509-1565 of the patent application WO2017/081082, or fragments or variants of these sequences. Suitable self-cleaving peptides may be selected from the list of amino acid sequences according to SEQ ID NOs: 1434-1508 of the patent application WO2017081082, or fragments or variants of these sequences. Suitable immunologic adjuvant sequences may be selected from the list of amino acid sequences according to SEQ ID NOs: 1360-1421 of the patent application WO2017081082, or fragments or variants of these sequences. Suitable dendritic cell (DCs) targeting sequences may be selected from the list of amino acid sequences according to SEQ ID NOs: 1344-1359 of the patent application WO2017081082, or fragments or variants of these sequences. Suitable secretory signal peptides may be selected from the list of amino acid sequences according to SEQ ID NOs: 1-1115 and SEQ ID NO: 1728 of published PCT patent application WO2017081082, or fragments or variants of these sequences.

In preferred embodiments, the at least one coding sequence additionally encodes one or more heterologous peptide or protein elements selected from a signal peptide, a linker peptide, a helper epitope, an antigen clustering element, a trimerization or multimerization element, a transmembrane element, or a VLP forming sequence.

Preferred antigenic peptide or proteins selected or derived from an RSV as defined above are provided in Table 11 (rows 1 to 10) below. Therein, each row 1 to 10 corresponds to a suitable RSV constructs. Column A of Table 11 provides a short description of suitable RSV antigen constructs. Column B of Table 11 provides protein (amino acid) SEQ ID NOs of respective RSV antigen constructs. Column D of Table 11 provides SEQ ID NO of the corresponding G/C optimized nucleic acid coding sequences (opt1, gc). Column E of Table 11 provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of Table 11, e.g. mRNA sequences comprising the coding sequences of Table 11 are provided in Table 12.

**Table 11: Preferred RSV constructs (amino acid sequences and nucleic acid coding sequences):**

| **row** | **A** | **B** | **D** | **E** |
|---|---|---|---|---|
| 1 | HRSV-A/RSVA/Homo sapiens/USA/841-215A-01/1984 fusion glycoprotein (F0) | 14542 | 14552 | 14562 |
| 2 | HRSV-A/A2 fusion glycoprotein (F0) | 14543 | 14553 | 14563 |
| 3 | HRSV-A/A2 fusion glycoprotein (F-del) | 14544 | 14554 | 14564 |
| 4 | HRSV-A/A2 fusion glycoprotein (F0_DSCav1) | 14545 | 14555 | 14565 |
| 5 | HRSV-A/A2 fusion glycoprotein (F-del_DSCav1) | 14546 | 14556 | 14566 |
| 6 | HRSV-A/A2 fusion glycoprotein (F_DSCav1_mut4) | 14547 | 14557 | 14567 |
| 7 | HRSV-A/A2 fusion glycoprotein (F-del_DSCav1_mutd4) | 14548 | 14558 | 14568 |
| 8 | HRSV-A/A2 fusion glycoprotein (F_DSCav1_mut5) | 14549 | 14559 | 14569 |
| 9 | HRSV-A/A2 fusion glycoprotein (F-del_DSCav1_mut5) | 14550 | 14560 | 14570 |
| 10 | HRSV-A/A2 matrix protein (M2-1) | 14551 | 14561 | 14571 |

In preferred embodiments, the at least one antigenic peptide or protein selected or derived from at least one RSV encoded by the at least one nucleic acid of component B (in particular component B-3a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14542-14551,** or an immunogenic fragment or immunogenic variant of any of these. Further information regarding said amino acid sequences is also provided in **Table 8** (see rows 1 to 10 of Column A and B), and under <223> identifier of the ST.25 sequence listing of respective sequence SEQ ID NOs.

Further RSV F proteins that may be encoded by the nucleic acid of component B (in particular, component B-3a) are provided in Table 1 of published PCT patent application WO2019202035. Accordingly, Table 1 of published PCT patent application WO2019202035, and the disclosure and explanations relating thereto, is herewith incorporated by reference.

In other embodiments, the at least one antigenic peptide or protein selected or derived from at least one RSV encoded by the at least one nucleic acid of component B (in particular, component B-3a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NO: 68, 483, 898, 1267, 1636, 2005, 2374, 2743, 3112, 3481, 3850, 4219, 4588, 4957, 5326, 5695, 6064, 6433, 6802, 7171, 7540, 7909, 11726, 12095, 12464, 12833, 13940, 14309, 14678, 15047, 15416, 15785, 13202, 13571, 16154, 16523, 16892, 17261, 17630, 17999, 18368, 18737, 19106, 19475, 8279-9683 of published PCT application WO2019202035, or an immunogenic fragment or immunogenic variant of any of these. Accordingly, SEQ ID NO: 68, 483, 898, 1267, 1636, 2005, 2374, 2743, 3112, 3481, 3850, 4219, 4588, 4957, 5326, 5695, 6064, 6433, 6802, 7171, 7540, 7909, 11726, 12095, 12464, 12833, 13940, 14309, 14678, 15047, 15416, 15785, 13202, 13571, 16154, 16523, 16892, 17261, 17630, 17999, 18368, 18737, 19106, 19475, 8279-9683 of WO2019202035 and the corresponding disclosure relating thereto (e.g. information provided in Table 1 of WO2019202035) are herewith incorporated by reference.

In other embodiments, the at least one antigenic peptide or protein selected or derived from at least one RSV encoded by the at least one nucleic acid of component B (in particular, component B-3) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-1428 of published PCT patent application WO2014160463 or a fragment or variant of any of these sequences. Accordingly, SEQ ID NOs: 1-1428 of WO2014160463 and the corresponding disclosure related thereto are herewith incorporated by reference.

In other embodiments, the at least one antigenic peptide or protein selected or derived from at least one RSV encoded by the at least one nucleic acid of component B (in particular, component B-3a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-11 of published PCT patent application WO2015024668 or a fragment or variant of any of these sequences. Accordingly, SEQ ID NOs: 1-11 of WO2015024668 and the corresponding disclosure related thereto are herewith incorporated by reference.

In other embodiments, the at least one antigenic peptide or protein selected or derived from at least one RSV encoded by the at least one nucleic acid of component B (in particular, component B-3a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NO: 3, 4, 6, 8, 10, 12, 14, 16, 18, 20, 22, 24, 26, 28, 243, or 245 of published PCT patent application WO2017070622 or a fragment or variant of any of these sequences. Accordingly, SEQ ID NO: 3, 4, 6, 8, 10, 12, 14, 16, 18, 20, 22, 24, 26, 28, 243, or 245 of WO2017070622 and the corresponding disclosure related thereto are herewith incorporated by reference.

In other embodiments, the at least one antigenic peptide or protein selected or derived from at least one RSV encoded by the at least one nucleic acid of component B (in particular, component B-3a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-65, 81-95, 110-116 of published PCT patent application WO2017172890 or a fragment or variant of any of these sequences. Accordingly, SEQ ID NOs: 1-65, 81-95, 110-116 of WO2017172890 and the corresponding disclosure related thereto are herewith incorporated by reference.

According to preferred embodiments, the nucleic acid of component B (in particular, component B-3a) comprises at least one coding sequence encoding at least one antigenic peptide or protein derived from at least one RSV as defined above, or fragments and variants thereof. In that context, any coding sequence encoding at least one RSV antigenic protein as defined herein, or fragments and variants thereof, may be understood as suitable coding sequence and may therefore be comprised in the nucleic acid of component B.

In embodiments, the nucleic acid of component B (in particular component B-3a) comprises or consists of at least one coding sequence encoding at least one antigenic peptide or protein selected or derived from an RSV as defined herein, preferably encoding any one of SEQ ID NO: 68, 483, 898, 1267, 1636, 2005, 2374, 2743, 3112, 3481, 3850, 4219, 4588, 4957, 5326, 5695, 6064, 6433, 6802, 7171, 7540, 7909, 11726, 12095, 12464, 12833, 13940, 14309, 14678, 15047, 15416, 15785, 13202, 13571, 16154, 16523, 16892, 17261, 17630, 17999, 18368, 18737, 19106, 19475, 8279-9683 of WO2019202035; SEQ ID NOs: 1-1428 of WO2014160463; SEQ ID NOs: 1-11 of WO2015024668; SEQ ID NO: 3, 4, 6, 8, 10, 12, 14, 16, 18, 20, 22, 24, 26, 28, 243, 245 of WO2017070622; and SEQ ID NOs: 1-65, 81-95, 110-116 of WO2017172890 or fragments of variants of any of these sequences. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NO: 68, 483, 898, 1267, 1636, 2005, 2374, 2743, 3112, 3481, 3850, 4219, 4588, 4957, 5326, 5695, 6064, 6433, 6802, 7171, 7540, 7909, 11726, 12095, 12464, 12833, 13940, 14309, 14678, 15047, 15416, 15785, 13202, 13571, 16154, 16523, 16892, 17261, 17630, 17999, 18368, 18737, 19106, 19475, 8279-9683 of WO2019202035; SEQ ID NOs: 1-1428 of WO2014160463; SEQ ID NOs: 1-11 of WO2015024668; SEQ ID NO: 3, 4, 6, 8, 10, 12, 14, 16, 18, 20, 22, 24, 26, 28, 243, or 245 of WO2017070622; and SEQ ID NOs: 1-65, 81-95, 110-116 of WO2017172890, or fragments or variants of any of these sequences, may be selected and may accordingly be used as suitable coding sequence of the invention.

In preferred embodiments, the nucleic acid of component B (in particular component B-3a) comprises or consists of at least one coding sequence encoding at least one antigenic peptide or protein selected or derived from an RSV as defined herein, preferably encoding any one of **SEQ ID NOs: 14542-14551** or fragments of variants of any of these. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14542-14551** or fragment or variants of any of these, may be selected and may accordingly be understood as suitable coding sequence of the invention.

In embodiments, the at least one coding sequence of the at least one nucleic acid of component B (in particular, component B-3a) comprises or consists at least one nucleic acid sequence encoding a RSV antigen, the nucleic acid being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 69-482, 484-897, 899-1266, 1268-1635, 1637-2004, 2006-2373, 2375-2742, 2744-3111, 3113-3480, 3482-3849, 3851-4218, 4220-4587, 4589-4956, 4958-5325, 5327-5694, 5696-6063, 6065-6432, 6434-6801, 6803-7170, 7172-7539, 7541-7908, 7910-8277, 8278, 11727-12094, 12096-12463, 12465-12832, 12834-13201, 13941-14308, 14310-14677, 14679-15046, 15048-15415, 15417-15784, 15786-16153, 13203-13570, 13572-13939, 16155-16522, 16524-16891, 16893-17260, 17262-17629, 17631-17998, 18000-17998, 18369-18736, 18738-19105, 19107-19474, 19476-19843, 21363-21706 of published PCT application WO2019202035, or an immunogenic fragment or immunogenic variant of any of these. Accordingly, SEQ ID NOs: 69-482, 484-897, 899-1266, 1268-1635, 1637-2004, 2006-2373, 2375-2742, 2744-3111, 3113-3480, 3482-3849, 3851-4218, 4220-4587, 4589-4956, 4958-5325, 5327-5694, 5696-6063, 6065-6432, 6434-6801, 6803-7170, 7172-7539, 7541-7908, 7910-8277, 8278, 11727-12094, 12096-12463, 12465-12832, 12834-13201, 13941-14308, 14310-14677, 14679-15046, 15048-15415, 15417-15784, 15786-16153, 13203-13570, 13572-13939, 16155-16522, 16524-16891, 16893-17260, 17262-17629, 17631-17998, 18000-17998, 18369-18736, 18738-19105, 19107-19474, 19476-19843, 21363-21706 of WO2019202035 and the corresponding disclosure relating thereto (see also Table 3-6 in WO2019202035) are herewith incorporated by reference. Additional information regarding each of these suitable nucleic acid sequences encoding may also be derived from the sequence listing of WO2019202035, in particular from the details provided therein under identifier <223> in the sequence listing of WO2019202035.

In other embodiments, the at least one coding sequence of the at least one nucleic acid of component B (in particular, component B-3a) comprises or consists at least one nucleic acid sequence encoding a RSV antigen, the nucleic acid being identical or at least 70%, 80%, 85%, 86%, 87%, 88%, 89%, 90%, 91%, 92%, 93%, 94%, 95%, 96%, 97%, 98%, or 99% identical to SEQ ID NOs: 383-388 of published patent application WO2014160463 or a fragment or a fragment or variant of any of these sequences. Accordingly, SEQ ID NOs: 383-388 of WO2014160463 and the corresponding disclosure relating thereto are herewith incorporated by reference.

In other embodiments, the at least one coding sequence of the at least one nucleic acid of component B (in particular, component B-3a) comprises or consists at least one nucleic acid sequence encoding a RSV antigen, the nucleic acid being identical or at least 70%, 80%, 85%, 86%, 87%, 88%, 89%, 90%, 91%, 92%, 93%, 94%, 95%, 96%, 97%, 98%, or 99% identical to SEQ ID NOs: 12-22 of published patent application WO2015024668 or a fragment or a fragment or variant of any of these sequences Accordingly, SEQ ID NOs: 12-22 of WO2015024668 and the corresponding disclosure relating thereto are herewith incorporated by reference.

In other embodiments, the at least one coding sequence of the at least one nucleic acid of component B (in particular, component B-3a) comprises or consists at least one nucleic acid sequence encoding an RSV antigen, the nucleic acid being identical or at least 70%, 80%, 85%, 86%, 87%, 88%, 89%, 90%, 91%, 92%, 93%, 94%, 95%, 96%, 97%, 98%, or 99% identical to SEQ ID NOs: 1, 2, 5, 7, 9, 11, 13, 15, 17, 19, 21, 23, 25, 27, 242, 244, 246, 257, 258-280 of published patent application WO2017070622 or a fragment or a fragment or variant of any of these sequences. Accordingly, SEQ ID NOs: 1, 2, 5, 7, 9, 11, 13, 15, 17, 19, 21, 23, 25, 27, 242, 244, 246, 257, 258-280 of WO2017070622 and the corresponding disclosure relating thereto are herewith incorporated by reference.

in other embodiments, the at least one coding sequence of the at least one nucleic acid of component B (in particular, component B-3a) comprises or consists at least one nucleic acid sequence encoding a RSV antigen, the nucleic acid being identical or at least 70%, 80%, 85%, 86%, 87%, 88%, 89%, 90%, 91%, 92%, 93%, 94%, 95%, 96%, 97%, 98%, or 99% identical to SEQ ID NOs: 96-99 of published patent application WO2017172890 or a fragment or a fragment or variant of any of these sequences. Accordingly, SEQ ID NOs: 96-99 of WO2017172890 and the corresponding disclosure relating thereto are herewith incorporated by reference.

In preferred embodiments, the at least one coding sequence of the at least one nucleic acid of component B (in particular component B-3a) comprises or consists of a nucleic acid sequence encoding an RSV antigen, the nucleic acid comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequence selected from **SEQ ID NOs:** 14552-14571, 14572-14611 or a fragment or variant of any of these sequences. Further information regarding said nucleic acid sequences is also provided in **Table 11** (Column C-F), **Table** 12, and under <223> identifier of the ST.25 sequence listing of respective sequence SEQ ID NOs.

In preferred embodiments, the at least one coding sequence of the nucleic acid of **component B** (in particular of component B-3) is a codon modified coding sequence as defined herein, wherein the amino acid sequence, that is the RSV peptide or protein, encoded by the at least one codon modified coding sequence is preferably not being modified compared to the amino acid sequence encoded by the corresponding wild type or reference coding sequence. In particularly preferred embodiments, the at least one coding sequence of the nucleic acid **component B** (in particular of component B-3) is a codon modified coding sequence, wherein the codon modified coding sequence is selected a G/C optimized coding sequence, a human codon usage adapted coding sequence, or a G/C modified coding sequence.

Preferred nucleic acid sequences of **component B** (in particular of component B-3a) including particularly preferred mRNA sequences, are provided in **Table 12** (column C and D). Therein, each row represents a specific suitable RSV construct of the invention (compare with **Table 11),** wherein the description of the RSV construct is indicated in column A of **Table 12** and the SEQ ID NOs of the amino acid sequence of the respective RSV virus construct is provided in column B. The corresponding SEQ ID NOs of the coding sequences encoding the respective RSV constructs are provided in Table 11. Further information is provided under <223> identifier of the respective SEQ ID NOs in the sequence listing.

**Table 12: Nucleic acid, preferably mRNA constructs encoding RSV antigens**

| **A** | **B** | **C** | **D** |
|---|---|---|---|
| HRSV-A/RSVA/Homo sapiens/USA/84I-215A-01/1984 fusion glycoprotein (F0) | 14542 | 14572, 14582 | 14592, 14602 |
| HRSV-A/A2 fusion glycoprotein (F0) | 14543 | 14573, 14583 | 14593, 14603 |
| HRSV-A/A2 fusion glycoprotein (F-del) | 14544 | 14574, 14584 | 14594, 14604 |
| HRSV-A/A2 fusion glycoprotein (F0_DSCav1) | 14545 | 14575, 14585 | 14595, 14605 |
| HRSV-A/A2 fusion glycoprotein (F-del_DSCav1) | 14546 | 14576, 14586 | 14596, 14606 |
| HRSV-A/A2 fusion glycoprotein (F_DSCav1_mut4) | 14547 | 14577, 14587 | 14597, 14607 |
| HRSV-A/A2 fusion glycoprotein (F-del_DSCav1_mut4) | 14548 | 14578, 14588 | 14598, 14608 |
| HRSV-A/A2 fusion glycoprotein (F_DSCav1_mut5) | 14549 | 14579, 14589 | 14599, 14609 |
| HRSV-A/A2 fusion glycoprotein (F-del_DSCav1_mut5) | 14550 | 14580, 14590 | 14600, 14610 |
| HRSV-A/A2 matrix protein (M2-1) | 14551 | 14581, 14591 | 14601, 14611 |

In preferred embodiments, the at least one nucleic acid of component B (in particular component B-3a) comprises or consists of a nucleic acid sequence encoding an RSV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14572-14611,** or a fragment or variant of any of these sequences. Further information regarding respective nucleic acid sequences is provided under <223> identifier of the respective SEQ ID NO in the sequence listing, and in **Table 12.** Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In further embodiments, the at least one nucleic acid of component B (in particular component B-3) comprises or consists of a nucleic acid sequence encoding an RSV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 78-482, 493-897, 907-1266, 1276-1635, 1645-2004, 2014-2373, 2383-2742, 2752-3111, 3121-3480, 3490-3849, 3859-4218, 4228-4587, 4597-4956, 4966-5325, 5335-5694, 5704-6063, 6073-6432, 6442-6801, 6811-7170, 7180-7539, 7549-7908, 7918-8277, 8278, 21415-21480, 21561-21626, 11735-12094, 12104-12463, 12473-12832, 12842-13201, 13949-14308, 14318-14677, 14687-15046, 15056-15415, 15425-15784, 15794-16153, 13211-13570, 13580-13939, 16163-16522, 16532-16891, 16901-17260, 17270-17629, 17639-17998, 18008-18367, 18377-18736, 18746-19105, 19115-19474, 19484-19843, 21489-21552, 21635-21698 of published PCT application WO2019202035 or a fragment or variant of any of these sequences. Additional information regarding each of these suitable nucleic acid sequences may also be derived from the sequence listing of WO2019202035, in particular from the details provided therein under identifier <223> in the sequence listing of WO02019202035, or from Table 5A, 5B, 6A, 6B in WO2019202035. Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In various embodiments, component B, in particular component B-3a as defined herein, may comprise a plurality or at least more than one of the nucleic acid sequences as defined herein. In embodiment, component B of the pharmaceutical composition may comprise at least 2, 3, 4, 5, 6, 7, 8, 9, 10 or even more different nucleic acid sequences as defined herein each encoding at least one antigenic peptide or protein derived from genetically the same RSV or a fragment or variant thereof. In various embodiments, component B, in particular component B-3 as defined herein, may comprise a plurality or at least more than one of the nucleic acid sequences as defined herein. In embodiment, component B of the pharmaceutical composition may comprise at least 2, 3, 4, 5, 6, 7, 8, 9, 10 or even more different nucleic acid sequences as defined herein each encoding at least one antigenic peptide or protein derived from genetically distinct RSV or a fragment or variant thereof. Particularly, said (genetically) distinct RSV expresses at least one different protein, peptide or polyprotein, wherein the at least one different protein, peptide or polyprotein preferably differs in at least one amino acid.

In some embodiments, component B (in particular component B-3a) comprises at least 1, 2, 3, 4, 5, 6, 7, 8, 9, 10 or even more further nucleic acid, e.g. DNA or RNA constructs encoding RSV antigens selected from glycoprotein G, short hydrophobic protein SH, matrix protein M, nucleoprotein N, large polymerase L, M2-1 protein, M2-2 protein, phosphoprotein P, non-structural protein NS1 or non-structural protein NS2, or any combination thereof.

According to preferred embodiments, component B (in particular component B-3a) comprises at least one, two or three further nucleic acid sequences comprising at least one coding sequence encoding at least one antigenic peptide or protein selected or derived from RSV matrix protein M, nucleoprotein N, M2-1 protein, and/or phosphoprotein P or combinations thereof, preferably selected from M2-1.

Accordingly, in some embodiments, component B may comprise at least one nucleic acid encoding an RSV F peptide or protein as defined above, and, additionally, at least one further nucleic acid comprising at least one coding sequence encoding at least one antigenic peptide or protein derived from RSV selected from matrix protein M, nucleoprotein N, M2-1 protein, and/or phosphoprotein P or combinations thereof.

The addition of a further nucleic acid comprising at least one coding sequence encoding at least one antigenic peptide or protein derived from RSV M, N, M2-1, and/or phosphoprotein P (or combinations thereof) is particularly advantageous to e.g. promote a T-cell immune response.

In preferred embodiments, the composition of the second aspect may suitably comprise at least one artificial RNA of the first aspect encoding RSF F, at least one further artificial RNA comprising at least one coding sequence encoding at least one antigenic peptide or protein derived from RSV M, at least one further artificial RNA comprising at least one coding sequence encoding at least one antigenic peptide or protein derived from RSV N, at least one further artificial RNA comprising at least one coding sequence encoding at least one antigenic peptide or protein derived from RSV P, and at least one further artificial RNA comprising at least one coding sequence encoding at least one antigenic peptide or protein derived from RSV M2-1.

In preferred, the coding sequence of the further nucleic acid of component B (in particular B-3a) encodes at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 9684, 10053-10133, 10134, 10503-10636, 10637, 11006-11182, 11183, 11552-11725, 19844, 20213, 20582, 20951 of published PCT application WO2019202035, or a fragment or variant of any of these sequences. Accordingly, SEQ ID NOs: 9684, 10053-10133, 10134, 10503-10636, 10637, 11006-11182, 11183, 11552-11725, 19844, 20213, 20582, 20951 of WO2019202035, and the disclosure relating to these sequences, are herewith incorporated by reference. Additional information regarding each of these suitable amino acid sequences encoding RSV proteins may also be derived from the sequence listing of WO2019202035, in particular from the details provided therein under identifier <223> as explained in the following.

In further embodiments, the coding sequence of the further nucleic acid encodes at least one antigenic peptide or protein as defined herein and additionally a heterologous secretory signal sequence or heterologous secretory signal peptide. The heterologous secretory signal sequence may increase the secretion of the encoded antigenic peptide or protein.

In preferred embodiments, the at least one coding sequence of the at least one further nucleic acid sequence of component B (in particular component B-3a) comprises or consists of a nucleic acid sequence encoding an RSV antigen, the nucleic acid comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from SEQ ID NOs: 9685-9692, 10135-10142, 10638-10645, 11184-11119, 19845-19852, 20214-20221, 20583-20590, 20952-20959, 21385-21388, 21411-21414 of published PCT application WO2019202035, or a fragment or variant of any of these sequences. Accordingly, SEQ ID NOs: 9685-9692, 10135-10142, 10638-10645, 11184-11119, 19845-19852, 20214-20221, 20583-20590, 20952-20959, 21385-21388, 21411-21414 of WO2019202035, and the disclosure relating to these sequences, are herewith incorporated by reference. Additional information regarding each of these suitable amino acid sequences encoding RSV proteins may also be derived from the sequence listing of WO2019202035, in particular from the details provided therein under identifier <223> as explained in the following. In that context, respective preferred RSV polypeptide, nucleic acid, and mRNA sequences are provided in Table 7A of published PCT application WO2019202035. The full content of Table 7A of WO2019202035 is herewith incorporated by reference.

In preferred embodiments, the at least one coding sequence of the at least one further nucleic acid sequence of component B (in particular component B-3a) comprises or consists of a nucleic acid sequence encoding an RSV antigen, the nucleic acid comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from SEQ ID NOs: 9693-10052, 10143-10502, 10646-11005, 11192-11551, 19853-20212, 20222-20581, 20591-20950, 20960-21319, 21481-21488, 21627-21634, 21553-21560, 21699-21706 of published PCT application WO2019202035, or a fragment or variant of any of these sequences. Accordingly SEQ ID NOs: 9693-10052, 10143-10502, 10646-11005, 11192-11551, 19853-20212, 20222-20581, 20591-20950, 20960-21319, 21481-21488, 21627-21634, 21553-21560, 21699-21706 of WO2019202035, and the disclosure relating to these sequences, are herewith incorporated by reference. Additional information regarding each of these suitable amino acid sequences encoding RSV proteins may also be derived from the sequence listing of WO2019202035, in particular from the details provided therein under identifier <223> as explained in the following. In that context, respective preferred RSV polypeptide, nucleic acid, and mRNA sequences are provided in Table 7A of published PCT application WO2019202035. The full content of Table 7A of WO2019202035 is herewith incorporated by reference.

Suitably, component B, in particular component B-3a comprises nucleic acid sequences encoding at least two different RSV antigens, wherein suitable combinations are provided in Table 7B of WO2019202035, the full content of Table 7A of WO2019202035 is herewith incorporated by reference.

In preferred embodiments, component B-3a comprises at least one nucleic acid encoding at least one antigenic peptide or protein that is selected or derived from at least one RSV, or an immunogenic fragment or immunogenic variant thereof, wherein said component B-3a is to be, preferably, administered intramuscularly or intradermal.

Preferably, intramuscular or intradermal administration of said component B-3a results in expression of the encoded RSV antigen construct in a subject. In embodiments where the nucleic acid is an RNA, administration of component B-3a results in translation of the RNA and to a production of the encoded RSV antigen in a subject. In embodiments where the nucleic acid is a DNA (e.g. plasmid DNA, adenovirus DNA), administration of said composition results in transcription of the DNA into RNA, and to a subsequent translation of the RNA into the encoded RSV antigen in a subject.

In preferred embodiments, administration of component B-3a elicits antigen-specific immune responses comprising T-cell responses and/or B-cell responses against the encoded RSV antigens provided by the at least one nucleic acid of component B-3a.

Preferably, the component B-3a is suitable for a vaccine, in particular, suitable for a RSV vaccine, preferably a combination vaccine of the invention.

In embodiments, the nucleic acid as comprised in component B-3a as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-3a comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component B-3b: Human metapneumovirus:

In preferred embodiments, the at least one further virus of component B is selected from at least one Metapneumovirus of the Pneumoviridae virus family (also herein referred to as component B-3b). Suitably, the Metapneumovirus is selected from human Metapneumovirus (hMPV).

Human metapneumovirus (hMPV) is a negative-sense single-stranded RNA virus of the family Pneumoviridae and is closely related to the Avian metapneumovirus (AMPV) subgroup C. It was isolated for the first time in 2001. It is the second most common cause after Human orthopneumovirus (RSV) of lower respiratory infection in young children. hMPV is nearly as common and as severe as influenza in older adults. hMPV is associated with more severe disease in people with asthma and adults with chronic obstructive pulmonary disease (COPD). Numerous outbreaks of hMPV have been reported in long-term care facilities for children and adults, causing fatalities.

In the context of the invention, any Human metapneumovirus (hMPV; NCBI Taxonomy ID: 162145) may be selected, irrespective of genotype, species, strain, isolate, or serotype. Non-limiting examples of strains of hMPV for use as provided herein include CAN98-75 (CAN75) and CAN97-83 (CAN83) hMPV strains, hMPV AI, A2, B I or B2 strain, hMPV isolate TN/92-4 (e.g., SEQ ID NO: 1 and 5 of WO2017070626), a hMPV isolate NL/1/99 (e.g. , SEQ ID NO: 2 and 6 of WO2017070626), a hMPV isolate PER/CFI0497/2010/B (e.g., SEQ ID NO: 3 and 7 of WO2017070626), or hMPV isolate 00-1.

In some embodiments, the Metapneumovirus is selected from hMPV AI, A2, B I or B2 strain, CAN98-75 (CAN75) hMPV strain, CAN97-83 (CAN83) hMPV, hMPV isolate TN/92-4 (e.g., SEQ ID NO: 1 and 5 of WO2017070626), hMPV isolate NL/1/99 (e.g., SEQ ID NO: 2 and 6 of WO2017070626), hMPV isolate PER/CFI0497/2010/B (e.g., SEQ ID NO: 3 and 7 of WO2017070626). In some embodiments, the Metapneumovirus is selected from HMPV/TN/92-4, HMPV/NL/1/99, HMPV/Sabana, or HMPV/00-1

In some embodiments, the at least one antigenic peptide or protein encoded by the at least one nucleic acid of component B comprises or consists at least one peptide or protein selected or derived from at least one hMPV F protein, G protein, M protein, P protein, N protein and/or SH protein, or an immunogenic fragment or immunogenic variant thereof.

In preferred embodiments, the at least one antigenic peptide or protein of component B (in particular component B-3b) is selected or derived from hMPV F protein, or an immunogenic fragment or variant thereof.

In preferred embodiments, the at least one antigenic peptide or protein selected or derived from hMPV F protein is a full-length F protein (F0) or an F protein with deleted C-terminus (F-del), or an immunogenic fragment or immunogenic variant thereof.

In preferred embodiments, the hMPV F protein is selected or derived from a pre-fusion stabilized F protein (F_stab) comprising at least one pre-fusion stabilizing mutation.

According to various preferred embodiments, the nucleic acid of component B encodes at least one antigenic peptide or protein from hMPV as defined herein, and, additionally, at least one heterologous peptide or protein element.

Suitably, the at least one heterologous peptide or protein element may promote or improve secretion of the encoded hMPV antigenic peptide or protein (e.g. via secretory signal sequences), promote or improve anchoring of the encoded hMPV antigenic peptide or protein in the plasma membrane (e.g. via transmembrane elements), promote or improve formation of antigen complexes (e.g. via multimerization domains or antigen clustering elements), or promote or improve virus-like particle formation (VLP forming sequence). In addition, the nucleic acid of component B (in particular, component B-3b) may additionally encode peptide linker elements, self-cleaving peptides, immunologic adjuvant sequences or dendritic cell targeting sequences.

Preferred antigenic peptide or proteins selected or derived from a hMPV as defined above are provided in **Table 13** (rows 1 to 29). Therein, each row 1 to 29 corresponds to a suitable hMPV constructs. Column A of **Table 13** provides a short description of suitable hMPV antigen constructs. Column B of **Table 13** provides protein (amino acid) SEQ ID NOs of respective hMPV antigen constructs. Column D of **Table 13** provides SEQ ID NO of the corresponding G/C optimized nucleic acid coding sequences (opt1, gc). Column E of **Table 13** provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of **Table 13,** e.g. mRNA sequences comprising the coding sequences of **Table 13** are provided in **Table 14.**

**Table 13: Preferred hMPV constructs (amino acid sequences and nucleic acid coding sequences).**

| **row** | **A** | **B** | **D** | **E** |
|---|---|---|---|---|
| 1 | HMPV/TN/92-4 fusion glycoprotein (F0) | 14626 | 14650, 26978 | 14674 |
| 2 | HMPV/TN/92-4 fusion glycoprotein (variant) | 14627 | 14651 | 14675 |
| 3 | HMPV/TN/92-4 fusion glycoprotein (variant) | 14628 | 14652 | 14676 |
| 4 | HMPV/TN/92-4 fusion glycoprotein (variant) | 14629 | 14653 | 14677 |
| 5 | HMPV/TN/92-4 fusion glycoprotein (variant) | 14630 | 14654 | 14678 |
| 6 | HMPV/TN/92-4 fusion glycoprotein (variant) | 14631 | 14655 | 14679 |
| 7 | HMPV/TN/92-4 fusion glycoprotein (variant) | 14632 | 14656 | 14680 |
| 8 | HMPV/TN/92-4 fusion glycoprotein (variant) | 14633 | 14657 | 14681 |
| 9 | HMPV/TN/92-4 fusion glycoprotein (variant) | 14634 | 14658 | 14682 |
| 10 | HMPV/TN/92-4 fusion glycoprotein (variant) | 14635 | 14659 | 14683 |
| 11 | HMPV/TN/92-4 fusion glycoprotein (variant) | 14636 | 14660 | 14684 |
| 12 | HMPV/TN/92-4 fusion glycoprotein (variant) | 14637 | 14661 | 14685 |
| 13 | HMPV/TN/92-4 fusion glycoprotein (variant) | 14638 | 14662 | 14686 |
| 14 | HMPV/TN/92-4 fusion glycoprotein (variant) | 14639 | 14663 | 14687 |
| 15 | HMPV/TN/92-4 fusion glycoprotein (variant) | 14640 | 14664 | 14688 |
| 16 | HMPV/TN/92-4 fusion glycoprotein (variant) | 14641 | 14665 | 14689 |
| 17 | HMPV/TN/92-4 fusion glycoprotein (variant) | 14642 | 14666 | 14690 |
| 18 | HMPV/TN/92-4 fusion glycoprotein (variant) | 14643 | 14667 | 14691 |
| 19 | HMPV/TN/92-4 fusion glycoprotein (variant) | 14644 | 14668 | 14692 |
| 20 | HMPV/TN/92-4 fusion glycoprotein (variant) | 14645 | 14669 | 14693 |
| 21 | HMPV/TN/92-4 fusion glycoprotein (variant) | 14646 | 14670 | 14694 |
| 22 | HMPV/TN/92-4 fusion glycoprotein (variant) | 14647 | 14671 | 14695 |
| 23 | HMPV/NL/1/99 fusion glycoprotein (F0) | 14648 | 14672, 26972 | 14696 |
| 24 | HMPV/Sabana fusion glycoprotein (F0) | 14649 | 14673 | 14697 |
| 25 | HMPV/NL/1/99 fusion glycoprotein (variant) | 26967 | 26973 | |
| 26 | HMPV/NL/1/99 fusion glycoprotein (variant) | 26968 | 26974 | |
| 27 | HMPV/00-1 fusion glycoprotein (F0) | 26969 | 26975 | |
| 28 | HMPV/00-1 fusion glycoprotein (variant) | 26970 | 26976 | |
| 29 | HMPV/00-1 fusion glycoprotein (variant) | 26971 | 26977 | |

In preferred embodiments, the at least one antigenic peptide or protein selected or derived from at least one hMPV encoded by the at least one nucleic acid of component B (in particular component B-3b) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14626-14649, 26966-26971** or an immunogenic fragment or immunogenic variant of any of these. Further information regarding said amino acid sequences is also provided in **Table 13** (see rows 1 to 29 of Column A and B), and under <223> identifier of the ST.25 sequence listing of respective sequence SEQ ID NOs.

In further embodiments, the at least one antigenic peptide or protein selected or derived from hMPV encoded by the at least one nucleic acid of component B (in particular component B-3b) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of SEQ ID NOs: 5-8 of published PCT patent application WO2017070626 or an immunogenic fragment or immunogenic variant of any of these. Accordingly, SEQ ID NOs: 5-8 of WO2017070626, and the corresponding disclosure relating thereto, are herewith incorporated by reference.

Further hMPV antigens may also be derived from or selected proteins identifiable by GenBank Accession Numbers provided in Table 4 of WO2017070626. The full content of Table 4 of WO2017070626 herewith incorporated by reference.

According to preferred embodiments, the nucleic acid of component B (in particular component B-3b) comprises at least one coding sequence encoding at least one antigenic peptide or protein derived from hMPV as defined above, or fragments and variants thereof. In that context, any coding sequence encoding at least one hMPV antigenic protein as defined herein, or fragments and variants thereof may be understood as suitable coding sequence of component B.

In preferred embodiments, the nucleic acid of component B (in particular component B-3b) comprise or consist of at least one coding sequence encoding at least one antigenic peptide or protein selected or derived from a hMPV as defined herein, preferably encoding any one of **SEQ ID NOs: 14626-14649, 26966-26971;** SEQ ID NOs: 5-8 of WO2017070626, or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14626-14649, 26966-26971;** SEQ ID NOs: 5-8 of WO2017070626, or fragments or variants thereof, may be selected and may accordingly be understood as suitable coding sequence of the invention. Further information regarding said amino acid sequences is also provided in **Table 13, Table 14,** and under <223> identifier of the ST.25 sequence listing of respective sequence SEQ ID NOs.

In preferred embodiments, the at least one coding sequence of the at least one nucleic acid of component B (in particular component B-3b) comprises or consists of a nucleic acid sequence encoding an hMPV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14650-14697, 14698-14793, 26972-26978, 26979-26991,** or a fragment or variant of any of these sequences. Further information regarding said nucleic acid sequences is also provided in **Table 13, Table 14,** and under <223> identifier of the ST.25 sequence listing of respective sequence SEQ ID NOs.

In further embodiments, the nucleic acid of component B (in particular component B-3b) comprises a coding sequence that comprises at least one of the nucleic acid sequences encoding a hMPV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from SEQ ID NOs: 1-4, 57-60 of published PCT patent application WO2017070626 or a fragment or a fragment or variant of any of these sequences. Accordingly, SEQ ID NOs: 1-4, 57-60 of WO2017070626, and the corresponding disclosure relating thereto, are herewith incorporated by reference.

In preferred embodiments, the at least one coding sequence of the nucleic acid of component B (in particular component B-3b) is a codon modified coding sequence as defined herein, wherein the amino acid sequence, that is the hMPV peptide or protein, encoded by the at least one codon modified coding sequence, is preferably not being modified compared to the amino acid sequence encoded by the corresponding wild type or reference coding sequence.

In particularly preferred embodiments, the at least one coding sequence of the nucleic acid component B (in particular component B-3b) is a codon modified coding sequence, wherein the codon modified coding sequence is selected a G/C optimized coding sequence, a human codon usage adapted coding sequence, or a G/C modified coding sequence.

Preferred nucleic acid sequences of component B (in particular component B-3b), including particularly preferred mRNA sequences, are provided in **Table 14** (column C and D). Therein, each row represents a specific suitable hMPV construct of the invention (compare with **Table 13),** wherein the description of the hMPV construct is indicated in column A of Table 14 and the SEQ ID NOs of the amino acid sequence of the respective hMPV construct is provided in column B. The corresponding SEQ ID NOs of the coding sequences encoding the respective hMPV constructs are provided in in Table 13. Further information is provided under <223> identifier of the respective SEQ ID NOs in the sequence listing.

**Table 14: Nucleic acid, preferably mRNA constructs encoding hMPV antigens**

| **A** | **B** | **C** | **D** |
|---|---|---|---|
| HMPV/TN/92-4 fusion glycoprotein (F0) | 14626 | 14698, 14722 | 14746, 14770 |
| HMPV/TN/92-4 fusion glycoprotein (variant) | 14627 | 14699, 14723 | 14747, 14771 |
| HMPV/TN/92-4 fusion glycoprotein (variant) | 14628 | 14700, 14724 | 14748, 14772 |
| HMPV/TN/92-4 fusion glycoprotein (variant) | 14629 | 14701, 14725 | 14749, 14773 |
| HMPV/TN/92-4 fusion glycoprotein (variant) | 14630 | 14702, 14726 | 14750, 14774 |
| HMPV/TN/92-4 fusion glycoprotein (variant) | 14631 | 14703, 14727 | 14751, 14775 |
| HMPV/TN/92-4 fusion glycoprotein (variant) | 14632 | 14704, 14728 | 14752, 14776 |
| HMPV/TN/92-4 fusion glycoprotein (variant) | 14633 | 14705, 14729 | 14753, 14777 |
| HMPV/TN/92-4 fusion glycoprotein (variant) | 14634 | 14706, 14730 | 14754, 14778 |
| HMPV/TN/92-4 fusion glycoprotein (variant) | 14635 | 14707, 14731 | 14755, 14779 |
| HMPV/TN/92-4 fusion glycoprotein (variant) | 14636 | 14708, 14732 | 14756, 14780 |
| HMPV/TN/92-4 fusion glycoprotein (variant) | 14637 | 14709, 14733 | 14757, 14781 |
| HMPV/TN/92-4 fusion glycoprotein (variant) | 14638 | 14710, 14734 | 14758, 14782 |
| HMPV/TN/92-4 fusion glycoprotein (variant) | 14639 | 14711, 14735 | 14759, 14783 |
| HMPV/TN/92-4 fusion glycoprotein (variant) | 14640 | 14712, 14736 | 14760, 14784 |
| HMPV/TN/92-4 fusion glycoprotein (variant) | 14641 | 14713, 14737 | 14761, 14785 |
| HMPV/TN/92-4 fusion glycoprotein (variant) | 14642 | 14714, 14738 | 14762, 14786 |
| HMPV/TN/92-4 fusion glycoprotein (variant) | 14643 | 14715, 14739 | 14763, 14787 |
| HMPV/TN/92-4 fusion glycoprotein (variant) | 14644 | 14716, 14740 | 14764, 14788 |
| HMPV/TN/92-4 fusion glycoprotein (variant) | 14645 | 14717, 14741 | 14765, 14789 |
| HMPV/TN/92-4 fusion glycoprotein (variant) | 14646 | 14718, 14742 | 14766, 14790 |
| HMPV/TN/92-4 fusion glycoprotein (variant) | 14647 | 14719, 14743 | 14767, 14791 |
| HMPV/NL/1/99 fusion glycoprotein (F0) | 14648 | 14720, 14744, 26979 | 14768, 14792, 26985 |
| HMPV/Sabana fusion glycoprotein (F0) | 14649 | 14721, 14745 | 14769, 14793 |
| HMPV/NL/1/99 fusion glycoprotein (variant) | 26967 | 26980 | 26986 |
| HMPV/NL/1/99 fusion glycoprotein (variant) | 26968 | 26981 | 26987 |
| HMPV/00-1 fusion glycoprotein (F0) | 26969 | 26982 | 26988 |
| HMPV/00-1 fusion glycoprotein (variant) | 26970 | 6983 | 26989 |
| HMPV/00-1 fusion glycoprotein (variant) | 26971 | 26984 | 26990 |

In preferred embodiments, the at least one nucleic acid of component B (in particular component B-3b) comprises or consists of a nucleic acid sequence encoding an hMPV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of **SEQ ID NOs: 14698-14793, 26979-26991** or a fragment or variant of any of these sequences. Further information regarding respective nucleic acid sequences is provided under <223> identifier of the respective SEQ ID NO in the sequence listing and in **Table 14** (Column C and D). Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In further embodiments, the nucleic acid of component B (in particular component B-3b), preferably the RNA, comprises or consists of a nucleic acid sequence encoding a hMPV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NOs: 57-60 of WO2017070626 or a fragment or variant of any of these sequences. Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In embodiments, component B (in particular component B-3b) comprises a plurality or at least more than one of the nucleic acid species comprising at least one coding sequence encoding at least one antigenic peptide or protein selected or derived from at least one hMPV, or an immunogenic fragment or immunogenic variant thereof, e.g. DNA or RNA species as defined herein. Preferably, component B (in particular component B-3b) as defined herein comprises 2, 3, 4, 5, 6, 7, 8, 9, or 10 different nucleic acids each defined as defined herein.

In embodiments, component B (in particular component B-3b) may comprise at least 2, 3, 4, 5, 6, 7, 8, 9, 10 or even more different nucleic acid species, e.g. DNA or RNA, as defined herein, each encoding at least one antigenic peptide or protein derived from the same hMPV, or a fragment or variant thereof. Particularly, said (genetically) same hMPV expresses (essentially) the same repertoire of proteins or peptides, wherein all proteins or peptides have (essentially) the same amino acid sequence. Particularly, said (genetically) same hMPV expresses essentially the same proteins, peptides or polyproteins, wherein these protein, peptide or polyproteins preferably do not differ in their amino acid sequence(s).

In embodiments, component B (in particular component B-3b) comprises at least 2, 3, 4, 5, 6, 7, 8, 9, 10 or even more different nucleic acid species, e.g. DNA or RNA, as defined herein, each encoding at least one peptide or protein derived from a genetically distinct hMPV (e.g. a distinct hMPV isolate), or a fragment or variant thereof. The terms "distinct" or "distinct hMPV" as used throughout the present specification have to be understood as the difference between at least two respective hMPV (e.g. a distinct hMPV isolate), wherein the difference is manifested on the genome of the respective distinct hMPV. Particularly, said (genetically) distinct hMPV may express at least one distinct protein, peptide or polyprotein, wherein the at least one distinct protein, peptide or polyprotein differs in at least one amino acid.

In preferred embodiments, the component B-3b comprises at least one nucleic acid encoding at least one antigenic peptide or protein that is selected or derived from at least one hMPV, or an immunogenic fragment or immunogenic variant thereof, wherein said component B-3b is to be, preferably, administered intramuscularly or intradermal.

Preferably, intramuscular or intradermal administration of said component B-3b results in expression of the encoded hMPV antigen construct in a subject. In embodiments where the nucleic acid is an RNA, administration of component B-3b results in translation of the RNA and to a production of the encoded hMPV antigen in a subject. In embodiments where the nucleic acid is a DNA (e.g. plasmid DNA, adenovirus DNA), administration of said composition results in transcription of the DNA into RNA, and to a subsequent translation of the RNA into the encoded hMPV antigen in a subject.

In preferred embodiments, administration of component B-3b elicits antigen-specific immune responses comprising T-cell responses and/or B-cell responses against the encoded hMPV antigens provided by the at least one nucleic acid of component B-3b.

Preferably, the component B-3b is suitable for a vaccine, in particular, suitable for a hMPV vaccine, preferably a combination vaccine of the invention.

In embodiments, the nucleic acid as comprised in component B-3 as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-3 comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component B-4: Paramyxoviridae virus

In preferred embodiments, the at least one further virus of component B is selected from at least one virus of the Paramyxoviridae virus family ("Paramyxovirus").

It has to be understood that generic embodiments and features that have been described in paragraph *"Component B"* may also apply to a nucleic acid encoding a Paramyxoviridae antigenic peptide or protein.

Paramyxoviridae has four subfamilies, 17 genera, and 77 species, three genera of which are unassigned to a subfamily. Diseases associated with this family include measles, mumps, and respiratory tract infections.

In embodiments, any protein selected or derived from a Paramyxovirus may be used in the context of the invention and may be encoded by the coding sequence or the nucleic acid of component B (in particular, component B-4).

Suitable in the context of the invention are pathogenic Paramyxoviruses, including human parainfluenza viruses (hPIV) or Henipaviruses.

### Component B-4a: Parainfluenzavirus (PIV):

In preferred embodiments, the at least one further virus of component B is selected from at least one Parainfluenza virus (PIV) of the Paramyxoviridae virus family, preferably a human Parainfluenzavirus (hPIV) of the Paramyxoviridae virus family (also herein referred to as component B-4a).

The human parainfluenza viruses (hPIV) are the second most common causes of respiratory tract disease in infants and children. hPIV viruses belong to the Respirovirus genus (NCBI Taxonomy ID: 186938). Parainfluenza viruses are also referred to as Respiroviruses. There are four types of hPIVs, known as HPIV-1, HPIV-2, HPIV-3 and HPIV-4. HPIV-1 and HPIV-2 may cause cold-like symptoms, along with croup in children. HPIV-3 is associated with bronchiolitis, bronchitis, and pneumonia. HPIV-4 is less common than the other types, and is known to cause mild to severe respiratory tract illnesses. In the context of the invention, any Parainfluenzavirus may be selected,
In the context of the invention, any Parainfluenzavirus, e.g. any hPIV may be selected, irrespective of genotype, species, strain, isolate, or serotype.

In embodiments, the at least one human hPIV is selected from a hPIV-1, a hPIV-2, a hPIV-3 or hPIV-4.

In embodiments, the at least one human hPIV may be selected from a hPIV-1 (alternative name, Human respirovirus 1, NCBI Taxonomy ID: 12730). In the context of the invention, hPIV-1 viruses may be selected from hPIV-1 (human/Washington/1957; NCBI Taxonomy ID: 11211), hPIV-1 (CI-14/83; NCBI Taxonomy ID: 31606), hPIV-1 (CI-5/73; NCBI Taxonomy ID: 31607), hPIV-1 (A1426 / 86-315 / 62M-753; NCBI Taxonomy ID: 36412), hPIV-1 (C35; NCBI Taxonomy ID: 31605), hPIV-1 (C39; NCBI Taxonomy ID: 11210), hPIV-1 (Washington/1964; NCBI Taxonomy ID: 188538).

In embodiments, the at least one human hPIV may be selected from a hPIV-3 (altemative name, Human respirovirus 3, NCBI Taxonomy ID: 11216). In the context of the invention, hPIV-3 viruses may be selected from hPIV-3 (AUS/124854/74; NCBI Taxonomy ID: 11218), hPIV-3 (NIH 47885; NCBI Taxonomy ID: 11217), hPIV-3 (TEX/12677/83; NCBI Taxonomy ID: 11221), hPIV-3 (TEX/545/80; NCBI Taxonomy ID: 11219), hPIV-3 (TEX/9305/82; NCBI Taxonomy ID: 11220), hPIV-3 (WASH/1511/73; NCBI Taxonomy ID: 11223), hPIV-3 (WASH/641/79; NCBI Taxonomy ID: 11222),hPIV-3 (Simian Agent 10; NCBI Taxonomy ID: 929831), hPIV-3 (HPIV3/Homo sapiens/PER/FLA4571/2008; NCBI Taxonomy ID: 11216), or hPIV-3 (HPIV3/USA/629-D01959/2007; NCBI Taxonomy ID: 11216).

In preferred embodiments, the at least one human hPIV is selected from a hPIV-3 virus as defined herein.

In preferred embodiments, the at least one antigenic peptide or protein encoded by the at least one nucleic acid of component B (in particular, component B-4a) comprises or consists at least one peptide or protein selected or derived from at least one PIV hemagglutinin-neuraminidase, fusion (F) glycoprotein, matrix protein (M), nucleocapsid protein (N), viral replicase (L), non-structural V protein, or an immunogenic fragment thereof.

PIV hemagglutinin-neuraminidase (PIV HN), a structural protein, is found on the viral envelope, where it is necessary for attachment and cell entry. It recognizes and binds to sialic acid-containing receptors on the host cell's surface. As a neuraminidase, HN removes sialic acid from virus particles, preventing self-aggregation of the virus, and promoting the efficient spread of the virus. Furthermore, HN promotes the activity of the fusion (F or F0) protein, contributing to the penetration of the host cell's surface.

PIV fusion protein (PIV F) is located on the viral envelope, where it facilitates the viral fusion and cell entry. The F protein is initially inactive, but proteolytic cleavage leads to its active forms, F1 and F2, which are linked by disulfide bonds. This occurs when the HN protein binds its receptor on the host cell's surface. During early phases of infection, the F glycoprotein mediates penetration of the host cell by fusion of the viral envelope to the plasma membrane. In later stages of the infection, the F protein facilitates the fusion of the infected cells with neighbouring uninfected cells, which leads to the formation of a syncytium and spread of the infection.

PIV matrix protein (PIV M) is found within the viral envelope and assists with viral assembly. It interacts with the nucleocapsid and envelope glycoproteins, where it facilitates the budding of progeny viruses through its interactions with specific sites on the cytoplasmic tail of the viral glycoproteins and nucleocapsid. It also plays a role in transporting viral components to the budding site.

PIV phosphoprotein (PIV P) and PIV large polymerase protein (L) are found in the nucleocapsid where they form part of the RNA polymerase complex. The L protein, a viral RNA-dependent RNA polymerase, facilitates genomic transcription, while the host cell's ribosomes translate the viral mRNA into viral proteins.

PIV V is a non-structural protein that blocks IFN signalling in the infected cell, therefore acting as a virulence factor.

PIV nucleoprotein (N) encapsulates the genome in a ratio of 1 N per 6 ribonucleotides, protecting it from nucleases. The nucleocapsid (NC) has a helical structure. The encapsulated genomic RNA is termed the NC and serves as template for transcription and replication. During replication, encapsulation by PIV3 N is coupled to RNA synthesis and all replicative products are resistant to nucleases. PrV3 N homo-multimerizes to form the nucleocapsid and binds to viral genomic RNA. PrV3 N binds the P protein and thereby positions the polymerase on the template.

According to various preferred embodiments, the nucleic acid of component B encodes at least one antigenic peptide or protein from PIV as defined herein, and, additionally, at least one heterologous peptide or protein element.

Suitably, the at least one heterologous peptide or protein element may promote or improve secretion of the encoded PIV antigenic peptide or protein (e.g. via secretory signal sequences), promote or improve anchoring of the encoded PIV antigenic peptide or protein in the plasma membrane (e.g. via transmembrane elements), promote or improve formation of antigen complexes (e.g. via multimerization domains or antigen clustering elements), or promote or improve virus-like partide formation (VLP forming sequence). In addition, the nucleic acid of component B (in particular, component B-4a) may additionally encode peptide linker elements, self-cleaving peptides, immunologic adjuvant sequences or dendritic cell targeting sequences.

Preferred antigenic peptide or proteins selected or derived from a hPIV as defined above are provided in Table 15. Therein, each row corresponds to a suitable hPIV constructs. Column A of Table 15 provides a short description of suitable hPIV antigen constructs. Column B of Table 15 provides protein (amino acid) SEQ ID NOs of respective hPIV antigen constructs. Column D of **Table 15** provides SEQ ID NO of the corresponding G/C optimized nucleic acid coding sequences (opt1, gc). Column E of **Table 15** provides SEQ ID NO of the corresponding human codon usage adapted nucleic acid coding sequences (opt 3, human).

Notably, the description of the invention explicitly includes the information provided under <223> identifier of the ST.25 sequence listing of the present application. Preferred nucleic acid constructs comprising coding sequences of **Table 15,** e.g. mRNA sequences comprising the coding sequences of **Table 15** are provided in **Table 16.**

**Table 15: Preferred PIV constructs (amino acid sequences and nucleic acid coding sequences):**

| **A** | **B** | **D** | **E** |
|---|---|---|---|
| HPIV3/Homo sapiens/PER/FLA4571/2008 fusion glycoprotein (F0) | 14612 | 14614, 26960 | 14616 |
| HPIV3/BJ/001/08 hemagglutinin-neuraminidase (HN) | 14613 | 14615 | 14617 |
| HPIV3/USA/629-D01959/2007 fusion glycoprotein (F0) | 26956 | 26958 | |
| HPIV3/USA/629-D01959/2007 fusion glycoprotein (variant) | 26957 | 26959 | |

In preferred embodiments, the at least one antigenic peptide or protein selected or derived from at least one PIV encoded by the at least one nucleic acid of component B (in particular component B-4a) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14612, 14613, 26956, 26957** or an immunogenic fragment or immunogenic variant of any of these. Further information regarding said amino acid sequences is also provided in **Table 15** (Column A and B), and under <223> identifier of the ST.25 sequence listing of respective sequence SEQ ID NOs.

In further embodiments, the at least one antigenic peptide or protein selected or derived from PIV encoded by the at least one nucleic acid of component B (in particular component B-4a) comprises or consists of at least one of the amino acid sequences being identical or at least 50%, 60%, 70%, 80%, 85%, 86%, 87%, 88%, 89%, 90%, 91%, 92%, 93%, 94%, 95%, 96%, 97%, 98%, or 99% identical to any one of SEQ ID NOs: 13-14 of published PCT patent application WO2017070626 or an immunogenic fragment or immunogenic variant of any of these. Accordingly, SEQ ID NOs: 13-14 of WO2017070626, and the corresponding disclosure relating thereto are herewith incorporated by reference. Further PIV antigen may also be derived from or selected proteins identifiable by GenBank Accession Numbers provided in Table 7 of WO2017070626. The full content of Table 7 of WO2017070626 herewith incorporated by reference.

According to preferred embodiments, the nucleic acid of component B (in particular component B-4a) comprises at least one coding sequence encoding at least one antigenic peptide or protein derived from PIV as defined above, or fragments and variants thereof. In that context, any coding sequence encoding at least one PIV antigenic protein as defined herein, or fragments and variants thereof may be understood as suitable coding sequence of component B.

In preferred embodiments, the nucleic acid of component B (in particular component B-4a) comprise or consist of at least one coding sequence encoding at least one antigenic peptide or protein selected or derived from a PIV as defined herein, preferably encoding any one of **SEQ ID NOs: 14612, 14613, 26956, 26957;** SEQ ID NOs: 13-14 of WO2017070626, or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14612, 14613, 26956, 26957;** SEQ ID NOs: 13-14 of WO2017070626, or fragments or variants thereof, may be selected and may accordingly be understood as suitable coding sequence of the invention. Further information regarding said amino acid sequences is also provided in **Table 15** (Column A and B), **Table 16,** and under <223> identifier of the ST.25 sequence listing of respective sequence SEQ ID NOs.

In preferred embodiments, the at least one coding sequence of the at least one nucleic acid of component B (in particular component B-4a) comprises or consists of a nucleic acid sequence encoding an PIV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14614-14625, 26958-26965** or a fragment or variant of any of these sequences. Further information regarding said nucleic acid sequences is also provided in **Table 15, Table 16,** and under <223> identifier of the ST.25 sequence listing of respective sequence SEQ ID NOs.

In preferred embodiments, the nucleic acid of component B (in particular component B-4a) comprises a coding sequence that comprises at least one of the nucleic acid sequences encoding a hPIV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from SEQ ID NOs: 9-12, 61-64 of published PCT patent application WO2017070626 or a fragment or a fragment or variant of any of these sequences. Accordingly, SEQ ID NOs: 9-12, 61-64 of WO2017070626, and the corresponding disclosure relating thereto, are herewith incorporated by reference.

In preferred embodiments, the at least one coding sequence of the nucleic acid of component B (in particular component B-4a) is a codon modified coding sequence as defined herein, wherein the amino acid sequence, that is the PIV peptide or protein, encoded by the at least one codon modified coding sequence, is preferably not being modified compared to the amino acid sequence encoded by the corresponding wild type or reference coding sequence.

In particularly preferred embodiments, the at least one coding sequence of the nucleic acid component B (in particular component B-4a) is a codon modified coding sequence, wherein the codon modified coding sequence is selected a G/C optimized coding sequence, a human codon usage adapted coding sequence, or a G/C modified coding sequence.

Preferred nucleic acid sequences of component B (in particular component B-4a), including particularly preferred mRNA sequences, are provided in **Table 16** (column C and D). Therein, each row represents a specific suitable PIV construct of the invention (compare with **Table 15**), wherein the description of the PIV construct is indicated in column A of **Table 16** and the SEQ ID NOs of the amino acid sequence of the respective PIV construct is provided in column B. The corresponding SEQ ID NOs of the coding sequences encoding the respective PIV constructs are provided in in **Table 15.** Further information is provided under <223> identifier of the respective SEQ ID NOs in the sequence listing.

**Table 16: Nucleic acid, preferably mRNA constructs encoding PIV antigens**

| **A** | **B** | **C** | **D** |
|---|---|---|---|
| HPIV3/Homo sapiens/PER/FLA4571/2008 fusion glycoprotein (F0) | 14612 | 14618, 14620 | 14622, 14624 |
| HPIV3/BJ/001/08 hemagglutinin-neuraminidase (HN) | 14613 | 14619, 14621 | 14623, 14625 |
| HPIV3/USA/629-DO1959/2007 fusion glycoprotein (F0) | 26956 | 26961 | 26963 |
| HPIV3/USA/629-D01959/2007 fusion glycoprotein (variant) | 26957 | 26962 | 26964 |

In preferred embodiments, the at least one nucleic acid of component B (in particular component B-4a) comprises or consists of a nucleic acid sequence encoding an PIV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of **SEQ ID NOs: 14618-14625, 26961-26965** or a fragment or variant of any of these sequences. Further information regarding respective nucleic acid sequences is provided under <223> identifier of the respective SEQ ID NO in the sequence listing and in **Table 16** (Column C and D). Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In further embodiments, the nucleic acid of component B (in particular component B-4a), preferably the RNA, comprises or consists of a nucleic acid sequence encoding a hPIV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from SEQ ID NOs: 61-64 of WO2017070626 or a fragment or variant of any of these sequences. Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In embodiments, the component B (in particular component B-4a) comprises a plurality or at least more than one of the nucleic acid species comprising at least one coding sequence encoding at least one antigenic peptide or protein selected or derived from at least one hPIV, or an immunogenic fragment or immunogenic variant thereof, e.g. DNA or RNA species as defined herein. Preferably, component B (in particular component B-4) as defined herein comprises 2, 3, 4, 5, 6, 7, 8, 9, or 10 different nucleic acids each defined as defined herein.

In embodiments, component B (in particular component B-4a) may comprise at least 2, 3, 4, 5, 6, 7, 8, 9, 10 or even more different nucleic acid species, e.g. DNA or RNA, as defined herein, each encoding at least one antigenic peptide or protein derived from the same hPIV, or a fragment or variant thereof. Particularly, said (genetically) same hPIV expresses (essentially) the same repertoire of proteins or peptides, wherein all proteins or peptides have (essentially) the same amino acid sequence. Particularly, said (genetically) same hPIV expresses essentially the same proteins, peptides or polyproteins, wherein these protein, peptide or polyproteins preferably do not differ in their amino acid sequence(s).

In embodiments, the component B (in particular component B-4a) comprises at least 2, 3, 4, 5, 6, 7, 8, 9, 10 or even more different nucleic acid species, e.g. DNA or RNA, as defined herein, each encoding at least one peptide or protein derived from a genetically distinct hPIV (e.g. a distinct hPIV isolate), or a fragment or variant thereof. The terms "distinct" or "distinct hPIV" as used throughout the present specification have to be understood as the difference between at least two respective hPIV (e.g. a distinct hPIV isolate), wherein the difference is manifested on the genome of the respective distinct hPIV. Particularly, said (genetically) distinct hPIV may express at least one distinct protein, peptide or polyprotein, wherein the at least one distinct protein, peptide or polyprotein differs in at least one amino acid.

In preferred embodiments, the component B-4a comprises at least one nucleic acid encoding at least one antigenic peptide or protein that is selected or derived from at least one PIV, or an immunogenic fragment or immunogenic variant thereof, wherein said component B-4a is to be, preferably, administered intramuscularly or intradermal.

Preferably, intramuscular or intradermal administration of said component B-4a results in expression of the encoded PIV antigen construct in a subject. In embodiments where the nucleic acid is an RNA, administration of component B-4a results in translation of the RNA and to a production of the encoded PIV antigen in a subject. In embodiments where the nucleic acid is a DNA (e.g. plasmid DNA, adenovirus DNA), administration of said composition results in transcription of the DNA into RNA, and to a subsequent translation of the RNA into the encoded PIV antigen in a subject.

In preferred embodiments, administration of component B-4a elicits antigen-specific immune responses comprising T-cell responses and/or B-cell responses against the encoded PIV antigens provided by the at least one nucleic acid of component B-4a.

Preferably, the component B-4a is suitable for a vaccine, in particular, suitable for a PIV vaccine, preferably a combination vaccine of the invention.

Preferably, the component B-4a is suitable for a vaccine, in particular, suitable for a RSV vaccine, preferably a combination vaccine of the invention.

In embodiments, the nucleic acid as comprised in component B-4a as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-4a comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Component B-4b: Henipavirus:

In embodiments, the at least one further virus of component B (in particular, component B-4b) is selected from at least one Henipavirus of the Paramyxoviridae virus family.

The term "Henipavirus" relates to any virus of the Henipavirus genus, irrespective of genotype, species, strain, isolate, or serotype (NCBI Taxonomy ID: 260964). The term Henipavirus relates to a virus genus comprising virus strains selected from but not limited to Cedar henipavirus or Cedar virus (NCBI Taxonomy ID: 1221391), Ghanaian bat henipavirus or Bat paramyxovirus (NCBI Taxonomy ID: 665603), Mojiang henipavirus or Mojiang virus (NCBI Taxonomy ID: 1474807), Hendra virus (NCBI Taxonomy ID: 928303), Nipah virus (NCBI Taxonomy ID: 121791).

In preferred embodiments, the at least one Henipavirus is selected from at least one Hendra virus and/or at least one Nipah virus.

In embodiments, the at least one antigenic peptide or protein encoded by the at least one nucleic acid of component B (in particular, component B-4b) comprises or consists at least one peptide or protein selected or derived from at least one Henipavirus RNA-directed RNA polymerase (L), Henipavirus fusion protein (F), Henipavirus non-structural protein (V), Henipavirus glycoprotein (G), Henipavirus nucleoprotein (N), Henipavirus matrix protein (M), Henipavirus phosphoprotein (P), Henipavirus protein C, and Henipavirus protein W.

In preferred embodiments, the at least one antigenic peptide or protein encoded by the at least one nucleic acid of component B (in particular, component B-4b) comprises or consists at least one peptide or protein selected or derived from at least one Henipavirus glycoprotein and/or at least one Henipavirus fusion protein or a fragment or variant thereof.

In preferred embodiments, the at least one antigenic peptide or protein selected or derived from at least one Henipavirus encoded by the at least one nucleic acid of component B (in particular, component B-4b) comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-26, 573-598, 807-832, 1041-1066, 1513-1515 of published PCT patent application WO2018115507, or an immunogenic fragment or immunogenic variant of any of these. Notably, SEQ ID NOs: 1-26, 573-598, 807-832, 1041-1066, 1513-1515 of published PCT patent application WO2018115507, and the corresponding disclosure relating thereto (e.g. Table 1, Table 1B, Table 2, Table 2B, or claims 1-43 of WO2018115507) are herewith incorporated by reference.

According to preferred embodiments, the at least one nucleic acid of component B (in particular component B-4b) comprises or consists of at least one coding sequence encoding at least one antigenic peptide or protein derived from a Henipavirus as defined above, in particular Hendra virus or Nipah virus, or fragments and variants thereof. In that context, any coding sequence encoding at least one Henipavirus antigenic protein as defined herein, or fragments and variants thereof, may be understood as suitable coding sequence of component B.

In preferred embodiments, the at least one nucleic acid of component B (in particular component B-4b) comprises or consists of at least one coding sequence encoding at least one antigenic peptide or protein selected or derived from a Henipavirus as defined herein, in particular Hendra virus or Nipah virus, preferably encoding any one of SEQ ID NOs: 1-26, 573-598, 807-832, 1041-1066, 1513-1515 of published PCT patent application WO2018115507, or fragments of variants thereof. It has to be understood that, on nucleic acid level, any sequence (DNA or RNA sequence) which encodes an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 1-26, 573-598, 807-832, 1041-1066, 1513-1515 of published PCT patent application WO2018115507, or fragments or variants thereof, may be selected and may accordingly be understood as suitable coding sequence of the invention.

In preferred embodiments, the at least one coding sequence of the at least one nucleic acid of component B (in particular, component B-4b) comprises or consists of a nucleic acid sequence encoding an Henipavirus antigen, preferably a Hendra virus or Nipah virus antigen, comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one SEQ ID NOs: 27-234, 599-806, 833-1040, 1067-1274, 1275-1508, 1516-1539, 1540-1548 of published PCT patent application WO2018115507, or a fragment or variant of any of these sequences. Notably, SEQ ID NOs: 27-234, 599-806, 833-1040, 1067-1274, 1275-1508, 1516-1539, 1540-1548 of published PCT patent application WO2018115507, and the corresponding disclosure relating thereto (e.g. Table 5, 6, 7 or claims 1-43 of WO2018115507) are herewith incorporated by reference.

In preferred embodiments, the at least one coding sequence of the nucleic acid of component B (in particular component B-4b) is a codon modified coding sequence as defined herein, wherein the amino acid sequence, that is the Henipavirus peptide or protein, encoded by the at least one codon modified coding sequence, is preferably not being modified compared to the amino acid sequence encoded by the corresponding wild type or reference coding sequence.

In particularly preferred embodiments, the at least one coding sequence of the nucleic acid component B (in particular component B-4b) is a codon modified coding sequence encoding an Henipavirus antigen, wherein the codon modified coding sequence is suitably selected from a G/C optimized coding sequence, a human codon usage adapted coding sequence, or a G/C modified coding sequence.

In preferred embodiments, at least one nucleic acid of component B (in particular component B-4b) comprises or consists of a nucleic acid sequence encoding an Henipavirus antigen, preferably a Hendra virus or Nipah virus antigen, which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 1275-1508, 1540-1548 of published PCT patent application WO2018115507, or a fragment or variant of any of these sequences. Notably, SEQ ID NOs: 1275-1508, 1540-1548 of published PCT patent application WO2018115507, and the corresponding disclosure relating thereto (e.g. Table 5, 6, 7, or claims 1-43 of WO2018115507) are herewith incorporated by reference. Optionally, said nucleic acid sequences comprise a cap1 structure as defined herein, and/or at least one, preferably all uracil nucleotides in said RNA sequences are replaced by pseudouridine (ψ) nucleotides and/or N1-methylpseudouridine (m1ψ) nucleotides

In embodiments, component B (in particular component B-4b) comprises a plurality or at least more than one of the nucleic acid species comprising at least one coding sequence encoding at least one antigenic peptide or protein selected or derived from at least one Henipavirus, preferably a Hendra virus or Nipah virus, or an immunogenic fragment or immunogenic variant thereof, e.g. DNA or RNA species as defined herein. Preferably, component B (in particular component B-4b) as defined herein comprises 2, 3, 4, 5, 6, 7, 8, 9, or 10 different nucleic acids each as defined herein.

In embodiments, component B (in particular component B-4b) may comprise at least 2, 3, 4, 5, 6, 7, 8, 9, 10 or even more different nucleic acid species, e.g. DNA or RNA, as defined herein, each encoding at least one antigenic peptide or protein derived from the same Henipavirus, or a fragment or variant thereof. Particularly, said (genetically) same Henipavirus expresses (essentially) the same repertoire of proteins or peptides, wherein all proteins or peptides have (essentially) the same amino acid sequence. Particularly, said (genetically) same Henipavirus expresses essentially the same proteins, peptides or polyproteins, wherein these protein, peptide or polyproteins preferably do not differ in their amino acid sequence(s).

In embodiments, component B (in particular component B-4b) comprises at least 2, 3, 4, 5, 6, 7, 8, 9, 10 or even more different nucleic acid species, e.g. DNA or RNA, as defined herein, each encoding at least one peptide or protein derived from a genetically distinct Henipavirus (e.g. a distinct Henipavirus strain or isolate), or a fragment or variant thereof. The terms "distinct" or "distinct Henipavirus" as used throughout the present specification have to be understood as the difference between at least two respective Henipavirus (e.g. a distinct Henipavirus strain or isolate isolate), wherein the difference is manifested on the genome of the respective distinct Henipavirus. Particularly, said (genetically) distinct Henipavirus may express at least one distinct protein, peptide or polyprotein, wherein the at least one distinct protein, peptide or polyprotein differs in at least one amino acid.

In preferred embodiments, the component B-4b comprises at least one nucleic acid encoding at least one antigenic peptide or protein that is selected or derived from at least one Henipavirus, or an immunogenic fragment or immunogenic variant thereof, wherein said component B-4b is to be, preferably, administered intramuscularly or intradermal.

Preferably, intramuscular or intradermal administration of said component B-4b results in expression of the encoded Henipavirus antigen construct in a subject. In embodiments where the nucleic acid is an RNA, administration of component B-4b results in translation of the RNA and to a production of the encoded Henipavirus antigen in a subject. In embodiments where the nucleic acid is a DNA (e.g. plasmid DNA, adenovirus DNA), administration of said composition results in transcription of the DNA into RNA, and to a subsequent translation of the RNA into the encoded Henipavirus antigen in a subject.

In preferred embodiments, administration of component B-4b elicits antigen-specific immune responses comprising T-cell responses and/or B-cell responses against the encoded Henipavirus antigens provided by the at least one nucleic acid of component B-4b.

Preferably, the component B-4b is suitable for a vaccine, in particular, suitable for a Henipavirus vaccine, preferably a combination vaccine of the invention.

In embodiments, the nucleic acid as comprised in component B-4b as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In case component B-4b comprises a plurality or at least more than one of the nucleic acid species as defined herein, the amount of nucleic acid for each nucleic acid species is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug, 65ug, 70ug, 75ug, 80ug, 85ug, 90ug, 95ug or 100ug.

In some embodiments, the amount of nucleic acid for each nucleic acid species is essentially equal in mass. In other embodiments, the amount of nucleic acid for each nucleic acid species is selected to be equimolar.

### Nucleic acid features and embodiments:

In the following, suitable features and embodiments relating to the nucleic acid comprised in the pharmaceutical composition are further specified. In particular, suitable features and embodiments relating to nucleic acid of component A (in particular, component A-1, A-2, A-3), and/or component B (in particular component B-1, B-2, B-3, B-3a, B-3b, B-4, B-4a, B-4b) are further specified.

Accordingly, features and embodiments provided in the following may be read as suitable features and embodiments of (i) the at least one nucleic acid comprising at least one coding sequence encoding at least one antigenic peptide or protein selected or derived from at least one Coronavirus, or an immunogenic fragment or immunogenic variant thereof (component A, in particular, component A-1, A-2, A-3). Features and embodiments provided in the following may be read as suitable features and embodiments of (ii) the at least one nucleic acid comprising at least one coding sequence encoding at least one antigenic peptide or protein selected or derived from at least one further virus, or an immunogenic fragment or immunogenic variant thereof (component B, in particular component B-1, B-2, B-3, B-3a, B-3b, B-4, B-4a, B-4b).

In preferred embodiments, the nucleic acid of component A and/or component B is an artificial nucleic acid, e.g. an artificial DNA or an artificial RNA.

The term "artificial nucleic acid" as used herein is intended to refer to a nucleic acid that does not occur naturally. In other words, an artificial nucleic acid may be understood as a non-natural nucleic acid molecule. Such nucleic acid molecules may be non-natural due to its individual sequence (e.g. G/C content modified coding sequence, UTRs) and/or due to other modifications, e.g. structural modifications of nucleotides. Typically, artificial nucleic acid may be designed and/or generated by genetic engineering to correspond to a desired artificial sequence of nucleotides. In this context, an artificial nucleic acid is a sequence that may not occur naturally, i.e. a sequence that differs from the wild type or reference sequence/the naturally occurring sequence by at least one nucleotide (via e.g. codon modification as further specified below). The term "artificial nucleic acid" is not restricted to mean "one single molecule" but is understood to comprise an ensemble of essentially identical nucleic acid molecules. Accordingly, it may relate to a plurality of essentially identical nucleic acid molecules. The term "artificial nucleic acid" as used herein may for example relate to an artificial DNA or, preferably, to an artificial RNA.

In preferred embodiments, the nucleic acid of component A and/or component B, e.g. the DNA or RNA, is a modified and/or stabilized nucleic acid, preferably a modified and/or stabilized artificial nucleic acid.

According to preferred embodiments, the nucleic acid of component A and/or component B may thus be provided as a "stabilized artificial nucleic acid" or "stabilized coding nucleic acid" that is to say a nucleic acid showing improved resistance to in vivo degradation and/or a nucleic acid showing improved stability in vivo, and/or a nucleic acid showing improved translatability in vivo. In the following, specific suitable modifications/adaptations in this context are described which are suitably to "stabilize" the nucleic acid. Preferably, the nucleic acid of the present invention may be provided as a "stabilized RNA", "stabilized coding RNA", "stabilized DNA" or "stabilized coding DNA".

In the following, suitable modifications are described that are capable of "stabilizing" the nucleic acid of component A and/or component B.

In preferred embodiments, the nucleic acid of component A and/or component B, e.g. the RNA or DNA, comprises at least one codon modified coding sequence.

In preferred embodiments, the at least one coding sequence of the nucleic acid of component A and/or component B is a codon modified coding sequence. Suitably, the amino acid sequence encoded by the at least one codon modified coding sequence is not being modified compared to the amino acid sequence encoded by the corresponding wild type or reference coding sequence.

The term "codon modified coding sequence" relates to coding sequences that differ in at least one codon (triplets of nucleotides coding for one amino acid) compared to the corresponding wild type or reference coding sequence. Suitably, a codon modified coding sequence in the context of the invention may show improved resistance to in vivo degradation and/or improved stability in vivo, and/or improved translatability in vivo. Codon modifications in the broadest sense make use of the degeneracy of the genetic code wherein multiple codons may encode the same amino acid and may be used interchangeably (cf. Table 1 of WO2020002525) to optimize/modify the coding sequence for in vivo applications as outlined above.

In preferred embodiments, the at least one coding sequence of the nucleic acid of component A and/or component B is a codon modified coding sequence, wherein the codon modified coding sequence is selected from C maximized coding sequence, CAI maximized coding sequence, human codon usage adapted coding sequence, G/C content modified coding sequence, and G/C optimized coding sequence, or any combination thereof.

In preferred embodiments, the at least one coding sequence of the nucleic acid of component A and/or component B has a G/C content of at least about 50%, 55%, or 60%. In particular embodiments, the at least one coding sequence of the nucleic acid of component A and/or component B has a G/C content of at least about 50%, 51%, 52%, 53%, 54%, 55%, 56%, 57%, 58%, 59%, 60%, 61%, 62%, 63%, 64%, 65%, 66%, 67%, 68%, 69%, or 70%.

When transfected into mammalian host cells, the nucleic acid of component A and/or component B comprising a codon modified coding sequence has a stability of between 12-18 hours, or greater than 18 hours, e.g., 24, 36, 48, 60, 72, or greater than 72 hours and are capable of being expressed by the mammalian host cell (e.g. a muscle cell).

When transfected into mammalian host cells, the nucleic acid of component A and/or component B comprising a codon modified coding sequence is translated into protein, wherein the amount of protein is at least comparable to, or preferably at least 10% more than, or at least 20% more than, or at least 30% more than, or at least 40% more than, or at least 50% more than, or at least 100% more than, or at least 200% or more than the amount of protein obtained by a naturally occurring or wild type or reference coding sequence transfected into mammalian host cells.

In embodiments, the nucleic acid of component A and/or component B may be modified, wherein the C content of the at least one coding sequence may be increased, preferably maximized, compared to the C content of the corresponding wild type or reference coding sequence (herein referred to as "C maximized coding sequence"). The amino acid sequence encoded by the C maximized coding sequence of the nucleic acid is preferably not modified compared to the amino acid sequence encoded by the respective wild type or reference coding sequence. The generation of a C maximized nucleic acid sequences may suitably be carried out using a modification method according to WO2015/062738. In this context, the disclosure of WO2015/062738 is included herewith by reference.

In preferred embodiments, the nucleic acid of component A and/or component B may be modified, wherein the G/C content of the at least one coding sequence may be optimized compared to the G/C content of the corresponding wild type or reference coding sequence (herein referred to as "G/C content optimized coding sequence"). "Optimized" in that context refers to a coding sequence wherein the G/C content is preferably increased to the essentially highest possible G/C content. The amino acid sequence encoded by the G/C content optimized coding sequence of the nucleic acid is preferably not modified as compared to the amino acid sequence encoded by the respective wild type or reference coding sequence. The generation of a G/C content optimized nucleic acid sequence (RNA or DNA) may be carried out using a method according to WO2002/098443. In this context, the disclosure of WO2002/098443 is included in its full scope in the present invention. Throughout the description, including the <223> identifier of the sequence listing, G/C optimized coding sequences are indicated by the abbreviations "opt1" or "gc".

In preferred embodiments, the nucleic acid of component A and/or component B may be modified, wherein the codons in the at least one coding sequence may be adapted to human codon usage (herein referred to as "human codon usage adapted coding sequence"). Codons encoding the same amino acid occur at different frequencies in humans. Accordingly, the coding sequence of the nucleic acid is preferably modified such that the frequency of the codons encoding the same amino acid corresponds to the naturally occurring frequency of that codon according to the human codon usage. For example, in the case of the amino acid Ala, the wild type or reference coding sequence is preferably adapted in a way that the codon "GCC" is used with a frequency of 0.40, the codon "GCT" is used with a frequency of 0.28, the codon "GCA" is used with a frequency of 0.22 and the codon "GCG" is used with a frequency of 0.10 etc. (see e.g. Table 1 of WO2020002525). Accordingly, such a procedure (as exemplified for Ala) is applied for each amino acid encoded by the coding sequence of the nucleic acid to obtain sequences adapted to human codon usage. Throughout the description, including the <223> identifier of the sequence listing, human codon usage adapted coding sequences are indicated by the abbreviation "opt3" or "human".

In embodiments, the nucleic acid of component A and/or component B may be modified, wherein the G/C content of the at least one coding sequence may be modified compared to the G/C content of the corresponding wild type or reference coding sequence (herein referred to as "G/C content modified coding sequence"). In this context, the terms "G/C optimization" or "G/C content modification" relate to a nucleic acid that comprises a modified, preferably an increased number of guanosine and/or cytosine nucleotides as compared to the corresponding wild type or reference coding sequence. Such an increased number may be generated by substitution of codons containing adenosine or thymidine nucleotides by codons containing guanosine or cytosine nucleotides. Advantageously, nucleic acid sequences having an increased G /C content are more stable or show a better expression than sequences having an increased A/U. The amino acid sequence encoded by the G/C content modified coding sequence of the nucleic acid of component A and/or component B is preferably not modified as compared to the amino acid sequence encoded by the respective wild type or reference sequence. Preferably, the G/C content of the coding sequence of the nucleic acid is increased by at least 10%, 20%, 30%, preferably by at least 40% compared to the G/C content of the coding sequence of the corresponding wild type or reference nucleic acid sequence (herein referred to "opt 10" or "gc mod")

In embodiments, the nucleic acid of component A and/or component B may be modified, wherein the codon adaptation index (CAI) may be increased or preferably maximised in the at least one coding sequence (herein referred to as "CAI maximized coding sequence"). It is preferred that all codons of the wild type or reference nucleic acid sequence that are relatively rare in e.g. a human are exchanged for a respective codon that is frequent in the e.g. a human, wherein the frequent codon encodes the same amino acid as the relatively rare codon. Suitably, the most frequent codons are used for each amino acid of the encoded protein (see Table 1 of WO2020002525, most frequent human codons are marked with asterisks). Suitably, the nucleic acid comprises at least one coding sequence, wherein the codon adaptation index (CAI) of the at least one coding sequence is at least 0.5, at least 0.8, at least 0.9 or at least 0.95. Most preferably, the codon adaptation index (CAI) of the at least one coding sequence is 1 (CAI=1). For example, in the case of the amino acid Ala, the wild type or reference coding sequence may be adapted in a way that the most frequent human codon "GCC" is always used for said amino acid. Accordingly, such a procedure (as exemplified for Ala) may be applied for each amino acid encoded by the coding sequence of the nucleic acid to obtain CAI maximized coding sequences.

In embodiments, the nucleic acid of component A and/or component B may be modified by altering the number of A and/or U nucleotides in the nucleic acid sequence with respect to the number of A and/or U nucleotides in the original nucleic acid sequence (e.g. the wild type or reference sequence). Preferably, such an AU alteration is performed to modify the retention time of the individual nucleic acids in a composition, to (i) allow co-purification using a HPLC method, and/or to allow analysis of the obtained nucleic acid composition. Such a method is described in detail in published PCT application WO2019092153A1. Claims 1 to 70 of WO2019092153A1 herewith incorporated by reference.

In particularly preferred embodiments, the at least one coding sequence of the nucleic acid of component A and/or component B is a codon modified coding sequence, wherein the codon modified coding sequence is selected a G/C optimized coding sequence, a human codon usage adapted coding sequence, or a G/C modified coding sequence

In preferred embodiments, the nucleic acid of component A and/or component B comprises at least one heterologous untranslated region (UTR).

The term "untranslated region" or "UTR" or "UTR element" will be recognized and understood by the person of ordinary skill in the art, and are e.g. intended to refer to a part of a nucleic acid molecule typically located 5' or 3' of a coding sequence. An UTR is not translated into protein. An UTR may be part of a nucleic acid, e.g. a DNA or an RNA. An UTR may comprise elements for controlling gene expression, also called regulatory elements. Such regulatory elements may be, e.g., ribosomal binding sites, miRNA binding sites, promotor elements etc.

In preferred embodiments, the nucleic acid of component A and/or component B comprises a protein-coding region ("coding sequence" or "cds"), and 5'-UTR and/or 3'-UTR. Notably, UTRs may harbor regulatory sequence elements that determine nucleic acid, e.g. RNA turnover, stability, and localization. Moreover, UTRs may harbor sequence elements that enhance translation. In medical application of nucleic acid sequences (including DNA and RNA), translation of the nucleic acid into at least one peptide or protein is of paramount importance to therapeutic efficacy. Certain combinations of 3'-UTRs and/or 5'-UTRs may enhance the expression of operably linked coding sequences encoding peptides or proteins of the invention. Nucleic acid molecules harboring said UTR combinations advantageously enable rapid and transient expression of antigenic peptides or proteins after administration to a subject, preferably after intramuscular administration. Accordingly, the nucleic acid of component A and/or component B comprising certain combinations of 3'-UTRs and/or 5'-UTRs as provided herein is particularly suitable for administration as a vaccine, in particular, suitable for administration into the muscle, the dermis, or the epidermis of a subject.

Suitably, the nucleic acid of component A and/or component B comprises at least one heterologous 5'-UTR and/or at least one heterologous 3'-UTR. Said heterologous 5'-UTRs or 3'-UTRs may be derived from naturally occurring genes or may be synthetically engineered. In preferred embodiments, the nucleic acid, preferably the RNA comprises at least one coding sequence as defined herein operably linked to at least one (heterologous) 3'-UTR and/or at least one (heterologous) 5'-UTR.

In preferred embodiments, the nucleic acid of component A and/or component B, e.g. the RNA or DNA, comprises at least one heterologous 3'-UTR.

The term "3'-untranslated region" or "3'-UTR" or "3'-UTR element" will be recognized and understood by the person of ordinary skill in the art, and are e.g. intended to refer to a part of a nucleic acid molecule located 3' (i.e. downstream) of a coding sequence and which is not translated into protein. A 3'-UTR may be part of a nucleic acid, e.g. a DNA or an RNA, located between a coding sequence and an (optional) terminal poly(A) sequence. A 3'-UTR may comprise elements for controlling gene expression, also called regulatory elements. Such regulatory elements may be, e.g., ribosomal binding sites, miRNA binding sites etc.

Preferably, the nucleic acid of component A and/or component B comprises a 3'-UTR, which may be derivable from a gene that relates to an RNA with enhanced half-life (i.e. that provides a stable RNA).

In some embodiments, a 3'-UTR comprises one or more of a polyadenylation signal, a binding site for proteins that affect a nucleic acid stability of location in a cell, or one or more miRNA or binding sites for miRNAs.

MicroRNAs (or miRNA) are 19-25 nucleotide long noncoding RNAs that bind to the 3'-UTR of nucleic acid molecules and down-regulate gene expression either by reducing nucleic acid molecule stability or by inhibiting translation. E.g., microRNAs are known to regulate RNA, and thereby protein expression, e.g. in liver (miR-122), heart (miR-ld, miR-149), endothelial cells (miR-17-92, miR-126), adipose tissue (let-7, miR-30c), kidney (miR-192, miR-194, miR-204), myeloid cells (miR-142-3p, miR-142-5p, miR-16, miR-21, miR-223, miR-24, miR-27), muscle (miR-133, miR-206, miR-208), and lung epithelial cells (let-7, miR-133, miR-126). The RNA may comprise one or more microRNA target sequences, microRNA sequences, or microRNA seeds. Such sequences may e.g. correspond to any known microRNA such as those taught in US20050261218 and US20050059005.

Accordingly, miRNA, or binding sites for miRNAs as defined above may be removed from the 3'-UTR or may be introduced into the 3'-UTR in order to tailor the expression of the nucleic acid, e.g. the DNA or RNA to desired cell types or tissues (e.g. muscle cells).

In preferred embodiments, the nucleic acid of component A and/or component B comprises at least one heterologous 3'-UTR, wherein the at least one heterologous 3'-UTR comprises a nucleic acid sequence is derived or selected from a 3'-UTR of a gene selected from PSMB3, ALB7, alpha-globin (referred to as "muag"), CASP1, COX6B1, GNAS, NDUFA1 and RPS9, or from a homolog, a fragment or variant of any one of these genes, preferably according to nucleic acid sequences being identical or at least 70%, 80%, 85%, 86%, 87%, 88%, 89%, 90%, 91%, 92%, 93%, 94%, 95%, 96%, 97%, 98%, or 99% identical to **SEQ ID NOs: 253-268, 22902-22905, 22892-22895** or a fragment or a variant of any of these. Particularly preferred nucleic acid sequences in that context can be derived from published PCT application WO2019077001A1, in particular, claim 9 of WO2019077001A1. The corresponding 3'-UTR sequences of claim 9 of WO2019077001A1 are herewith incorporated by reference (e.g., SEQ ID NOs: 23-34 of WO2019077001A1, or fragments or variants thereof).

In preferred embodiments, the nucleic acid of component A and/or component B comprises a 3'-UTR derived from an alpha-globin gene. Said 3'-UTR derived from a alpha-globin gene ("muag") may comprise or consist of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 267, 268, 22896-22901, 22906-22911** or a fragment or a variant thereof.

In further embodiments, the nucleic acid comprises a 3'-UTR derived from a RPS9 gene. Said 3'-UTR derived from a RPS9 gene may comprise or consist of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 263, 264, 22894, 22895, 22904, 22905** or a fragment or a variant thereof.

In other embodiments, the nucleic acid comprise a 3'-UTR which comprise or consist of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NO: 22876-22891** or a fragment or a variant thereof.

In preferred embodiments, the nucleic acid of component A and/or component B comprises a 3'-UTR derived from a PSMB3 gene. Said 3'-UTR derived from a PSMB3 gene may comprise or consist of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 253 or 254** or a fragment or a variant thereof.

In other embodiments, the nucleic acid of component A and/or component B may comprise a 3'-UTR as described in WO2016107877, the disclosure of WO2016107877 relating to 3'-UTR sequences herewith incorporated by reference. Suitable 3'-UTRs are SEQ ID NOs: 1-24 and SEQ ID NOs: 49-318 of WO2016107877, or fragments or variants of these sequences. In other embodiments, the nucleic acid comprises a 3'-UTR as described in WO2017036580, the disclosure of WO2017036580 relating to 3'-UTR sequences herewith incorporated by reference. Suitable 3'-UTRs are SEQ ID NOs: 152-204 of WO2017036580, or fragments or variants of these sequences. In other embodiments, the nucleic acid comprises a 3'-UTR as described in WO2016022914, the disclosure of WO2016022914 relating to 3'-UTR sequences herewith incorporated by reference. Particularly preferred 3'-UTRs are nucleic acid sequences according to SEQ ID NOs: 20-36 of WO2016022914, or fragments or variants of these sequences.

In preferred embodiments, the nucleic acid of component A and/or component B, e.g. the RNA or DNA, comprises at least one heterologous 5'-UTR.

The terms "5'-untranslated region" or "5'-UTR" or "5'-UTR element" will be recognized and understood by the person of ordinary skill in the art, and are e.g. intended to refer to a part of a nucleic acid molecule located 5' (i.e. "upstream") of a coding sequence and which is not translated into protein. A 5'-UTR may be part of a nucleic acid located 5' of the coding sequence. Typically, a 5'-UTR starts with the transcriptional start site and ends before the start codon of the coding sequence. A 5'-UTR may comprise elements for controlling gene expression, also called regulatory elements. Such regulatory elements may be, e.g., ribosomal binding sites, miRNA binding sites etc. The 5'-UTR may be post-transcriptionally modified, e.g. by enzymatic or post-transcriptional addition of a 5'-cap structure (e.g. for mRNA as defined below).

Preferably, the nucleic acid of component A and/or component B comprises a 5'-UTR, which may be derivable from a gene that relates to an RNA with enhanced half-life (i.e. that provides a stable RNA).

In some embodiments, a 5'-UTR comprises one or more of a binding site for proteins that affect an RNA stability or RNA location in a cell, or one or more miRNA or binding sites for miRNAs (as defined above).

Accordingly, miRNA or binding sites for miRNAs as defined above may be removed from the 5'-UTR or introduced into the 5'-UTR in order to tailor the expression of the nucleic acid to desired cell types or tissues (e.g. muscle cells).

In preferred embodiments, the nucleic acid of component A and/or component B comprises at least one heterologous 5'-UTR, wherein the at least one heterologous 5'-UTR comprises a nucleic acid sequence is derived or selected from a 5'-UTR of gene selected from HSD17B4, RPL32, ASAH1, ATP5A1, MP68, NDUFA4, NOSIP, RPL31, SLC7A3, TUBB4B, and UBQLN2, or from a homolog, a fragment or variant of any one of these genes according to nucleic acid sequences being identical or at least 70%, 80%, 85%, 86%, 87%, 88%, 89%, 90%, 91%, 92%, 93%, 94%, 95%, 96%, 97%, 98%, or 99% identical to **SEQ ID NOs: 231-252, 22870-22875** or a fragment or a variant of any of these. Particularly preferred nucleic acid sequences in that context can be selected from published PCT application WO2019077001A1, in particular, claim 9 of WO2019077001A1. The corresponding 5'-UTR sequences of claim 9 of WO2019077001A1 are herewith incorporated by reference (e.g., SEQ ID NOs: 1-20 of WO2019077001A1, or fragments or variants thereof).

In preferred embodiments, the nucleic acid of component A and/or component B comprises a 5'-UTR derived from a RPL31 gene, wherein said 5'-UTR derived from a RPL31 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 243, 244, 22872, 22873** or a fragment or a variant thereof.

In embodiments, the nucleic acid of component A and/or component B may comprise a 5'-UTR derived from a SLC7A3 gene, wherein said 5'-UTR derived from a SLC7A3 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 246, 246, 22874, 22875** or a fragment or a variant thereof.

In particularly preferred embodiments, the nucleic acid of component A and/or component B comprises a 5'-UTR derived or selected from a HSD17B4 gene, wherein said 5'-UTR derived from a HSD17B4 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 231,232, 22870, 22871** or a fragment or a variant thereof.

In other embodiments, the nucleic acid of component A and/or component B comprises a 5'-UTR which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NO: 22848-22869** or a fragment or a variant thereof.

In other embodiments, the nucleic acid of component A and/or component B may comprise a 5'-UTR as described in WO2013143700, the disclosure of WO2013143700 relating to 5'-UTR sequences herewith incorporated by reference. Particularly preferred 5'-UTRs are nucleic acid sequences derived from SEQ ID NOs: 1-1363, SEQ ID NO: 1395, SEQ ID NO: 1421 and SEQ ID NO: 1422 of WO2013143700, or fragments or variants of these sequences. In other embodiments, the nucleic acid comprises a 5'-UTR as described in WO2016107877, the disclosure of WO2016107877 relating to 5'-UTR sequences herewith incorporated by reference. Particularly preferred 5'-UTRs are nucleic acid sequences according to SEQ ID NOs: 25-30 and SEQ ID NOs: 319-382 of WO2016107877, or fragments or variants of these sequences. In other embodiments, the nucleic acid comprises a 5'-UTR as described in WO2017036580, the disclosure of WO2017036580 relating to 5'-UTR sequences herewith incorporated by reference. Particularly preferred 5'-UTRs are nucleic acid sequences according to SEQ ID NOs: 1-151 of WO2017036580, or fragments or variants of these sequences. In other embodiments, the nucleic acid comprises a 5'-UTR as described in WO2016022914, the disclosure of WO2016022914 relating to 5'-UTR sequences herewith incorporated by reference. Particularly preferred 5'-UTRs are nucleic acid sequences according to SEQ ID NOs: 3-19 of WO2016022914, or fragments or variants of these sequences.

In preferred embodiments, the nucleic acid of component A and/or component B comprises at least one coding sequence as specified herein encoding at least one antigenic protein as defined herein, operably linked to a 3'-UTR and/or a 5'-UTR selected from the following 5'UTR/3'UTR combinations ("also referred to UTR designs"):
a-1 (HSD17B4/PSMB3), a-2 (NDUFA4/PSMB3), a-3 (SLC7A3/PSMB3), a4 (NOSIP/PSMB3), a-5 (MP68/PSMB3), b-1 (UBQLN2/RPS9), b-2 (ASAH1/RPS9), b-3 (HSD17B4/RPS9), b-4 (HSD17B4/CASP1), b-5 (NOSIP/COX6B1), c-1 (NDUFA4/RPS9), c-2 (NOSIP/NDUFA1), c-3 (NDUFA4/COX6B1), c-4 (NOUFA4/NDUFA1), c-5 (ATP5A1/PSMB3), d-1 (Rpl31/PSMB3), d-2 (ATP5A1/CASP1), d-3 (SLC7A3/GNAS), d-4 (HSD17B4/NDUFA1), d-5 (Slc7a3/Ndufa1), e-1 (TUBB4B/RPS9), e-2 (RPL31/RPS9), e-3 (MP68/RPS9), e-4 (NOSIP/RPS9), e-5 (ATP5A1/RPS9), e-6 (ATP5A1/COX6B1), f-1 (ATP5A1/GNAS), f-2 (ATP5A1/NDUFA1), f-3 (HSD17B4/COX6B1), f-4 (HSD17B4/GNAS), f-5 (MP68/COX6B1), g-1 (MP68/NDUFA1), g-2 (NDUFA4/CASP1), g-3 (NDUFA4/GNAS), g-4 (NOSIP/CASP1), g-5 (RPL31/CASP1), h-1 (RPL31/COX6B1), h-2 (RPL31/GNAS), h-3 (RPL31/NDUFA1), h-4 (Sic7a3/CASP1), h-5 (SLC7A3/COX6B1), i-1 (SLC7A3/RPS9), i-2 (RPL32/ALB7), i-2 (RPL32/ALB7), or i-3 (alpha-globin gene).

In particularly preferred embodiments, the nucleic acid of component A and/or component B comprises at least one coding sequence as defined herein encoding at least one antigenic protein as defined herein, wherein said coding sequence is operably linked to a HSD17B4 5'-UTR and a PSMB3 3'-UTR (HSD17B4/PSMB3 (UTR design a-1)).

In further preferred embodiments, the nucleic acid of component A and/or component B comprises at least one coding sequence as specified herein encoding at least one antigenic protein as defined herein, preferably derived from SARS-CoV-2 (nCoV-2019) coronavirus, wherein said coding sequence is operably linked to a SLC7A3 5'-UTR and a PSMB3 3'-UTR (SLC7A3/PSMB3 (UTR design a-3)).

In further preferred embodiments, the nucleic acid of component A and/or component B comprises at least one coding sequence as specified herein encoding at least one antigenic protein as defined herein, preferably derived from SARS-CoV-2 (nCoV-2019) coronavirus, wherein said coding sequence is operably linked to a RPL31 5'-UTR and a RPS9 3'-UTR (RPL31/ RPS9 (UTR design e-2)).

In particularly preferred embodiments, the nucleic acid of component A and/or component B comprises at least one coding sequence as defined herein encoding at least one antigenic protein as defined herein, wherein said coding sequence is operably linked to an alpha-globin ("muag") 3'-UTR.

In embodiments, the nucleic acid of component A and/or component B, e.g. the DNA or RNA may be monocistronic, bicistronic, or multicistronic.

The term "monocistronic" will be recognized and understood by the person of ordinary skill in the art, and is e.g. intended to refer to a nucleic acid that comprises only one coding sequence. The terms "bicistronic", or "multicistronic" as used herein will be recognized and understood by the person of ordinary skill in the art, and are e.g. intended to refer to a nucleic acid that may comprise two (bicistronic) or more (multicistronic) coding sequences.

In preferred embodiments, the nucleic acid of component A and/or component B is monocistronic.

In embodiments, the nucleic acid of component A and/or component B is monocistronic and the coding sequence of said nucleic acid encodes at least two different antigenic peptides or proteins. Accordingly, said coding sequence may encode at least two, three, four, five, six, seven, eight and more antigenic peptides or proteins, linked with or without an amino acid linker sequence, wherein said linker sequence can comprise rigid linkers, flexible linkers, cleavable linkers, or a combination thereof. Such constructs are herein referred to as "multi-antigen-constructs".

In embodiments, the nucleic acid of component A and/or component B may be bicistronic or multicistronic and comprises at least two coding sequences, wherein the at least two coding sequences encode two or more different antigenic peptides or proteins as specified herein. Accordingly, the coding sequences in a bicistronic or multicistronic nucleic acid suitably encodes distinct antigenic proteins or peptides as defined herein or immunogenic fragments or immunogenic variants thereof. Preferably, the coding sequences in said bicistronic or multicistronic constructs may be separated by at least one IRES (internal ribosomal entry site) sequence. Thus, the term "encoding two or more antigenic peptides or proteins" may mean, without being limited thereto, that the bicistronic or multicistronic nucleic acid encodes e.g. at least two, three, four, five, six or more (preferably different) antigenic peptides or proteins of virus isolates. Alternatively, the bicistronic or multicistronic nucleic acid may encode e.g. at least two, three, four, five, six or more (preferably different) antigenic peptides or proteins derived from the same virus. In that context, suitable IRES sequences may be selected from the list of nucleic acid sequences according to SEQ ID NOs: 1566-1662 of the patent application WO2017081082, or fragments or variants of these sequences. In this context, the disclosure of WO2017081082 relating to IRES sequences is herewith incorporated by reference.

It has to be understood that, in the context of the invention, certain combinations of coding sequences may be generated by any combination of monocistronic, bicistronic and multicistronic DNA and/or RNA constructs and/or multi-antigen-constructs to obtain a nucleic acid set encoding multiple antigenic peptides or proteins as defined herein.

In embodiments, the A/U (A/T) content in the environment of the ribosome binding site of the nucleic acid of component A and/or component B may be increased compared to the A/U (A/T) content in the environment of the ribosome binding site of its respective wild type or reference nucleic acid. This modification (an increased A/U (A/T) content around the ribosome binding site) increases the efficiency of ribosome binding to the nucleic acid, e.g. to an RNA. An effective binding of the ribosomes to the ribosome binding site in tum has the effect of an efficient translation the nucleic acid.

Accordingly, in a particularly preferred embodiment, the nucleic acid of component A and/or component B comprises a ribosome binding site, also referred to as "Kozak sequence" identical to or at least 80%, 85%, 90%, 95% identical to any one of the sequences SEQ ID NOs: 180,181, 22845-22847 or fragments or variants thereof.

In preferred embodiments, the nucleic acid of component A and/or component B comprises at least one poly(N) sequence, e.g. at least one poly(A) sequence, at least one poly(U) sequence, at least one poly(C) sequence, or combinations thereof.

In preferred embodiments, the nucleic acid of component A and/or component B, preferably the RNA comprises at least one poly(A) sequence.

The terms "poly(A) sequence", "poly(A) tail" or "3'-poly(A) tail" as used herein will be recognized and understood by the person of ordinary skill in the art, and are e.g. intended to be a sequence of adenosine nucleotides, typically located at the 3'-end of a linear RNA (or in a circular RNA), of up to about 1000 adenosine nucleotides. Preferably, said poly(A) sequence is essentially homopolymeric, e.g. a poly(A) sequence of e.g. 100 adenosine nucleotides has essentially the length of 100 nucleotides. In other embodiments, the poly(A) sequence may be interrupted by at least one nucleotide different from an adenosine nucleotide, e.g. a poly(A) sequence of e.g. 100 adenosine nucleotides may have a length of more than 100 nucleotides (comprising 100 adenosine nucleotides and in addition said at least one nucleotide - or a stretch of nucleotides - different from an adenosine nucleotide). It has to be understood that "poly(A) sequence" as defined herein typically relates to RNA - however in the context of the invention, the term likewise relates to corresponding sequences in a DNA molecule (e.g. a "poly(T) sequence").

The poly(A) sequence may comprise about 10 to about 500 adenosine nucleotides, about 10 to about 200 adenosine nucleotides, about 40 to about 200 adenosine nucleotides, or about 40 to about 150 adenosine nucleotides. Suitably, the length of the poly(A) sequence may be at least about or even more than about 10, 50, 64, 75, 100, 200, 300, 400, or 500 adenosine nucleotides.

In preferred embodiments, the nucleic acid of component A and/or component B comprises at least one poly(A) sequence comprising about 30 to about 200 adenosine nucleotides. In particularly preferred embodiments, the poly(A) sequence comprises about 64 adenosine nucleotides (A64). In other particularly preferred embodiments, the poly(A) sequence comprises about 100 adenosine nucleotides (A100). In other embodiments, the poly(A) sequence comprises about 150 adenosine nucleotides.

In further embodiments, the nucleic acid of component A and/or component B comprises at least one poly(A) sequence comprising about 100 adenosine nucleotides, wherein the poly(A) sequence is interrupted by non-adenosine nucleotides, preferably by 10 non-adenosine nucleotides (A30-N10-A70).

The poly(A) sequence as defined herein may be located directly at the 3' terminus of the nucleic acid of component A and/or component B, preferably directly located at the 3' terminus of an RNA. In preferred embodiments, the 3'-terminal nucleotide (that is the last 3'-terminal nucleotide in the polynucleotide chain) is the 3'-terminal A nucleotide of the at least one poly(A) sequence. The term "directly located at the 3' terminus" has to be understood as being located exactly at the 3' terminus - in other words, the 3' terminus of the nucleic acid consists of a poly(A) sequence terminating with an A nucleotide.

In a particularly preferred embodiment the nucleic acid sequence of component A and/or component B, preferably the RNA comprises a poly(A) sequence of at least 70 adenosine nucleotides, preferably consecutive at least 70 adenosine nucleotides, wherein the 3'-terminal nucleotide is an adenosine nucleotide.

In this context it has been shown that ending on an adenosine nucleotide decreases the induction of IFNalpha by the RNA vaccine. This is particularly important as the induction of IFNalpha is thought to be the main factor for induction of fever in vaccinated subjects, which of course has to be avoided.

In embodiments where the nucleic acid of component A and/or component B is an RNA, the poly(A) sequence of the nucleic acid is preferably obtained from a DNA template during RNA in vitro transcription. In other embodiments, the poly(A) sequence is obtained in vitro by common methods of chemical synthesis without being necessarily transcribed from a DNA template. In other embodiments, poly(A) sequences are generated by enzymatic polyadenylation of the RNA (after RNA *in vitro* transcription) using commercially available polyadenylation kits and corresponding protocols known in the art, or alternatively, by using immobilized poly(A)polymerases e.g. using a methods and means as described in WO2016174271.

The nucleic acid of component A and/or component B may comprise a poly(A) sequence obtained by enzymatic polyadenylation, wherein the majority of nucleic acid molecules comprise about 100 (+/-20) to about 500 (+/-50), preferably about 250 (+/-20) adenosine nucleotides.

In embodiments, the nucleic acid of component A and/or component B comprises a poly(A) sequence derived from a template DNA and, optionally, additionally comprises at least one additional poly(A) sequence generated by enzymatic polyadenylation, e.g. as described in WO2016091391.

In embodiments, the nucleic acid of component A and/or component B comprises at least one polyadenylation signal.

In embodiments, the nucleic acid of component A and/or component B comprises at least one poly(C) sequence.

The term "poly(C) sequence" as used herein is intended to be a sequence of cytosine nucleotides of up to about 200 cytosine nucleotides. In preferred embodiments, the poly(C) sequence comprises about 10 to about 200 cytosine nucleotides, about 10 to about 100 cytosine nucleotides, about 20 to about 70 cytosine nucleotides, about 20 to about 60 cytosine nucleotides, or about 10 to about 40 cytosine nucleotides. In a particularly preferred embodiment, the poly(C) sequence comprises about 30 cytosine nucleotides.

In preferred embodiments, the nucleic acid of component A and/or component B comprises at least one histone stem-loop (hSL) or histone stem loop structure.

The term "histone stem-loop" (abbreviated as "hSL" in e.g. the sequence listing) is intended to refer to nucleic acid sequences that form a stem-loop secondary structure predominantly found in histone mRNAs.

Histone stem-loop sequences/structures may suitably be selected from histone stem-loop sequences as disclosed in WO2012019780, the disclosure relating to histone stem-loop sequences/histone stem-loop structures incorporated herewith by reference. A histone stem-loop sequence that may be used within the present invention may preferably be derived from formulae (I) or (II) of WO2012019780. According to a further preferred embodiment, the nucleic acid of component A and/or component B comprises at least one histone stem-loop sequence derived from at least one of the specific formulae (la) or (IIa) of the patent application WO2012019780.

In preferred embodiments, the nucleic acid of component A and/or component B comprises at least one histone stem-loop, wherein said histone stem-loop (hSL) comprises or consists a nucleic acid sequence identical or at least 70%, 80%, 90%, 91%, 92%, 93%, 94%, 95%, 96%, 97%, 88%, or 99% identical to **SEQ ID NOs: 178 or 179,** or fragments or variants thereof.

In other embodiments, the nucleic acid of component A and/or component B does not comprise a hsL as defined herein.

In embodiments, in particular in embodiments that relate to RNA, the nucleic acid of component A and/or component B comprises a 3'-terminal sequence element. Said 3'-terminal sequence element comprises a poly(A) sequence and optionally a histone-stem-loop sequence. Accordingly, the nucleic acid of component A and/or component B comprises at least one 3'-terminal sequence element comprising or consisting of a nucleic acid sequence being identical or at least 70%, 80%, 90%, 91%, 92%, 93%, 94%, 95%, 96%, 97%, 98%, or 99% identical to **SEQ ID NOs: 182-230, 22912, 22913** or a fragment or variant thereof.

In preferred embodiments, in particular in embodiments that relate to RNA, the nucleic acid of component A and/or component B comprises a 3'-terminal sequence element. Said 3'-terminal sequence element comprises a poly(A) sequence and optionally a histone-stem-loop sequence. Accordingly, the nucleic acid of component A and/or component B may comprise at least one 3'-terminal sequence element comprising or consisting of a nucleic acid sequence being identical or at least 70%, 80%, 90%, 91%, 92%, 93%, 94%, 95%, 96%, 97%, 98%, or 99% identical to **SEQ ID NOs: 182, 187, 189, 192, 199, 207,** or a fragment or variant thereof.

In various embodiments, in particular in embodiments that relate to RNA, the nucleic acid of component A and/or component B may comprise a 5'-terminal sequence element according to **SEQ ID NOs: 176,177, 22840-22844,** or a fragment or variant thereof. Such a 5'-terminal sequence element comprises e.g. a binding site for T7 RNA polymerase. Further, the first nucleotide of said 5'-terminal start sequence may preferably comprise a 2'O methylation, e.g. 2'O methylated guanosine or a 2'O methylated adenosine.

Preferably, the nucleic acid of component A and/or component B, e.g. the RNA or DNA, typically comprises about 50 to about 20000 nucleotides, or about 500 to about 10000 nucleotides, or about 1000 to about 10000 nucleotides, or preferably about 1000 to about 5000 nucleotides, or even more preferably about 2000 to about 5000 nucleotides.

In embodiments, the nucleic acid of component A and/or component B is a DNA or an RNA.

In embodiments, the DNA is a plasmid DNA or a linear coding DNA construct, wherein the DNA comprises or consists of the nucleic acid elements as defined herein (e.g. including coding sequences, UTRs, poly(A/T), polyadenylation signal, a promoter).

In embodiments, the nucleic acid of component A and/or component B is a DNA expression vector. Such a DNA expression vector may be selected from the group consisting of a bacterial plasmid, an Adenovirus, a Poxvirus, a Parapoxivirus (orf virus), a Vaccinia virus, a Fowlpox virus, a Herpes virus, an Adeno-associated virus (AAV), an Alphavirus, a Lentivirus, a Lambda phage, a Lymphocytic choriomeningitis virus, a Listeria sp and Salmonella sp.

Suitably, the DNA may also comprise a promoter that is operably linked to the respective antigen coding sequence of component A and/or component B. The promoter operably linked to the antigen coding sequence can be e.g. a promoter from simian virus 40 (SV40), a mouse mammary tumor virus (MMTV) promoter, a human immunodeficiency virus (HIV) promoter such as the bovine immunodeficiency virus (BIV) long terminal repeat (LTR) promoter, a Moloney virus promoter, an avian leukosis virus (ALV) promoter, a cytomegalovirus (CMV) promoter such as the CMV immediate early promoter, Epstein Barr virus (EBV) promoter, or a Rous sarcoma virus (RSV) promoter. The promoter can also be a promoter from a human gene such as human actin, human myosin, human hemoglobin, human muscle creatine, or human metalothionein. The promoter can also be a tissue specific promoter, such as a muscle or skin specific promoter, natural or synthetic. Examples of such promoters are described in US patent application publication no. US20040175727. In preferred embodiments, the vector can be pVAX, pcDNA3.0, or provax, or any other expression vector capable of expressing DNA encoding the coronavirus antigen and enabling a cell to translate the sequence to an antigen that is recognized by the immune system.

Further suitable plasmid DNA may be generated to allow efficient production of the encoded antigens in cell lines, e.g. in insect cell lines, for example using vectors as described in WO2009150222A2 and as defined in PCT claims 1 to 33, the disclosure relating to claim 1 to 33 of WO2009150222A2 herewith incorporated by reference.

In embodiments, the nucleic acid of component A and/or component B is an adenovirus based vector. Such an adenovirus based vector may comprise at least one coding sequence encoding at least one antigenic peptide or protein as defined herein (e.g. Coronavirus antigen of component A, further viral antigen of component B).

In the context of the invention, any suitable adenovirus based vector may be used such as those described in WO2005071093 or WO2006048215. Suitably, the adenovirus based vector used is a simian adenovirus, thereby avoiding dampening of the immune response after vaccination by pre-existing antibodies to common human entities such as AdHu5. Suitable simian adenovirus vectors include AdCh63 (see WO2005071093) or AdCh68 (Cohen et al J. Gen Virol 2002 83:151) but others may also be used. Suitably the adenovirus vector will have the E1 region deleted, rendering it replication-deficient in human cells. Other regions of the adenovirus such as E3 and E4 may also be deleted.

In embodiments, the nucleic acid of component A and/or component B is an orf virus based vector. Such an orf virus based vector may comprise at least one coding sequence encoding at least one antigenic peptide or protein as defined herein (e.g. Coronavirus antigen of component A, further viral antigen of component B).

In particularly preferred embodiments of the invention, the nucleic acid of component A and/or component B is an RNA.

Preferably, the RNA of component A and/or component B typically comprises about 50 to about 20000 nucleotides, or about 500 to about 10000 nucleotides, or about 1000 to about 10000 nucleotides, or preferably about 1000 to about 5000 nucleotides, or even more preferably about 2000 to about 5000 nucleotides.

According to preferred embodiments, the nucleic acid of component A and/or component B is an RNA, preferably a coding RNA.

In preferred embodiments, the (coding) RNA of component A and/or component B is selected from an mRNA, a (coding) self-replicating RNA, a (coding) circular RNA, a (coding) viral RNA, or a (coding) replicon RNA.

In embodiments, the RNA is a circular RNA. As used herein, "circular RNA" or "circRNAs" have to be understood as a circular polynucleotide constructs that encode at least one antigenic peptide or protein as defined herein. Preferably, such a circRNA is a single stranded RNA molecule. In preferred embodiments, said circRNA comprises at least one coding sequence encoding at least one antigenic protein as defined herein, or an immunogenic fragment or an immunogenic variant thereof.

In embodiments, the RNA is a replicon RNA. The term "replicon RNA" will be recognized and understood by the person of ordinary skill in the art, and is e.g. intended to be an optimized self-replicating RNA. Such constructs may include replicase elements derived from e.g. alphaviruses (e.g. SFV, SIN, VEE, or RRV) and the substitution of the structural virus proteins with the nucleic acid of interest (that is, the coding sequence encoding an antigenic peptide or protein as defined herein). Alternatively, the replicase may be provided on an independent coding RNA construct or a coding DNA construct. Downstream of the replicase may be a sub-genomic promoter that controls replication of the replicon RNA.

In particularly preferred embodiments, the nucleic acid of component A and/or component B is not a replicon RNA or a self-replicating RNA.

In particularly preferred embodiments, the nucleic acid of component A and/or component B is an mRNA.

Preferably, the mRNA of component A and/or component B does not comprise a replicase element (e.g. a nucleic acid encoding a replicase).

The terms "RNA" and "mRNA" will be recognized and understood by the person of ordinary skill in the art, and are e.g. intended to be a ribonucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. The mRNA (messenger RNA) provides the nucleotide coding sequence that may be translated into an amino-acid sequence of a particular peptide or protein.

In the context of the invention, the RNA of component A and/or component B, preferably the mRNA, may provide at least one coding sequence encoding an antigenic protein as defined herein that is translated into a (functional) antigen after administration (e.g. after administration to a subject, e.g. a human subject).

Accordingly, the RNA of component A and/or component B, preferably the mRNA, is suitable for a vaccine, preferably a combination vaccine of the invention.

Suitably, the RNA of component A and/or component B may be modified by the addition of a 5'-cap structure, which preferably stabilizes the RNA and/or enhances expression of the encoded antigen and/or reduces the stimulation of the innate immune system (after administration to a subject). A 5'-cap structure is of particular importance in embodiments where the nucleic acid is an RNA, in particular a linear coding RNA, e.g. a linear mRNA or a linear coding replicon RNA.

Accordingly, in preferred embodiments, the at least one nucleic acid of component A and/or the at least one nucleic acid of component B comprises a 5'-cap structure, preferably m7G, cap0, cap1, cap2, a modified cap0 or a modified cap1 structure.

The term "5'-cap structure" as used herein will be recognized and understood by the person of ordinary skill in the art, and is e.g. intended to refer to a 5' modified nucleotide, particularly a guanine nucleotide, positioned at the 5'-end of an RNA, e.g. an mRNA. Preferably, the 5'-cap structure is connected via a 5'-5'-triphosphate linkage to the RNA.

5'-cap structures which may be suitable in the context of the present invention are cap0 (methylation of the first nucleobase, e.g. m7GpppN), cap1 (additional methylation of the ribose of the adjacent nucleotide of m7GpppN), cap2 (additional methylation of the ribose of the 2nd nucleotide downstream of the m7GpppN), cap3 (additional methylation of the ribose of the 3rd nucleotide downstream of the m7GpppN), cap4 (additional methylation of the ribose of the 4th nucleotide downstream of the m7GpppN), ARCA (anti-reverse cap analogue), modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

A 5'-cap (cap0 or cap1) structure may be formed in chemical RNA synthesis or in RNA in vitro transcription (co-transcriptional capping) using cap analogues.

The term "cap analogue" as used herein will be recognized and understood by the person of ordinary skill in the art, and is e.g. intended to refer to a non-polymerizable di-nucleotide or tri-nucleotide that has cap functionality in that it facilitates translation or localization, and/or prevents degradation of a nucleic acid molecule, particularly of an RNA molecule, when incorporated at the 5'-end of the nucleic acid molecule. Non-polymerizable means that the cap analogue will be incorporated only at the 5'-terminus because it does not have a 5' triphosphate and therefore cannot be extended in the 3'-direction by a template-dependent polymerase, particularly, by template-dependent RNA polymerase. Examples of cap analogues include, but are not limited to, a chemical structure selected from the group consisting of m7GpppG, m7GpppA, m7GpppC; unmethylated cap analogues (e.g. GpppG); dimethylated cap analogue (e.g. m2,7GpppG), trimethylated cap analogue (e.g. m2,2,7GpppG), dimethylated symmetrical cap analogues (e.g. m7Gpppm7G), or anti reverse cap analogues (e.g. ARCA; m7,2'OmeGpppG, m7,2'dGpppG, m7,3'OmeGpppG, m7,3'dGpppG and their tetraphosphate derivatives). Further cap analogues have been described previously (WO2008016473, WO2008157688, WO2009149253, WO2011015347, and WO2013059475). Further suitable cap analogues in that context are described in WO2017066793, WO2017066781, WO2017066791, WO2017066789, WO2017/053297, WO2017066782, WO2018075827 and WO2017066797 wherein the disclosures referring to cap analogues are incorporated herewith by reference.

In embodiments, a modified cap1 structure is generated using tri-nucleotide cap analogue as disclosed in WO2017053297, WO2017066793, WO2017066781, WO2017086791, WO2017066789, WO2017066782, WO2018075827 and WO2017066797. In particular, any cap structures derivable from the structure disclosed in claim 1-5 of WO2017053297 may be suitably used to co-transcriptionally generate a modified cap1 structure. Further, any cap structures derivable from the structure defined in claim 1 or claim 21 of WO2018075827 may be suitably used to co-transcriptionally generate a modified cap1 structure.

In preferred embodiments, the RNA of component A and/or component B, in particular the mRNA comprises a cap1 structure.

In preferred embodiments, the 5'-cap structure may suitably be added co-transcriptionally using tri-nucleotide cap analogue as defined herein, preferably in an RNA *in vitro* transcription reaction as defined herein.

In preferred embodiments, the cap1 structure of the RNA of component A and/or component B, is formed using co-transcriptional capping using tri-nucleotide cap analogues m7G(5')ppp(5')(2'OMeA)pG or m7G(5')ppp(5')(2'OMeG)pG. A preferred cap1 analogues in that context is m7G(5')ppp(5')(2'OMeA)pG.

In other preferred embodiments, the cap1 structure of the RNA of the invention is formed using co-transcriptional capping using tri-nucleotide cap analogue 3'OMe-m7G(5')ppp(5')(2'OMeA)pG.

In other embodiments, a cap0 structure of the RNA of the invention is formed using co-transcriptional capping using cap analogue 3'OMe-m7G(5')ppp(5')G.

In other embodiments, the 5'-cap structure is formed via enzymatic capping using capping enzymes (e.g. vaccinia virus capping enzymes and/or cap-dependent 2'-O methyltransferases) to generate cap0 or cap1 or cap2 structures. The 5'-cap structure (cap0 or cap1) may be added using immobilized capping enzymes and/or cap-dependent 2'-O methyltransferases using methods and means disclosed in WO2016193226.

In preferred embodiments, about 70%, 75%, 80%, 85%, 90%, 95% of the RNA (species) of component A and/or component B comprises a cap1 structure as determined using a capping assay. In preferred embodiments, less than about 20%, 15%, 10%, 5%, 4%, 3%, 2%, 1% of the RNA (species) of component A and/or component B does not comprises a cap1 structure as determined using a capping assay. In other preferred embodiments, about 70%, 75%, 80%, 85%, 90%, 95% of the RNA (species) of component A and/or component B comprises a cap0 structure as determined using a capping assay. In preferred embodiments, less than about 20%, 15%, 10%, 5%, 4%, 3%, 2%, 1% of the RNA (species) of component A and/or component B does not comprises a cap0 structure as determined using a capping assay.

In the context of the invention, the term "RNA species" is not restricted to mean "one single molecule" but is understood to comprise an ensemble of essentially identical RNA molecules. Accordingly, it may relate to a plurality of essentially identical (coding) RNA molecules.

For determining the presence/absence of a cap0 or a cap1 structure, a capping assays as described in published PCT application WO2015101416, in particular, as described in claims 27 to 46 of published PCT application WO2015101416 can be used. Other capping assays that may be used to determine the presence/absence of a cap0 or a cap1 structure of an RNA are described in PCT/EP2018/08667, or published PCT applications WO2014152673 and WO2014152659.

In preferred embodiments, the RNA of component A and/or component B comprises an m7G(5')ppp(5')(2'OMeA) cap structure. In such embodiments, the RNA comprises a 5'-terminal m7G cap, and an additional methylation of the ribose of the adjacent nucleotide of m7GpppN, in that case, a 2'O methylated Adenosine. Preferably, about 70%, 75%, 80%, 85%, 90%, 95% of the RNA (species) comprises such a cap1 structure as determined using a capping assay.

In other preferred embodiments, the RNA of component A and/or component B comprises an m7G(5')ppp(5')(2'OMeG) cap structure. In such embodiments, the RNA comprises a 5'-terminal m7G cap, and an additional methylation of the ribose of the adjacent nucleotide, in that case, a 2'O methylated guanosine. Preferably, about 70%, 75%, 80%, 85%, 90%, 95% of the coding RNA (species) comprises such a cap1 structure as determined using a capping assay.

Accordingly, the first nucleotide of said RNA or mRNA sequence, that is, the nucleotide downstream of the m7G(5')ppp structure, may be a 2'O methylated guanosine or a 2'O methylated adenosine.

According to embodiments, the RNA of component A and/or component B is a modified RNA, wherein the modification refers to chemical modifications comprising backbone modifications as well as sugar modifications or base modifications.

A modified RNA may comprise nucleotide analogues/modifications, e.g. backbone modifications, sugar modifications or base modifications. A backbone modification in the context of the invention is a modification, in which phosphates of the backbone of the nucleotides of the RNA are chemically modified. A sugar modification in the context of the invention is a chemical modification of the sugar of the nucleotides of the RNA. Furthermore, a base modification in the context of the invention is a chemical modification of the base moiety of the nucleotides of the RNA. In this context, nucleotide analogues or modifications are preferably selected from nucleotide analogues which are applicable for transcription and/or translation.

In particularly preferred embodiments, the nucleotide analogues/modifications which may be incorporated into a modified RNA of component A and/or component B are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5' -triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine, 5'-O-(1-thiophosphate)-adenosine, 5'-O-(1-thiophosphate)-cytidine, 5'-O-(1-thiophosphate)-guanosine, 5'-O-(1-thiophosphate)-uridine, 5'-O-(1-thiophosphate)-pseudouridine, 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha -thio-uridine, 4-thio-uridine, 6-aza-uridine, 5-hydroxy-uridine, deoxy-thymidine, 5-methyl-uridine, Pyrrolo-cytidine, inosine, alpha -thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-aminopurine, Pseudo-iso-cytidine, 6-Chloro-purine, N6-methyl-adenosine, alpha -thio-adenosine, 8-azido-adenosine, 7-deazaadenosine.

In some embodiments, the at least one modified nucleotide is selected from pseudouridine, N1-methylpseudouridine, N1-ethylpseudouridine, 2-thiouridine, 4'-thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine and 2'-O-methyl uridine.

Particularly preferred in that context are pseudouridine (ψ), N1-methylpseudouridine (m1ψ), 5-methylcytosine, and 5-methoxyuridine.

Accordingly, in embodiments, the at least one nucleic acid of component A and/or the at least one nucleic acid of component B comprises at least one modified nucleotide.

In some embodiments, essentially all, e.g. essentially 100% of the uracil in the coding sequence of component A and/or component B have a chemical modification, preferably a chemical modification is in the 5-position of the uracil.

Incorporating modified nucleotides such as e.g. pseudouridine (ψ), N1-methylpseudouridine (m1ψ), 5-methylcytosine, and/or 5-methoxyuridine into the coding sequence of component A and/or component B may be advantageous as unwanted innate immune responses (upon administration of the coding RNA or the vaccine) may be adjusted or reduced (if required).

In embodiments, the RNA of component A and/or component B comprises at least one coding sequence encoding at least one antigenic protein as defined herein, wherein said coding sequence comprises at least one modified nucleotide selected from pseudouridine (ψ) and N1-methylpseudouridine (m1ψ), preferably wherein all uracil nucleotides are replaced by pseudouridine (ψ) nucleotides and/or N1-methylpseudouridine (m1ψ) nucleotides, optionally wherein all uracil nucleotides are replaced by pseudouridine (Ψ) nucleotides and/or N1-methylpseudouridine (m1 Ψ) nucleotides.

In preferred embodiments, the RNA of component A and/or component B does not comprise N1-methylpseudouridine (m1Ψ) substituted positions. In further embodiments, the RNA of component A and/or component B does not comprise pseudouridine (ψ), N1-methylpseudouridine (m1ψ), 5-methylcytosine, and 5-methoxyuridine substituted position.

In preferred embodiments, the RNA of component A and/or component B comprises a coding sequence that consists only of G, C, A and U nucleotides and therefore does not comprise modified nucleotides (except of the 5' terminal cap structure (cap0, cap1, cap2)).

In embodiments, the nucleic acid of component A and/or component B is an RNA, wherein the RNA may be prepared using any method known in the art, including chemical synthesis such as e.g. solid phase RNA synthesis, as well as in vitro methods, such as RNA in vitro transcription reactions. Accordingly, in a preferred embodiment, the RNA is obtained by RNA in vitro transcription.

Accordingly, in preferred embodiments, the nucleic acid of component A and/or component B is preferably an in vitro transcribed RNA.

The terms "RNA in vitro transcription" or "in vitro transcription" relate to a process wherein RNA is synthesized in a cell-free system (in vitro). RNA may be obtained by DNA-dependent in vitro transcription of an appropriate DNA template, which according to the present invention is a linearized plasmid DNA template or a PCR-amplified DNA template. The promoter for controlling RNA in vitro transcription can be any promoter for any DNA-dependent RNA polymerase. Particular examples of DNA-dependent RNA polymerases are the T7, T3, SP6, or Syn5 RNA polymerases. In a preferred embodiment of the present invention the DNA template is linearized with a suitable restriction enzyme, before it is subjected to RNA in vitro transcription.

Reagents used in RNA in vitro transcription typically include: a DNA template (linearized plasmid DNA or PCR product) with a promoter sequence that has a high binding affinity for its respective RNA polymerase such as bacteriophage-encoded RNA polymerases (T7, T3, SP6, or Syn5); ribonucleotide triphosphates (NTPs) for the four bases (adenine, cytosine, guanine and uracil); optionally, a cap analogue as defined herein; optionally, further modified nucleotides as defined herein; a DNA-dependent RNA polymerase capable of binding to the promoter sequence within the DNA template (e.g. T7, T3, SP6, or Syn5 RNA polymerase); optionally, a ribonuclease (RNase) inhibitor to inactivate any potentially contaminating RNase; optionally, a pyrophosphatase to degrade pyrophosphate, which may inhibit RNA in vitro transcription; MgCl2, which supplies Mg2+ ions as a co-factor for the polymerase; a buffer (TRIS or HEPES) to maintain a suitable pH value, which can also contain antioxidants (e.g. DTT), and/or polyamines such as spermidine at optimal concentrations, e.g. a buffer system comprising TRIS-Citrate as disclosed in WO2017109161.

In preferred embodiments, the cap1 structure of the RNA of component A and/or component B is formed using co-transcriptional capping using tri-nucleotide cap analogues m7G(5')ppp(5')(2'OMeA)pG or m7G(5')ppp(5')(2'OMeG)pG. A preferred cap1 analogue that may suitably be used in manufacturing the coding RNA of the invention is m7G(5')ppp(5')(2'OMeA)pG.

In other preferred embodiments, the cap1 structure of the RNA of the invention is formed using co-transcriptional capping using tri-nucleotide cap analogue 3'OMe-m7G(5')ppp(5')(2'OMeA)pG.

In other embodiments, a cap0 structure of the RNA of the invention is formed using co-transcriptional capping using cap analogue 3'OMe-m7G(5')ppp(5')G.

In embodiments, the nucleotide mixture used in RNA in vitro transcription may additionally comprise modified nucleotides as defined herein. In that context, preferred modified nucleotides may be selected from pseudouridine (ψ), N1-methylpseudouridine (m1ψ), 5-methylcytosine, and 5-methoxyuridine. In particular embodiments, uracil nucleotides in the nucleotide mixture are replaced (either partially or completely) by pseudouridine (ψ) and/or N1-methylpseudouridine (m1ψ) to obtain a modified RNA.

In preferred embodiments, the nucleotide mixture used in RNA in vitro transcription does not comprise modified nucleotides as defined herein. In preferred embodiments, the nucleotide mixture used in RNA in vitro transcription does only comprise G, C, A and U nucleotides, and, optionally, a cap analog as defined herein.

In preferred embodiments, the nucleotide mixture (i.e. the fraction of each nucleotide in the mixture) used for RNA in vitro transcription reactions may be optimized for the given RNA sequence, preferably as described WO2015188933.

In this context, the in vitro transcription has been performed in the presence of a sequence optimized nucleotide mixture and optionally a cap analog, preferably wherein the sequence optimized nucleotide mixture does not comprise chemically modified nucleotides.

In this context a sequence-optimized nucleoside triphosphate (NTP) mix is a mixture of nucleoside triphosphates (NTPs) for use in an in vitro transcription reaction of an RNA molecule of a given sequence comprising the four nucleoside triphosphates (NTPs) GTP, ATP, CTP and UTP, wherein the fraction of each of the four nucleoside triphosphates (NTPs) in the sequence-optimized nucleoside triphosphate (NTP) mix corresponds to the fraction of the respective nucleotide in said RNA molecule. If a ribonucleotide is not present in the RNA molecule, the corresponding nucleoside triphosphate is also not present in the sequence-optimized nucleoside triphosphate (NTP) mix.

In embodiment where more than one different RNA as defined herein have to be produced, e.g. where 2, 3, 4, 5, 6, 7, 8, 9, 10 or even more different RNAs have to be produced, procedures as described in WO2017109134 may suitably be used.

In the context of nucleic acid-based vaccine production, it may be required to provide GMP-grade nucleic acid, e.g. a GMP grade RNA or DNA. GMP-grade RNA or DNA may be produced using a manufacturing process approved by regulatory authorities. Accordingly, in a particularly preferred embodiment, RNA production is performed under current good manufacturing practice (GMP), implementing various quality control steps on DNA and RNA level, preferably according to WO2016180430. In preferred embodiments, the RNA of the invention is a GMP-grade RNA, particularly a GMP-grade mRNA. Accordingly, an RNA for a vaccine is preferably a GMP grade RNA.

The obtained RNA products are preferably purified using PureMeassenger^{®} (CureVac, Tübingen, Germany; RP-HPLC according to WO2008077592) and/or tangential flow filtration (as described in WO2016193206) and/or oligo d(T) purification (see WO2016180430).

Preferably, the RNA according to the invention is purified using RP-HPLC, preferably using Reversed-Phase High pressure liquid chromatography (RP-HPLC) with a macroporous styrene/divinylbenzene column (e.g. particle size 30µm, pore size 4000 A and additionally using a filter cassette with a cellulose based membrane with a molecular weight cutoff of about 100kDa.

In a further preferred embodiment, the nucleic acid of component A and/or component B, preferably the RNA, is lyophilized (e.g. according to WO2016165831 or WO2011069586) to yield a temperature stable dried nucleic acid (powder) as defined herein (e.g. RNA or DNA). The nucleic acid of the invention, particularly the RNA may also be dried using spray-drying or spray-freeze drying (e.g. according to WO2016184575 or WO2016184576) to yield a temperature stable RNA (powder) as defined herein. Accordingly, in the context of manufacturing and purifying nucleic acid, in particular RNA, the disclosures of WO2017109161, WO2015188933, WO2016180430, WO2008077592, WO2016193206, WO2016165831, WO2011069586, WO2016184575, and WO2016184576 are incorporated herewith by reference.

Accordingly, in preferred embodiments, the nucleic acid of component A and/or component B is a dried nucleic acid, particularly a dried RNA.

The term "dried RNA" as used herein has to be understood as RNA that has been lyophilized, or spray-dried, or spray-freeze dried as defined above to obtain a temperature stable dried RNA (powder).

In preferred embodiments, the nucleic acid of component A and/or component B is a purified nucleic acid, particularly a purified RNA.

The term "purified nucleic acid" as used herein has to be understood as nucleic acid which has a higher purity after certain purification steps than the starting material. Typical impurities that are essentially not present in purified nucleic acid comprise peptides or proteins, spermidine, BSA, abortive nucleic acid sequences, nucleic acid fragments, free nucleotides, bacterial impurities, or impurities derived from purification procedures. Accordingly, it is desirable in this regard for the "degree of nucleic acid purity" to be as close as possible to 100%. It is also desirable for the degree of nucleic acid purity that the amount of full-length nucleic acid is as close as possible to 100%. Accordingly "purified nucleic acid" as used herein has a degree of purity of more than 75%, 80%, 85%, very particularly 90%, 91%, 92%, 93%, 94%, 95%, 96%, 97%, 98% and most favorably 99% or more. The degree of purity may for example be determined by an analytical HPLC, wherein the percentages provided above correspond to the ratio between the area of the peak for the target nucleic acid and the total area of all peaks representing the by-products. Alternatively, the degree of purity may for example be determined by an analytical agarose gel electrophoresis or capillary gel electrophoresis.

In preferred embodiments, the nucleic acid of component A and/or component B is a purified RNA, preferably a purified mRNA.

The term "purified RNA" or "purified mRNA" as used herein has to be understood as RNA which has a higher purity after certain purification steps (e.g. HPLC, TFF, Oligo d(T) purification, precipitation steps) than the starting material (e.g. in vitro transcribed RNA). Typical impurities that are essentially not present in purified RNA comprise peptides or proteins (e.g. enzymes derived from DNA dependent RNA in vitro transcription, e.g. RNA polymerases, RNases, pyrophosphatase, restriction endonuclease, DNase), spermidine, BSA, abortive RNA sequences, RNA fragments (short double stranded RNA fragments, abortive sequences etc.), free nucleotides (modified nucleotides, conventional NTPs, cap analogue), template DNA fragments, buffer components (HEPES, TRIS, MgCl2) etc. Other potential impurities that may be derived from e.g. fermentation procedures comprise bacterial impurities (bioburden, bacterial DNA) or impurities derived from purification procedures (organic solvents etc.). Accordingly, it is desirable in this regard for the "degree of RNA purity" to be as close as possible to 100%. It is also desirable for the degree of RNA purity that the amount of full-length RNA transcripts is as close as possible to 100%. Accordingly, "purified RNA" as used herein has a degree of purity of more than 75%, 80%, 85%, very particularly 90%, 91%, 92%, 93%, 94%, 95%, 96%, 97%, 98% and most favorably 99% or more. The degree of purity may for example be determined by an analytical HPLC, wherein the percentages provided above correspond to the ratio between the area of the peak for the target RNA and the total area of all peaks representing the by-products. Alternatively, the degree of purity may for example be determined by an analytical agarose gel electrophoresis or capillary gel electrophoresis.

It has to be understood that "dried RNA" as defined herein and "purified RNA" as defined herein or "GMP-grade RNA" as defined herein may have superior stability characteristics (in vitro, in vivo) and improved efficiency (e.g. better translatability of the mRNA in vivo) and are therefore particularly suitable for a medical purpose, e.g. a combination vaccine.

In particularly preferred embodiments where the nucleic acid of component A and/or component B is an RNA, the RNA has been purified by RP-HPLC and/or TFF to remove double-stranded RNA, non-capped RNA and/or RNA fragments.

The formation of double stranded RNA as side products during e.g. RNA in vitro transcription can lead to an induction of the innate immune response, particularly IFNalpha which is the main factor of inducing fever in vaccinated subjects, which is of course an unwanted side effect. Current techniques for immunoblotting of dsRNA (via dot Blot, serological specific electron microscopy (SSEM) or ELISA for example) are used for detecting and sizing dsRNA species from a mixture of nucleic acids.

Suitably, the RNA has been purified by RP-HPLC and/or TFF as described herein to reduce the amount of dsRNA.

In preferred embodiments, the RNA comprises about 5%, 10%, or 20% less double stranded RNA side products as an RNA that has not been purified with RP-HPLC and/or TFF.

In preferred embodiments, the RP-HPLC and/or TFF purified RNA of component A and/or component B comprises about 5%, 10%, or 20% less double stranded RNA side products as an RNA that has been purified with Oligo dT purification, precipitation, filtration and/or AEX.

In embodiments, RNA of a composition has an RNA integrity ranging from about 40% to about 100%.

The term "RNA integrity" generally describes whether the complete RNA sequence is present in the composition. Low RNA integrity could be due to, amongst others, RNA degradation, RNA cleavage, incorrect or incomplete chemical synthesis of the RNA, incorrect base pairing, integration of modified nucleotides or the modification of already integrated nucleotides, lack of capping or incomplete capping, lack of polyadenylation or incomplete polyadenylation, or incomplete RNA in vitro transcription. RNA is a fragile molecule that can easily degrade, which may be caused e.g. by temperature, ribonucleases, pH or other factors (e.g. nucleophilic attacks, hydrolysis etc.), which may reduce the RNA integrity and, consequently, the functionality of the RNA.

The skilled person can choose from a variety of different chromatographic or electrophoretic methods for determining an RNA integrity. Chromatographic and electrophoretic methods are well-known in the art. In case chromatography is used (e.g. RP-HPLC), the analysis of the integrity of the RNA may be based on determining the peak area (or "area under the peak") of the full length RNA in a corresponding chromatogram. The peak area may be determined by any suitable software which evaluates the signals of the detector system. The process of determining the peak area is also referred to as integration. The peak area representing the full length RNA is typically set in relation to the peak area of the total RNA in a respective sample. The RNA integrity may be expressed in % RNA integrity.

In the context of aspects of the invention, RNA integrity may be determined using analytical (RP)HPLC. Typically, a test sample of the composition comprising lipid based carrier encapsulating RNA may be treated with a detergent (e.g. about 2% Triton X100) to dissociate the lipid based carrier and to release the encapsulated RNA. The released RNA may be captured using suitable binding compounds, e.g. Agencourt AMPure XP beads (Beckman Coulter, Brea, CA, USA) essentially according to the manufacturer's instructions. Following preparation of the RNA sample, analytical (RP)HPLC may be performed to determine the integrity of RNA. Typically, for determining RNA integrity, the RNA samples may be diluted to a concentration of 0.1g/l using e.g. water for injection (WFI). About 10µl of the diluted RNA sample may be injected into an HPLC column (e.g. a monolithic poly(styrene-divinylbenzene) matrix). Analytical (RP)HPLC may be performed using standard conditions, for example: Gradient 1: Buffer A (0.1M TEAA (pH 7.0)); Buffer B (0.1M TEAA (pH 7.0) containing 25% acetonitrile). Starting at 30% buffer B the gradient extended to 32% buffer B in 2min, followed by an extension to 55% buffer B over 15 minutes at a flow rate of 1ml/min. HPLC chromatograms are typically recorded at a wavelength of 260nm. The obtained chromatograms may be evaluated using a software and the relative peak area may be determined in percent (%) as commonly known in the art. The relative peak area indicates the amount of RNA that has 100% RNA integrity. Since the amount of the RNA injected into the HPLC is typically known, the analysis of the relative peak area provides information on the integrity of the RNA. Thus, if e.g. 100ng RNA have been injected in total, and 100ng are determined as the relative peak area, the RNA integrity would be 100%. If, for example, the relative peak area would correspond to 80ng, the RNA integrity would be 80%. Accordingly, RNA integrity in the context of the invention is determined using analytical HPLC, preferably analytical RP-HPLC.

In embodiments, RNA of a composition has an RNA integrity ranging from about 40% to about 100%. In embodiments, the RNA has an RNA integrity ranging from about 50% to about 100%. In embodiments, the RNA has an RNA integrity ranging from about 60% to about 100%. In embodiments, the RNA has an RNA integrity ranging from about 70% to about 100%. In embodiments, the RNA integrity is for example about 50%, about 60%, about 70%, about 80%, or about 90%. RNA is suitably determined using analytical HPLC, preferably analytical RP-HPLC.

In preferred embodiments, the RNA of a composition has an RNA integrity of at least about 50%, preferably of at least about 60%, more preferably of at least about 70%, most preferably of at least about 80% or ABOUT 90%. RNA is suitably determined using analytical HPLC, preferably analytical RP-HPLC.

Following co-transcriptional capping as defined herein, and following purification as defined herein, the capping degree of the obtained RNA may be determined using capping assays as described in published PCT application WO2015101416, in particular, as described in Claims 27 to 46 of published PCT application WO2015101416 can be used. Alternatively, a capping assays described in PCT/EP2018/08667 may be used.

In embodiments, an automated device for performing RNA in vitro transcription may be used to produce and purify the nucleic acid of component A and/or component B. Such a device may also be used to produce the composition or the vaccine (see aspects 2 and 3). Preferably, a device as described in WO2020002598, in particular, a device as described in claims 1 to 59 and/or 68 to 76 of WO2020002598 (and Figures 1-18) may suitably be used.

The methods described herein may preferably applied to a method of producing an pharmaceutical composition or combination vaccine as described in further detail below.

In various embodiments the nucleic acid of component A and/or component B, preferably the mRNA of component A and component B comprises, preferably in 5'- to 3'-direction, the following elements:
A) 5'-cap structure, *preferably* as specified herein;
B) 5'-terminal start element, preferably as specified herein;
C) optionally, a 5'-UTR, preferably as specified herein;
D) a ribosome binding site, preferably as specified herein;
E) at least one coding sequence, preferably as specified herein;
F) 3'-UTR, preferably as specified herein;
G) optionally, poly(A) sequence, preferably as specified herein;
H) optionally, poly(C) sequence, preferably as specified herein;
I) optionally, histone stem-loop preferably as specified herein;
J) optionally, 3'-terminal sequence element, preferably as specified herein.

In preferred embodiments the nucleic acid of component A and/or component B, preferably the mRNA of component A and component B, comprises the following elements preferably in 5'- to 3'-direction:
A) 5'-cap structure selected from m7G(5'), m7G(5')ppp(5')(2'OMeA), or m7G(5')ppp(5')(2'OMeG);
B) 5'-terminal start element selected from **SEQ ID NOs: 176 or 177** or fragments or variants thereof,
C) optionally, a 5'-UTR derived from a HSD17B4 gene;
D) a ribosome binding site selected from **SEQ ID NOs: 180, 181, 22845-22847or** fragments or variants thereof;
E) at least one coding sequence as specified herein, preferably a codon optimized coding sequence;
F) 3'-UTR derived from a 3'-UTR of a PSMB3 gene or an alpha-globin gene ("muag");
G) optionally, poly(A) sequence comprising about 30 to about 500 adenosines;
H) optionally, poly(C) sequence comprising about 10 to about 100 cytosines;
I) optionally, histone stem-loop selected from **SEQ ID NOs: 178 or 179;**
J) optionally, 3'-terminal sequence element selected from **SEQ ID NOs: 182-230.**

In particularly preferred embodiments the nucleic acid of component A and/or component B, preferably the mRNA of component A and component B, comprises the following elements in 5'- to 3'-direction:
A) cap1 structure as defined herein;
B) coding sequence as specified herein, preferably a codon optimized coding sequence;
C) 3'-UTR derived from a 3'-UTR of a muag gene as defined herein, preferably according to SEQ ID NO: **267, 268, 22896-22901, 22906-22911;**
D) poly(A) sequence comprising about 64 A nucleotides.
E) poly(C) sequence comprising about 10 to about 100 cytosines;
F) histone stem-loop selected from **SEQ ID NOs: 178 or 179;**

In preferred embodiments the nucleic acid of component A and/or component B, preferably the mRNA of component A and component B, comprises the following elements in 5'- to 3'-direction:
A) cap1 structure as defined herein, preferably a codon optimized coding sequence;
B) 5'-UTR derived from a HSD17B4 gene as defined herein, preferably according to **SEQ ID NO: 231 or 232;**
C) coding sequence as specified herein;
D) 3'-UTR derived from a 3'-UTR of a PSMB3 gene as defined herein, preferably according to **SEQ ID NO: 253 or 254;**
E) poly(A) sequence comprising about 64 A nucleotides.
F) optionally a poly(C) sequence comprising about 10 to about 100 cytosines;
G) histone stem-loop selected from **SEQ ID NOs: 178 or 179;**
H) optionally, 3'-terminal sequence element **SEQ ID NOs: 182-230.**

In particularly preferred embodiments the nucleic acid of component A and/or component B, preferably the mRNA of component A and component B, comprises the following elements in 5'- to 3'-direction:
A) cap1 structure as defined herein;
B) 5'-UTR derived from a HSD17B4 gene as defined herein, preferably according to **SEQ ID NO: 231 or 232;**
C) coding sequence selected as specified herein, preferably a codon optimized coding sequence;
D) 3'-UTR derived from a 3'-UTR of a PSMB3 gene as defined herein, preferably according to **SEQ ID NO: 253 or 254;**
E) a histone stem-loop selected from **SEQ ID NOs: 178 or 179;**
F) poly(A) sequence comprising about 100 A nucleotides, preferably representing the 3' terminus.

In even more particularly preferred embodiments the nucleic acid of component A and/or component B, preferably the mRNA of component A and component B, comprises the following elements in 5'- to 3'-direction:
A) cap1 structure as defined herein;
B) 5'-UTR derived from a HSD17B4 gene as defined herein, preferably according to **SEQ ID NO: 231 or 232;**
C) coding sequence selected as specified herein, preferably a codon optimized coding sequence;
D) 3'-UTR derived from a 3'-UTR of a PSMB3 gene as defined herein, preferably according to **SEQ ID NO: 253 or 254;**
E) poly(A) sequence comprising about 100 A nucleotides, preferably representing the 3' terminus.

In further preferred embodiments the nucleic acid of component A and/or component B, preferably the mRNA of component A and component B, comprises the following elements in 5'- to 3'-direction:
A) cap1 structure as defined herein;
B) 5'-UTR derived from a SLC7A3 gene as defined herein, preferably according to **SEQ ID NO: 245 or 246;**
C) coding sequence selected as specified herein, preferably a codon optimized coding sequence;
D) 3'-UTR derived from a 3'-UTR of a PSMB3 gene as defined herein, preferably according to **SEQ ID NO: 253 or 254;**
E) optionally, a histone stem-loop selected from **SEQ ID NOs: 178 or 179;**
F) poly(A) sequence comprising about 100 A nucleotides, preferably representing the 3' terminus.

In further preferred embodiments the nucleic acid of component A and/or component B, preferably the mRNA of component A and component B, comprises the following elements in 5'- to 3'-direction:
A) cap1 structure as defined herein;
B) 5'-UTR derived from a RPL31 gene as defined herein, preferably according to **SEQ ID NO: 243 or 243;**
C) coding sequence selected as specified herein, preferably a codon optimized coding sequence;
D) 3'-UTR derived from a 3'-UTR of a RPS9 gene as defined herein, preferably according to **SEQ ID NO: 263 or 264;**
E) optionally, a histone stem-loop selected from **SEQ ID NOs: 178 or 179;**
F) poly(A) sequence comprising about 100 A nucleotides, preferably representing the 3' terminus.

### Formulation and complexation:

In the following, suitable features and embodiments relating to the formulation and/or complexation of the pharmaceutical composition are further specified. In particular, suitable features and embodiments relating to formulation and/or complexation of component A (in particular, component A-1, A-2, A-3), and/or formulation and/or complexation of component B (in particular component B-1, B-2, B-3, B-3a, B-3b, B-4, B-4a, B-4b) are further specified.

In preferred embodiments, the pharmaceutical composition comprises at least one pharmaceutically acceptable carrier or excipient.

The term "pharmaceutically acceptable carrier" or "pharmaceutically acceptable excipient" as used herein preferably includes the liquid or non-liquid basis of the composition for administration. If the composition is provided in liquid form, the carrier may be water, e.g. pyrogen-free water; isotonic saline or buffered (aqueous) solutions, e.g. phosphate, citrate etc. buffered solutions. Water or preferably a buffer, more preferably an aqueous buffer, may be used, containing a sodium salt, preferably at least 50mM of a sodium salt, a calcium salt, preferably at least 0.01mM of a calcium salt, and optionally a potassium salt, preferably at least 3mM of a potassium salt. According to preferred embodiments, the sodium, calcium and, optionally, potassium salts may occur in the form of their halogenides, e.g. chlorides, iodides, or bromides, in the form of their hydroxides, carbonates, hydrogen carbonates, or sulfates, etc. Examples of sodium salts include NaCl, Nal, NaBr, Na₂CO₃, NaHCOs, Na₂SO₄, examples of the optional potassium salts include KCI, Kl, KBr, K₂CO₃, KHCO₃, K₂SO₄, and examples of calcium salts include CaCl₂, Cal₂, CaBr₂, CaCO₃, CaSO₄, Ca(OH)₂.

Furthermore, organic anions of the aforementioned cations may be in the buffer. Accordingly, in embodiments, the pharmaceutical composition may comprise pharmaceutically acceptable carriers or excipients using one or more pharmaceutically acceptable carriers or excipients to e.g. increase stability, increase cell transfection, permit the sustained or delayed, increase the translation of encoded antigenic peptides or proteins in vivo, and/or alter the release profile of encoded antigenic peptides or proteins protein in vivo. In addition to traditional excipients such as any and all solvents, dispersion media, diluents, or other liquid vehicles, dispersion or suspension aids, surface active agents, isotonic agents, thickening or emulsifying agents, preservatives, excipients of the present invention can include, without limitation, lipidoids, liposomes, lipid nanoparticles, polymers, lipoplexes, core-shell nanoparticles, peptides, proteins, cells transfected with polynucleotides, hyaluronidase, nanoparticle mimics and combinations thereof. In embodiments, one or more compatible solid or liquid fillers or diluents or encapsulating compounds may be used as well, which are suitable for administration to a subject. The term "compatible" as used herein means that the constituents of the composition are capable of being mixed with the at least one nucleic acid of component A and/or component B and, optionally, a plurality of nucleic acids of the composition, in such a manner that no interaction occurs, which would substantially reduce the biological activity or the pharmaceutical effectiveness of the composition under typical use conditions (e.g., intramuscular or intradermal administration). Pharmaceutically acceptable carriers or excipients must have sufficiently high purity and sufficiently low toxicity to make them suitable for administration to a subject to be treated. Compounds which may be used as pharmaceutically acceptable carriers or excipients may be sugars, such as, for example, lactose, glucose, trehalose, mannose, and sucrose; starches, such as, for example, com starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, com oil and oil from theobroma; polyols, such as, for example, polypropylene glycol, glycerol, sorbitol, mannitol and polyethylene glycol; alginic acid.

The at least one pharmaceutically acceptable carrier or excipient of the pharmaceutical composition may preferably be selected to be suitable for intramuscular or intradermal delivery/administration of said pharmaceutical composition. The pharmaceutical composition is preferably a composition suitable for intramuscular administration to a subject.

Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as poultry, chickens, ducks, geese, and/or turkeys.

Pharmaceutical compositions of the present invention may suitably be sterile and/or pyrogen-free.

In a preferred embodiments, the at least one nucleic acid of component A and/or component B is complexed or associated with further compound to obtain a formulated composition. A formulation in that context may have the function of a transfection agent. A formulation in that context may also have the function of protecting the nucleic acid from degradation.

In embodiments, the at least one nucleic acid of component A and the at least one nucleic acid of component B
are formulated separately.

In preferred embodiments, the at least one nucleic acid of component A and the at least one nucleic acid of component B are co-formulated.

In a preferred embodiment, the at least one nucleic acid of component A and/or component B, preferably the at least one RNA of component A and/or component B, is complexed or associated with, or at least partially complexed or partially associated with one or more cationic or polycationic compound. *Preferably,* the at least one nucleic acid of component A and component B are complexed or associated with, or at least partially complexed or partially associated with one or more cationic or polycationic compound, wherein component A and component B are formulated separately or wherein component A and component B are co-formulated.

In preferred embodiments, the one or more cationic or polycationic compound is selected from a cationic or polycationic polymer, cationic or polycationic polysaccharide, cationic or polycationic lipid, cationic or polycationic protein, cationic or polycationic peptide, or any combinations thereof.

The term "cationic or polycationic compound" as used herein will be recognized and understood by the person of ordinary skill in the art, and is for example intended to refer to a charged molecule, which is positively charged at a pH value ranging from about 1 to 9, at a pH value ranging from about 3 to 8, at a pH value ranging from about 4 to 8, at a pH value ranging from about 5 to 8, more preferably at a pH value ranging from about 6 to 8, even more preferably at a pH value ranging from about 7 to 8, most preferably at a physiological pH, e.g. ranging from about 7.2 to about 7.5. Accordingly, a cationic component, e.g. a cationic peptide, cationic protein, cationic polymer, cationic polysaccharide, cationic lipid (including lipidoids) may be any positively charged compound or polymer which is positively charged under physiological conditions. A "cationic or polycationic peptide or protein" may contain at least one positively charged amino acid, or more than one positively charged amino acid, e.g. selected from Arg, His, Lys or Om. Accordingly, "polycationic" components are also within the scope exhibiting more than one positive charge under the given conditions.

Cationic or polycationic compounds, being particularly preferred in this context may be selected from the following list of cationic or polycationic peptides or proteins of fragments thereof: protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides, pAntp, plsl, FGF, Lactoferrin, Transportan, Buforin-2, Bac715-24, SynB, SynB(1), pVEC, hCT-derived peptides, SAP, or histones. More preferably, the nucleic acid (e.g. DNA or RNA) of component A and/or component B, e.g. the coding RNA, preferably the mRNA, is complexed with one or more polycations, preferably with protamine or oligofectamine, most preferably with protamine. In preferred embodiment, the at least one nucleic acid component A and/or component B (e.g. DNA or RNA), preferably the at least one RNA, is complexed with protamine.

Further preferred cationic or polycationic compounds, which can be used as transfection or complexation agent may include cationic polysaccharides, for example chitosan, polybrene etc.; cationic lipids, e.g. DOTMA, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS, DIMRI, DOTAP, DC-6-14, CLIP1, CLIP6, CLIP9, oligofectamine; or cationic or polycationic polymers, e.g. modified polyaminoacids, such as beta-aminoacid-polymers or reversed polyamides, etc., modified polyethylenes, such as PVP etc., modified acrylates, such as pDMAEMA etc., modified amidoamines such as pAMAM etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI, poly(propyleneimine), etc., polyallylamine, sugar backbone based polymers, such as cyclodextrin based polymers, dextran based polymers, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

In this context it is particularly preferred that the at least one nucleic acid of component A and/or component B (e.g. DNA or RNA), preferably the at least one RNA, is complexed or at least partially complexed with a cationic or polycationic compound and/or a polymeric carrier, preferably cationic proteins or peptides. In this context, the disclosure of WO2010037539 and WO2012113513 is incorporated herewith by reference. Partially means that only a part of the nucleic acid is complexed with a cationic compound and that the rest of the nucleic acid is in uncomplexed form ("free").

In embodiments, the pharmaceutical composition comprises at least one nucleic acid of component A and/or component B (e.g. DNA or RNA), preferably at least one RNA, complexed with one or more cationic or polycationic compounds, preferably protamine, and at least one free (non-complexed) nucleic acid.

In this context it is particularly preferred that the at least one nucleic acid of component A and/or component B (e.g. DNA or RNA), preferably the at least one RNA is complexed, or at least partially complexed with protamine. Preferably, the molar ratio of the nucleic acid of component A and/or component B, particularly the RNA of the protamine-complexed RNA to the free RNA may be selected from a molar ratio of about 0.001:1 to about 1:0.001, including a ratio of about 1:1. Suitably, the complexed RNA is complexed with protamine by addition of protamine-trehalose solution to the RNA sample at a RNA:protamine weight to weight ratio (w/w) of 2:1.

Further preferred cationic or polycationic proteins or peptides that may be used for complexation of the nucleic acid of component A and/or component B can be derived from formula (Arg)I;(Lys)m;(His)n;(Om)o;(Xaa)x of the patent application WO2009030481 or WO2011026641, the disclosure of WO2009030481 or WO2011026641 relating thereto incorporated herewith by reference.

In preferred embodiments, the at least one nucleic acid of component A and/or component B (e.g. DNA or RNA), preferably the at least one RNA is complexed, or at least partially complexed, with at least one cationic or polycationic proteins or peptides preferably selected from **SEQ ID NOs: 269-273,** or any combinations thereof.

According to various embodiments, the composition of the present invention comprises at least one nucleic acid of component A and component B (e.g. DNA or RNA), preferably at least one RNA as defined herein, and a polymeric carrier.

The term "polymeric carrier" as used herein will be recognized and understood by the person of ordinary skill in the art, and are e.g. intended to refer to a compound that facilitates transport and/or complexation of another compound (e.g. cargo nucleic acid of component A and/or component B). A polymeric carrier is typically a carrier that is formed of a polymer. A polymeric carrier may be associated to its cargo (e.g. DNA, or RNA of component A and/or component B) by covalent or non-covalent interaction. A polymer may be based on different subunits, such as a copolymer.

Suitable polymeric carriers in that context may include, for example, polyacrylates, polyalkycyanoacrylates, polylactide, polylactide-polyglycolide copolymers, polycaprolactones, dextran, albumin, gelatin, alginate, collagen, chitosan, cyclodextrins, protamine, PEGylated protamine, PEGylated PLL and polyethylenimine (PEI), dithiobis(succinimidylpropionate) (DSP), Dimethyl-3,3'-dithiobispropionimidate (DTBP), poly(ethylene imine) biscarbamate (PEIC), poly(L-lysine) (PLL), histidine modified PLL, poly(N-vinylpyrrolidone) (PVP), poly(propylenimine (PPI), poly(amidoamine) (PAMAM), poly(amido ethylenimine) (SS-PAEI), triehtylenetetramine (TETA), poly(β-aminoester), poly(4-hydroxy-L-proine ester) (PHP), poly(allylamine), poly(α-(4-aminobutyl]-L-glycolic acid (PAGA), Poly(D,L-lactic-co-glycolid acid (PLGA), Poly(N-ethyl-4-vinylpyridinium bromide), poly(phosphazene)s (PPZ), poly(phosphoester)s (PPE), poly(phosphoramidate)s (PPA), poly(N-2-hydroxypropylmethacryfamide) (pHPMA), poly(2-(dimethylamino)ethyl methacrylate) (pDMAEMA), poly(2-aminoethyl propylene phosphate) PPE_EA), galactosylated chitosan, N-dodecylated chitosan, histone, collagen and dextran-spermine. In one embodiment, the polymer may be an inert polymer such as, but not limited to, PEG. In one embodiment, the polymer may be a cationic polymer such as, but not limited to, PEI, PLL, TETA, poly(allylamine), Poly(N-ethyl-4-vinylpyridinium bromide), pHPMA and pDMAEMA. In one embodiment, the polymer may be a biodegradable PEI such as, but not limited to, DSP, DTBP and PEIC. In one embodiment, the polymer may be biodegradable such as, but not limited to, histine modified PLL, SS-PAEI, poly(β-aminoester), PHP, PAGA, PLGA, PPZ, PPE, PPA and PPE-EA.

A suitable polymeric carrier may be a polymeric carrier formed by disulfide-crosslinked cationic compounds. The disulfide-crosslinked cationic compounds may be the same or different from each other. The polymeric carrier can also contain further components. The polymeric carrier used according to the present invention may comprise mixtures of cationic peptides, proteins or polymers and optionally further components as defined herein, which are crosslinked by disulfide bonds (via -SH groups).

In this context, polymeric carriers according to formula ((Arg)l;(Lys)m;(His)n;(Orn)o;(Xaa)x(Cys)y} and formula Cys,{(Arg)l;(Lys)m;(His)n;(Orn)o;(Xaa)x}Cys₂ of the patent application WO2012/013326 are preferred, the disclosure of WO2012/013326 relating thereto incorporated herewith by reference.

In embodiments, the polymeric carrier used to complex the at least one nucleic acid of component A and/or component B (e.g. DNA or RNA), preferably the at least one RNA of component A and/or component B may be derived from a polymeric carrier molecule according formula (L-P¹-S-[S-P²-S]ₙ-S-P³-L) of the patent application WO2011026641, the disclosure of WO2011026641 relating thereto incorporated herewith by reference.

In embodiments, the polymeric carrier compound is formed by, or comprises or consists of the peptide elements CysArg 12Cys **(SEQ ID NO: 269)** or CysArg12 **(SEQ ID NO: 270)** or TrpArg12Cys (**SEQ ID NO: 271**). In particularly preferred embodiments, the polymeric carrier compound consists of a (R₁₂C)-(R₁₂C) dimer, a (WR₁₂C)-(WR₁₂C) dimer, or a (CR₁₂)-(CR₁₂C)-(CR₁₂) trimer, wherein the individual peptide elements in the dimer (e.g. (WR12C)), or the trimer (e.g. (CR12)), are connected via -SH groups.

In a embodiments, at least one nucleic acid of component A and/or component B (e.g. DNA or RNA), preferably the at least one RNA is complexed or associated with a polyethylene glycol/peptide polymer comprising HO-PEG5000-S-(S-CHHHHHHRRRRHHHHHHC-S-)7-S-PEG5000-OH (**SEQ ID NO: 272** as peptide monomer), HO-PEG5000-S-(S-CHHHHHHRRRRHHHHHHC-S-)4-S-PEG5000-OH (**SEQ ID NO: 272** as peptide monomer), HO-PEG5000-S-(S-CGHHHHHRRRRHHHHHGC-S-)7-S-PEG5000-OH (**SEQ ID NO: 273** as peptide monomer) and/or a polyethylene glycol/peptide polymer comprising HO-PEG5000-S-(S-CGHHHHHRRRRHHHHHGC-S-)4-S-PEG5000-OH (**SEQ ID NO: 273** of the peptide monomer).

In other embodiments, the composition comprises at least one nucleic acid of component A and/or component B (e.g. DNA or RNA), wherein the at least one nucleic acid of component A and/or component B, preferably the at least one RNA is complexed or associated with polymeric carriers and, optionally, with at least one lipid component as described in WO2017212008A1, WO2017212006A1, WO2017212007A1, and WO2017212009A1. In this context, the disclosures of WO2017212008A1, WO2017212006A1, WO2017212007A1, and WO2017212009A1 are herewith incorporated by reference.

In preferred embodiments, the polymeric carrier (of the first and/or second component) is a peptide polymer, preferably a polyethylene glycol/peptide polymer as defined above, and a lipid component, preferably a lipidoid component.

A lipidoid (or lipidoit) is a lipid-like compound, i.e. an amphiphilic compound with lipid-like physical properties. The lipidoid is preferably a compound, which comprises two or more cationic nitrogen atoms and at least two lipophilic tails. In contrast to many conventional cationic lipids, the lipidoid may be free of a hydrolysable linking group, in particular linking groups comprising hydrolysable ester, amide or carbamate groups. The cationic nitrogen atoms of the lipidoid may be cationisable or permanently cationic, or both types of cationic nitrogens may be present in the compound. In the context of the present invention, the term lipid is considered to also encompass lipidoids.

In some embodiments of the inventions, the lipidoid may comprise a PEG moiety.

In preferred embodiments, the at least one nucleic acid of component A and/or component B (e.g. DNA or RNA), preferably the at least one RNA acid of component A and/or component B is complexed or associated with a polymeric carrier, preferably with a polyethylene glycol/peptide polymer as defined above, and a lipidoid component.

In embodiments, the at least one nucleic acid of component A and component B are complexed or associated with, or at least partially complexed or partially associated with polymer as defined above, and a lipid or lipidoid component as defined above, wherein component A and component B are formulated separately or wherein component A and component B are co-formulated.

Suitably, the lipidoid is cationic, which means that it is cationisable or permanently cationic. In one embodiment, the lipidoid is cationisable, i.e. it comprises one or more cationisable nitrogen atoms, but no permanently cationic nitrogen atoms. In another embodiment, at least one of the cationic nitrogen atoms of the lipidoid is permanently cationic. Optionally, the lipidoid comprises two permanently cationic nitrogen atoms, three permanently cationic nitrogen atoms, or even four or more permanently cationic nitrogen atoms.

In a preferred embodiment, the lipidoid component may be any one selected from the lipidoids of the lipidoids provided in the table of page 50-54 of published PCT patent application WO2017212009A1, the specific lipidoids provided in said table, and the specific disclosure relating thereto herewith incorporated by reference.

In preferred embodiments, the lipidoid component may be any one selected from 3-C12-OH, 3-C12-OH-cat, 3-C12-amide, 3-C12-amide monomethyl, 3-C12-amide dimethyl, RevPEG(10)-3-C12-OH, RevPEG(10)-DLin-pAbenzoic, 3C12amide-TMA cat., 3C12amide-DMA, 3C12amide-NH2, 3C12amide-OH, 3C12Ester-OH, 3C12 Ester-amin, 3C12Ester-DMA, 2C12Amid-DMA, 3C12-lin-amid-DMA, 2C12-sperm-amid-DMA, or 3C12-sperm-amid-DMA (see table of published PCT patent application WO2017212009A1 (pages 50-54)). Particularly preferred are 3-C12-OH or 3-C12-OH-cat.

In preferred embodiments, the polyethylene glycol/peptide polymer comprising a lipidoid as specified above (e.g. 3-C12-OH or 3-C12-OH-cat), is used to complex the at least one nucleic acid to form complexes having an N/P ratio from about 0.1 to about 20, or from about 0.2 to about 15, or from about 2 to about 15, or from about 2 to about 12, wherein the N/P ratio is defined as the mole ratio of the nitrogen atoms of the basic groups of the cationic peptide or polymer to the phosphate groups of the nucleic acid. In that context, the disclosure of published PCT patent application WO2017212009A1, in particular claims 1 to 10 of WO2017212009A1, and the specific disclosure relating thereto is herewith incorporated by reference.

Further suitable lipidoids may be derived from published PCT patent application WO2010053572. In particular, lipidoids derivable from claims 1 to 297 of published PCT patent application WO2010053572 may be used in the context of the invention, e.g. incorporated into the peptide polymer as described herein, or e.g. incorporated into the lipid nanoparticle (as described below). Accordingly, claims 1 to 297 of published PCT patent application WO2010053572, and the specific disclosure relating thereto, is herewith incorporated by reference.

### Encapsulation/Complexation in liposomes or LNPs:

In preferred embodiments, the at least one nucleic acid of component A and/or the at least one nucleic acid of component B (e.g. DNA or RNA), preferably the at least one RNA of component A and/or the at least one RNA of component B is complexed, encapsulated, partially encapsulated, or associated with one or more lipids (e.g. cationic lipids and/or neutral lipids), thereby forming lipid-based carriers such as liposomes, lipid nanoparticles (LNPs), lipoplexes, and/or nanoliposomes, *preferably* lipid nanoparticles.

In embodiments, the at least one nucleic acid of component A and the at least one nucleic acid of component B are complexed, encapsulated, partially encapsulated, or associated with one or more lipids (e.g. cationic lipids and/or neutral lipids), thereby forming liposomes, lipid nanoparticles (LNPs), lipoplexes, and/or nanoliposomes, *preferably* lipid nanoparticles.

In embodiments, the at least one nucleic acid of component A and/or the at least one nucleic acid of component B are formulated separately (in any formulation or complexation agent defined herein), *preferably* wherein the at least one nucleic acid of component A and/or the at least one nucleic acid of component B are formulated in separate liposomes, lipid nanoparticles (LNP), lipoplexes, and/or nanoliposomes

In embodiments, the at least one nucleic acid of component A and the at least one nucleic acid of component B are co-formulated (in any formulation or complexation agent defined herein), *preferably* wherein the at least one nucleic acid of component A and the at least one nucleic acid of component B are formulated in separate liposomes, lipid nanoparticles (LNP), lipoplexes, and/or nanoliposomes

The liposomes, lipid nanoparticles (LNPs), lipoplexes, and/or nanoliposomes - incorporated nucleic acid (e.g. DNA or RNA) may be completely or partially located in the interior space of the liposomes, lipid nanoparticles (LNPs), lipoplexes, and/or nanoliposomes, within the lipid layer/membrane, or associated with the exterior surface of the lipid layer/membrane. The incorporation of a nucleic acid into liposomes/LNPs is also referred to herein as "encapsulation" wherein the nucleic acid, e.g. the RNA is entirely contained within the interior space of the liposomes, lipid nanoparticles (LNPs), lipoplexes, and/or nanoliposomes. The purpose of incorporating nucleic acid into liposomes, lipid nanoparticles (LNPs), lipoplexes, and/or nanoliposomes is to protect the nucleic acid, preferably RNA from an environment which may contain enzymes or chemicals or conditions that degrade nucleic acid and/or systems or receptors that cause the rapid excretion of the nucleic acid. Moreover, incorporating nucleic acid, preferably RNA into liposomes, lipid nanoparticles (LNPs), lipoplexes, and/or nanoliposomes may promote the uptake of the nucleic acid, and hence, may enhance the therapeutic effect of the nucleic acid of component A and/or component B, e.g. the nucleic acid encoding at least one antigenic peptide or protein. Accordingly, incorporating a nucleic acid of component A or B, e.g. RNA or DNA of component A and/or component B, into liposomes, lipid nanoparticles (LNPs), lipoplexes, and/or nanoliposomes may be particularly suitable for combination vaccine of the invention, e.g. for intramuscular and/or intradermal administration. In this context, the terms "complexed" or "associated" refer to the essentially stable combination of nucleic acid (of component A and/or component B) with one or more lipids into larger complexes or assemblies without covalent binding.

The term "lipid nanoparticle", also referred to as "LNP", is not restricted to any particular morphology, and include any morphology generated when a cationic lipid and optionally one or more further lipids are combined, e.g. in an aqueous environment and/or in the presence of a nucleic acid, e.g. an RNA. For example, a liposome, a lipid complex, a lipoplex and the like are within the scope of a lipid nanoparticle (LNP).

Liposomes, lipid nanoparticles (LNPs), lipoplexes, and/or nanoliposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50nm in diameter, and a large unilamellar vesicle (LUV) which may be between 50nm and 500nm in diameter

LNPs of the invention are suitably characterized as microscopic vesicles having an interior aqua space sequestered from an outer medium by a membrane of one or more bilayers. Bilayer membranes of LNPs are typically formed by amphiphilic molecules, such as lipids of synthetic or natural origin that comprise spatially separated hydrophilic and hydrophobic domains. Bilayer membranes of the liposomes can also be formed by amphophilic polymers and surfactants (e.g., polymerosomes, niosomes, etc.). In the context of the present invention, an LNP typically serves to transport the at least one nucleic acid of component A and/or the at least one nucleic acid of component B, preferably the at least one RNA, to a target tissue.

Accordingly, in preferred embodiments, the at least one nucleic acid of component A and/or the at least one nucleic acid of component B, preferably the at least one RNA, is complexed with one or more lipids thereby forming lipid nanoparticles (LNP), liposomes, nanoliposomes, lipoplexes, preferably LNPs. Preferably, LNPs (liposomes, nanoliposomes, lipoplexes) are particularly suitable for intramuscular and/or intradermal administration.

In embodiments, at least about 80%, 85%, 90%, 95% of lipid-based carriers, preferably the LNPs, have a spherical morphology, preferably comprising a solid core or partially solid core.

LNPs (or liposomes, nanoliposomes, lipoplexes) typically comprise a cationic lipid and one or more excipients selected from neutral lipids, charged lipids, steroids and polymer conjugated lipids (e.g. PEGylated lipid). The nucleic acid (e.g. RNA, DNA) may be encapsulated in the lipid portion of the LNP or an aqueous space enveloped by some or the entire lipid portion of the LNP. The nucleic acid (e.g. RNA, DNA) or a portion thereof may also be associated and complexed with the LNP. An LNP may comprise any lipid capable of forming a particle to which the nucleic acids are attached, or in which the one or more nucleic acids are encapsulated. Preferably, the LNP comprising nucleic acids comprises one or more cationic lipids, and one or more stabilizing lipids. Stabilizing lipids include neutral lipids and PEGylated lipids.

Preferably, the LNP (or liposomes, nanoliposomes, lipoplexes) comprises
(i) at least one cationic lipid;
(ii) at least one neutral lipid;
(iii) at least one steroid or steroid analogue, preferably cholesterol; and
(iv) at least one polymer conjugated lipid, preferably a PEG-lipid;
wherein (i) to (iv) are in a molar ratio of about 20-60% cationic lipid, 5-25% neutral lipid, 25-55% sterol, and 0.5-15% polymer conjugated lipid.

The cationic lipid of an LNP (or liposomes, nanoliposomes, lipoplexes) may be cationisable, i.e. it becomes protonated as the pH is lowered below the pK of the ionizable group of the lipid, but is progressively more neutral at higher pH values. At pH values below the pK, the lipid is then able to associate with negatively charged nucleic acids. In certain embodiments, the cationic lipid comprises a zwitterionic lipid that assumes a positive charge on pH decrease.

Such lipids (for liposomes, lipid nanoparticles (LNP), lipoplexes, and/or nanoliposomes) include, but are not limited to, DSDMA, N,N-dioleyl-N,N-dimethylammonium chloride (DODAC), N,N-distearyl-N,N-dimethylammonium bromide (DDAB), 1,2-dioleoyltrimethyl ammonium propane chloride (DOTAP) (also known as N-(2,3-dioleoyloxy)propyl)-N,N,N-trimethylammonium chloride and 1,2-Dioleyloxy-3-trimethylaminopropane chloride salt), N-(1-(2,3-<Jioleyloxy)propyl)-N,N,N-trimethylammonium chloride (DOTMA), N,N-dimethyl-2,3-dioleyloxy)propylamine (DODMA), ckk-E12, ckk, 1,2-DiLinoleyloxy-N,N-dimethylaminopropane (DLinDMA), 1,2-Dilinolenyloxy-N,N-dimethylaminopropane (DLenDMA), 1,2-di-y-linolenyloxy-N,N-dimethylaminopropane (y-DLenDMA), 98N12-5, 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.CI), ICE (Imidazol-based), HGT5000, HGT5001, DMDMA, CLinDMA, CpLinDMA, DMOBA, DOcarbDAP, DLincarbDAP, DLinCDAP, KLin-K-DMA, DLin-K-XTC2-DMA, XTC (2,2-Dilinoleyl-4-dimethylaminoethyl-[1,3]-dioxolane) HGT4003, 1,2-Dilinoleoyl-3-trimethylaminopropane chloride salt (DLin-TAP.CI), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DM A), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine, (6Z,9Z,28Z,31Z)-heptatriaconta-6,9,28,31-tetraen-19-yl-4-(dimethylamino)butanoate (MC3), ALNY-100 ((3aR,5s,6aS)-N,N-dimethyl-2,2-di((9Z,12Z)-octadeca-9,12-dienyl)tetrahydro-3aH-cyclopenta[d][1,3]dioxol-5-amine)), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), NC98-5 (4,7, 13-tris(3-oxo-3-(undecylamino)propyl)-N ,N 16-diundecyl-4,7, 10,13-tetraazahexadecane-1,16-diamide), (6Z,9Z,282,31Z)-heptatriaconta-6,9,28,31-tetraen-19-yl 4-(dimethylamino) butanoate (DLin-M-C3-DMA), 3-((6Z,92,28Z,312)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), LIPOFECTIN^{®} (commercially available cationic liposomes comprising DOTMA and 1,2-dioleoyl-sn-3phosphoethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.) or any combination of any of the foregoing. Further suitable cationic lipids for use in the compositions and methods of the invention include those described in international patent publications WO2010053572 (and particularly, Cl 2-200 described at paragraph [00225]) and WO2012170930, both of which are incorporated herein by reference, HGT4003, HGT5000, HGTS001, HGT5001, HGT5002 (see US20150140070A1).

In embodiments, the cationic lipid of the liposomes, lipid nanoparticles (LNP), lipoplexes, and/or nanoliposomes may be an amino lipid.

Representative amino lipids include, but are not limited to, 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.CI), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,Ndilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA); dilinoleyl-methyl-4-dimethylaminobutyrate (DLin-MC3-DMA); MC3 (US20100324120).

In embodiments, the cationic lipid of the liposomes, lipid nanoparticles (LNP), lipoplexes, and/or nanoliposomes may an aminoalcohol lipidoid.

Aminoalcohol lipidoids which may be used in the present invention may be prepared by the methods described in U.S. Patent No. 8,450,298, herein incorporated by reference in its entirety. Suitable (ionizable) lipids can also be the compounds as disclosed in Tables 1, 2 and 3 and as defined in claims 1-24 of WO2017075531A1, hereby incorporated by reference.

In another embodiment, suitable lipids can also be the compounds as disclosed in WO2015074085A1 (i.e. ATX-001 to ATX-032 or the compounds as specified in claims 1-26), U.S. Appl. Nos. 61/905,724 and 15/614,499 or U.S. Patent Nos. 9,593,077 and 9,567,296 hereby incorporated by reference in their entirety.

In other embodiments, suitable cationic lipids can also be the compounds as disclosed in WO2017117530A1 (i.e. lipids 13, 14, 15, 16, 17, 18, 19, 20, or the compounds as specified in the claims), hereby incorporated by reference in its entirety.

In preferred embodiments, ionizable or cationic lipids may also be selected from the lipids disclosed in WO2018078053A1 (i.e. lipids derived from formula I, II, and III of WO2018078053A1, or lipids as specified in Claims 1 to 12 of WO2018078053A1), the disclosure of WO2018078053A1 hereby incorporated by reference in its entirety. In that context, lipids disclosed in Table 7 of WO2018078053A1 (e.g. lipids derived from formula I-1 to 1-41) and lipids disclosed in Table 8 of WO2018078053A1 (e.g. lipids derived from formula II-1 to II-36) may be suitably used in the context of the invention. Accordingly, formula I-1 to formula I-41 and formula II-1 to formula II-36 of WO2018078053A1, and the specific disclosure relating thereto, are herewith incorporated by reference.

In preferred embodiments, cationic lipids may be derived from formula III of published PCT patent application WO2018078053A1. Accordingly, formula III of WO2018078053A1, and the specific disclosure relating thereto, are herewith incorporated by reference.

In particularly preferred embodiments, the at least one nucleic acid of component A and/or component B (e.g. DNA or RNA), *preferably* the at least one RNA of of component A and/or component B is complexed with one or more lipids thereby forming LNPs (or liposomes, nanoliposomes, lipoplexes), wherein the cationic lipid of the LNP is selected from structures III-1 to III-36 of Table 9 of published PCT patent application WO2018078053A1. Accordingly, formula III-1 to III-36 of WO2018078053A1, and the specific disclosure relating thereto, are herewith incorporated by reference.

In particularly preferred embodiments, the at least one nucleic acid of component A and/or the at least one nucleic acid of component B (e.g. DNA or RNA), preferably the at least one RNA acid of component A and/or the at least one RNA component B is complexed with one or more lipids thereby forming liposomes, lipid nanoparticles (LNP), lipoplexes, and/or nanoliposomes, *preferably* LNPs, wherein the liposomes, lipid nanoparticles (LNP), lipoplexes, and/or nanoliposomes, *preferably* the LNPs comprise a cationic lipid according to formula III-3:

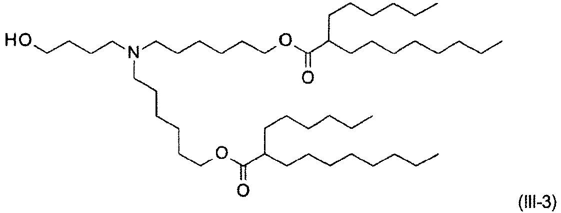

The lipid of formula III-3 as suitably used herein has the chemical term ((4-hydroxybutyl)azanediyl)bis(hexane-6,1-diyl)bis(2-hexyldecanoate), also referred to as ALC-0315.

In certain embodiments, the cationic lipid as defined herein, more preferably cationic lipid compound III-3, is present in the LNP (or liposomes, nanoliposomes, lipoplexes) in an amount from about 30 to about 95 mole percent, relative to the total lipid content of the LNP. If more than one cationic lipid is incorporated within the LNP, such percentages apply to the combined cationic lipids.

In embodiments, the cationic lipid is present in the LNP (or liposomes, nanoliposomes, lipoplexes) in an amount from about 30 to about 70 mole percent. In one embodiment, the cationic lipid is present in the LNP (or liposomes, nanoliposomes, lipoplexes) in an amount from about 40 to about 60 mole percent, such as about 40, 41, 42, 43, 44, 45, 46, 47, 48, 49, 50, 51, 52, 53, 54, 55, 56, 57, 58, 59 or 60 mole percent, respectively. In embodiments, the cationic lipid is present in the LNP (or liposomes, nanoliposomes, lipoplexes) in an amount from about 47 to about 48 mole percent, such as about 47.0, 47.1, 47.2, 47.3, 47.4, 47.5, 47.6, 47.7, 47.8, 47.9, 50.0 mole percent, respectively, wherein 47.7 mole percent are particularly preferred.

In some embodiments, the cationic lipid is present in a ratio of from about 20mol% to about 70 or 75mol% or from about 45 to about 65mol% or about 20, 25, 30, 35, 40, 45, 50, 55, 60, 65, or about 70mol% of the total lipid present in the LNP (or liposomes, nanoliposomes, lipoplexes). In further embodiments, the LNPs (or liposomes, nanoliposomes, lipoplexes) comprise from about 25% to about 75% on a molar basis of cationic lipid, e.g., from about 20 to about 70%, from about 35 to about 65%, from about 45 to about 65%, about 60%, about 57.5%, about 57.1%, about 50% or about 40% on a molar basis (based upon 100% total moles of lipid in the lipid nanoparticle). In some embodiments, the ratio of cationic lipid to nucleic acid (e.g. coding RNA or DNA) is from about 3 to about 15, such as from about 5 to about 13 or from about 7 to about 11.

Other suitable (cationic or ionizable) lipids are disclosed in WO2009086558, WO2009127060, WO2010048536, WO2010054406, WO2010088537, WO2010129709, WO2011153493, WO 2013063468, US20110256175, US20120128760, US20120027803, US8158601, WO2016118724, WO2016118725, WO2017070613, WO2017070620, WO2017099823, WO2012040184, WO2011153120, WO2011149733, WO2011090965, WO2011043913, WO2011022460, WO2012061259, WO2012054365, WO2012044638, WO2010080724, WO201021865, WO2008103276, WO2013086373, WO2013086354, US Patent Nos. 7,893,302, 7,404,969, 8,283,333, 8,466,122 and 8,569,256 and US Patent Publication No. US20100036115, US20120202871, US20130064894, US20130129785, US20130150625, US20130178541, US20130225836, US20140039032 and WO2017112865. In that context, the disclosures of WO2009086558, WO2009127060, WO2010048536, WO2010054406, WO2010088537, WO2010129709, WO2011153493, WO 2013063468, US20110256175, US20120128760, US20120027803, US8158601, WO2016118724, WO2016118725, WO2017070613, WO2017070620, WO2017099823, WO2012040184, WO2011153120, WO2011149733, WO2011090965, WO2011043913, WO2011022460, WO2012061259, WO2012054365, WO2012044638, WO2010080724, WO201021865, WO2008103276, WO2013086373, WO2013086354, US Patent Nos. 7,893,302, 7,404,969, 8,283,333, 8,466,122 and 8,569,256 and US Patent Publication No. US20100036115, US20120202871, US20130064894, US20130129785, US20130150625, US20130178541, US20130225836 and US20140039032 and WO2017112865 specifically relating to (cationic) lipids suitable for LNPs (or liposomes, nanoliposomes, lipoplexes) are incorporated herewith by reference.

In embodiments, amino or cationic lipids as defined herein have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of lipids have to be present in the charged or neutral form. Lipids having more than one protonatable or deprotonatable group, or which are zwitterionic, are not excluded and may likewise suitable in the context of the present invention. In some embodiments, the protonatable lipids have a pKa of the protonatable group in the range of about 4 to about 11, e.g., a pKa of about 5 to about 7.

LNPs (or liposomes, nanoliposomes, lipoplexes) can comprise two or more (different) cationic lipids as defined herein. Cationic lipids may be selected to contribute to different advantageous properties. For example, cationic lipids that differ in properties such as amine pKa, chemical stability, half-life in circulation, half-life in tissue, net accumulation in tissue, or toxicity can be used in the LNP (or liposomes, nanoliposomes, lipoplexes). In particular, the cationic lipids can be chosen so that the properties of the mixed-LNP are more desirable than the properties of a single-LNP of individual lipids.

The amount of the permanently cationic lipid or lipidoid may be selected taking the amount of the nucleic acid cargo into account. In one embodiment, these amounts are selected such as to result in an N/P ratio of the nanoparticle(s) or of the composition in the range from about 0.1 to about 20. In this context, the N/P ratio is defined as the mole ratio of the nitrogen atoms ("N") of the basic nitrogen-containing groups of the lipid or lipidoid to the phosphate groups ("P") of the nucleic acid which is used as cargo. The N/P ratio may be calculated on the basis that, for example, 1ug RNA typically contains about 3nmol phosphate residues, provided that the RNA exhibits a statistical distribution of bases. The "N"-value of the lipid or lipidoid may be calculated on the basis of its molecular weight and the relative content of permanently cationic and - if present - cationisable groups.

In vivo characteristics and behavior of LNPs (or liposomes, nanoliposomes, lipoplexes) can be modified by addition of a hydrophilic polymer coating, e.g. polyethylene glycol (PEG), to the LNP surface to confer steric stabilization. Furthermore, LNPs (or liposomes, nanoliposomes, lipoplexes) can be used for specific targeting by attaching ligands (e.g. antibodies, peptides, and carbohydrates) to its surface or to the terminal end of the attached PEG chains (e.g. via PEGylated lipids or PEGylated cholesterol).

In some embodiments, the LNPs (or liposomes, nanoliposomes, lipoplexes) comprise a polymer conjugated lipid. The term "polymer conjugated lipid" refers to a molecule comprising both a lipid portion and a polymer portion. An example of a polymer conjugated lipid is a PEGylated lipid. The term "PEGylated lipid" refers to a molecule comprising both a lipid portion and a polyethylene glycol portion. PEGylated lipids are known in the art and include 1-(monomethoxy-polyethyleneglycol)-2,3-dimyristoylglycerol (PEG-s-DMG) and the like.

A polymer conjugated lipid as defined herein, e.g. a PEG-lipid, may serve as an aggregation reducing lipid.

In certain embodiments, the LNP (or liposomes, nanoliposomes, lipoplexes) comprises a stabilizing-lipid which is a polyethylene glycol-lipid (PEGylated lipid). Suitable polyethylene glycol-lipids include PEG-modified phosphatidylethanolamine, PEG-modified phosphatidic acid, PEG-modified ceramides (e.g. PEG-CerC14 or PEG-CerC20), PEG-modified dialkylamines, PEG-modified diacylglycerols, PEG-modified dialkylglycerols. Representative polyethylene glycol-lipids include PEG-c-DOMG, PEG-c-DMA, and PEG-s-DMG. In one embodiment, the polyethylene glycol-lipid is N-[(methoxy poly(ethylene glycol)2000)carbamyll-1,2-dimyristyloxlpropyl-3-amine (PEG-c-DMA). In a preferred embodiment, the polyethylene glycol-lipid is PEG-2000-DMG. In one embodiment, the polyethylene glycol-lipid is PEG-c-DOMG). In other embodiments, the LNPs comprise a PEGylated diacylglycerol (PEG-DAG) such as 1-(monomethoxy-polyethyleneglycol)-2,3-dimyristoylglycerol (PEG-DMG), a PEGylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedicate (PEG-S-DMG), a PEGylated ceramide (PEG-cer), or a PEG dialkoxypropylcarbamate such as ω-methoxy(polyethoxy)ethyl-N-(2,3di(tetradecanoxy)propyl)carbamate or 2,3-di(tetradecanoxy)propyl-N-(ω-methoxy(polyethoxy)ethyl)carbamate.

In preferred embodiments, the PEGylated lipid is preferably derived from formula (IV) of published PCT patent application WO2018078053A1. Accordingly, PEGylated lipids derived from formula (IV) of published PCT patent application WO2018078053A1, and the respective disclosure relating thereto, are herewith incorporated by reference.

In a preferred embodiments, the at least one nucleic acid of component A and/or the at least one nucleic acid of component B (e.g. RNA or DNA) of the pharmaceutical composition is complexed with one or more lipids thereby forming LNPs (or liposomes, nanoliposomes, lipoplexes), wherein the LNP comprises a polymer conjugated lipid, preferably a PEGylated lipid, wherein the PEG lipid is preferably derived from formula (IVa) of published PCT patent application WO2018078053A1. Accordingly, PEGylated lipid derived from formula (IVa) of published PCT patent application WO2018078053A1, and the respective disclosure relating thereto, is herewith incorporated by reference.

In a preferred embodiment, the at least one nucleic acid of component A and/or the at least one nucleic acid of component B, preferably the at least one RNA component A and/or component B, is complexed with one or more lipids thereby forming lipid nanoparticles (or liposomes, nanoliposomes, lipoplexes), wherein the LNP (or liposomes, nanoliposomes, lipoplexes) comprises a polymer conjugated lipid, preferably a PEGylated lipid / PEG lipid. In preferred embodiments, said PEG lipid or PEGylated lipid is of formula (IVa): 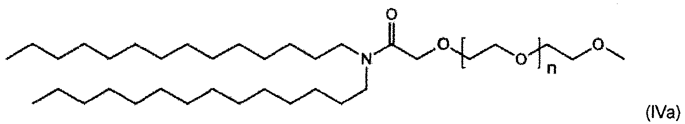 wherein n has a mean value ranging from 30 to 60, such as about 30±2, 32±2, 34±2, 36±2, 38±2, 40±2, 42±2, 44±2, 46±2, 48±2, 50±2, 52±2, 54±2, 56±2, 58±2, or 60±2. In a most preferred embodiment n is about 49. In another preferred embodiment n is about 45. In further preferred embodiments, said PEG lipid is of formula (IVa) wherein n is an integer selected such that the average molecular weight of the PEG lipid is about 2000g/mol to about 3000 g/mol or about 2300g/mol to about 2700g/mol, even more preferably about 2500g/mol.

The lipid of formula IVa as suitably used herein has the chemical term 2[(polyethylene glycol)-2000]-N,N-ditetradecylacetamide, also referred to as ALC-0159.

Further examples of PEG-lipids suitable in that context are provided in US20150376115A1 and WO2015199952, each of which is incorporated by reference in its entirety.

In some embodiments, LNPs (or liposomes, nanoliposomes, lipoplexes) include less than about 3, 2, or 1 mole percent of PEG or PEG-modified lipid, based on the total moles of lipid in the LNP. In further embodiments, LNPs (or liposomes, nanoliposomes, lipoplexes) comprise from about 0.1% to about 20% of the PEG-modified lipid on a molar basis, e.g., about 0.5 to about 10%, about 0.5 to about 5%, about 10%, about 5%, about 3.5%, about 3%, about 2,5%, about 2%, about 1.5%, about 1%, about 0.5%, or about 0.3% on a molar basis (based on 100% total moles of lipids in the LNP). In preferred embodiments, LNPs (or liposomes, nanoliposomes, lipoplexes) comprise from about 1.0% to about 2.0% of the PEG-modified lipid on a molar basis, e.g., about 1.2 to about 1.9%, about 1.2 to about 1.8%, about 1.3 to about 1.8%, about 1.4 to about 1.8%, about 1.5 to about 1.8%, about 1.6 to about 1.8%, in particular about 1.4%, about 1.5%, about 1.6%, about 1.7%, about 1.8%, about 1.9%, most preferably 1.7% (based on 100% total moles of lipids in the LNP). In various embodiments, the molar ratio of the cationic lipid to the PEGylated lipid ranges from about 100:1 to about 25:1.

In preferred embodiments, the LNP (or liposomes, nanoliposomes, lipoplexes) comprises one or more additional lipids, which stabilize the formation of particles during their formulation or during the manufacturing process (e.g. neutral lipid and/or one or more steroid or steroid analogue).

In preferred embodiments, the at least one nucleic acid of component A and/or the at least one nucleic acid of component B, preferably the at least one RNA is complexed with one or more lipids thereby forming lipid nanoparticles (or liposomes, nanoliposomes, lipoplexes), wherein the LNP (or liposomes, nanoliposomes, lipoplexes) comprises one or more neutral lipid and/or one or more steroid or steroid analogue.

Suitable stabilizing lipids include neutral lipids and anionic lipids. The term "neutral lipid" refers to any one of a number of lipid species that exist in either an uncharged or neutral zwitterionic form at physiological pH. Representative neutral lipids include diacylphosphatidylcholines, diacylphosphatidylethanolamines, ceramides, sphingomyelins, dihydro sphingomyelins, cephalins, and cerebrosides.

In embodiments, the LNP (or liposome, nanoliposome, lipoplexe) comprises one or more neutral lipids, wherein the neutral lipid is selected from the group comprising distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoylphosphatidylethanolamine (DSPE), 16-O-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE), or mixtures thereof.

In some embodiments, the LNPs (or liposomes, nanoliposomes, lipoplexes) comprise a neutral lipid selected from DSPC, DPPC, DMPC, DOPC, POPC, DOPE and SM. In various embodiments, the molar ratio of the cationic lipid to the neutral lipid ranges from about 2:1 to about 8:1.

In preferred embodiments, the neutral lipid is 1,2-distearoyl-sn-glycero-3-phosphocholine (DSPC). Suitably, the molar ratio of the cationic lipid to DSPC may be in the range from about 2:1 to about 8:1.

In preferred embodiments, the steroid is cholesterol. Suitably, the molar ratio of the cationic lipid to cholesterol may be in the range from about 2:1 to about 1:1. In some embodiments, the cholesterol may be PEGylated.

The sterol can be about 10mol% to about 60mol% or about 25mol% to about 40mol% of the lipid particle. In one embodiment, the sterol is about 10, 15, 20, 25, 30, 35, 40, 45, 50, 55, or about 60mol% of the total lipid present in the lipid particle. In another embodiment, the LNPs include from about 5% to about 50% on a molar basis of the sterol, e.g., about 15% to about 45%, about 20% to about 40%, about 48%, about 40%, about 38.5%, about 35%, about 34.4%, about 31.5% or about 31% on a molar basis (based upon 100% total moles of lipid in the lipid nanoparticle).

Preferably, lipid LNPs (or liposomes, nanoliposomes, lipoplexes) comprise:
(a) the at least one nucleic acid of component A and/or at least one nucleic acid of component B, preferably the at least one RNA of component A and/or component B, (b) a cationic lipid, (c) an aggregation reducing agent (such as polyethylene glycol (PEG) lipid or PEG-modified lipid), (d) optionally a non-cationic lipid (such as a neutral lipid), and (e) optionally, a sterol.

In some embodiments, the cationic lipids (as defined above), non-cationic lipids (as defined above), cholesterol (as defined above), and/or PEG-modified lipids (as defined above) may be combined at various relative molar ratios. For example, the ratio of cationic lipid to non-cationic lipid to cholesterol-based lipid to PEGylated lipid may be between about 30-60:20-35:20-30:1-15, or at a ratio of about 40:30:25:5, 50:25:20:5, 50:27:20:3, 40:30:20:10, 40:32:20:8, 40:32:25:3 or 40:33:25:2, or at a ratio of about 50:25:20:5, 50:20:25:5, 50:27:20:3 40:30:20: 10,40:30:25:5 or 40:32:20:8, 40:32:25:3 or 40:33:25:2, respectively.

In some embodiments, the LNPs (or liposomes, nanoliposomes, lipoplexes) comprise a lipid of formula (III), the at least one nucleic acid of component A and/or component B, *preferably* the at least one RNA as defined herein, a neutral lipid, a steroid and a PEGylated lipid. In preferred embodiments, the lipid of formula (III) is lipid compound III-3 (ALC-0315), the neutral lipid is DSPC, the steroid is cholesterol, and the PEGylated lipid is the compound of formula (Iva ALC-0159).

In a preferred embodiment, the LNP (or liposomes, nanoliposomes, lipoplexes) consists essentially of (i) at least one cationic lipid; (ii) a neutral lipid; (iii) a sterol, e.g. , cholesterol; and (iv) a PEG-lipid, e.g. PEG-DMG or PEG-cDMA, in a molar ratio of about 20-60% cationic lipid: 5-25% neutral lipid: 25-55% sterol; 0.5-15% PEG-lipid.

In particularly preferred embodiments, the at least one nucleic acid of component A and/or component B, preferably the at least one RNA of component A and/or component B is complexed with one or more lipids thereby forming lipid nanoparticles (or liposomes, nanoliposomes, lipoplexes), wherein the LNP (or liposome, nanoliposome, lipoplex) comprises
(i) at least one cationic lipid as defined herein, preferably a lipid of formula (III), more preferably lipid III-3 (ALC-0315);
(ii) at least one neutral lipid as defined herein, preferably 1,2-distearoyl-sn-glycero-3-phosphocholine (DSPC);
(iii) at least one steroid or steroid analogue as defined herein, preferably cholesterol; and
(iv) at least one polymer conjugated lipid, *preferably* a PEG-lipid as defined herein, e.g. PEG-DMG or PEG-cDMA, preferably a PEGylated lipid that is or is derived from formula (IVa ALC-0159).

In particularly preferred embodiments, the at least one nucleic acid of component A and/or component B, preferably the at least one RNA of component A and/or component B is complexed with one or more lipids thereby forming lipid nanoparticles (LNP), wherein the LNP comprises (i) to (iv) in a molar ratio of about 20-60% cationic lipid: 5-25% neutral lipid: 25-55% sterol; 0.5-15% polymer conjugated lipid, *preferably* PEG-lipid.

In one preferred embodiment, the lipid nanoparticle (or liposome, nanoliposome, lipoplexe) comprises: a cationic lipid with formula (III) and/or PEG lipid with formula (IV), optionally a neutral lipid, preferably 1,2-distearoyl-sn-glycero-3-phosphocholine (DSPC) and optionally a steroid, preferably cholesterol, wherein the molar ratio of the cationic lipid to DSPC is optionally in the range from about 2:1 to 8:1, wherein the molar ratio of the cationic lipid to cholesterol is optionally in the range from about 2:1 to 1:1.

In a particular preferred embodiment, the composition comprises the at least one nucleic acid of component A and/or at least one nucleic acid of component B, preferably the at least one RNA, comprises lipid nanoparticles (LNPs), which have a molar ratio of approximately 50:10:38.5:1.5, preferably 47.5:10:40.8:1.7 or more preferably 47.4:10:40.9:1.7 (i.e. proportion (mol%) of cationic lipid (preferably lipid III-3 (ALC-0315)), DSPC, cholesterol and polymer conjugated lipid, preferably PEG-lipid (preferably PEG-lipid of formula (IVa) with n = 49, even more preferably PEG-lipid of formula (IVa) with n = 45; ALC-0159); solubilized in ethanol).

The total amount of nucleic acid in the lipid nanoparticles may vary and is defined depending on the e.g. nucleic acid to total lipid w/w ratio. In one embodiment of the invention the nucleic acid, in particular the RNA to total lipid ratio is less than 0.06 w/w, preferably between 0.03 w/w and 0.04 w/w.

In preferred embodiments, the wt/wt ratio of lipid to nucleic acid is from about 10:1 to about 60:1, preferably from about 20:1 to about 30:1, for example about 25:1.

In preferred embodiments, the n/p ratio of the liposome, lipid nanoparticle (LNP), lipoplex, and/or nanoliposome encapsulating the nucleic acid is in a range from about 1 to about 10, preferably in a range from about 5 to about 7, more preferably about 6.

In some embodiments, the lipid nanoparticles (or liposomes, nanoliposomes, lipoplexes) are composed of only three lipid components, namely imidazole cholesterol ester (ICE), 1,2-dioleoyl-sn-glycero-3-phosphoethanolamine (DOPE), and 1,2-dimyristoyl-sn-glycerol, methoxypolyethylene glycol (DMG-PEG-2K)

In one embodiment, the lipid nanoparticle (or liposomes, nanoliposomes, lipoplexes) of the composition comprises a cationic lipid, a steroid, a neutral lipid, and a polymer conjugated lipid, preferably a pegylated lipid. Preferably, the polymer conjugated lipid is a pegylated lipid or PEG-lipid. In a specific embodiment, lipid nanoparticles comprise a cationic lipid resembled by the cationic lipid COATSOME^{®} SS-EC (former name: SS-33/4PE-15; NOF Corporation, Tokyo, Japan), in accordance with the following formula

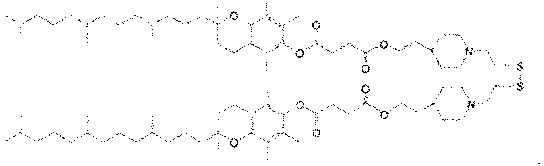

As described further below, those lipid nanoparticles are termed "GN01".

Furthermore, in a specific embodiment, the GN01 lipid nanoparticles comprise a neutral lipid being resembled by the structure 1,2-diphytanoyl-sn-glycero-3-phosphoethanolamine (DPhyPE):

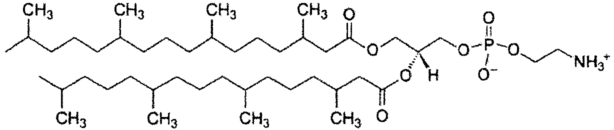

Furthermore, in a specific embodiment, the GN01 lipid nanoparticles comprise a polymer conjugated lipid, preferably a pegylated lipid, being 1,2-dimyristoyl-rac-glycero-3-methoxypolyethylene glycol 2000 (DMG-PEG 2000) having the following structure:

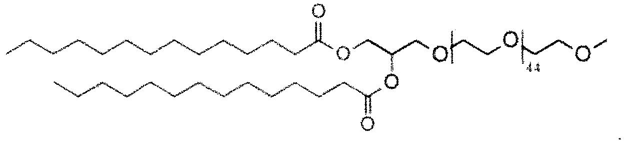

As used in the art, "DMG-PEG 2000" is considered a mixture of 1,2-DMG PEG2000 and 1,3-DMG PEG2000 in -97:3 ratio.

Accordingly, GN01 lipid nanoparticles (GN01-LNPs) according to one of the preferred embodiments comprise a SS-EC cationic lipid, neutral lipid DPhyPE, cholesterol, and the polymer conjugated lipid (pegylated lipid) 1,2-dimyristoyl-rac-glycero-3-methoxypolyethylene glycol (PEG-DMG).

In a preferred embodiment, the GN01 LNPs comprise:
(a) cationic lipid SS-EC (former name: SS-33/4PE-15; NOF Corporation, Tokyo, Japan) at an amount of 45-65 mol%;
(b) cholesterol at an amount of 25-45 mol%;
(c) DPhyPE at an amount of 8-12 mol%; and
(d) PEG-DMG 2000 at an amount of 1-3 mol%;
each amount being relative to the total molar amount of all lipidic excipients of the GN01 lipid nanoparticles.

In a further preferred embodiment, the GN01 lipid nanoparticles as described herein comprises 59mol% cationic lipid, 10mol% neutral lipid, 29.3mol% steroid and 1.7mol% polymer conjugated lipid, preferably pegylated lipid. In a most preferred embodiment, the GN01 lipid nanoparticles as described herein comprise 59mol% cationic lipid SS-EC, 10mol% DPhyPE, 29.3mol% cholesterol and 1.7mol% DMG-PEG 2000.

The amount of the cationic lipid relative to that of the nucleic acid in the GN01 lipid nanoparticle may also be expressed as a weight ratio (abbreviated e.g. "m/m"). For example, the GN01 lipid nanoparticles comprise the at least one nucleic acid, preferably the at least one RNA at an amount such as to achieve a lipid to RNA weight ratio in the range of about 20 to about 60, or about 10 to about 50. In other embodiments, the ratio of cationic lipid to nucleic acid or RNA is from about 3 to about 15, such as from about 5 to about 13, from about 4 to about 8 or from about 7 to about 11. In a very preferred embodiment of the present invention, the total lipid/RNA mass ratio is about 40 or 40, i.e. about 40 or 40 times mass excess to ensure RNA encapsulation. Another preferred RNA/lipid ratio is between about 1 and about 10, about 2 and about 5, about 2 and about 4, or preferably about 3.

Further, the amount of the cationic lipid may be selected taking the amount of the nucleic acid cargo such as the RNA compound into account. In one embodiment, the N/P ratio can be in the range of about 1 to about 50. In another embodiment, the range is about 1 to about 20, about 1 to about 10, about 1 to about 5. In one preferred embodiment, these amounts are selected such as to result in an N/P ratio of the GN01 lipid nanoparticles or of the composition in the range from about 10 to about 20. In a further very preferred embodiment, the N/P is 14 (i.e. 14 times mol excess of positive charge to ensure nucleic acid encapsulation).

In a preferred embodiment, GN01 lipid nanoparticles comprise 59mol% cationic lipid COATSOME^{®} SS-EC (former name: SS-33/4PE-15 as apparent from the examples section; NOF Corporation, Tokyo, Japan), 29.3mol% cholesterol as steroid, 10mol% DPhyPE as neutral lipid / phospholipid and 1.7mol% DMG-PEG 2000 as polymer conjugated lipid. A further inventive advantage connected with the use of DPhyPE is the high capacity for fusogenicity due to its bulky tails, whereby it is able to fuse at a high level with endosomal lipids. For "GN01", N/P (lipid to nucleic acid, e.g. RNA mol ratio) preferably is 14 and total lipid/RNA mass ratio preferably is 40 (m/m).

In other embodiments, the at least one nucleic acid of component A and/or the at least one nucleic acid of component B, preferably the at least one RNA is complexed with one or more lipids thereby forming lipid nanoparticles (or liposomes, nanoliposomes, lipoplexes), wherein the LNP (or liposomes, nanoliposomes, lipoplexes) comprises
I. at least one cationic lipid;
Ii. at least one neutral lipid;
Iii. at least one steroid or steroid analogue; and
Iiii. at least one PEG-lipid as defined herein,
wherein the cationic lipid is DLin-KC2-DMA (50mol%) or DLin-MC3-DMA (50mol%), the neutral lipid is DSPC (10mol%), the PEG lipid is PEG-DOMG (1.5mol%) and the structural lipid is cholesterol (38.5mol%).

In other embodiments, the at least one nucleic acid of component A and/or component B (e.g. DNA or RNA), preferably the at least one RNA is complexed with one or more lipids thereby forming lipid nanoparticles (LNP), wherein the LNP comprises SS15 / Chol / DOPE (or DOPC) / DSG-5000 at mol% 50/38.5/10/1.5.

In other embodiments, the nucleic acid of component A and/or component B may be formulated in liposomes, e.g. in liposomes as described in WO2019222424, WO2019226925, WO2019232095, WO2019232097, or WO2019232208, the disclosure of WO2019222424, WO2019226925, WO2019232095, WO2019232097, or WO2019232208 relating to liposomes or lipid-based carrier molecules herewith incorporated by reference.

In various embodiments, LNPs that suitably encapsulates the at least one nucleic acid of component A and/or component B have a mean diameter of from about 50nm to about 200nm, from about 60nm to about 200nm, from about 70nm to about 200nm, from about 80nm to about 200nm, from about 90nm to about 200nm, from about 90nm to about 190nm, from about 90nm to about 180nm, from about 90nm to about 170nm, from about 90nm to about 160nm, from about 90nm to about 150nm, from about 90nm to about 140nm, from about 90nm to about 130nm, from about 90nm to about 120nm, from about 90nm to about 100nm, from about 70nm to about 90nm, from about 80nm to about 90nm, from about 70nm to about 80nm, or about 30nm, 35nm, 40nm, 45nm, 50nm, 55nm, 60nm, 65nm, 70nm, 75nm, 80nm, 85nm, 90nm, 95nm, 100nm, 105nm, 110nm, 115nm, 120nm, 125nm, 130nm, 135nm, 140nm, 145nm, 150nm, 160nm, 170nm, 180nm, 190nm, or 200nm and are substantially non-toxic. As used herein, the mean diameter may be represented by the z-average size as determined by dynamic light scattering as commonly known in the art.

In another preferred embodiment of the invention the lipid nanoparticles have a hydrodynamic diameter in the range from about 50nm to about 300nm, or from about 60nm to about 250nm, from about 60nm to about 150nm, or from about 60nm to about 120nm, respectively.

In another preferred embodiment of the invention the lipid nanoparticles have a hydrodynamic diameter in the range from about 50nm to about 300nm, or from about 60nm to about 250nm, from about 60nm to about 150nm, or from about 60nm to about 120nm, respectively.

In another preferred embodiments, the lipid nanoparticles have a Z-average size in a range of about 60nm to about 120nm, preferably less than about 120nm, more preferably less than about 100nm, most preferably less than about 80nm.

Suitably, LNPs comprise less than about 10%, 9%, 8%, 7%, 6%, 5%, 4%, 3%, 2%, 1% LNPs that have a particle size exceeding about 500nm. Suitably, the LNPs comprise less than about 10%, 9%, 8%, 7%, 6%, 5%, 4%, 3%, 2%, 1% LNPs that have a particle size smaller than about 20nm.

The polydispersity index (PDI) of the nanoparticles (e.g. LNPs) is typically in the range of 0.1 to 0.5. In a particular embodiment, a PDI is below 0.2. Typically, the PDI is determined by dynamic light scattering.

In preferred embodiments, the composition has a polydispersity index (PDI) value of less than about 0.4, preferably of less than about 0.3, more preferably of less than about 0.2, most preferably of less than about 0.1.

In various embodiments, nucleic acid, e.g., RNA of the pharmaceutical composition does not exceed a certain proportion of free RNA.

In this context, the term "free RNA" or "non-complexed RNA" or "non-encapsulated RNA" comprise the RNA molecules that are not encapsulated in the lipid-based carriers as defined herein. During formulation of the composition (e.g. during encapsulation of the RNA into the lipid-based carriers), free RNA may represent a contamination or an impurity.

The skilled person can choose from a variety of different methods for determining the amount and/or the proportion of free nucleic acid of free RNA in the composition. Free RNA in the composition may be determined by chromatographic methods (e.g. AEX, SEC) or by using probes (e.g. dyes) that bind to free RNA in the composition. In the context of the invention, the amount of free RNA or non-encapsulated RNA may be determined using a dye based assay. Suitable dyes that may be used to determine the amount and/or the proportion of free RNA comprise RiboGreen^{®}, PicoGreen^{®} dye, OliGreen^{®}dye, QuantiFluor^{®} RNA dye, Qubit^{®} RNA dye, Quant-iT^{™} RNA dye, TOTO^{®}-1 dye, YOYO^{®}-1 dye. Such dyes are suitable to discriminate between free RNA and encapsulated RNA. Reference standards consisting of defined amounts of free RNA or encapsulated RNA may be used and mixed with the respective reagent (e.g. RiboGreen^{®} reagent (Excitation 500nm/Emission 525nm)) as recommended by the supplier's instructions. Typically, the free RNA of the composition is quantitated using the Quant-iT RiboGreen RNA Reagent according to the manufacturer's instructions. The proportion of free RNA in the context of the invention is typically determined using a RiboGreen assay.

In embodiments, a composition comprises free nucleic acid, such as free RNA ranging from about 30% to about 0%. In embodiments, the composition comprises about 20% free RNA (and about 80% encapsulated RNA), about 15% free RNA (and about 85% encapsulated RNA), about 10% free RNA (and about 90% encapsulated RNA), or about 5% free RNA (and about 95% encapsulated RNA). In preferred embodiments, the composition comprises less than about 20% free RNA, preferably less than about 15% free RNA, more preferably less than about 10% free RNA, most preferably less than about 5% free RNA.

In aspects comprising RNA nucleic acids, the term "encapsulated RNA" comprise the RNA molecules that are encapsulated in the lipid-based carriers as defined herein. The proportion of encapsulated RNA in the context of the invention is typically determined using a RiboGreen assay.

Accordingly, in embodiments, about 70% to about 100% of the RNA in the composition is encapsulated in the lipid-based carriers. In embodiments, the composition comprises about 80% encapsulated RNA (and about 20% free RNA), about 85% encapsulated RNA (and about 15% free RNA), about 90% encapsulated RNA (and about 10% free RNA), or about 95% encapsulated RNA (and 5% about free RNA).

In preferred embodiments, 80% of the nucleic acid (e.g., RNA) comprised in the composition is encapsulated, preferably 85% of the RNA comprised in the composition is encapsulated, more preferably 90% of the RNA comprised in the composition is encapsulated, most preferably 95% of the RNA comprised in the composition is encapsulated.

In embodiments, the lipid-based carriers encapsulating the RNA has been purified by at least one purification step, preferably by at least one step of TFF and/or at least one step of clarification and/or at least one step of filtration. Suitably, the purified composition w comprises less than about 500ppM ethanol, preferably less than about 50ppM ethanol, more preferably less than about 5ppM ethanol.

In various embodiments, the pharmaceutical composition of the first aspect comprises a sugar in a concentration of about 50mM to about 300mM, preferably sucrose in a concentration of about 150mM.

In embodiments where more than one or a plurality, e.g. 2, 3, 4, 5, 6, 7, 8, 9, 10, 11, 12, 13, 14, 15 of nucleic acid species of component A and/or component B of the invention are comprised in the composition, said more than one or said plurality e.g. 2, 3, 4, 5, 6, 7, 8, 9, 10, 11, 12, 13, 14, 15 of nucleic acid species may be complexed within one or more lipids thereby forming LNPs comprising more than one or a plurality, e.g. 2, 3, 4, 5, 6, 7, 8, 9, 10, 11, 12, 13, 14, 15 of different nucleic acid species.

In embodiments, the nucleic acid formulation (e.g. Polymers, LNPs, liposomes, nanoliposomes, lipoplexes) described herein may be lyophilized in order to improve storage stability of the formulation and/or the nucleic acid of component A and/or component B. In embodiments, the nucleic acid formulation (e.g. Polymers, LNPs, liposomes, nanoliposomes, lipoplexes) described herein may be spray dried in order to improve storage stability of the formulation and/or the nucleic acid of component A and/or B. Lyoprotectants for lyophilization and or spray drying may be selected from trehalose, sucrose, mannose, dextran and inulin. A preferred lyoprotectant is sucrose, optionally comprising a further lyoprotectant. A further preferred lyoprotectant is trehalose, optionally comprising a further lyoprotectant.

Suitably, the pharmaceutical composition, e.g. the composition comprising LNPs, is lyophilized (e.g. according to WO2016165831 or WO2011069586) to yield a temperature stable dried nucleic acid (powder) composition as defined herein (e.g. RNA or DNA). The composition, e.g. the composition comprising LNPs, may also be dried using spray-drying or spray-freeze drying (e.g. according to WO2016184575 or WO2016184576) to yield a temperature stable composition (powder) as defined herein.

Accordingly, in preferred embodiments, the pharmaceutical composition is a dried composition.

The term "dried composition" as used herein has to be understood as composition that has been lyophilized, or spray-dried, or spray-freeze dried as defined above to obtain a temperature stable dried composition (powder) e.g. comprising LNP complexed RNA (as defined above).

In embodiments, lyophilized or spray-dried composition has a water content of less than about 10%.

In preferred embodiments, lyophilized or spray-dried composition has a water content of between about 0.5% and 5%.

In preferred embodiments, the lyophilized or spray-dried composition is stable for at least 2 months after storage at about 5 °C, preferably for at least 3 months, 4 months, 5 months, 6 months.

According to further embodiments, the pharmaceutical composition may comprise at least one adjuvant.

Suitably, the adjuvant is preferably added to enhance the immunostimulatory properties of the composition.

The term "adjuvant" as used herein will be recognized and understood by the person of ordinary skill in the art, and is for example intended to refer to a pharmacological and/or immunological agent that may modify, e.g. enhance, the effect of other agents or that may be suitable to support administration and delivery of the composition. The term "adjuvant" refers to a broad spectrum of substances. Typically, these substances are able to increase the immunogenicity of antigens. For example, adjuvants may be recognized by the innate immune systems and, e.g., may elicit an innate immune response (that is, a nonspecific immune response). "Adjuvants" typically do not elicit an adaptive immune response. In the context of the invention, adjuvants may enhance the effect of the antigenic peptide or protein provided by the nucleic acid. In that context, the at least one adjuvant may be selected from any adjuvant known to a skilled person and suitable for the present case, i.e. supporting the induction of an immune response in a subject, e.g. in a human subject.

Accordingly, the pharmaceutical composition may comprise at least one adjuvant, wherein the at least one adjuvant may be suitably selected from any adjuvant provided in WO2016203025. Adjuvants disclosed in any of the claims 2 to 17 of WO2016203025, preferably adjuvants disclosed in claim 17 of WO2016203025 are particularly suitable, the specific content relating thereto herewith incorporated by reference. Adjuvants thay suitably used and comprised in the composition of the first aspect, or the combination vaccine of the second aspect, to e.g. reduce the amount of nucleic acid (of component A and/or component B) required for a sufficient immune response against the encoded protein and/or the improve the efficacy of the composition / the vaccine for treatment/vaccination, in particular of the elderly. A suitable adjuvant in the context of a coronavirus composition or vaccine (in particular for compositions comprising a polypeptide of the third aspect) may be a Toll-like receptor 9 (TLR9) agonist adjuvant, CpG 1018TM.

The pharmaceutical composition may comprise, besides the components specified herein, at least one further component which may be selected from the group consisting of further antigens (e.g. in the form of a peptide or protein, preferably derived from a coronavirus as specified herein and/or a further virus as specified herein) or further antigen-encoding nucleic acids; a further immunotherapeutic agent; one or more auxiliary substances (cytokines, such as monokines, lymphokines, interleukins or chemokines); or any further compound, which is known to be immune stimulating due to its binding affinity (as ligands) to human Toll-like receptors; and/or an adjuvant nucleic acid, preferably an immunostimulatory RNA (isRNA), e.g. CpG-RNA etc.

In embodiments, the pharmaceutical composition comprises at least one antagonist of at least one RNA sensing pattern recognition receptor. Such an antagonist may preferably be co-formulated in lipid-based carriers as defined herein.

Suitable antagonist of at least one RNA sensing pattern recognition receptor are disclosed in PCT patent application PCT/EP2020/072516, the full disclosure herewith incorporated by reference. In particular, the disclosure relating to suitable antagonist of at least one RNA sensing pattern recognition receptors as defined in any one of the claims 1 to 94 of PCT/EP2020/072516 are incorporated.

In preferred embodiments, the composition comprises at least one antagonist of at least one RNA sensing pattern recognition receptor selected from a Toll-like receptor, preferably TLR7 and/or TLR8.

In embodiments, the at least one antagonist of at least one RNA sensing pattern recognition receptor is selected from a nucleotide, a nucleotide analog, a nucleic acid, a peptide, a protein, a small molecule, a lipid, or a fragment, variant or derivative of any of these.

In preferred embodiments, the at least one antagonist of at least one RNA sensing pattern recognition receptor is a single stranded oligonucleotide, preferably a single stranded RNA Oligonucleotide.

In embodiments, the antagonist of at least one RNA sensing pattern recognition receptor is a single stranded oligonucleotide that comprises or consists of a nucleic acid sequence identical or at least 70%, 80%, 85%, 86%, 87%, 88%, 89%, 90%, 91%, 92%, 93%, 94%, 95%, 96%, 97%, 98%, or 99% identical to a nucleic acid sequence
selected from the group consisting of SEQ ID NOs: 85-212 of PCT/EP2020/072516, or fragments of any of these sequences. In preferred embodiments, the antagonist of at least one RNA sensing pattern recognition receptor is a single stranded oligonucleotide that comprises or consists of a nucleic acid sequence identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 85-87, 149-212 of PCT/EP2020/072516, or fragments of any of these sequences.

A particularly preferred antagonist of at least one RNA sensing pattern recognition receptor in the context of the invention is 5'-GAG CGmG CCA-3' (SEQ ID NO: 85 of PCT/EP20201072516), or a fragment thereof.

In embodiments, the molar ratio of the at least one antagonist of at least one RNA sensing pattern recognition receptor as defined herein to the at least one nucleic acid, preferably RNA encoding an antigenic peptide or protein as defined herein suitably ranges from about 1:1, to about 100:1, or ranges from about 20:1, to about 80:1.

In embodiments, the wherein the weight to weight ratio of the at least one antagonist of at least one RNA sensing pattern recognition receptor as defined herein to the at least one nucleic acid, preferably RNA encoding an antigenic peptide or protein as defined herein suitably ranges from about 1:1, to about 1:30, or ranges from about 1:2, to about 1:10.

### Combinations of component A and component B:

In the following, suitable features and embodiments relating to suitable combinations of component A (in particular, component A-1, A-2, A-3) and component B (in particular component B-1, B-2, B-3, B-3a, B-3b, B-4, B-4a, B-4b) of the pharmaceutical composition (and the combination vaccine) are further specified.

As outlined herein, the invention relates to a pharmaceutical composition comprising at least one component A and at least one component B, wherein component A comprises at least one nucleic acid comprising at least one coding sequence encoding at least one antigenic peptide or protein selected or derived from at least one Coronavirus, or an immunogenic fragment or immunogenic variant thereof.

Suitably, component A is selected from at least one of component
A-1 at least one nucleic acid comprising at least one coding sequence encoding at least one antigenic peptide or protein selected or derived from at least one SARS-CoV-2 or an immunogenic fragment or immunogenic variant thereof, preferably as defined herein, and/or
A-2 at least one nucleic acid comprising at least one coding sequence encoding at least one antigenic peptide or protein selected or derived from at least one SARS-associated virus (e.g. SARS-CoV-1) or an immunogenic fragment or immunogenic variant thereof, preferably as defined herein, and/or
A-3 at least one nucleic acid comprising at least one coding sequence encoding at least one antigenic peptide or protein selected or derived from at least one MERS-CoV or an immunogenic fragment or immunogenic variant thereof, preferably as defined herein.

It has to be understood that component A-1 comprises at least one nucleic acid encoding at least one antigenic peptide or protein selected or derived from at least one SARS-CoV-2 as defined in paragraph "*Component A-1: SARS-CoV-2',* preferably having a nucleic acid sequence, preferably an mRNA sequence, as defined in the context of component A-1. Suitable features of said nucleic acid sequence encoding a SARS-CoV-2 antigen are provided in paragraph *"Nucleic acid features and embodiments".* Further, it is preferred that the at least one nucleic acid encoding a SARS-CoV-2 antigen is formulated and/or complexed. Suitable formulations or complexations of said nucleic acid encoding a SARS-CoV-2 antigen are provided in paragraph *"Formulation and complexation".*

It has to be understood that component A-2 comprises at least one nucleic acid encoding at least one antigenic peptide or protein selected or derived from at least one SARS-associated virus (e.g. SARS-CoV-1) as defined in paragraph "*Component A-2: SARS-associated virus*", preferably having a nucleic acid sequence, preferably an mRNA sequence, as defined in the context of component A-2. Suitable features of said nucleic acid sequence encoding at least one SARS-associated virus antigen are provided in paragraph *"*N*ucleic acid features and embodiments*"*.* Further, it is preferred that the at least one nucleic acid encoding at least one SARS-associated virus antigen is formulated and/or complexed. Suitable formulations or complexations of said nucleic acid encoding at least one SARS-associated virus antigen are provided in paragraph "*Formulation and complexation*"*.*

It has to be understood that component A-3 comprises at least one nucleic acid encoding at least one antigenic peptide or protein selected or derived from at least one MERS-CoV as defined in paragraph "*Component A-3: MERS-CoV"*, preferably having a nucleic acid sequence, preferably an mRNA sequence, as defined in the context of component A-3. Suitable features of said nucleic acid sequence encoding at least one MERS-CoV antigen are provided in paragraph "*Nucleic acid features and embodiments*". Further, it is preferred that the at least one nucleic acid encoding at least one MERS-CoV antigen is formulated and/or complexed. Suitable formulations or complexations of said nucleic acid encoding at least one MERS-CoV antigen are provided in paragraph "*Formulation and complexation*".

In embodiments, component A is selected from A-1 and A-2; A-1 and A-3; A-2 and A-3; or A-1 and A-2 and A-3.

In preferred embodiments, component A is selected from component A-1 (SARS-CoV-2).

As outlined herein, the invention relates to a pharmaceutical composition comprising at least one component A and at least one component B, wherein component B comprises at least one nucleic acid comprising at least one coding sequence encoding at least one antigenic peptide or protein selected or derived from at least one further virus, or an immunogenic fragment or immunogenic variant thereof.

Suitably, component B is selected from at least one of component
B-1 at least one nucleic acid comprising at least one coding sequence encoding at least one antigenic peptide or protein selected or derived from at least one different Coronavirus or an immunogenic fragment or immunogenic variant thereof, preferably as defined herein, and/or
B-2 at least one nucleic acid comprising at least one coding sequence encoding at least one antigenic peptide or protein selected or derived from at least one Influenza virus or an immunogenic fragment or immunogenic variant thereof, preferably as defined herein, and/or
B-3 at least one nucleic acid comprising at least one coding sequence encoding at least one antigenic peptide or protein selected or derived from at least one Pneumoviridae virus or an immunogenic fragment or immunogenic variant thereof, preferably as defined herein, and/or
B-4 at least one nucleic acid comprising at least one coding sequence encoding at least one antigenic peptide or protein selected or derived from at least one Paramyxoviridae virus or an immunogenic fragment or immunogenic variant thereof, preferably as defined herein.

It has to be understood that component B-1 comprises at least one nucleic acid encoding at least one antigenic peptide or protein selected or derived from at least one different Coronavirus as defined in paragraph "*Component B-1: Different Coronavirus*", preferably having a nucleic acid sequence, preferably an mRNA sequence, as defined in the context of component B-1. Suitable features of said nucleic acid sequence encoding an antigen of a different Coronavirus are provided in paragraph "*Nucleic acid features and embodiments*". Further, it is preferred that the at least one nucleic acid encoding a different Coronavirus antigen is formulated and/or complexed. Suitable formulations or complexations of said nucleic acid encoding a different Coronavirus antigen are provided in paragraph "*Formulation and complexation*".

It has to be understood that component B-2 comprises at least one nucleic acid encoding at least one antigenic peptide or protein selected or derived from at least one Influenza virus as defined in paragraph "*Component B-2: Influenza virus*", preferably having a nucleic acid sequence, preferably an mRNA sequence, as defined in the context of component B-2. Suitable features of said nucleic acid sequence encoding at least one Influenza virus antigen are provided in paragraph "*Nucleic acid features and embodiments*". Further, it is preferred that the at least one nucleic acid encoding at least one Influenza virus antigen is formulated and/or complexed. Suitable formulations or complexations of said nucleic acid encoding at least one Influenza virus antigen are provided in paragraph "*Formulation and complexation*".

It has to be understood that component B-3 comprises at least one nucleic acid encoding at least one antigenic peptide or protein selected or derived from at least one Pneumoviridae virus (e.g. RSV and/or hMPV) as defined in paragraph "*Component B-3: Pneumoviridae virus*", preferably having a nucleic acid sequence, preferably an mRNA sequence, as defined in the context of component B-3. Suitable features of said nucleic acid sequence encoding at least one Pneumoviridae virus (e.g. RSV and/or hMPV) antigen are provided in paragraph "*Nucleic acid features and embodiments*". Further, it is preferred that the at least one nucleic acid encoding at least one Pneumoviridae virus (e.g. RSV and/or hMPV) antigen is formulated and/or complexed. Suitable formulations or complexations of said nucleic acid encoding at least one Pneumoviridae virus (e.g. RSV and/or hMPV) antigen are provided in paragraph "*Formulation and complexation*".

It has to be understood that component B-4 comprises at least one nucleic acid encoding at least one antigenic peptide or protein selected or derived from at least one Paramyxoviridae virus (e.g. PIV and/or Henipavirus) as defined in paragraph "*Component B-4: Paramyxoviridae virus*", preferably having a nucleic acid sequence, preferably an mRNA sequence, as defined in the context of component B-4. Suitable features of said nucleic acid sequence encoding at least one Paramyxoviridae virus (e.g. PIV and/or Henipavirus) antigen are provided in paragraph "*Nucleic acid features and embodiments*". Further, it is preferred that the at least one nucleic acid encoding at least one Paramyxoviridae virus (e.g. PIV and/or Henipavirus) antigen is formulated and/or complexed. Suitable formulations or complexations of said nucleic acid encoding at least one Paramyxoviridae virus (e.g. PIV and/or Henipavirus) antigen are provided in paragraph "*Formulation and complexation*".

In preferred embodiments, component B of the pharmaceutical composition is selected from component B-1 and component B-2. Accordingly, in preferred embodiments, the pharmaceutical composition comprises at least one component B-1 as defined herein and at least one component B-2 as defined herein. In particularly preferred embodiments, the pharmaceutical composition comprises at least one component B-1 as defined herein and at least one component B-2 as defined herein and at least one component A-1 as defined herein.

In preferred embodiments, component B of the pharmaceutical composition is selected from component B-1 and component B-3. Accordingly, in preferred embodiments, the pharmaceutical composition comprises at least one component B-1 as defined herein and at least one component B-3 as defined herein. In particularly preferred embodiments, the pharmaceutical composition comprises at least one component B-1 as defined herein and at least one component B-3 as defined herein and at least one component A-1 as defined herein.

In preferred embodiments, component B of the pharmaceutical composition is selected from component B-1 and component B-4. Accordingly, in preferred embodiments, the pharmaceutical composition comprises at least one component B-1 as defined herein and at least one component B-4 as defined herein. In particularly preferred embodiments, the pharmaceutical composition comprises at least one component B-1 as defined herein and at least one component B-4 as defined herein and at least one component A-1 as defined herein.

In preferred embodiments, component B of the pharmaceutical composition is selected from component B-2 and component B-3. Accordingly, in preferred embodiments, the pharmaceutical composition comprises at least one component B-2 as defined herein and at least one component B-3 as defined herein. In particularly preferred embodiments, the pharmaceutical composition comprises at least one component B-2 as defined herein and at least one component B-3 as defined herein and at least one component A-1 as defined herein.

In preferred embodiments, component B of the pharmaceutical composition is selected from component B-2 and component B-4. Accordingly, in preferred embodiments, the pharmaceutical composition comprises at least one component B-2 as defined herein and at least one component B-4 as defined herein. In particularly preferred embodiments, the pharmaceutical composition comprises at least one component B-2 as defined herein and at least one component B-4 as defined herein and at least one component A-1 as defined herein.

In preferred embodiments, component B of the pharmaceutical composition is selected from component B-3 and component B-4. Accordingly, in preferred embodiments, the pharmaceutical composition comprises at least one component B-3 as defined herein and at least one component B-4 as defined herein. In particularly preferred embodiments, the pharmaceutical composition comprises at least one component B-3 as defined herein and at least one component B-4 as defined herein and at least one component A-1 as defined herein.

In preferred embodiments, component B of the pharmaceutical composition is selected from component B-1 and component B-2 and component B3. Accordingly, in preferred embodiments, the pharmaceutical composition comprises at least one component B-1 as defined herein and at least one component B-2 as defined herein and at least one component B-3 as defined herein. In particularly preferred embodiments, the pharmaceutical composition comprises at least one component B-1 as defined herein and at least one component B-2 as defined herein and at least one component B-3 as defined herein and at least one component A-1 as defined herein.

In preferred embodiments, component B of the pharmaceutical composition is selected from component B-1 and component B-2 and component B4. Accordingly, in preferred embodiments, the pharmaceutical composition comprises at least one component B-1 as defined herein and at least one component B-2 as defined herein and at least one component B-4 as defined herein. In particularly preferred embodiments, the pharmaceutical composition comprises at least one component B-1 as defined herein and at least one component B-2 as defined herein and at least one component B-4 as defined herein and at least one component A-1 as defined herein.

In preferred embodiments, component B of the pharmaceutical composition is selected from component B-1 and component B-3 and component B4. Accordingly, in preferred embodiments, the pharmaceutical composition comprises at least one component B-1 as defined herein and at least one component B-3 as defined herein and at least one component B-4 as defined herein. In particularly preferred embodiments, the pharmaceutical composition comprises at least one component B-1 as defined herein and at least one component B-3 as defined herein and at least one component B-4 as defined herein and at least one component A-1 as defined herein.

In preferred embodiments, component B of the pharmaceutical composition is selected from component B-2 and component B-3 and component B4. Accordingly, in preferred embodiments, the pharmaceutical composition comprises at least one component B-2 as defined herein and at least one component B-3 as defined herein and at least one component B-4 as defined herein. In particularly preferred embodiments, the pharmaceutical composition comprises at least one component B-2 as defined herein and at least one component B-3 as defined herein and at least one component B-4 as defined herein and at least one component A-1 as defined herein.

Suitably, component B-3 is selected from at least one of component
B-3a at least one nucleic acid comprising at least one coding sequence encoding at least one antigenic peptide or protein selected or derived from at least one RSV virus or an immunogenic fragment or immunogenic variant thereof, preferably as defined herein, and/or
B-3b at least one nucleic acid comprising at least one coding sequence encoding at least one antigenic peptide or protein selected or derived from at least one hMPV virus or an immunogenic fragment or immunogenic variant thereof, preferably as defined herein.

It has to be understood that component B-3a comprises at least one nucleic acid encoding at least one antigenic peptide or protein selected or derived from at least one RSV as defined in paragraph "*Component B-3a: Respiratory syncytial virus (RSV)*", preferably having a nucleic acid sequence, preferably an mRNA sequence, as defined in the context of component B-3a. Suitable features of said nucleic acid sequence encoding at least one RSV antigen are provided in paragraph "*Nucleic acid features and embodiments*". Further, it is preferred that the at least one nucleic acid encoding at least one RSV antigen is formulated and/or complexed. Suitable formulations or complexations of said nucleic acid encoding at least one RSV antigen are provided in paragraph "*Formulation and complexation*".

It has to be understood that component B-3b comprises at least one nucleic acid encoding at least one antigenic peptide or protein selected or derived from at least one hMPV as defined in paragraph "*Component B-3b: Human metapneumovirus*", preferably having a nucleic acid sequence, preferably an mRNA sequence, as defined in the context of component B-3a. Suitable features of said nucleic acid sequence encoding at least one hMPV antigen are provided in paragraph "*Nucleic acid features and embodiments*". Further, it is preferred that the at least one nucleic acid encoding at least one hMPV antigen is formulated and/or complexed. Suitable formulations or complexations of said nucleic acid encoding at least one hMPV antigen are provided in paragraph "*Formulation and complexation*".

In preferred embodiments, component B-3 is selected from component B-3a. Accordingly, in preferred embodiments, the pharmaceutical composition comprises at least one component B-3a as defined herein.

Suitably, component B-4 is selected from at least one of component
B-4a at least one nucleic acid comprising at least one coding sequence encoding at least one antigenic peptide or protein selected or derived from at least one PIV virus or an immunogenic fragment or immunogenic variant thereof, preferably as defined herein, and/or
B-4b at least one nucleic acid comprising at least one coding sequence encoding at least one antigenic peptide or protein selected or derived from at least one Henipavirus or an immunogenic fragment or immunogenic variant thereof, preferably as defined herein.

It has to be understood that component B-4a comprises at least one nucleic acid encoding at least one antigenic peptide or protein selected or derived from at least one hPIV as defined in paragraph "*Component B-4a: Parainfluenzavirus*", preferably having a nucleic acid sequence, preferably an mRNA sequence, as defined in the context of component B-4a. Suitable features of said nucleic acid sequence encoding at least one PIV antigen are provided in paragraph "*Nucleic acid features and embodiments*". Further, it is preferred that the at least one nucleic acid encoding at least one PIV antigen is formulated and/or complexed. Suitable formulations or complexations of said nucleic acid encoding at least one PIV antigen are provided in paragraph "*Formulation and complexation*".

It has to be understood that component B-4b comprises at least one nucleic acid encoding at least one antigenic peptide or protein selected or derived from at least one Henipavirus as defined in paragraph "*Component B-4b: Henipavirus*", preferably having a nucleic acid sequence, preferably an mRNA sequence, as defined in the context of component B-4b. Suitable features of said nucleic acid sequence encoding at least one Henipavirus antigen are provided in paragraph "*Nucleic acid features and embodiments*". Further, it is preferred that the at least one nucleic acid encoding at least one Henipavirus antigen is formulated and/or complexed. Suitable formulations or complexations of said nucleic acid encoding at least one Henipavirus antigen are provided in paragraph "Formulation and complexation".

In preferred embodiments, component B-4 is selected from component B-4a. Accordingly, in preferred embodiments, the pharmaceutical composition comprises at least one component B-4a as defined herein.

In preferred embodiments, the pharmaceutical composition of comprises or consists of component A-1 as defined
herein and component B-3a as defined herein.

In preferred embodiments, the pharmaceutical composition comprises or consists of component A-1 as defined
herein and component B-2 as defined herein.

In preferred embodiments, the Pharmaceutical composition comprises or consists of component A-1 as defined
herein and component B-2 as defined herein and component B-3a as defined herein.

In preferred embodiments of the pharmaceutical composition or vaccine, the at least one nucleic acid of component A (in particular, component A-1, A-2, A-3) and the at least one nucleic acid of component B (in particular component B-1, B-2, B-3, B-3a, B-3b, B-4, B-4a, B-4b) are translated into an antigenic peptide or protein when administered to a subject.

When administered to a subject, it is preferred that the pharmaceutical composition or vaccine induces an antigen-specific humoral immune responses against the peptide or protein encoded by component A (in particular, component A-1, A-2, A-3) and an antigen-specific humoral immune responses against the peptide or protein encoded by component B (in particular component B-1, B-2, B-3, B-3a, B-3b, B-4, B-4a, B-4b) in said subject

When administered to a subject, it is preferred that the pharmaceutical composition or vaccine induces an antigen-specific T-cell responses against the peptide or protein encoded by component A (in particular, component A-1, A-2, A-3) and an antigen-specific T-cell responses against the peptide or protein encoded by component B (in particular component B-1, B-2, B-3, B-3a, B-3b, B-4, B-4a, B-4b) in said subject.

When administered to a subject, it is preferred that the pharmaceutical composition or vaccine induces / establishes an antigen-specific B-cell memory against the peptide or protein encoded by component A (in particular, component A-1, A-2, A-3) and an antigen-specific B-cell memory against the peptide or protein encoded by component B (in particular component B-1, B-2, B-3, B-3a, B-3b, B-4, B-4a, B-4b) in said subject.

### Combination vaccine:

In a second aspect, the invention relates to a nucleic acid based combination vaccine or multipathogen vaccine.

It has to be noted that specific features and embodiments that are described in the context of the first aspect of the invention, that is the pharmaceutical composition of the invention comprising component A and component B, are likewise applicable to the second aspect (combination vaccine), the third aspect (kit or kit of parts of the invention), or further aspects including e.g. medical uses (first and second medical uses) and e.g. method of treatments.

In preferred embodiments of the second aspect, the combination vaccine comprises at least one component A (in particular, component A-1, A-2, A-3) as defined in the context of the first aspect, and at least one component B (in particular component B-1, B-2, B-3, B-3a, B-3b, B-4, B-4a, B-4b) as defined in the context of the first aspect

The term "combination vaccine" or "multipathogen vaccine" or "multivalent vaccine" will be recognized and understood by the person of ordinary skill in the art, and is for example intended to be a prophylactic or therapeutic material providing at least two epitopes or antigens, preferably an immunogen. In the context of the invention the at least two antigens or antigenic functions are suitably provided by component A and component B as defined herein, and are selected or derived from at least two different viruses (e.g. a SARS-CoV-2 (of component A) and an Influenza virus (of component B)).

Accordingly, in preferred embodiments, the combination vaccine of the second aspect comprises at least one component A (in particular, component A-1, A-2, A-3) as defined in the context of the first aspect and at least one component B (in particular component B-1, B-2, B-3, B-3a, B-3b, B4, B-4a, B-4b) as defined in the context of the first aspect.

According to a preferred embodiment, the combination vaccine as defined herein may further comprise a pharmaceutically acceptable carrier or excipient and optionally at least one adjuvant as specified in the context of the first aspect. Suitable adjuvants in that context may be selected from adjuvants disclosed in claim 17 of WO2016203025.

The combination vaccine typically comprises a safe and effective amount of nucleic acid (e.g. DNA or RNA), preferably RNA of the first aspect (provided by component A and component B). As used herein, "safe and effective amount" means an amount of nucleic acid component sufficient to significantly induce a positive modification of a disease or disorder related to an infection with a virus as specified herein. At the same time, a "safe and effective amount" is small enough to avoid serious side-effects. In relation to the nucleic acid, composition, or vaccine of the present invention, the expression "safe and effective amount" preferably means an amount of nucleic acid, composition, or vaccine that is suitable for stimulating the adaptive immune system against a virus as specified herein in such a manner that no excessive or damaging immune reactions (e.g. innate immune responses) are achieved.

A "safe and effective amount" of the nucleic acid, composition, or vaccine as defined herein will vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the skilled person. Moreover, the "safe and effective amount" of the nucleic acid, the composition, or vaccine may depend from application/delivery route (intradermal, intramuscular, intranasal), application device (jet injection, needle injection, microneedle patch, electroporation device) and/or complexation/formulation (protamine complexation or LNP encapsulation, DNA or RNA). Moreover, the "safe and effective amount" of the nucleic acid, the composition, or the vaccine may depend from the physical condition of the treated subject (infant, pregnant women, immunocompromised human subject etc.).

The pharmaceutically acceptable carrier as used herein preferably includes the liquid or non-liquid basis of the inventive coronavirus vaccine. If the inventive vaccine is provided in liquid form, the carrier will be water, typically pyrogen-free water; isotonic saline or buffered (aqueous) solutions, e.g. phosphate, citrate etc. buffered solutions. Preferably, Ringer-Lactate solution is used as a liquid basis for the vaccine or the composition according to the invention as described in WO2006122828, the disclosure relating to suitable buffered solutions incorporated herewith by reference. Other preferred solutions used as a liquid basis for the vaccine or the composition, in particular for compositions/vaccines comprising LNPs, comprise sucrose and/or trehalose.

The choice of a pharmaceutically acceptable carrier or excipient as defined herein is determined, in principle, by the manner, in which the pharmaceutical composition(s) or vaccine according to the invention is administered. The coronavirus vaccine is preferably administered locally. Routes for local administration in general include, for example, topical administration routes but also intradermal, transdermal, subcutaneous, or intramuscular injections or intralesional, intracranial, intrapulmonal, intracardial, intraarticular and sublingual injections. More preferably, composition or vaccines according to the present invention may be administered by an intradermal, subcutaneous, or intramuscular route, preferably by injection, which may be needle-free and/or needle injection. Preferred in the context of the invention is intramuscular injection. Compositions/vaccines are therefore preferably formulated in liquid or solid form. The suitable amount of the vaccine or composition according to the invention to be administered can be determined by routine experiments, e.g. by using animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4.

In embodiments, the combination vaccine is provided in lyophilized or spray-dried form. Such a lyophilized or spray-dried vaccine may comprise trehalose and/or sucrose and may be re-constituted in a suitable liquid buffer before administration to a subject. In some embodiments, a lyophilized vaccine comprises RNA complexed with LNPs. In some embodiments, a lyophilized composition has a water content of less than about 10%. For example, a lyophilized composition can have a water content of about 0.1% to 10%, 0.1% to 7.5%, or 0.5% to 7.5%, preferably the lyophilized composition has a water content of about 0.5% to about 5.0%.

The combination vaccine can be used according to the invention for human medical purposes and also for veterinary medical purposes (mammals, vertebrates, or avian species).

In preferred embodiments, the combination vaccine elicits an adaptive immune response against at least one Coronavirus, preferably against a pandemic Coronavirus, *preferably* against SARS-CoV-2, SARS-CoV-1, or MERS-CoV. In preferred embodiments, the combination vaccine elicits an adaptive immune response against at least one Influenza virus, preferably against at least one Influenza A virus and/or at least one Influenza B virus. In preferred embodiments, the combination vaccine elicits an adaptive immune response against at least one Pneumoviridae virus, preferably against at least one RSV and/or at least one hMPV. In embodiments, the combination vaccine elicits an adaptive immune response against at least one Paramyxoviridae virus, preferably against at least one PIV and/or at least one Henipavirus.

In preferred embodiments, the combination vaccine elicits neutralizing antibody titers against at least one Coronavirus, preferably against a pandemic Coronavirus, *preferably* against SARS-CoV-2, SARS-CoV-1, or MERS-CoV. In preferred embodiments, the combination vaccine elicits neutralizing antibody titers against at least one Influenza virus, preferably against at least one Influenza A virus and/or at least one Influenza B virus. In preferred embodiments, the combination vaccine elicits neutralizing antibody titers against at least one Pneumoviridae virus, preferably against at least one RSV and/or at least one hMPV. In embodiments, the combination vaccine elicits neutralizing antibody titers against at least one Paramyxoviridae virus, preferably against at least one PIV and/or at least one Henipavirus.

In some embodiments, the neutralizing antibody titer is at least 100 neutralizing units per milliliter (NU/mL), at least 500NU/mL, or at least 1000NU/mL.

In some embodiments, detectable levels of the respective antigen are produced in a subject at about 1 to about 72 hours post administration of the composition or the combination vaccine.

In some embodiments, a neutralizing antibody titer (against coronavirus) of at least 100NU/ml, at least 500NU/ml, or at least 1000NU/ml is produced in the serum of the subject at about 1 day to about 72 days post administration of the composition or the combination vaccine.

In particularly preferred embodiments, the combination vaccine or the composition elicits functional antibodies that can effectively neutralize the respective viruses (e.g. SARS-CoV-2 coronavirus and at least one further virus).

In further preferred embodiments, the combination vaccine or the composition elicits mucosal IgA immunity by inducing of mucosal IgA antibodies.

In particularly preferred embodiments, the combination vaccine or the composition elicits functional antibodies that can effectively neutralize the respective viruses (e.g. SARS-CoV-2 coronavirus and at least one further virus).

In further particularly preferred embodiments, the combination vaccine or the composition induces broad, functional cellular T-cell responses against the respective viruses (e.g. against SARS-CoV-2 coronavirus and at least one further virus).

In further particularly preferred embodiments, the combination vaccine or the composition induces a well-balanced B cell and T cell response against the respective viruses (e.g. against SARS-CoV-2 coronavirus and at least one further virus).

In some embodiments, the neutralizing antibody titer is sufficient to reduce virus infection by at least 50% relative to a neutralizing antibody titer of an unvaccinated control subject or relative to a neutralizing antibody titer of a subject vaccinated with a live attenuated viral vaccine, an inactivated viral vaccine, or a protein sub unit viral vaccine.

In some embodiments, the neutralizing antibody titer and/or a T cell immune response is sufficient to reduce the rate of asymptomatic viral infection relative to the neutralizing antibody titer of unvaccinated control subjects.

In some embodiments, the neutralizing antibody titer and/or a T cell immune response is sufficient to prevent viral latency in the subject.

In some embodiments, the neutralizing antibody titer is sufficient to block fusion of virus with epithelial cells of the subject.

In preferred embodiments, the neutralizing antibody titer is induced following a single 1ug-100ug dose of the composition, or the combination vaccine. In further preferred embodiments, the neutralizing antibody titer is induced following a single 2ug-20ug dose of the composition, or the combination vaccine.

In some embodiments, the neutralizing antibody titer is induced within 20 days following a single 1 ug-100ug dose of the composition, or the combination vaccine, or within 40 days following a second 1ug-100µg dose of the composition, or the combination vaccine.

In preferred embodiments, administration of a therapeutically effective amount of the composition, or the combination vaccine to a subject induces a T cell immune response against coronavirus in the subject. In preferred embodiments, the T cell immune response comprises a CD4+ T cell immune response and/or a CD8+ T cell immune response.

In preferred embodiments, the combination vaccine of the second aspect elicits antigen-specific immune responses comprising T-cell responses and/or B-cell responses against all encoded antigens provided by the at least one nucleic acid of component A (in particular, component A-1, A-2, A-3) and additionally eliciting an antigen-specific immune responses comprising T-cell responses and/or B-cell responses against all encoded antigens provided by the at least one nucleic acid of component B (in particular component B-1, B-2, B-3, B-3a, B-3b, B-4, B-4a, B-4b).

In preferred embodiments, the combination vaccine of the second aspect elicits antigen-specific immune responses comprising T-cell responses and/or B-cell responses against all encoded antigens upon administration of a low dose of the combination vaccine. In preferred embodiments, a low dose comprises less that about 100µg, preferably less than about 50µg, more preferably less than about 20µg of nucleic acid, even more preferably less than about 10µg.

In preferred embodiments, the combination vaccine of the second aspect elicits antigen-specific immune responses comprising T-cell responses and/or B-cell responses against all encoded antigens upon administration of a low dose of the combination vaccine. In preferred embodiments, a low dose comprises less that about 100µg, preferably less than about 50µg, more preferably less than about 20µg of nucleic acid, even more preferably less than about 10µg of nucleic acid.

In preferred embodiments, the combination vaccine of the second aspect elicits antigen-specific immune responses
in a subject that has an age of about 5 years old or younger. Accordingly, the nucleic acid based combination vaccines are particularly suitable for infants.

In preferred embodiments, the combination vaccine of the second aspect elicits antigen-specific immune responses
in a subject that has an age of about 60 years old or older. Accordingly, the nucleic acid based combination vaccines are particularly suitable for the elderly.

In various embodiments, the combination vaccine is against at least one Coronavirus, and, additionally against at least one additional Coronavirus, and/or at least one Influenza virus and/or at least one Pneumoviridae virus, and/or at least one Paramyxoviridae virus. Accordingly, the combination vaccine is a combination vaccine against multiple respiratory viruses.

In preferred embodiments, the combination vaccine is against at least one Coronavirus, *preferably* SARS-CoV-2, and at least one Influenza virus and/or RSV virus. Accordingly, the combination vaccine is a Coronavirus - Influenza vaccine, a Coronavirus - RSV vaccine, or a Coronavirus - Influenza - RSV vaccine.

In embodiments, the combination vaccine is against at least one Coronavirus, *preferably* SARS-CoV-2, and at least one Influenza virus and/or RSV virus and, optionally, against at least one PIV and/or at least one hMPV. Accordingly, the combination vaccine is a Coronavirus - Influenza - PIV vaccine, a Coronavirus - RSV - PIV vaccine, a Coronavirus - Influenza - RSV - PIV vaccine, a Coronavirus - Influenza - hMPV vaccine, a Coronavirus - RSV - hMPV vaccine, a Coronavirus - Influenza - RSV - hMPV vaccine, a Coronavirus - Influenza - PIV - hMPV vaccine, a Coronavirus - RSV - PIV - hMPV vaccine, or a Coronavirus - Influenza - RSV - PIV - hMPV vaccine.

### Kit or kit of parts, application, medical uses, method of treatment:

In a third aspect, the present invention provides a kit or kit of parts suitable for treating or preventing virus infections. Preferably, said kit or kit of parts is suitable for treating or preventing a Coronavirus, preferably a SARS-CoV-2 infection and, additionally, a further infection.

Notably, embodiments relating to the pharmaceutical composition of the first aspect and the combination vaccine of the second aspect may likewise be read on and be understood as suitable embodiments of the kit or kit of parts of the third aspect of the invention.

In preferred embodiments, the kit or kit of parts comprises at least one pharmaceutical composition of the first aspect, and/or at least one combination vaccine of the second aspect.

In addition, the kit or kit of parts may comprise a liquid vehicle for solubilising, and/or technical instructions providing information on administration and dosage of the components.

The technical instructions of said kit may contain information about administration and dosage and patient groups. Such kits, preferably kits of parts, may be applied e.g. for any of the applications or uses mentioned herein, preferably for the use of the pharmaceutical composition of the first aspect or the combination vaccine of the second aspect, for the treatment or prophylaxis of an infection or diseases caused by a coronavirus, *preferably* SARS-CoV-2 coronavirus, and a further virus infection (e.g. caused by an Influenza virus, and/or an RSV, and/or a PIV and /or a hMPV)

Preferably, the pharmaceutical composition or the vaccine is provided in a separate part of the kit, wherein the pharmaceutical composition or the combination vaccine is preferably lyophilised or spray-dried or spray-freeze dried.

The kit may further contain as a part a vehicle (e.g. buffer solution) for solubilising the dried or lyophilized nucleic composition or the vaccine.

In preferred embodiments, the kit or kit of parts as defined herein comprises Ringer lactate solution.

In preferred embodiments, the kit or kit of parts as defined herein comprises a multi-dose container for administration of the composition/the vaccine and/or an administration device (e.g. an injector for intradermal injection).

Any of the above kits may be used in a treatment or prophylaxis as defined herein.

### First and second/further medical use:

A further aspect relates to the first medical use of the pharmaceutical composition of the first aspect, the combination vaccine of the second aspect, and the kit or kit of parts of the third aspect.

Notably, embodiments relating to the composition of the first aspect, the vaccine of the second aspect, or the kit or kit of parts of the third aspect may likewise be read on and be understood as suitable embodiments of medical uses of the invention.

Accordingly, the invention provides a pharmaceutical composition as defined in the first aspect for use as a medicament, the combination vaccine as defined in the second aspect for use as a medicament, and the kit or kit of parts as defined in the third aspect for use as a medicament.

The present invention furthermore provides several applications and uses of the composition, vaccine, or kit.

In particular, pharmaceutical compositions, polypeptide, vaccine, or kit may be used for human medical purposes and also for veterinary medical purposes, preferably for human medical purposes.

In particular, the pharmaceutical composition, the combination vaccine, or kit or kit of parts is for use as a medicament for human medical purposes, wherein said composition, vaccine, or kit or kit of parts may be suitable for young infants, newborns, immunocompromised recipients, as well as pregnant and breast-feeding women and elderly people. In particular, the pharmaceutical composition, combination vaccine, or kit or kit of parts is for use as a medicament for human medical purposes, wherein said composition, vaccine, or kit or kit of parts is particularly suitable for elderly human subjects.

Said pharmaceutical composition, combination vaccine, or kit is for use as a medicament for human medical purposes, wherein said composition, vaccine, or the kit or kit of parts is particularly suitable for intramuscular injection or intradermal injection.

In yet another aspect, the invention relates to the second medical use of the provided pharmaceutical composition, combination vaccine, or kit.

In an aspect, the invention relates to a pharmaceutical composition of the first aspect, a combination vaccine of the second aspect, a kit or kit of parts of the third aspect, for use in the treatment or prophylaxis of an infection with a coronavirus, *preferably* SARS-CoV-2 (or a disorder related to such an infection) and in the treatment or prophylaxis of an infection with at least one further virus (or a disorder related to such an infection), *preferably* wherein said further virus is selected from at least one different Coronavirus, at least one Influenza virus, at least one Pneumoviridae virus, and/or at least one Paramyxoviridae virus.

In an aspect, the invention relates to a pharmaceutical composition of the first aspect, a combination vaccine of the second aspect, a kit or kit of parts of the third aspect, for use in the treatment or prophylaxis of an infection with a coronavirus, *preferably* SARS-CoV-2 (or a disorder related to such an infection) and in the treatment or prophylaxis of an infection with at least one Influenza virus (or a disorder related to such an infection), and, optionally, treatment or prophylaxis of an infection with at least one PIV and/or hMPV.

In an aspect, the invention relates to a pharmaceutical composition of the first aspect, a combination vaccine of the second aspect, a kit or kit of parts of the third aspect, for use in the treatment or prophylaxis of an infection with a coronavirus, *preferably* SARS-CoV-2 (or a disorder related to such an infection) and in the treatment or prophylaxis of an infection with at least one RSV (or a disorder related to such an infection), and, optionally, treatment or prophylaxis of an infection with at least one PIV and/or hMPV.

In an aspect, the invention relates to a pharmaceutical composition of the first aspect, a combination vaccine of the second aspect, a kit or kit of parts of the third aspect, for use in the treatment or prophylaxis of an infection with a coronavirus, *preferably* SARS-CoV-2 (or a disorder related to such an infection) and in the treatment or prophylaxis of an infection with at least one Influenza virus (or a disorder related to such an infection) and at least one RSV (or a disorder related to such an infection), and, optionally, treatment or prophylaxis of an infection with at least one PIV and/or hMPV.

Particularly, the pharmaceutical composition of the first aspect, the combination vaccine of the second aspect, or the kit or kit of parts of the third aspect may be used in a method of prophylactic (pre-exposure prophylaxis or post-exposure prophylaxis) and/or therapeutic treatment of infections caused by at least one Coronavirus, preferably SARS-CoV-2 coronavirus, and by at least one further virus (e.g. different Coronavirus, at least one Influenza virus, at least one Pneumoviridae virus, and/or at least one Paramyxoviridae virus).

The pharmaceutical composition or the combination vaccine may preferably be administered locally. In particular, composition or vaccines may be administered by an intradermal, subcutaneous, intranasal, or intramuscular route. In embodiments, the inventive nucleic acid, composition, polypeptide, vaccine may be administered by conventional needle injection or needle-free jet injection. Preferred in that context is intramuscular injection.

In embodiments where plasmid DNA is used and comprised in the pharmaceutical composition or vaccine, the composition/vaccine may be administered by a device, e.g. an electroporation device, preferably an electroporation device for intradermal or intramuscular delivery. Suitably, a device as described in US7245963B2 may be used, in particular a device as defined by claims 1 to 68 of US7245963B2.

In embodiments where adenovirus DNA is used and comprised in the pharmaceutical composition or combination vaccine, the composition/vaccine may be administered by intranasal administration.

In embodiments, the nucleic acid as comprised in a composition or vaccine as defined herein is provided in an amount of about 100ng to about 500ug, in an amount of about 1ug to about 200ug, in an amount of about 1ug to about 100ug, in an amount of about 5ug to about 100ug, preferably in an amount of about 10ug to about 50ug, specifically, in an amount of about 1ug, 2ug, 3ug, 4ug, 5ug, 6ug, 7ug, 8ug, 9ug, 10ug, 11ug, 12ug, 13ug, 14ug, 15ug, 20ug, 25ug, 30ug, 35ug, 40ug, 45ug, 50ug, 55ug, 60ug. 65ug, 70ug, 75ug, 80ug, 85ug, 90ug, 95ug or 100ug.

Notably, the amount relates to the total amount of nucleic acid, provided by the at least one component A and component B.

In some embodiments, the vaccine comprising the nucleic acid, or the composition comprising the nucleic acid is formulated in an effective amount to produce an antigen specific immune response in a subject. In some embodiments, the effective amount is a total dose of 1µg to 200µg, 1µg to 100µg, or 5µg to 100µg.

Notably, the effective amount relates to the total amount of nucleic acid, provided by the at least one component A and component B.

In embodiments where the nucleic acid is provided in a lipid-based carrier, e.g. an LNP, the amount of PEG-lipid as defined herein comprised in one dose is lower than about 50µg PEG lipid, preferably lower than about 45µg PEG lipid, more preferably lower than about 40µg PEG lipid.

Having a low amount of PEG lipid in one dose may reduce the risk of adverse effects (e.g. allergies).

In particularly preferred embodiments, the amount of PEG-lipid comprised in one dose is in a range from about 3.5µg PEG lipid to about 35µg PEG lipid.

In embodiments where the nucleic acid is provided in a lipid-based carrier, e.g. an LNP, the amount of cationic lipid as defined herein comprised in one dose is lower than about 400µg cationic lipid, preferably lower than about 350µg cationic lipid, more preferably lower than about 300µg cationic lipid.

Having a low amount of cationic lipid in one dose may reduce the risk of adverse effects (e.g. fewer).

In particularly preferred embodiments, the amount of cationic-lipid comprised in one dose is in a range from about 30µg PEG lipid to about 300µg PEG lipid.

In one embodiment, the immunization protocol for the treatment or prophylaxis of a subject against at least one Coronavirus, *preferably* SARS-CoV-2 and against at least one further virus comprises one single dose of the pharmaceutical composition or the combination vaccine.

In some embodiments, the effective amount is a dose of 1 ug administered to the subject in one vaccination. In some embodiments, the effective amount is a dose of 2ug administered to the subject in one vaccination. In some embodiments, the effective amount is a dose of 3ug administered to the subject in one vaccination. In some embodiments, the effective amount is a dose of 4ug administered to the subject in one vaccination. In some embodiments, the effective amount is a dose of 5ug administered to the subject in one vaccination. In some embodiments, the effective amount is a dose of 6ug administered to the subject in one vaccination. In some embodiments, the effective amount is a dose of 7ug administered to the subject in one vaccination. In some embodiments, the effective amount is a dose of 8ug administered to the subject in one vaccination. In some embodiments, the effective amount is a dose of 9ug administered to the subject in one vaccination. In some embodiments, the effective amount is a dose of 10ug administered to the subject in one vaccination. In some embodiments, the effective amount is a dose of 11ug administered to the subject in one vaccination. In some embodiments, the effective amount is a dose of 12ug administered to the subject in one vaccination. In some embodiments, the effective amount is a dose of 13ug administered to the subject in one vaccination. In some embodiments, the effective amount is a dose of 14ug administered to the subject in one vaccination. In some embodiments, the effective amount is a dose of 15ug administered to the subject in one vaccination. In some embodiments, the effective amount is a dose of 20ug administered to the subject in one vaccination. In some embodiments, the effective amount is a dose of 25ug administered to the subject in one vaccination. In some embodiments, the effective amount is a dose of 30ug administered to the subject in one vaccination. In some embodiments, the effective amount is a dose of 40ug administered to the subject in one vaccination. In some embodiments, the effective amount is a dose of 50ug administered to the subject in one vaccination. In some embodiments, the effective amount is a dose of 100ug administered to the subject in one vaccination. In some embodiments, the effective amount is a dose of 200ug administered to the subject in one vaccination. Notably, the dose relates to the total dose of nucleic acid, provided by the at least one component A and component B.

In preferred embodiments, the immunization protocol for the treatment or prophylaxis of at least one Coronavirus infection and at least one further virus infection comprises a series of single doses or dosages of the pharmaceutical composition or the combination vaccine. A single dosage, as used herein, refers to the initial/first dose, a second dose or any further doses, respectively, which are preferably administered in order to "boost" the immune reaction.

In some embodiments, the effective amount is a dose of 1ug administered to the subject a total of two times. In some embodiments, the effective amount is a dose of 2ug administered to the subject a total of two times. In some embodiments, the effective amount is a dose of 3ug administered to the subject a total of two times. In some embodiments, the effective amount is a dose of 4ug administered to the subject a total of two times. In some embodiments, the effective amount is a dose of 5ug administered to the subject a total of two times. In some embodiments, the effective amount is a dose of 6ug administered to the subject a total of two times. In some embodiments, the effective amount is a dose of 7ug administered to the subject a total of two times. In some embodiments, the effective amount is a dose of 8ug administered to the subject a total of two times. In some embodiments, the effective amount is a dose of 9ug administered to the subject a total of two times. In some embodiments, the effective amount is a dose of 10ug administered to the subject a total of two times. In some embodiments, the effective amount is a dose of 11ug administered to the subject a total of two times. In some embodiments, the effective amount is a dose of 12ug administered to the subject a total of two times. In some embodiments, the effective amount is a dose of 13ug administered to the subject a total of two times. In some embodiments, the effective amount is a dose of 14ug administered to the subject a total of two times. In some embodiments, the effective amount is a dose of 15ug administered to the subject a total of two times. In some embodiments, the effective amount is a dose of 20ug administered to the subject a total of two times. In some embodiments, the effective amount is a dose of 25ug administered to the subject a total of two times. In some embodiments, the effective amount is a dose of 30ug administered to the subject a total of two times. In some embodiments, the effective amount is a dose of 40ug administered to the subject a total of two times. In some embodiments, the effective amount is a dose of 50ug administered to the subject a total of two times. In some embodiments, the effective amount is a dose of 100ug administered to the subject a total of two times. In some embodiments, the effective amount is a dose of 200ug administered to the subject a total of two times. Notably, the dose relates to the total dose of nucleic acid, provided by the at least one component A and component B.

In preferred embodiments, the vaccine/composition immunizes the subject against a Coronavirus infection and against one further virus infection (upon administration as defined herein) for at least 1 year, preferably at least 2 years. In preferred embodiments, the vaccine/composition immunizes the subject against a Coronavirus infection and against one further virus infection for more than 2 years, more preferably for more than 3 years, even more preferably for more than 4 years, even more preferably for more than 5-10 years.

### Method of treatment and use, diagnostic method and use:

In another aspect, the present invention relates to a method of treating or preventing a disorder.

Notably, embodiments relating to the pharmaceutical composition of the first aspect, the combination vaccine of the second aspect, the kit or kit of parts of the third aspect, or medical uses may likewise be read on and be understood as suitable embodiments of methods of treatments as provided herein. Furthermore, specific features and embodiments relating to method of treatments as provided herein may also apply for medical uses of the invention.

Preventing (Inhibiting) or treating a disease, in particular a virus infection relates to inhibiting the full development of a disease or condition, for example, in a subject who is at risk for a disease such as a virus infection. "Treatment" refers to a therapeutic intervention that ameliorates a sign or symptom of a disease or pathological condition after it has begun to develop. The term "ameliorating", with reference to a disease or pathological condition, refers to any observable beneficial effect of the treatment. Inhibiting a disease can include preventing or reducing the risk of the disease, such as preventing or reducing the risk of viral infection. The beneficial effect can be evidenced, for example, by a delayed onset of clinical symptoms of the disease in a susceptible subject, a reduction in severity of some or all clinical symptoms of the disease, a slower progression of the disease, a reduction in the viral load, an improvement in the overall health or well-being of the subject, or by other parameters that are specific to the particular disease. A "prophylactic" treatment is a treatment administered to a subject who does not exhibit signs of a disease or exhibits only early signs for the purpose of decreasing the risk of developing pathology.

In preferred embodiments, the present invention relates to a method of treating or preventing a disorder, wherein the method comprises applying or administering to a subject in need thereof at least one pharmaceutical composition of the first aspect, the combination vaccine of the second aspect, or the kit or kit of parts of the third aspect.

In preferred embodiments, the disorder is an infection with a Coronavirus, or a disorder related to such infections, in particular an infection with SARS-CoV-2, or a disorder related to such infections (e.g. COVID-19), and an additional further virus infection, *optionally* wherein said further virus is selected from at least one different Coronavirus, at least one Influenza virus, at least one Pneumoviridae virus, and/or at least one Paramyxoviridae virus, or of a disorder related to any of these infections.

In preferred embodiments, the present invention relates to a method of treating or preventing a disorder as defined above, wherein the method comprises applying or administering to a subject in need thereof at least pharmaceutical composition of the first aspect, the combination vaccine of the second aspect, or the kit or kit of parts of the third aspect, wherein the subject in need is preferably a mammalian subject.

In particularly preferred embodiments, the subject in need is a mammalian subject, preferably a human subject, e.g. new-bom, pregnant, immunocompromised, and/or elderly.

In particularly preferred embodiments, the human subject is an elderly human subject.

In certain embodiments, a method of treating or preventing disease by applying or administering to a subject in need thereof at least pharmaceutical composition of the first aspect, the combination vaccine of the second aspect, or the kit or kit of parts of the third aspect is further defined as a method of reducing disease burden in the subject. For example, the method preferably reduces the severity and/or duration of one or more symptom of COVID-19 disease. In some aspects, a method reduces the probability that a subject will require hospital admission, intensive care unit admission, treatment with supplemental oxygen and/or treatment with a ventilator. In further aspects, the method reduces the probability that a subject will develop a fever, breathing difficulties; loss of smell and/or loss of taste. In preferred aspects, the method reduces the probability that a subject will develop severe or moderate COVID-19 disease. In certain aspects, a method of the embodiments prevents severe or moderate COVID-19 disease in the subject between about 2 weeks and 1 month, 2 months, 3 months, 4 months, 5 months, 6 months, 1 year or 2 years after the subject is administered a composition of the embodiments. In preferred aspects, a method of the embodiments prevents symptomatic COVID-19 disease. In further aspects, a method of the embodiment prevents detectable levels of SARS CoV-2 nucleic acid in the subject between about 2 weeks and 1 month, 2 months, 3 months, 4 months, 5 months, 6 months, 1 year or 2 years after the subject is administered a composition of the embodiments. In further aspects, a method of the embodiments is defined as a method for providing protective immunity to a coronavirus infection (e.g., SARS CoV-2 infection) in the subject In still further aspects, a method of the embodiments prevents moderate and severe COVID-19 disease in at least 80%, 85%, 90% or 95% of treated subjects. In yet further aspects, a method of the embodiments prevents moderate and severe COVID-19 disease in at least 80%, 85%, 90% or 95% of treated subjects from about 2 weeks to about 1 year after administering the second or subsequent immunogenic composition (e.g., a booster administration). In yet further aspects, a method of the embodiments prevents moderate and severe COVID-19 disease in at least 80%, 85%, 90% or 95% of treated subjects from about 2 weeks to about 3 month, 6 months, 9 months, 1 year, 1.5 years, 2 years or 3 years after administering the second or subsequent composition.

As used herein severe COVID-19 disease is defined as a subject experiencing one or more of the following:
- Clinical signs at rest indicative of severe systemic illness (respiratory rate ≥ 30 breaths per minute, heart rate ≥ 125 per minute, SpO2 ≤ 93% on room air at sea level or PaO2/FIO2 < 300mm Hg (adjusted according to altitude))
- Respiratory failure (defined as needing high flow-oxygen, noninvasive ventilation, mechanical ventilation or ECMO)
- Evidence of shock (SBP < 90mm Hg, DBP < 60mmHg, or requiring vasopressors)
- Significant renal, hepatic, or neurologic dysfunction
- Admission to ICU
- Death

As used herein moderate COVID-19 disease is defined as a subject experiencing one or more of the following:
- Shortness of breath or difficulty breathing
- Respiratory rate ≥ 20 breaths per minute
- Abnormal SpO2 but still > 93% on room air at sea level (adjusted according to altitude)
- Clinical or radiographic evidence of lower respiratory tract disease
- Radiologic evidence of deep vein thrombosis (DVT)

As used herein mild COVID-19 disease is defined as a subject experiencing all of the following:
- Symptomatic AND
- No shortness of breath or difficulty breathing AND
- No hypoxemia (adjusted according to altitude) AND
- Does not meet the case definition of moderate or severe COVID-19 disease

In a further aspects, a method of the embodiments comprises (i) obtaining a composition (e.g., a vaccine composition) of the embodiments, wherein the composition is lyophilized; (ii) solubilizing the lyophilized composition in a pharmaceutically acceptable liquid carrier to produce a liquid composition; and (iii) administering an effective amount of the liquid composition to the subject. In some aspects, the lyophilized composition comprises less than about 10% water content. For example, the lyophilized composition can preferably comprise about 0.1 % to about 10%, 0.5% to 7.5% or 0.5% to 5.0% water.

In particular, such the method of treatment may comprise the steps of.
a) providing at least one pharmaceutical composition of the first aspect, at least one combination vaccine of the second aspect, or the kit or kit of parts of the third aspect;
b) applying or administering said composition, vaccine, or kit or kit of parts to a subject as a first dose
c) optionally, applying or administering said composition, vaccine, or kit or kit of parts to a subject as a second dose or a further dose, preferably at least 1, 2, 3, 4, 5, 6, 7, 8, 9, 10, 11, 12, months after the first dose.

The first dosage, as used herein, refers to the initial/first dose, a second dose or any further doses, respectively, which are preferably administered in order to "boost" the immune reaction.

In certain aspects, the vaccine/composition is administered to a subject one, two three, four or more times. In some aspects, the vaccine/composition is administered to the subject at least first and a second time (e.g., a prime and boost). I some aspects, the send administration is at least 10 days, 14 days, 21 days, 28 days, 35 days, 42 days, 49 days or 56 days after the first administration. In some aspects, the time between the first administration and the second administration is between about 7 days and about 56 days; about 14 days and about 56 days; about 21 days and about 56 days; or about 28 days and about 56 days. In further aspects, the vaccine/composition is administered to a subject three or more times. In certain aspects, there is at least 10 days, 14 days, 21 days, 28 days, 35 days, 42 days, 49 days or 56 days between each administration of the vaccine/composition.

In some aspects, a subject for treatment according to the embodiments was previously infected with SARS CoV-2 or was previously treated with at least a first SARS CoV-2 vaccine composition. In some aspects, the subject was treated with one, two, three or more doses of a first SARS CoV-2 vaccine composition. In some aspects, the composition of the embodiments used to treat a subject is a different type of vaccine composition than the composition previously used to treat the subject. In some aspects, the subject was previously treated with a mRNA vaccine, such as BNT162 or mRNA-1273. In further aspects, the subject was previously treated with a protein subunit vaccine, such as spike protein based vaccine, e.g., NVX-CoV2373 or COVAX. In further aspects, the subject was previously treated with a viral vector vaccine, such as an adenovirus vector based vaccine, e.g., ADZ1222 or Ad26.COV-2.S. In further aspects, a subject previously treated with a vaccine composition has detectable SARS CoV-2 binding antibodies, such as SARS CoV-2 S protein-binding antibodies or SARS CoV-2 N protein-binding antibodies. In further aspects, a subject for treatment according the embodiments was treated with a first SARS CoV-2 vaccine composition at least about 3 month, 6 months, 9 months, 1 year, 1.5 years, 2 years or 3 years ago. In still further aspects, a subject for treatment according the embodiments was treated with a first SARS CoV-2 vaccine composition between about 3 months and 2 years ago or between about 6 months and 2 years ago. In some aspects, a subjects treated with a further vaccine composition of the embodiments are protected from moderate and severe COVID-19 disease in at least 80%, 85%, 90% or 95% of treated subjects. For example, the treated subjects can be protected from moderate and severe COVID-19 disease in at least 80%, 85%, 90% or 95% of treated subjects from about 2 weeks to about 1 year after administration of the further composition. In still further aspects, administering the further vaccine composition of the embodiments prevents moderate and severe COVID-19 disease in at least 80%, 85%, 90% or 95% of treated subjects from about 2 weeks to about 3 month, 6 months, 9 months, 1 year, 1.5 years, 2 years or 3 years after said administration.

In certain aspects, a method of the embodiments is further defined as a method of stimulating an antibody or CD8+ T-cell response in a subject. In some aspects, the method is defined as a method of stimulating a neutralizing antibody response in a subject. In further aspects, the method is defined as a method of stimulating a protective immune response in a subject In yet further aspects, the method is defined as a method of stimulating TH2 directed immune response in a subject.

In further aspects, administration of a vaccine/composition of the embodiments stimulates an antibody response that produces between about 10 and about 500 coronavirus spike protein-binding antibodies for every coronavirus neutralizing antibody in the subject. For example, the administration can stimulate an antibody response that produces no more than about 200 spike protein-binding antibodies for every coronavirus neutralizing antibody. In further aspects, the administration stimulates an antibody response that produces between about 10 and about 300; about 20 and about 300; about 20 and about 200; about 30 and about 100; or about 30 and about 80 coronavirus spike protein-binding antibodies for every coronavirus neutralizing antibody. In still further aspects, administration of composition of the embodiments stimulates an antibody response in a subject that includes a ratio of spike protein-binding antibodies to coronavirus neutralizing antibodies that is with 20%, 15%, 10% or 5% of the ratio of spike protein-binding antibodies to coronavirus neutralizing antibodies found in average convalescent patient serum (from a subject who has recovered from coronavirus infection).

In yet further aspects, administration of a composition of the embodiments stimulates an antibody response that produces between about 1 and about 500 coronavirus spike protein receptor binding domain (RBD)-binding antibodies for every coronavirus neutralizing antibody in the subject. In further aspects, the administration stimulates an antibody response that produces no more than about 50 spike protein RBD-binding antibodies for every coronavirus neutralizing antibody. In still further aspects, administration stimulates an antibody response that produces between about 1 and about 200; about 2 and about 100; about 3 and about 200; about 5 and about 100; about 5 and about 50; or about 5 and about 20 spike protein RBD-binding antibodies for every coronavirus neutralizing antibody. In still further aspects, administration of composition of the embodiments stimulates an antibody response in a subject that includes a ratio of spike protein RBD-binding antibodies to coronavirus neutralizing antibodies that is with 20%, 15%, 10% or 5% of the ratio of spike protein RBD-binding antibodies to coronavirus neutralizing antibodies found in average convalescent patient serum (from a subject who has recovered from coronavirus infection).

In still further aspects, administration of a vaccine/composition of the embodiments induces essentially no increase in IL-4, IL-13, TNF and/or IL-1β in the subject. In further aspects, the administration of a vaccine/composition of the embodiments induces essentially no increase in serum IL-4, IL-13, TNF and/or IL-1β in the subject In some aspects, the administration of a vaccine/composition of the embodiments induces essentially no increase in IL-4, IL-13, TNF and/or IL-1β at the injection site (*e*.*g*., an intramuscular injection site) in the subject.

In still further aspects, a method of the embodiments comprises administration of a vaccine/composition of the embodiments to a human subject having a disease. In certain aspects, the subject has cardiovascular disease, kidney disease, lung disease or an autoimmune disease. In some aspects, a vaccine/composition of the embodiments is administered to a subject who is receiving anti-coagulation therapy.

According to a further aspect, the present invention also provides a method of manufacturing a composition or a vaccine, comprising the steps of
a) optionally, adaptation of AU count of the respective sequences of component A and B according to WO2019092153A1 (claims 1 to 70), thereby modifying the retention time of the nucleic acid components on HPLC, allowing co-purification of the nucleic acid composition in step c);
b) RNA in vitro transcription step (of component A and B) using a DNA template in the presence of a cap analogue to obtain capped mRNA, preferably having a nucleic acid sequence as provided in **Table 17,** optionally wherein 2, 3, 4, 5, 6, 7, 8, 9, 10 or even more different RNAs are simultaneously produced in the RNA in vitro transcription step using , 3, 4, 5, 6, 7, 8, 9, 10 different DNA templates
c) Purifying the obtained capped RNA of step a) using RP-HPLC, and/or TFF, and/or Oligo(dT) purification and/or AEX, *preferably* using RP-HPLC and TFF;
d) Providing a first liquid composition comprising the purified capped RNA (of component A and B) of step b);
e) Providing a second liquid composition comprising at least one cationic lipid as defined herein, a neutral lipid as defined herein, a steroid or steroid analogue as defined herein, and a PEG-lipid as defined herein;
f) Introducing the first liquid composition and the second liquid composition into at least one mixing means to allow the formation of LNPs comprising capped RNA;
g) Purifying the obtained LNPs comprising capped RNA;
h) optionally, lyophilizing the purified LNPs comprising capped RNA.

Preferably, the mixing means of step e) is a T-piece connector or a microfluidic mixing device. Preferably, the purifying step f) comprises at least one step selected from precipitation step, dialysis step, filtration step, TFF step. Optionally, an enzymatic polyadenylation step may be performed after step a) or b). Optionally, further purification steps may be implemented to e.g. remove residual DNA, buffers, small RNA by-products etc. Optionally, RNA in vitro transcription is performed in the absence of a cap analog, and an enzymatic capping step is performed after RNA vitro transcription. Optionally, RNA in vitro transcription is performed in the presence of at least one modified nucleotide as defined herein.

In embodiments, step a, preferably steps a-c, more preferably all steps outlined above (a-g) are performed in an automated device for RNA in vitro transcription. Such a device may also be used to produce the composition or the vaccine. Preferably, a device as described in WO2020002598, in particular, a device as described in claims 1 to 59 and/or 68 to 76 of WO2020002598 (and Figures 1-18) may suitably be used.

In a further aspect, the present invention provides a method of stabilizing a pharmaceutical composition or a combination vaccine comprising lyophilizing or spray-drying the composition or vaccine to produce a stabilized composition or vaccine. Preferably, the stabilized composition or vaccine has a water content of less than about 10%, preferably a water content of between about 0.5% and 5.0%. Accordingly, the invention provides a stabilized, lyophilized composition or vaccine produced by a method of stabilizing as defined herein.

### Brief description of lists and tables

- **List A:**: Exemplary SARS-CoV-2 coronavirus isolates (EPI/GISAID)
- **List B:**: GenBank Accession Numbers of different SARS-CoV-2 isolates
- **List 1:**: Exemplary suitable SARS-CoV-2 antigen designs
- **Table 1:**: Preferred SARS-CoV-2 constructs (amino acid sequences and nucleic acid coding sequences)
- **Table 3A:**: Nucleic acid, preferably mRNA constructs encoding SARS-CoV-2 antigens
- **Table 3B:**: Nucleic acid, preferably mRNA constructs encoding SARS-CoV-2 antigens
- **Table 4:**: SARS-CoV-1 and SARS-associated constructs (amino acid sequences, nucleic acid coding sequences)
- **Table 5:**: Nucleic acid, preferably mRNA constructs encoding SARS-associated or SARS-CoV-1 antigens
- **Table 6:**: Preferred MERS-CoV constructs (amino acid sequences and nucleic acid coding sequences)
- **Table 7:**: Nucleic acid, preferably mRNA constructs encoding MERS-CoV antigens
- **Table 8:**: Preferred Influenza virus constructs (amino acid sequences and nucleic acid coding sequences)
- **Table 9:**: Nucleic acid, preferably mRNA constructs encoding Influenza virus antigens
- **Table 10:**: Preferred Influenza virus RNA sequences
- **Table 11:**: Preferred RSV constructs (amino acid sequences and nucleic acid coding sequences)
- **Table 12:**: Nucleic acid, preferably mRNA constructs encoding RSV antigens
- **Table 13:**: Preferred hMPV constructs (amino acid sequences and nucleic acid coding sequences)
- **Table 14:**: Nucleic acid, preferably mRNA constructs encoding hMPV antigens
- **Table 15:**: Preferred hPIV constructs (amino acid sequences and nucleic acid coding sequences)
- **Table 16:**: Nucleic acid, preferably mRNA constructs encoding hPIV antigens
- **Table 17:**: Overview of mRNA constructs used in Examples
- **Table 18:**: Overview of the nucleic acid based combination vaccines and vaccination scheme
- **Table A:**: Lipid-based carrier composition of the examples
- **Table 19:**: Overview of the nucleic acid based combination vaccines and vaccination scheme
- **Table 20:**: Overview of the nucleic acid based combination vaccines and vaccination scheme

### Brief description of the drawings

- **Figure 1**: Combination vaccines comprising component A-1 induce humoral immune responses against SARS-CoV-2 spike protein, shown by ELISA on day 21 after first vaccination (Figure 1 A) and by VNT-Assay on day 42 after second vaccination (Figure 1 B). Further details provided in Example 3 and Table 19.
- **Figure 2**: Combination vaccines comprising component B-3a induce humoral immune responses against RSV F protein, shown by ELISA on day 21 after first vaccination (Figure 2 A) and on day 42 after second vaccination (Figure 2 B). Further details provided in Example 3 and Table 19.
- **Figure 3**: Combination vaccines comprising component B-2 induce humoral immune responses against Influenza HA (A1 California/07/09), shown by ELISA on day 21 after first vaccination (Figure 3 A) and on day 42 after second vaccination (Figure 3 B). Further details provided in Example 3 and Table 19.
- **Figure 4**: Combination vaccines comprising component A-1 induce humoral immune responses against SARS-CoV-2 spike protein, shown by ELISA on day 21 after first vaccination (Figure 4 A) and by VNT-Assay on day 42 after second vaccination (Figure 4 B). Further details provided in Example 4 and Table 20.
- **Figure 5**: Combination vaccines comprising component B-3a induce humoral immune responses against RSV F protein, shown by ELISA on day 21 after first vaccination (Figure 5 A) and on day 42 after second vaccination (Figure 5 B). Further details provided in Example 4 and Table 20.
- **Figure 6**: Combination vaccines comprising component B-3b induce humoral immune responses against hMPV F protein, shown by FACS-based Immunoassay on day 42 after second vaccination for hMPV-A1 (Figure 6 A) and for hMPV-B1 (Figure 6 B). Further details provided in Example 4 and Table 20.
- **Figure 7**: Combination vaccines comprising component B-4a induce humoral immune responses against PIV3 F protein, shown by FACS-based Immunoassay on day 42 after second vaccination. Further details provided in Example 4 and Table 20.
- **Figure 8**: Combination vaccines comprising component B-2 induce humoral immune responses against Influenza HA (A1 Califomia/07/09), shown by ELISA on day 42 after second vaccination. Further details provided in Example 4 and Table 20.

### Abbreviations

FLUAV: Influenza A virus
FLUBV: Influenza B virus
RSV: Human respiratory syncytial virus
HRSV-A, RSV-A: Human respiratory syncytial virus A
HPIV, hPIV: Human respirovirus (Human parainfluenzavirus)
HPIV3: Human respirovirus 3
HMPV, hMPV: Human metapneumovirus
MERS-CoV: Middle East respiratory syndrome-related coronavirus
SARS-CoV: Severe acute respiratory syndrome-related coronavirus
HCoV: Human coronavirus
Bat SARS-like CoV: Bat SARS-like coronavirus
BatCoV: Rousettus bat coronavirus
PDCoV: Porcine deltacoronavirus
PEDV: Porcine epidemic diarrhea virus
MHV: Murine hepatitis virus

### Examples

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods, which are functionally equivalent are within the scope of the invention. Indeed, various modifications of the invention in addition to those described herein will become readily apparent to those skilled in the art from the foregoing description and the examples below. All such modifications fall within the scope of the appended claims.

### Example 1: Preparation of DNA and RNA constructs, compositions, and vaccines

The present example provides methods of obtaining the RNA of the invention as well as methods of generating a composition or a vaccine of the invention.

### 1.1. Preparation of DNA and RNA constructs:

DNA sequences encoding different virus antigen designs were prepared and used for subsequent RNA *in vitro* transcription reactions. Said DNA sequences were prepared by modifying the wild type or reference encoding DNA sequences by introducing a G/C optimized or modified coding sequence (e.g., "cds opt1") for stabilization and expression optimization. Sequences were introduced into a pUC derived DNA vector to comprise stabilizing 3'-UTR sequences and 5'-UTR sequences, additionally comprising a stretch of adenosines (e.g. A64 or A100), and optionally a histone-stem-loop (hSL) structure, and optionally a stretch of 30 cytosines (e.g. C30) (see **Table 17**). The obtained plasmid DNA constructs were transformed and propagated in bacteria using common protocols known in the art. Eventually, the plasmid DNA constructs were extracted, purified, and used for subsequent RNA *in vitro* transcription.

### 1.2. RNA in vitro transcription from plasmid DNA templates:

DNA plasmids prepared according to section 1.1 were enzymatically linearized using a restriction enzyme and used for DNA dependent RNA *in vitro* transcription using T7 RNA polymerase in the presence of a nucleotide mixture (ATP/GTP/CTP/UTP) and cap analog (e.g. m7GpppG, m7G(5')ppp(5')(2'OMeA)pG or m7G(5')ppp(5')(2'OMeG)pG)) under suitable buffer conditions. The obtained RNA constructs were purified using RP-HPLC (PureMessenger^{®}, CureVac AG, Tübingen, Germany; WO2008077592) and used for *in vitro* and *in vivo* experiments. The generated RNA sequences/constructs are provided in **Table 17** with the encoded antigenic protein and the respective UTR elements indicated therein.

**Table 17: Overview of mRNA constructs used in Examples**

| **RNA ID** | **Name** | **SEQ ID NO: Protein** | **SEQ ID NO: CDS (gc)** | **SEQ ID NO: RNA** |
|---|---|---|---|---|
| R1 | FLUAV/H1N1/A/California/7/2009 hemagglutinin (HA) | 14178 | 14230 | 14438 |
| R2 | FLUAV/H3N2/A/Hong Kong/4801/2014 hemagglutinin (HA) | 14181 | 14233 | 14441 |
| R3 | FLUBV/HON0/B/Brisbane/60/2008 hemagglutinin (HA) | 14182 | 14234 | 14442 |
| R4 | FLUBV/H0N0/B/Phuket/3073/2013 hemagglutinin (HA) | 14183 | 14235 | 14443 |
| R5 | FLUAV/H1N1/A/California/7/2009 neuraminidase (NA) | 14210 | 14262 | 14470 |
| R6 | FLUAV/H3N2/A/Hong Kong/4801/2014 neuraminidase (NA) | 14213 | 14265 | 14473 |
| R7 | FLUBV/H0N0/B/Brisbane/60/2008 neuraminidase (NA) | 14214 | 14266 | 14474 |
| R8 | HRSV-A/A2 fusion glycoprotein (F-del_DSCav1_mut5) | 14550 | 14560 | 14600 |
| R9 | HPIV3/Homo sapiens/PER/FLA4571/2008 fusion glycoprotein (F0) | 14612 | 14614 | 14622 |
| R10 | HPIV3/BJ/001/08 hemagglutinin-neuraminidase (HN) | 14613 | 14615 | 14623 |
| R11 | HMPV/TN/92-4 fusion glycoprotein (F0) | 14626 | 14650 | 14746 |
| R12 | MERS-CoV/MERS-CoV-Jeddah-human-1 spike protein (1-1353)(V1060P_L1061P) | 14795 | 14811 | 14875 |
| R13 | SARS-CoV/Tor2 spike protein (1-1255)(K968P_V969P) | 14910 | 14955 | 15135 |
| R14 | SARS-CoV-2/Wuhan/WIV05/2019 spike protein (1-1273)(K986P _V987P); R9515 | 10 | 137 | 163 |
| R15 | SARS-CoV-2/Wuhan/WIV05/2019 spike protein (1-1273)(K986P_V987P); R9709 | 10 | 137 | 149 |
| R16 | SARS-CoV-2/Wuhan/WIV05/2019 spike protein (1-1273)(K986P_V987P_L18F_D80A_D215G_L242del_A243del_L244del_R246I_K4 17N_E484K_N501Y_D614G_A701V); R10384 | 22961 | 23091 | 23531 |
| R17 | SARS-CoV-2/Wuhan/WIV05/2019 spike protein (1-1273)(K986P_V987P_H69del_V70del_Y144del_N501Y_A570D_D614G_P681H_T 716I_S982A_D1118H); R10357 | 22959 | 23089 | 23529 |
| R18 | FLUAV/H1N1/A/California/7/2009 hemagglutinin (HA); R8849 | 14178 | 14230 | 26949 |
| R19 | FLUAV/H3N2/A/Hong Kong/4801/2014 hemagglutinin (HA); R8853 | 14181 | 26946 | 26950 |
| R20 | FLUBV/HON0/B/Brisbane/60/2008 hemagglutinin (HA); R8852 | 14182 | 14234 | 26951 |
| R21 | FLUBV/H0N0/B/Phuket/3073/2013 hemagglutinin (HA); R8854 | 14183 | 14235 | 26952 |
| R22 | FLUAVIH1N1/A/California/7/2009 neuraminidase (NA); R8858 | 14210 | 14262 | 26953 |
| R23 | FLUAV/H3N2/A/Hong Kong/4801/2014 neuraminidase (NA); R8856 | 14213 | 14265 | 26954 |
| R24 | FLUBV/HON0/B/Brisbane/60/2008 neuraminidase (NA); R8860 | 14214 | 14266 | 26955 |
| R25 | HRSV-A/A2 fusion glycoprotein (F-del_DSCav1_mut5); R8898 | 14550 | 14560 | 14580 |
| R26 | HPIV3/USA/629-D01959/2007 fusion glycoprotein (variant); R10331 | 26957 | 26959 | 26962 |
| R27 | HMPV/00-1 fusion glycoprotein (variant); R10326 | 26971 | 26977 | 26984 |
| R28 | HMPV/NL/1/99 fusion glycoprotein (variant); R10329 | 26968 | 26974 | 26981 |

### 1.4. Preparation of an LNP formulated mRNA composition:

LNPs were prepared using cationic lipids, structural lipids, a PEG-lipids, and cholesterol. Lipid solution (in ethanol) was mixed with RNA solution (aqueous buffer) using T-piece formulation as outlined below. Obtained LNPs were re-buffered in a carbohydrate buffer via dialysis, and optionally up-concentrated to a target concentration using ultracentrifugation tubes. LNP-formulated mRNA was stored at -80°C prior to use in *in vitro* or *in vivo* experiments.

In short, lipid nanoparticles were prepared and tested according to the general procedures described in PCT Pub. Nos. WO2015199952, WO 2017004143 and WO2017075531, the full disclosures of which are incorporated herein by reference. Lipid nanoparticle (LNP)-formulated mRNA was prepared using an ionizable amino lipid (cationic lipid), phospholipid, cholesterol and a PEGylated lipid (lipids see **Table A**). LNPs were prepared as follows. Cationic lipid according to formula III-3 (ALC-0315), DSPC, cholesterol and PEG-lipid according to formula IVa (ALC-0159) were solubilized in ethanol at a molar ratio of approximately 47.5:10:40.8:1.7. Lipid nanoparticles (LNP) comprising compound III-3 (ALC-0315) were prepared at a ratio of mRNA to total Lipid of 0.03-0.04 w/w. Briefly, the mRNA was diluted to 0.05 to 0.2mg/mL in 10 to 50mM citrate buffer, pH 4. Syringe pumps were used to mix the ethanolic lipid solution with the mRNA aqueous solution at a ratio of about 1:5 to 1:3 (vol/vol) with total flow rates above 15ml/min. The ethanol was then removed and the external buffer replaced with PBS by dialysis. Finally, the lipid nanoparticles were filtered through a 0.2µm pore sterile filter. Lipid nanoparticle particle diameter size was 60-90nm as determined by quasi-elastic light scattering using a Malvem Zetasizer Nano (Malvern, UK).

**Table A: Lipid-based carrier composition of the examples**

| | **Compounds** | **Ratio (mol%)** | **Structure** | **Mass** |
|---|---|---|---|---|
| 1 | Cholesterol | 40.9 | 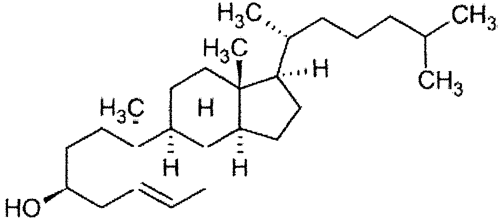 | 386.4 |
| 2 | 1,2-distearoyl-sn-glycero-3-phosphocholin e (DSPC) | 10 | 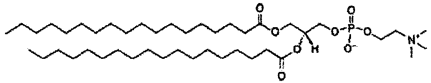 | 789.6 |
| 3 | Cationic Lipid | 47.4 | 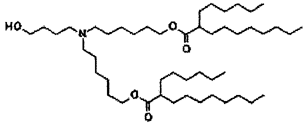 | 765.7 |
| 4 | PEG Lipid | 1.7 | 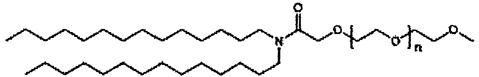 Average n = ~49 | 2010.1 |

### 1.5. Preparation of combination mRNA vaccines:

Combination mRNA vaccines were formulated with LNPs either in a separate or co-formulated way. For separately mixed or formulated mRNA vaccines, each mRNA component was prepared and separately LNP formulated as described in **Example 1.4,** followed by mixing of the different LNP-formulated components. For co-formulated mRNA vaccine, the different mRNA components were firstly mixed together, followed by a co-formulation in LNPs as described in **Example 1.4.**

### Example 2: Vaccination of mice with nucleic acid based combination vaccines

### Preparation of LNP formulated mRNA vaccine:

mRNA constructs for a combination vaccine are prepared as described in **Example 1** (RNA *in vitro* transcription). HPLC purified mRNA is formulated with LNPs according to **Example 1.4** and **Example 1.5** prior to use in *in vivo* vaccination experiments.

### Immunization:

Female BALB/c mice (6-8 weeks old) are injected intramuscularly (i.m.) with mRNA combination vaccine (respective RNA identifiers see **Table 17**) with doses as indicated in **Table 18.** As a negative control, one group of mice is vaccinated with buffer. All animals are vaccinated on day 0 and 21. Blood samples are collected on day 21 (post prime) and 42 (post boost) for the determination of antibody titers. After vaccination experiments, the efficiency of the combination vaccines is determined.

**Table 18: Overview of the nucleic acid based combination vaccines and vaccination scheme**

| | | **Component A (mRNA)** | **Component B (mRNA)** | **Formulation** | **Dose** |
|---|---|---|---|---|---|
| 1 | Combination vaccine: SARS-CoV-2 - Influenza | R14 | R1-R7 | LNP co-formulated | 5µg |
| 2 | Combination vaccine: SARS-CoV-2 - Influenza - RSV | R14 | R1-R7 | LNP co-formulated | 5µg |
| | | | R8 | | |
| 3 | Combination vaccine: SARS-CoV-2 - RSV | R14 | R8 | LNP co-formulated | 5µg |
| 4 | Combination vaccine: SARS-CoV-2 - MERS - SARS-CoV-1 | R14 | R12 | LNP co-formulated | 5µg |
| | | | R13 | | |
| 5 | Combination vaccine: SARS-CoV-2 - PIV - hMPV | R14 | R9-10, R11 | LNP co-formulated | 5µg |
| 6 | Buffer | | | | |

### Determination of IgG1 and IgG2 antibody titers using ELISA:

ELISA is performed using recombinant antigenic proteins for coating. Coated plates are incubated using respective serum dilutions, and binding of specific antibodies coated protein are detected using biotinylated isotype specific anti-mouse antibodies followed by streptavidin-HRP (horse radish peroxidase) with Amplex as substrate. Endpoint titers of antibodies (IgG1, IgG2a) are measured by ELISA on day 21, and 42 post vaccinations.

### Detection of spike protein-specific immune responses (SARS-CoV-1, SARS-CoV-2, MERS-CoV):

Hela cells are transfected with 2µg of mRNA encoding the spike protein using lipofectamine. The cells are harvested 20h post transfection, and seeded at 1x10⁵ per well into a 96 well plate. The cells are incubated with serum samples of vaccinated mice (diluted 1:50) followed by a FITC-conjugated anti-mouse IgG antibody. Cells were acquired on BD FACS Canto II using DIVA software and analyzed by FlowJo.

### Intracellular cytokine staining:

Splenocytes from vaccinated mice are isolated according to a standard protocol known in the art. Briefly, isolated spleens are grinded through a cell strainer and washed in PBS/1%FBS followed by red blood cell lysis. After an extensive washing step with PBS/1%FBS, splenocytes are seeded into 96-well plates (2x10⁶ cells per well). Cells are stimulated with a mixture of protein specific peptide epitopes (5µg/ml of each peptide) in the presence of 2.5µg/ml of an anti-CD28 antibody (BD Biosciences) for 6 hours at 37°C in the presence of a protein transport inhibitor. After stimulation, cells are washed and stained for intracellular cytokines using the Cytofix/Cytoperm reagent (BD Biosciences) according to the manufacturer's instructions. The following antibodies are used for staining: Thy1.2-FITC (1:200), CD8-APC-H7 (1:100), TNF-PE (1:100), IFNy-APC (1:100) (eBioscience), CD4-BD Horizon V450 (1:200) (BD Biosciences) and incubated with Fcy-block diluted 1:100. Aqua Dye is used to distinguish liveldead cells (Invitrogen). Cells are acquired using a Canto II flow cytometer (Beckton Dickinson). Flow cytometry data is analyzed using FlowJo software package (Tree Star, Inc.)

### Determination of virus neutralization titers:

Serum is collected for determination of neutralization titers (VNTs) detected via CPE (cytopathic effect) or via a pseudotyped particle-based assay.

### Determination of HA specific immune responses

Detection of an HA-specific immune response (B-cell immune response) is carried out by detecting IgG2a antibodies directed against the particular influenza virus. Therefore, blood samples is taken from the vaccinated mice three weeks after vaccination and sera is prepared. MaxiSorb plates (Nalgene Nunc international) are coated with the particular recombinant HA protein. After blocking with 1xPBS containing 0.05% Tween-20 and 1% BSA the plates are incubated with diluted mice serum (as indicated). Subsequently a biotin-coupled secondary antibody (anti-mouse-lgG2a, Pharmingen) was added. After washing, the plate was incubated with horseradish peroxidase-streptavidin, followed by addition of the Amplex UltraRed Reagent (Invitrogen) and subsequent quantification of the fluorescent product

### Determination of virus neutralization RSV titers:

RSV virus neutralization titers (VNTs) are measured on serum samples using a plaque reduction neutralization test (PRNT). Diluted serum samples are incubated with RSV/A2 for 1 hour at room temperature and inoculated in duplicates onto confluent HEp-2 monolayers in 24 well plates. After one hour incubation at 37°C in a 5% CO2 incubator, the wells are overlayed with 0.75% Methylcellulose medium. After 4 days of incubation, the overlays are removed and the cells are fixed and stained. The corresponding reciprocal neutralizing antibody titers are determined at the 60% reduction end-point of the virus control.

### Example 3: Vaccination of mice with nucleic acid based combination vaccines (mRNA vaccines for SARS-CoV-2, RSV and/or Influenza)

The present example shows that combination mRNA vaccines induce strong and antigen-specific immune responses against all components of the combination vaccine (mRNA comprising at least one coding sequence encoding at least one antigenic peptide or protein selected from component A and/or from component B).

### Preparation of LNP formulated mRNA vaccine compositions/combinations:

mRNA constructs for a combination vaccine were prepared as described in **Example 1** (RNA *in vitro* transcription). HPLC purified mRNA was formulated with LNPs according to **Example 1.4** and/or **Example 1.5** prior to use in *in vivo* vaccination experiments.

### Immunization:

Female BALB/c mice (6-8 weeks old) were injected intramuscularly (i.m.) with mRNA combination vaccine (respective RNA identifiers see **Table 17**) with doses as indicated in **Table 19.** As a negative control, one group of mice was vaccinated with buffer (0.9 % NaCl). All animals received an injection volume of 25µl and were vaccinated on day 0 and 21. Blood samples are collected on day 21 (post first vaccination) and 42 (post second vaccination) for the determination of antibody titers.

**Table 19: Overview of the nucleic acid based combination vaccines and vaccination scheme**

| Group | Vaccine composition: | **Component A-1 (mRNA)** | **Component B-2 (mRNA)** | **Component B-3a (mRNA)** | **Formulation** | **Total Dose** |
|---|---|---|---|---|---|---|
| 1 | SARS-CoV-2 mRNA vaccine | R15 | - | - | separately formulated | 0.5µg |
| 2 | RSV mRNA vaccine | | - | R25 | separately formulated | 0.5µg |
| 3 | Combination vaccine: SARS-CoV-2 mRNA vaccine + RSV mRNA vaccine | R15 | - | R25 | separately formulated | 1µg (0.5µg each) |
| 4 | Heptavalent Influenza mRNA vaccine | - | R18-R24 | - | co-formulated | 3.5µg (0.5µg each) |
| 5 | Combination vaccine: SARS-CoV-2 mRNA vaccine + heptavalent Influenza mRNA vaccine | R15 | R18-R24 | - | A-1 and B-2 separately formulated | 4µg (0.5µg each) |
| 6 | Combination vaccine: SARS-CoV-2 mRNA vaccine + RSV mRNA vaccine + heptavalent Influenza mRNA vaccine | R15 | R18-R24 | R25 | separately formulated | 4.5µg (0.5µg each) |
| 7 | Control 0.9% NaCl | - | - | - | - | - |

### Determination of total IgG antibody titers for SARS-CoV-2 and RSV using ELISA:

S_{RBD} or RSV-F protein specific total IgG binding antibodies were quantified using an Amplex ELISA. Samples were pre-diluted 1:5 and consecutive 1:10 dilutions were tested (8 dilutions). SARS-CoV-2 (2019-nCoV) Spike RBD-His was used for coating (1µg/ml) or 2.5µg/ mL rec. RSV-F GP A2 (SinoBiological, Cat 11049-V08B). Plates were thoroughly washed after each incubation step. The coating was done at 37°C for 4-5 hours. This was followed by an overnight blocking at 4°C. Serum samples were pre-diluted 1:5 in blocking buffer on a separate uncoated plate. The titrated sera samples were added into the pre-coated plate and incubated for 2-4h at RT. Detection antibodies were added into each well (1:300) and incubated for 1-1.5h at RT. Incubation with HRP-Streptavidin (1:1000) in sterile blocking buffer (1% BSA without NaN₃) was carried out for 30 minutes. Amplex^{®} UltraRed reagent (dissolved in DMSO; 1:200, 30% hydrogen peroxide H₂O₂ 1:2000 in substrate buffer) was added into each well. The fluorescence was measured after a minimum of 45 minutes and a maximum of 24 hours. Endpoint titers of antibodies were measured by ELISA on day 21 post prime vaccination for SARS-CoV-2 and on day 21 and 42 for RSV.

### Determination of virus neutralization titers for SARS-CoV-2:

Serum was collected for determination of SARS-CoV-2 neutralization titers (VNTs) detected via CPE (cytopathic effect). Serial dilutions of heat-inactivated sera (56°C for 30min) tested in duplicates with a starting dilution of 1:10 followed by 1:2 serial dilutions were incubated with 100 TCID₅₀ of wild type SARS-CoV-2 (strain 2019-nCov/Italy-INMI1) for 1 hour at 37°C. Every plate contained a dedicated row (8 wells) for cell control which contains only cells and medium, and a dedicated row of virus control which contain only cells and virus. Infectious virus was quantified upon incubation of 100µl of virus-serum mixture with a confluent layer of Vero E6 cells (ATCC, Cat.1586) followed by incubation for 3 days at 37°C and microscopical scoring for CPE formation. A back titration was performed for each run in order to verify the correct range of TCID50 of the working virus solution. VN titres were calculated according to the method described by Reed & Muench. If no neutralization was observed (MNt <10), an arbitrary value of 5 was reported. Analyses were carried out at VisMederi srl (Siena, Italy). Virus neutralization titers were measured with sera collected on day 42.

### Determination of Influenza HA specific immune responses (via Hemagglutinin Inhibition Assay)

Functional anti-HA antibody titers were analyzed by hemagglutination inhibition (HI) assay. Sera were incubated with receptor destroying enzyme (RDEII, Denka Seiken) at 37°C overnight, inactivated (56 °C, 60min). Dilutions of pre-treated sera were incubated for 45min with 4 HAU of inactivated influenza A H1N1 California/07/09 (NIBSC Material 09/174) and 50µl 0.5% erythrocyte solution was added. The plates were incubated for 45-60 minutes and HI titers are determined by the highest dilution of serum that leads to the complete inhibition of agglutination.

### Results:

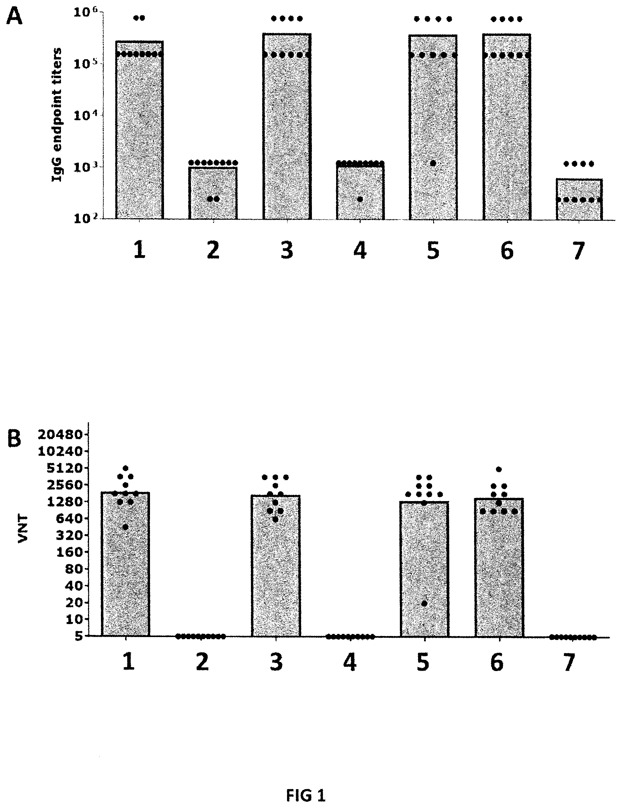

As shown in **Figure 1** **A** the vaccination with mRNA encoding full length S stabilized protein (S_stab) or combination vaccine comprising mRNA encoding full length S stabilized protein (S_stab) induced high titers of Spike specific binding antibodies after a single vaccination (day 21) **(****Figure 1** **A:** total IgG). **Figure 1** **B** shows the induction of functional antibodies after the second vaccination (day 42) (**Figure 1** **B:** Virus neutralizing antibodies VNTs). The combination of component A-1 with component B-2 (Heptavalent Influenza mRNA vaccine (group 5)) and/or **B-3a** (RSV mRNA vaccine (group 3), (group 6)) does not influence the induction of humoral antibody responses in a negative way. The data shows that the observed immune responses of the respective combination vaccines comprising mRNA encoding full length S stabilized protein are comparable to those observed for the monovalent vaccine group (group 1).

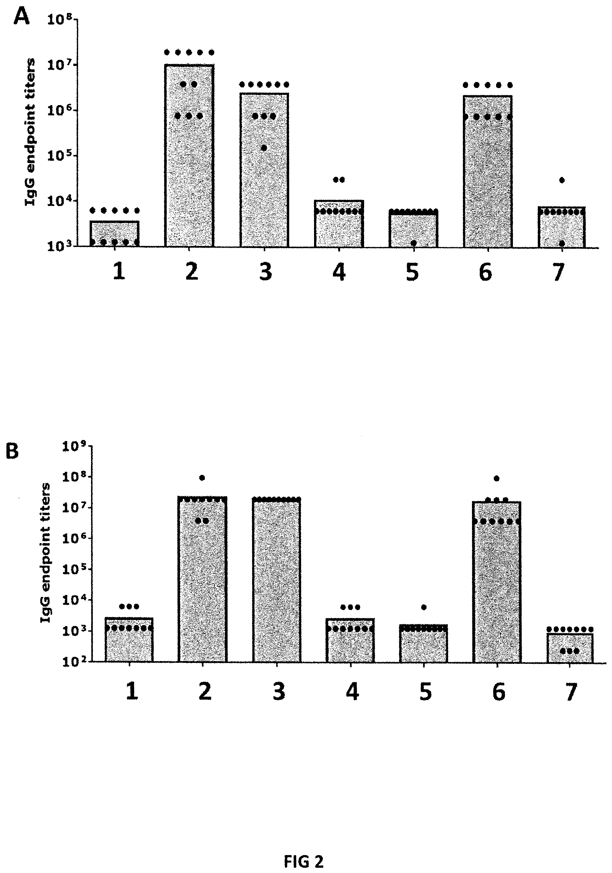

The induced antibody responses against RSV F protein (component B-3a) are shown in **Figure 2** **A** (day 21, after prime vaccination) and **B** (day 42, after second vaccination). All the combination vaccines comprising mRNA encoding RSV F protein induced high titers of IgG antibodies (group 3 and group 6). The data shows that the observed immune responses of the respective combination vaccines comprising mRNA encoding RSV F protein protein are comparable to those observed for the monovalent vaccine (group 2).

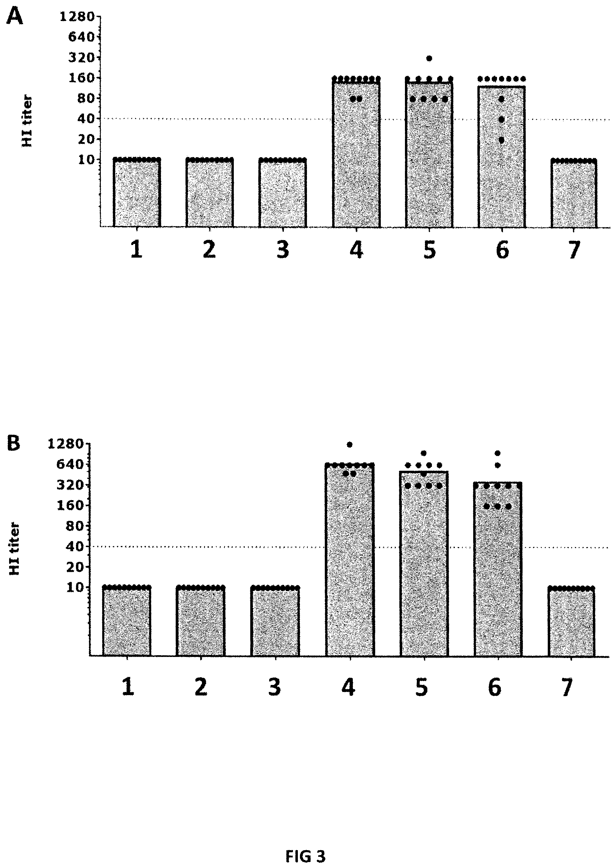

As shown in **Figure 3** **A** and **B** the induction of functional antibodies against Influenza virus measured by Hemagglutinin Inhibition Assay was induced by vaccine combinations comprising the heptavalent Influenza vaccine component B-2. HI titers were determined on day 21 after one vaccination (**Figure 3** **A**) and on day 42 after two vaccinations (**Figure 3** **B**). The data shows that the observed immune responses of the combination vaccines comprising mRNA encoding influenza antigens (group 5 and group 6) are comparable to those observed for the heptavalent influenza vaccine (group 4).

As demonstrated in the present example, the tested combination vaccines are suitable to deliver the different mRNA components to the host cells to translate the mRNA components into antigenic proteins or antigenic peptides, which induces antigen-specific immune responses against all the encoded proteins.

Summarizing the above, the data demonstrates that the combination of antigens of SARS CoV-2, Influenza virus and RSV is possible and that the combination vaccines induce strong immune responses against each of the encoded antigens. In addition to that, all components of the tested combination vaccines induce efficient immune responses which indicates that single components were not immunodominant, or that the components of the combination vaccines do not show immune-interference.

### Example 4: Vaccination of mice with nucleic acid based combination vaccines (mRNA vaccines for SARS-CoV-2, RSV, Influenza, hMPV, and/or PIV3)

The present example shows that combination mRNA vaccines induce strong and antigen-specific immune responses against all components of the combination vaccine (mRNA comprising at least one coding sequence encoding at least one antigenic peptide or protein selected from component A and/or from component B). The combination vaccine induced high antibody responses against all encoded proteins, independent if co-formulated or separately mixed after LNP formulation. The present example also shows the combination of five different mRNA vaccine components (component A-1: SARS-CoV-2, component B-3a: RSV, component B-2: Influenza, heptavalent component B-3b: hMPV (bivalent), and component B-4a: PIV). This combination vaccine includes 12 different antigens (12-valent).

### Preparation of LNP formulated mRNA vaccine:

mRNA constructs for a combination vaccine were prepared as described in **Example 1** (RNA *in vitro* transcription). HPLC purified mRNA was formulated with LNPs according to **Example 1.4** and/or **Example 1.5** prior to use in *in vivo* vaccination experiments.

The co-formulated 12-valent combination vaccine (see group 7, **Table 20**,) was prepared by mixing by molarity, so equal numbers of RNA molecules were mixed (equimolar). The separately formulated 12-valent combination vaccine (see group 6, **Table 20),** the total amount of RNA was used and no adjustment according to molarity was performed (equal mass).

### Immunization:

Female BALB/c mice (6-8 weeks old) were injected intramuscularly (i.m.) with mRNA combination vaccine (respective RNA identifiers see **Table 17**) with doses as indicated in **Table 20.** As a negative control, one group of mice was vaccinated with buffer (0.9% NaCl). All animals received 25µl and were vaccinated on day 0 and 21. Blood samples are collected on day 7 and day 21 (post first vaccination) and 42 (post second vaccination) for the determination of antibody titers.

**Table 20: Overview of the nucleic acid based combination vaccines and vaccination scheme**

| | **Vaccine composition:** | **Component A (mRNA)** | **Component B-2 (mRNA)** | **Component B-3a (mRNA)** | **Component B-3b (mRNA)** | **Component B-4a (mRNA)** | **LNP formulation** | **Total Dose** |
|---|---|---|---|---|---|---|---|---|
| 1 | SARS-CoV-2 mRNA vaccine | R15 | - | - | - | - | separately formulated | 0.5µg |
| 2 | Bivalent hMPV mRNA vaccine | - | - | - | R27+R28 | - | co-formulated | 1µg |
| 3 | Combination vaccine: SARS-CoV-2 mRNA vaccine + Bivalent hMPV mRNA vaccine | R15 | - | - | R27+R28 | - | separately formulated | 1.5µg (0.5µg each) |
| 4 | PIV3 mRNA vaccine | - | - | - | - | R26 | | 0.5µg |
| 5 | Combination vaccine: SARS-CoV-2 mRNA vaccine PIV3 mRNA vaccine | R15 | - | - | - | R26 | separately formulated | 1µg (0.5µg each) |
| 6 | Combination vaccine: 12-valent cocktail mRNA vaccine | R15 | R18-R24 | R25 | R27+R28 | R26 | separately formulated; equal mass. | 6µg (0.5µg each) |
| 7 | Combination vaccine: 12-valent cocktail mRNA vaccineL | R15 | R18-R24 | R25 | R27+R28 | R26 | co-formulated; equimolar | 6µg |
| 8 | Control 0.9% NaCl | - | - | - | - | - | - | - |

SARS-CoV-2, Influenza and RSV-specific immune responses (VNT, total IgG and HI titer) were analyzed as described in **Example 3.**

### FACS-based ELISA for determination of PIV-3 or hMPV-A1 and hMPV-B1 specific immune responses

HeLa cells were transfected with 17µg mRNA per T75 flask of R27 (R10326 (hMPV-A1 stabilized fusion protein with furin cleavage - F(19-539)PFs_GCv3,1)), R28 (R10329 (hMPV-B1 stabilized fusion protein with furin cleavage - F(19-539)_PFs_GCv3.1)), R26 (R10331 (PIV-3 fusion protein stabilized - F(19-539)DS2-Cav_GCv3.1)) using Lipofectamine 2000. Transfected cells were harvested 18-24h post lipofection and seeded in 96 V-bottom plate at 2x10⁵ cells/well The mouse serum samples collected in on day 42 were used as primary antibody followed by anti-mouse FITC-conjugated secondary antibody.

### Results

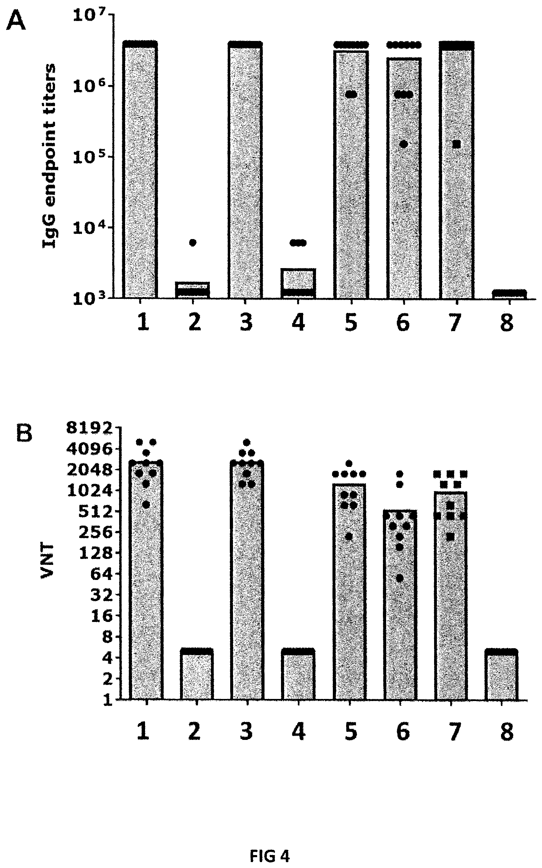

As shown in **Figure 4** **A** the vaccination with mRNA encoding full length S stabilized protein (S_stab) alone or the combination vaccines comprising mRNA encoding full length S stabilized protein (S_stab) induced high titers of Spike specific binding antibodies after a single vaccination (day 21) (**Figure 4** **A:** total IgG, ELISA). **Figure 4** **B** shows the induction of functional antibodies after the second vaccination (d42) (**Figure 4** **B:** Virus neutralizing antibodies VNTs). The combination with component B-3b (hMPV, bivalent mRNA vaccine (A+D)) or **B-4a** (PIV-3 mRNA vaccine (A+E)) does not influence the induction of humoral antibody responses in a negative way. The data shows that the observed immune responses of the respective combination vaccines comprising mRNA encoding full length S stabilized protein are comparable to those observed for the monovalent vaccine group (group 1).

Even the 12-valent vaccine combinations (group 6 and group 7) induced high SARS-CoV-2 specific immune responses (binding antibodies and VNTs). The response of the LNP co-formulated vaccine combination (group7) was slightly higher compared to separately LNP-formulated and mixed mRNA combinations (group 6).

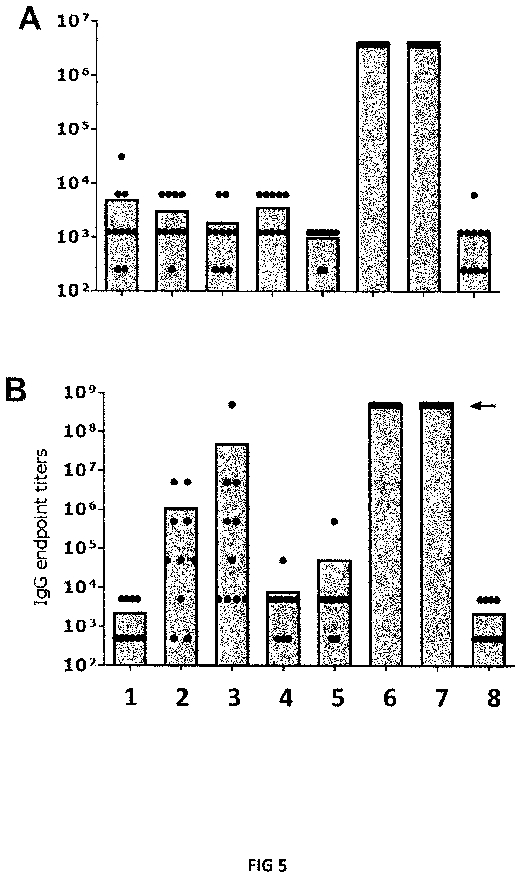

RSV-F specific immune responses measured via ELISA are shown in **Figure 5** **A** (day 21) and **B** (day 42). Both 12-valent combination vaccine (group 6 and group 7) induced high titers of total IgG. The titers on day 42 (**Figure 5** **B**) for both groups might be even higher (indicated by the arrow, due to assay conditions). Antibody titers against combination vaccines comprising component B-3b (comprising mRNA encoding hMPV antigens) raised also slight IgG titers (group 2 and 4). This might be due to cross-immunity between F proteins of related virus strains. RSV and hMPV both are belonging to Pneumoviridae virus family.

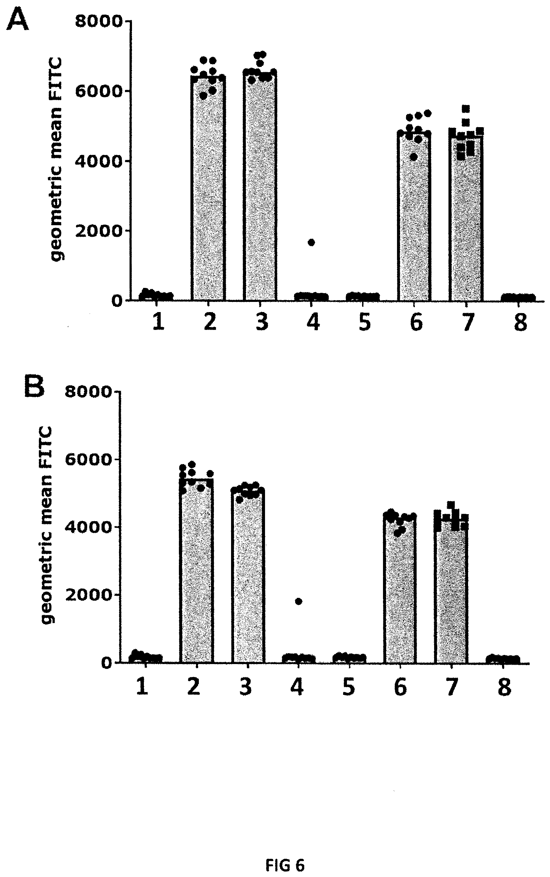

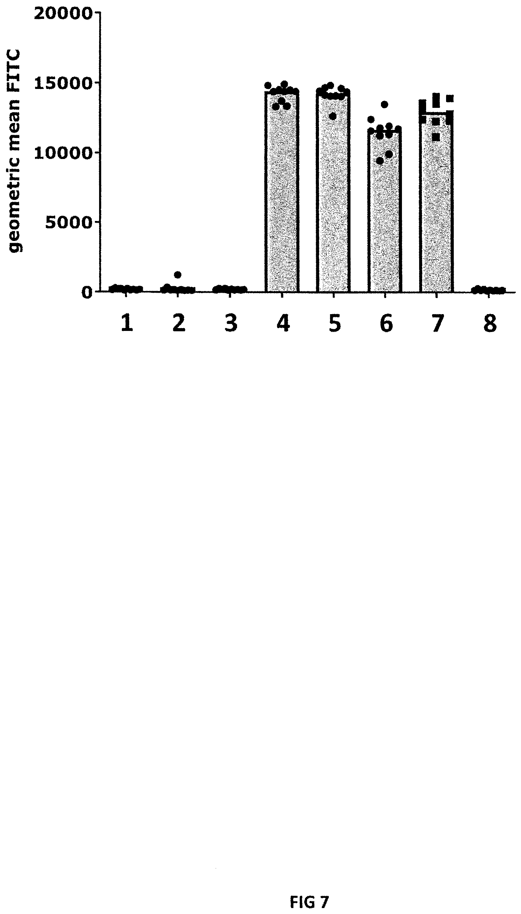

As shown in **Figure** 6 **A** and **B** all the vaccine combinations comprising component B3-b (comprising mRNA encoding bivalent hMPV antigen (group 2, 3, 6, and 7) induced strong humoral immune responses against F protein of hMPV A1 and B1 strains **(****Figure 6** **A:** hMPV A1, **6 B:** hMPV B1, day 42). **Figure 7** demonstrates the immune responses elicited against PIV3 F protein on day 42 after first vaccination. All vaccine combinations comprising component B-4a (comprising mRNA encoding PIV3 F protein (group 4, 5, 6, and 7) induced humoral immune responses against PIV3 F protein.

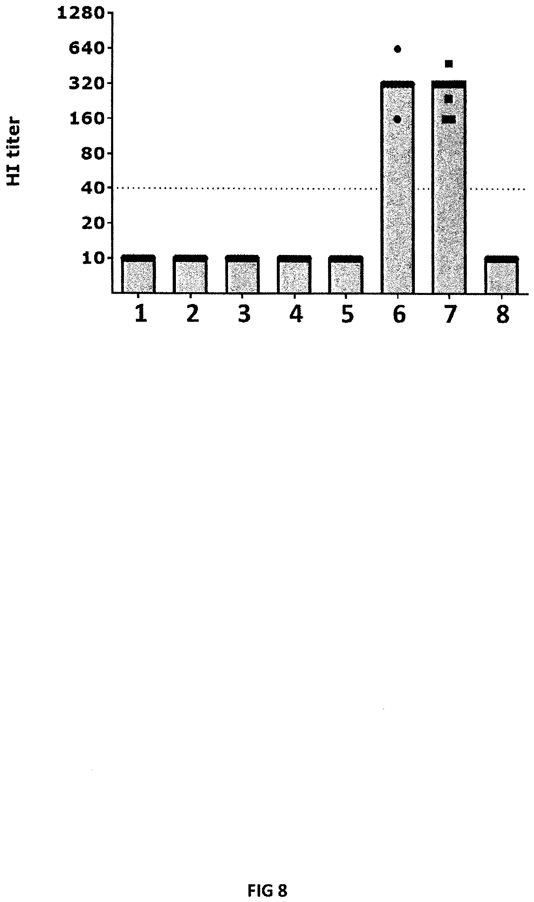

As shown in **Figure 8** the induction of functional antibodies against Influenza virus measured by Hemagglutinin Inhibition Assay was induced by vaccine combinations comprises the heptavalent Influenza vaccine component B-2 (group 6 and group 7). HI titers were determined on day 42 after two vaccinations.

As demonstrated in the present example, the tested combination vaccines are suitable to deliver the different mRNA components to the host cells to translate the mRNA components into antigenic proteins or antigenic peptides, which induces antigen-specific immune responses against all the encoded proteins.

Summarizing the above, the data demonstrates that the combination of antigens of SARS CoV-2, Influenza virus, hMPV, PIV, and RSV is possible and that the combination vaccines induce strong immune responses against each of the encoded antigens. In addition to that, all components of the tested combination vaccines induce efficient immune responses which indicates that single components were not immunodominant, or that the components of the combination vaccines do not show immune-interference.

### Example 5: Clinical development of a nucleic acid based combination vaccine

To demonstrate safety and efficiency of the nucleic acid based combination vaccine, a clinical trial (phase 1) is initiated. In the clinical trial, a cohort of human volunteers is intramuscularly injected for at least two times (e.g. day 0 and day 28 with a dose of 2µg, 6µg, or 18µg nucleic acid based combination vaccine (formulated in LNPs according to the invention). In order to assess the safety profile of the vaccine, subjects are monitored after administration (vital signs, vaccination site tolerability assessments, hematologic analysis). The efficacy of the immunization is analyzed by determination of virus neutralizing titers (VNT) in sera from vaccinated subjects. Blood samples are collected on day 0 as baseline and after completed vaccination. Sera are analyzed for virus neutralizing antibodies.

### Items

**1.** A pharmaceutical composition comprising or consisting of
   - at least one **component A** comprising at least one nucleic acid comprising at least one coding sequence encoding at least one antigenic peptide or protein selected or derived from at least one Coronavirus, or an immunogenic fragment or immunogenic variant thereof, and
   - at least one **component B** comprising at least one nucleic acid comprising at least one coding sequence encoding at least one antigenic peptide or protein selected or derived from at least one further virus, or an immunogenic fragment or immunogenic variant thereof.
**2.** Pharmaceutical composition of **item 1,** wherein the at least one further virus of **component B** is selected from at least one different Coronavirus, at least one Influenza virus, at least one Pneumoviridae virus, and/or at least one Paramyxoviridae virus.
**3.** Pharmaceutical composition of **item 2,** wherein the at least one Pneumoviridae virus is selected from at least one Respiratory syncytial virus, and/or at least one Metapneumovirus.
**4.** Pharmaceutical composition of **item 2,** wherein the at least one Paramyxoviridae virus is selected from at least one Parainfluenza virus, and/or at least one Henipavirus.
**5.** Pharmaceutical composition of **any one of the preceding items,** wherein the at least one Coronavirus of **component A** is selected from at least one pandemic Coronavirus.
**6.** Pharmaceutical composition of **any one of the preceding items,** wherein the at least one Coronavirus of **component A** is selected from at least one Alphacoronavirus, at least one Betacoronavirus, at least one Gammacoronavirus, and/or at least one Deltacoronavirus.
**7.** Pharmaceutical composition of **any one of the preceding items,** wherein the at least one Coronavirus of **component A,** *preferably* the Betacoronavirus, is selected from at least one Sarbecovirus, at least one Merbecovirus, at least one Embecovirus, at least one Nobecovirus, and/or at least one Hibecovirus.
**8.** Pharmaceutical composition of **item 7,** wherein the at least one Sarbecovirus is selected from a SARS-CoV-1 virus and/or a SARS-CoV-2 virus.
**9.** Pharmaceutical composition of **item 7,** wherein the at least one Merbecovirus is selected from a MERS-CoV virus.
**10.** Pharmaceutical composition of **any one of the preceding items,** wherein the at least one Coronavirus of **component A** is a SARS-CoV-2 virus.
**11.** Pharmaceutical composition of **item 10,** wherein the at least one antigenic peptide or protein encoded by the at least one nucleic acid of **component A** comprises or consists at least one peptide or protein selected or derived from a SARS-CoV-2 spike protein (S, S1, S2, or S1 and S2), or an immunogenic fragment or immunogenic variant of any of these.
**12.** Pharmaceutical composition of **item 11,** wherein the spike protein is selected or derived from a pre-fusion stabilized spike protein (S_stab) comprising at least one pre-fusion stabilizing mutation.
**13.** Pharmaceutical composition of **item 12** wherein the at least one pre-fusion stabilizing mutation comprises the following amino acid substitutions: K986P and V987P.
**14.** Pharmaceutical composition of **items 12 to 13,** wherein the at least one pre-fusion stabilizing mutation comprises a cavity filling mutation, *preferably* selected from the list comprising T887W; A1020W; T887W and A1020W; or P1069F.
**15.** Pharmaceutical composition of **items 12 to 14,** wherein the at least one pre-fusion stabilizing mutation comprises a mutated protonation site, *preferably* selected from the list comprising H1048Q and H1064N; H1083N and H1101N; or H1048Q and H1064N and H1083N and H1101N.
**16.** Pharmaceutical composition of **items 12 to 15,** wherein the at least one pre-fusion stabilizing mutation comprises at least one artificial intramolecular disulfide bond, *preferably* selected from the list comprising I712C and T1077C; I714C and Y1110C; P715C and P1069C; G889C and L1034C; I909C and Y1047C; Q965C and S1003C; F970C and G999C; A972C and R995C; A890C and V1040C; T874C and S1055C; N914C and S1123C; or N914C and S1123C.
**17.** Pharmaceutical composition of **items 10 to 16,** wherein the at least one nucleic acid of **component A** additionally encodes at least one heterologous peptide or protein element selected or derived from a signal peptide, a linker, a helper epitope, an antigen clustering element, a trimerization element, a transmembrane element, and/or a VLP-forming sequence.
**18.** Pharmaceutical composition of **item 17,** wherein the at least one heterologous antigen clustering element is selected or derived from a ferritin element, a lumazine synthase element, a surface antigen of Hepatitis B virus (HBsAg), or encapsulin and/or wherein the at least one heterologous trimerization element is selected or derived from a foldon element, preferably a fibritin foldon element and/or wherein the at least one VLP-forming sequence is selected or derived from a Woodchuck hepatitis core antigen element (WhcAg).
**19.** Pharmaceutical composition of **items 10 to 18,** wherein the at least one antigenic peptide or protein selected or derived from SARS-CoV-2 encoded by the at least one nucleic acid of **component A** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 1-111, 274-11663, 13176-13510, 13521-14123, 22732-22758, 22917, 22923, 22929-22964, 26938, or 26939** or an immunogenic fragment or immunogenic variant of any of these.
**20.** Pharmaceutical composition of **items 10 to 19,** wherein the at least one antigenic peptide or protein selected or derived from SARS-CoV-2 encoded by the at least one nucleic acid of **component A** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 10-26, 40-48, 85-111, 341-1278, 1681-2618, 2686-3623, 3691-4628, 4696-5633, 5701-6638, 6706-7643, 7711-8648, 8716-9653, 9721-10658, 10726-11663, 13377-13510, 13521-14123, 22732, 22738, 22740, 22742, 22744, 22746, 22748, 22750, 22752, 22754, 22756, 22758, 22947-22964** or an immunogenic fragment or immunogenic variant of any of these.
**21.** Pharmaceutical composition of **items 10 to 20,** wherein the at least one antigenic peptide or protein is an S protein comprising a pre-fusion stabilizing K986P and V987P mutation comprising or consisting of at least one amino acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 10, 341, 22960, 22961, 22963** or an immunogenic fragment or immunogenic variant thereof.
**22.** Pharmaceutical composition of **items 10 to 21,** wherein the at least one coding sequence of the at least one nucleic acid of **component A** comprises or consists at least one nucleic acid sequence encoding a SARS-CoV-2 antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 116-132, 134-138, 140-143, 145-147, 148-175, 11664-11813, 11815, 11817-12050, 12052, 12054-13147, 13514, 13515, 13519, 13520, 14124-14177, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or variant of any of these sequences.
**23.** Pharmaceutical composition of **items 10 to 22,** wherein the at least one coding sequence of the at least one nucleic acid of **component A** is a codon modified coding sequence comprising or consisting of a nucleic acid sequence encoding a SARS-CoV-2 antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 136-138, 140-143, 145-147, 148-175, 11731-11813, 11815, 11817-12050, 12052, 12054-13147, 14142-14177, 22759, 22764-22786, 22791-22813, 22818-22839, 22969-23184, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or variant of any of these sequences.
**24.** Pharmaceutical composition of **items 10 to 23,** wherein the at least one nucleic acid of **component A** comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 148-175, 12204-13147, 14142-14177, 22786-22839, 23189-23404, 23409-23624, 23629-23844, 23849-24064, 24069-24284, 24289-24504, 24509-24724, 24729-24944, 24949-25164, 25169-25384, 25389-25604, 25609-25824, 25829-26044, 26049-26264, 26269-26484, 26489-26704, 26709-26937** or a fragment or variant of any of these sequences.
**25.** Pharmaceutical composition of **items 10 to 24,** wherein the at least one nucleic acid of **component A** comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 149, 163, 24837, 26633, 26907,** or a fragment or variant of any of these sequences.
**26.** Pharmaceutical composition of **items 10 to 24,** wherein the at least one nucleic acid of **component A** comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 23311, 23531, 24851, 23310, 23530, 24850, 23313, 23533, 24853, 23314, 23534, 24854** or a fragment or variant of any of these sequences.
**27.** Pharmaceutical composition of **any one of the preceding items,** wherein the Coronavirus of **component A** is a SARS-associated virus, *preferably* a SARS-CoV-1 virus.
**28.** Pharmaceutical composition of **item 27,** wherein the at least one antigenic peptide or protein encoded by the at least one nucleic acid of **component A** comprises or consists at least one peptide or protein selected or derived from a spike protein (S, S1, S2, or S1 and S2), or an immunogenic fragment or immunogenic variant of any of these.
**29.** Pharmaceutical composition of **item 28,** wherein the spike protein is selected or derived from a pre-fusion stabilized spike protein (S_stab) comprising at least one pre-fusion stabilizing mutation.
**30.** Pharmaceutical composition of **item 29** wherein the at least one pre-fusion stabilizing mutation comprises the following amino acid substitutions: K968P and V969P.
**31.** Pharmaceutical composition of **items 29 to 30,** wherein the at least one pre-fusion stabilizing mutation comprises at least one selected from at least one cavity filling mutation, at least one mutated protonation site, and/or at least one artificial intramolecular disulfide bond.
**32.** Pharmaceutical composition of **items 27 to 31,** wherein the at least one antigenic peptide or protein selected or derived from a SARS associated virus encoded by the at least one nucleic acid of **component A** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14906-14950** or an immunogenic fragment or immunogenic variant of any of these.
**33.** Pharmaceutical composition of **items 27 to 32,** wherein the at least one coding sequence of the at least one nucleic acid of **component A** comprises or consists at least one nucleic acid sequence encoding a SARS associated virus antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14951-15220** or a fragment or variant of any of these sequences.
**34.** Pharmaceutical composition of **items 27 to 33,** wherein the at least one nucleic acid of **component A** comprises or consists of a nucleic acid sequence encoding a SARS associated virus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 15041-15220** or a fragment or variant of any of these sequences.
**35.** Pharmaceutical composition of **any one of the preceding items,** wherein the Coronavirus of **component A** is a MERS-CoV virus.
**36.** Pharmaceutical composition of **item 35,** wherein the at least one antigenic peptide or protein encoded by the at least one nucleic acid of **component A** comprises or consists at least one peptide or protein selected or derived from a MERS-CoV coronavirus spike protein (S, S1, S2, or S1 and S2), or an immunogenic fragment or immunogenic variant of any of these.
**37.** Pharmaceutical composition of **item 36,** wherein the spike protein is selected or derived from a pre-fusion stabilized spike protein (S_stab) comprising at least one pre-fusion stabilizing mutation.
**38.** Pharmaceutical composition of **item 37** wherein the at least one pre-fusion stabilizing mutation comprises the following amino acid substitutions: V1060P and L1061P.
**39.** Pharmaceutical composition of **items 37 to 38,** wherein the at least one pre-fusion stabilizing mutation comprises at least one selected from at least one cavity filling mutation, at least one mutated protonation site, and/or at least one artificial intramolecular disulfide bond.
**40.** Pharmaceutical composition of **items 35 to 39,** wherein the at least one antigenic peptide or protein of MERS-CoV encoded by the at least one nucleic acid of **component A** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14794-14809** or an immunogenic fragment or immunogenic variant of any of these.
**41.** Pharmaceutical composition of **items 35 to 40,** wherein the at least one coding sequence of the at least one nucleic acid of **component A** comprises or consists at least one nucleic acid sequence encoding a MERS-CoV antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14810-14905** or a fragment or variant of any of these sequences.
**42.** Pharmaceutical composition of **items 35 to 41,** wherein the at least one nucleic acid of **component A** comprises or consists of a nucleic acid sequence encoding a MERS-CoV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14842-14905** or a fragment or variant of any of these sequences.
**43.** Pharmaceutical composition of **any one of the preceding items,** wherein the at least one further virus **of component B** is selected from at least one different Coronavirus, wherein said at least one different Coronavirus is not the Coronavirus of **component A,** *preferably* wherein the at least one different Coronavirus is not SARS-CoV-2.
**44.** Pharmaceutical composition of **item 43,** wherein the at least one nucleic acid of **component B** is selected from at least one nucleic acid encoding a Coronavirus antigen as defined in any one of **items 5 to 42.**
**45.** Pharmaceutical composition of **any one of the preceding items,** wherein the at least one further virus **of component B** is selected from at least one Influenza virus.
**46.** Pharmaceutical composition of **item 45,** wherein at least one **Influenza virus** is selected from Influenza A, Influenza B or Influenza C, *preferably* wherein the at least one Influenza virus is selected from at least one Influenza A virus and/or at least one Influenza B virus.
**47.** Pharmaceutical composition of **item 45 or 46,** wherein the at least one Influenza virus is selected from at least one Influenza A virus selected from at least one of the list comprising H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N7 and H10N8, *preferably* from at least one of H1N1, H3N2, H5N1, H5N8.
**48.** Pharmaceutical composition of **items 45 to 47,** wherein the at least one antigenic peptide or protein encoded by the at least one nucleic acid of **component B** comprises or consists at least one peptide or protein selected or derived from at least one hemagglutinin (HA) or neuraminidase (NA) or an immunogenic fragment or immunogenic variant of any of these.
**49.** Pharmaceutical composition of **items 45 to 48,** wherein the at least one antigenic peptide or protein selected or derived from at least one Influenza virus encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14178-14229,** or an immunogenic fragment or immunogenic variant of any of these.
**50.** Pharmaceutical composition of **items 45 to 49,** wherein the at least one coding sequence of the at least one nucleic acid of **component B** comprises or consists at least one nucleic acid sequence encoding a Influenza virus antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14230-14333, 14334-14541, 26946, 26947-26955** or a fragment or variant of any of these sequences.
**51.** Pharmaceutical composition of **items 45 to 50,** wherein the at least one coding sequence of the at least one nucleic acid of **component B** comprises or consists at least one nucleic acid sequence encoding an Influenza A HA antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14230-14233, 14236-14256, 14282-14285, 14288-14308, 14334-14337, 14340-14360, 14386-14389, 14392-14412, 14438-14441, 14444-14464, 14490-14493, 14496-14516, 26946-26950** or a fragment or variant of any of these sequences.
**52.** Pharmaceutical composition of **items 45 to 51,** wherein the at least one coding sequence of the at least one nucleic acid of **component B** comprises or consists at least one nucleic acid sequence encoding an Influenza A NA antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14366-14369, 14372-14383, 14418-14421, 14424-14435, 14470-14473, 14476-14487, 14522-14525, 14528-14539, 26953, 26954** or a fragment or variant of any of these sequences.
**53.** Pharmaceutical composition of **items 45 to 52,** wherein the at least one coding sequence of the at least one nucleic acid of **component B** comprises or consists at least one nucleic acid sequence encoding an Influenza B antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14234, 14235, 14257-14261, 14266, 14267, 14280, 14281, 14286, 14287, 14309-14313, 14318, 14319, 14332, 14333, 14338, 14339, 14361-14365, 14370, 14371, 14384, 14385, 14390, 14391, 14413-14417, 14422, 14423, 14436, 14437, 14442, 14443, 14465-14469, 14474, 14475, 14488, 14489, 14494, 14495, 14517-14521, 14526, 14527, 14540, 14541, 26951, 26952, 26955,** or a fragment or variant of any of these sequences.
**54.** Pharmaceutical composition of **items 45 to 53,** wherein the at least one nucleic acid of **component B** comprises or consists of a nucleic acid sequence encoding an Influenza virus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14334-14541, 26947-26955,** or a fragment or variant of any of these sequences.
**55.** Pharmaceutical composition of **items 45 to 54,** wherein **component B** comprises a plurality of nucleic acid sequences encoding an Influenza virus antigen, *preferably* 2, 3, 4, 5, 6, 7, 6, 9, 10, 11, 12, 13, 14, 15, 16, 17, 18, 19, 20, 25, 30, 35, 40, 45, 50, 60, 70, 80, or 100 of nucleic acid sequences encoding an Influenza virus antigen, *preferably* wherein each of the plurality is selected from nucleic acid sequences defined in **items 45 to 54.**
**56.** Pharmaceutical composition of **item 55,** wherein the plurality of nucleic acid sequences encodes at least one, *preferably* 2, 3, 4, 5, 6 antigenic peptide or protein selected or derived from HA and at least one, *preferably* 2, 3, 4, 5, 6 antigenic peptide or protein selected or derived from NA of at least one Influenza A virus.
**57.** Pharmaceutical composition of **item 55 or 56,** wherein the plurality of nucleic acid sequences encodes at least one, *preferably* 2, 3, 4, 5, 6 antigenic peptide or protein selected or derived from HA and at least one, *preferably* 2, 3, 4, 5, 6 antigenic peptide or protein selected or derived from NA of at least one Influenza B virus.
**58.** Pharmaceutical composition of **items 45 to 57,** wherein **component B** comprises a plurality of nucleic acid sequences encoding an Influenza virus antigen, wherein the Influenza virus is selected from the list comprising FLUAV/H1N1/A/California/7/2009, FLUAV/H1N1/A/Michigan/45/2015, FLUAV/H1N1/A/Netherlands/602/2009, FLUAV/H3N2/A/Hong Kong/4801/2014, FLUBV/HONO/B/Brisbane/60/2008, FLUBV/H0N0/B/Phuket/3073/2013.
**59.** Pharmaceutical composition of **items 45 to 57,** wherein **component B** comprises a plurality of nucleic acid sequences, *preferably* seven nucleic acid sequences, encoding a plurality of Influenza virus antigens, *preferably* seven Influenza virus antigens, wherein the influenza virus antigens are selected from
   - HA protein of FLUAV/H3N2/A/Hong Kong/4801/2014,
   - HA protein of FLUAV/H1 N1/A/California/7/2009,
   - HA protein of FLUBV/H0N0/B/Phuket/3073/2013,
   - HA protein of FLUBV/H0N0/B/Brisbane/60/2008,
   - NA protein of FLUAV/H3N2/A/Hong Kong/4801/2014,
   - NA protein of FLUAV/H1N1/A/California/7/2009, and
   - NA protein of FLUBV/H0N0/B/Brisbane/60/2008.
**60.** Pharmaceutical composition of **any one of the preceding items,** wherein the at least one further virus **of component B** is selected from at least one Respiratory syncytial virus (RSV) of the Pneumoviridae virus family.
**61.** Pharmaceutical composition of **item 60** wherein the at least one antigenic peptide or protein encoded by the at least one nucleic acid of **component B** comprises or consists at least one peptide or protein selected or derived from at least one RSV fusion protein F, matrix protein M, nucleoprotein N, M2-1 protein, and/or phosphoprotein P, or an immunogenic fragment or immunogenic variant of any of these, *preferably* selected or derived from RSV fusion protein F, or an immunogenic fragment or immunogenic variant thereof.
**62.** Pharmaceutical composition of **item 61,** wherein the at least one antigenic peptide or protein selected or derived from RSV F protein is a full-length F protein (F0) or an F protein with deleted C-terminus (F-del), or an immunogenic fragment or immunogenic variant thereof.
**63.** Pharmaceutical composition of **items 61 to 62,** wherein the RSV F protein is selected or derived from a pre-fusion stabilized F protein (F_stab) comprising at least one pre-fusion stabilizing mutation.
**64.** Pharmaceutical composition of **items 61 to 63,** wherein the RSV F protein comprises the two subunits F2 and F1 in a single polypeptide chain, wherein F2 and F1 are connected via a linker element, *preferably* a GS linker to generate a stable F2-linker-F1 protein, *optionally* wherein said F2-linker-F1 protein lacks aa104-aa144.
**65.** Pharmaceutical composition of **items 60 to 64,** wherein the at least one antigenic peptide or protein selected or derived from at least one RSV encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14542-14551,** or an immunogenic fragment or immunogenic variant of any of these.
**66.** Pharmaceutical composition of **items 60 to 65,** wherein the at least one coding sequence of the at least one nucleic acid of **component B** comprises or consists of a nucleic acid sequence encoding an RSV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14552-14571, 14572-14611,** or a fragment or variant of any of these sequences.
**67.** Pharmaceutical composition of **items 60 to 66,** wherein the at least one nucleic acid of **component B** comprises or consists of a nucleic acid sequence encoding an RSV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14572-14611,** or a fragment or variant of any of these sequences.
**68.** Pharmaceutical composition of **items 60 to 67** comprising at least one, two or three further nucleic acid sequences comprising at least one coding sequence encoding at least one antigenic peptide or protein selected or derived from RSV matrix protein M, nucleoprotein N, M2-1 protein, and/or phosphoprotein P or combinations thereof, preferably selected from M2-1.
**69.** Pharmaceutical composition of **item 68,** wherein the at least one coding sequence of the at least one further artificial nucleic acid sequences encodes at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs:** 9684, 10053-10133, 10134, 10503-10636, 10637, 11006-11182, 11183, 11552-11725, 19844, 20213, 20582, 20951 of published PCT application WO2019202035**,** or a fragment or variant of any of these sequences.
**70.** Pharmaceutical composition of **items 68 to 69,** wherein the at least one coding sequence of the at least one further nucleic acid sequence comprises or consists of a nucleic acid sequence encoding an RSV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs:** 9685-9692, 10135-10142, 10638-10645, 11184-11119, 19845-19852, 20214-20221, 20583-20590, 20952-20959, 21385-21388, 21411-21414 of published PCT application WO2019202035**,** or a fragment or variant of any of these sequences.
**71.** Pharmaceutical composition of **items 68 to 70,** wherein the at least one further nucleic acid comprises or consists of a nucleic acid sequence encoding an RSV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs:** 9693-10052, 10143-10502, 10646-11005, 11192-11551, 19853-20212, 20222-20581, 20591-20950, 20960-21319, 21481-21488, 21627-21634, 21553-21560, 21699-21706 of published PCT application WO2019202035**,** or a fragment or variant of any of these sequences.
**72.** Pharmaceutical composition of **any one of the preceding items,** wherein the at least one further virus **of component B** is selected from at least one Metapneumovirus, *preferably* a human Metapneumovirus (hMPV) of the Pneumoviridae virus family.
**73.** Pharmaceutical composition of **item 72** wherein the at least one antigenic peptide or protein encoded by the at least one nucleic acid of **component B** comprises or consists at least one peptide or protein selected or derived from at least one hMPV F protein, G protein, M protein, P protein, N protein and SH protein, or an immunogenic fragment or immunogenic variant thereof.
**74.** Pharmaceutical composition of **items 72 to 73,** wherein the at least one antigenic peptide or protein selected or derived from at least one hMPV encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14626-14649, 26966-26971,** or an immunogenic fragment or immunogenic variant of any of these.
**75.** Pharmaceutical composition of **items 72 to 74,** wherein the at least one coding sequence of the at least one nucleic acid of **component B** comprises or consists of a nucleic acid sequence encoding an hMPV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14650-14697, 14698-14793, 26972-26978, 26979-26991,** or a fragment or variant of any of these sequences.
**76.** Pharmaceutical composition of **items 72 to 75,** wherein the at least one nucleic acid **of component B** comprises or consists of a nucleic acid sequence encoding an hMPV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14698-14793, 26979-26991,** or a fragment or variant of any of these sequences.
**77.** Pharmaceutical composition of **any one of the preceding items,** wherein the at least one further virus **of component B** is selected from at least one Parainfluenza virus (PIV), *preferably* a human Parainfluenzavirus (hPIV) of the Paramyxoviridae virus family.
**78.** Pharmaceutical composition of **item 77,** wherein the at least one PIV is selected from hPIV-1, hPIV-2, or hPIV-3 serotype, *preferably* hPIV-3 serotype.
**79.** Pharmaceutical composition of **item 77 or 78** wherein the at least one antigenic peptide or protein encoded by the at least one nucleic acid of **component B** comprises or consists at least one peptide or protein selected or derived from at least one PIV hemagglutinin-neuraminidase (HN), PIV fusion (F) glycoprotein, PIV matrix protein (M), PIV nucleocapsid protein (N), PIV viral replicase (L), PIV non-structural V protein, or an immunogenic fragment thereof.
**80.** Pharmaceutical composition of **items 77 to 79,** wherein the at least one antigenic peptide or protein selected or derived from at least one PIV encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14612, 14613, 26956, 26957,** or an immunogenic fragment or immunogenic variant of any of these.
**81.** Pharmaceutical composition of **items 77 to 80,** wherein the at least one coding sequence of the at least one nucleic acid of **component B** comprises or consists of a nucleic acid sequence encoding an PIV antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14614-14617, 14618-14625, 26958-26965,** or a fragment or variant of any of these sequences.
**82.** Pharmaceutical composition of **items 77 to 81,** wherein the at least one nucleic acid of **component B** comprises or consists of a nucleic acid sequence encoding an PIV antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 14618-14625, 26961-26965,** or a fragment or variant of any of these sequences.
**83.** Pharmaceutical composition of **any one of the preceding items,** wherein the at least one further virus **of component B** is selected from at least one Henipavirus of the Paramyxoviridae virus family.
**84.** Pharmaceutical composition **of item 83,** wherein the at least one Henipavirus is selected from at least one Hendra virus and/or at least one Nipah virus.
**85.** Pharmaceutical composition of **item 83 or 84,** wherein the at least one antigenic peptide or protein encoded by the at least one nucleic acid of **component B** comprises or consists at least one peptide or protein selected or derived from at least one Henipavirus glycoprotein and/or at least one Henipavirus fusion protein or a fragment or variant thereof.
**86.** Pharmaceutical composition of **items 83 to 85,** wherein the at least one antigenic peptide or protein selected or derived from at least one Henipavirus encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs:** 1-26, 573-598, 807-832, 1041-1066, 1513-1515 of published PCT patent application WO2018115507 or an immunogenic fragment or immunogenic variant of any of these.
**87.** Pharmaceutical composition of **items 83 to 86,** wherein the at least one coding sequence of the at least one nucleic acid of **component B** comprises or consists of a nucleic acid sequence encoding an Henipavirus antigen comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs:** 27-234, 599-806, 833-1040, 1067-1274, 1275-1508, 1516-1539, 1540-1548 of published PCT patent application WO2018115507 or a fragment or variant of any of these sequences.
**88.** Pharmaceutical composition of **items 83 to 87,** wherein the at least one nucleic acid of **component B** comprises or consists of a nucleic acid sequence encoding an Henipavirus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs:** 1275-1508, 1540-1548 of published PCT patent application WO2018115507 or a fragment or variant of any of these sequences.
**89.** Pharmaceutical composition of **any one of the preceding items,** wherein **component A** is selected from at least one of component
   **A-1** at least one nucleic acid comprising at least one coding sequence encoding at least one antigenic peptide or protein selected or derived from at least one SARS-CoV-2 or an immunogenic fragment or immunogenic variant thereof, *preferably* as defined in **items 10 to 26,** and/or
   **A-2** at least one nucleic acid comprising at least one coding sequence encoding at least one antigenic peptide or protein selected or derived from at least one SARS associated virus or an immunogenic fragment or immunogenic variant thereof, *preferably* as defined in **items 27 to 34,** and/or
   **A-3** at least one nucleic acid comprising at least one coding sequence encoding at least one antigenic peptide or protein selected or derived from at least one MERS-CoV or an immunogenic fragment or immunogenic variant thereof, *preferably* as defined in **items 35 to 42.**
**90.** Pharmaceutical composition of **item 89,** wherein **component A** is selected from **A-1** and **A-2; A-1** and **A-3; A-2** and **A-3;** or **A-1** and **A-2 and A-3.**
**91.** Pharmaceutical composition of **item 89,** wherein **component A** is selected from **component A-1.**
**92.** Pharmaceutical composition of **any one of the preceding items,** wherein **component B** is selected from at least one of component
   **B-1** at least one nucleic acid comprising at least one coding sequence encoding at least one antigenic peptide or protein selected or derived from at least one different Coronavirus or an immunogenic fragment or immunogenic variant thereof, *preferably* as defined in **items 43 to 44,** and/or
   **B-2** at least one nucleic acid comprising at least one coding sequence encoding at least one antigenic peptide or protein selected or derived from at least one Influenza virus or an immunogenic fragment or immunogenic variant thereof, *preferably* as defined in **items 45 to 59,** and/or
   **B-3** at least one nucleic acid comprising at least one coding sequence encoding at least one antigenic peptide or protein selected or derived from at least one Pneumoviridae virus or an immunogenic fragment or immunogenic variant thereof, *preferably* as defined in **items 60 to 76,** and/or
   **B-4** at least one nucleic acid comprising at least one coding sequence encoding at least one antigenic peptide or protein selected or derived from at least one Paramyxoviridae virus or an immunogenic fragment or immunogenic variant thereof, *preferably* as defined in **items 77 to 88.**
**93.** Pharmaceutical composition of **item 92,** wherein **component B** is selected from **B-1** and **B-2**; **B-1** and **B-3; B-1** and **B-4; B-2** and **B-3; B-2** and **B-4;** or **B-3** and **B-4.**
**94.** Pharmaceutical composition of **item 92,** wherein the at least one nucleic acid of **component B** is selected from **B-1** and **B-2** and **B-3; B-1** and **B-2** and **B-4; B-1** and **B-3** and **B-4; B-2** and **B-3** and **B-4.**
**95.** Pharmaceutical composition of **items 92 to 94** wherein **component B-3** is selected from at least one of component
   **B-3a** at least one nucleic acid comprising at least one coding sequence encoding at least one antigenic peptide or protein selected or derived from at least one RSV or an immunogenic fragment or immunogenic variant thereof, *preferably* as defined in **items 60 to 71,** and/or
   **B-3b** at least one nucleic acid comprising at least one coding sequence encoding at least one antigenic peptide or protein selected or derived from at least one hMPV or an immunogenic fragment or immunogenic variant thereof, *preferably* as defined in **items 72 to 76.**
**96.** Pharmaceutical composition of **item 95,** wherein **component B-3** is selected from **B-3a.**
**97.** Pharmaceutical composition of **items 92 to 96,** wherein **component B-4** is selected from
   **B-4a** at least one nucleic acid comprising at least one coding sequence encoding at least one antigenic peptide or protein selected or derived from at least one PIV or an immunogenic fragment or immunogenic variant thereof, *preferably* as defined in **items 77 to 82,** and/or
   **B-4b** at least one nucleic acid comprising at least one coding sequence encoding at least one antigenic peptide or protein selected or derived from at least one Henipavirus or an immunogenic fragment or immunogenic variant thereof, *preferably* as defined in **items 83 to 88.**
**98.** Pharmaceutical composition of **item 97,** wherein **component B4** is selected from **component B-4a.**
**99.** Pharmaceutical composition of **any one of the preceding items,** comprising or consisting of **component A-1** and **component B-3a.**
**100.** Pharmaceutical composition of **any one of the preceding items,** comprising or consisting of **component A-1** and **component B-2.**
**101.** Pharmaceutical composition of **any one of the preceding items,** comprising or consisting of **component A-1** and **component B-2** and **component B-3a.**
**102.** Pharmaceutical composition of **any one of the preceding items,** wherein the at least one coding sequence of the nucleic acid of **component A** and/or the at least one coding sequence of the nucleic acid of **component B** is a codon modified coding sequence, *preferably* wherein the amino acid sequence encoded by the at least one codon modified coding sequence is not being modified compared to the amino acid sequence encoded by the corresponding wild type or reference coding sequence.
**103.** Pharmaceutical composition of **item 102,** wherein the at least one codon modified coding sequence is selected from C maximized coding sequence, CAI maximized coding sequence, human codon usage adapted coding sequence, G/C content modified coding sequence, and G/C optimized coding sequence, or any combination thereof.
**104.** Pharmaceutical composition of **item 102 or 103,** wherein the at least one codon modified coding sequence is a G/C optimized coding sequence, a human codon usage adapted coding sequence, or a G/C content modified coding sequence.
**105.** Pharmaceutical composition of **any one of the preceding items,** wherein the at least one coding sequence **of component A** and/or the at least one nucleic acid **of component B** has a G/C content of at least about 50%, 55%, or 60%.
**106.** Pharmaceutical composition of **any one of the preceding items,** wherein the at least one nucleic acid **of component A** and/or the at least one nucleic acid **of component B** comprises at least one heterologous untranslated region selected from at least one heterologous 5'-UTR and/or at least one heterologous 3'-UTR.
**107.** Pharmaceutical composition of **item 106,** wherein the at least one heterologous 3'-UTR comprises or consists a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.
**108.** Pharmaceutical composition of **item 106 or 107,** wherein the at least one heterologous 5'-UTR comprises or consists of a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes.
**109.** Pharmaceutical composition of **any one of the preceding items, wherein** the nucleic acid of **component A** and/or **component B** comprises at least one coding sequence operably linked to
   - an alpha-globin 3'-UTR; or
   - a HSD17B4 5'-UTR and a PSMB3 3'-UTR (HSD17B4/PSMB3); or
   - a SLC7A3 5'-UTR and a PSMB3 3'-UTR (SLC7A3/PSMB3); or
   - a RPL31 5'-UTR and a RPS9 3'-UTR (RPL31/RPS9).
**110.** Pharmaceutical composition **any one of the preceding items, wherein** the nucleic acid of **component A** and/or **component B** comprises at least one coding sequence operably linked to a HSD17B4 5'-UTR and a PSMB3 3'-UTR (HSD17B4/PSMB3).
**111.** Pharmaceutical composition of **any one of the preceding items,** wherein the at least one nucleic acid of **component A** and/or the at least one nucleic acid of **component B** comprises at least one poly(A) sequence, *preferably* comprising 30 to 200 adenosine nucleotides, *optionally*
   wherein the at least one nucleic acid of **component A** and/or the at least one nucleic acid of **component B** comprises at least one poly(C) sequence, *preferably* comprising 10 to 40 cytosine nucleotides.
**112.** Pharmaceutical composition of **any one of the preceding items,** wherein the at least one nucleic acid of **component A** and/or the at least one nucleic acid of **component B** comprises at least one histone stem-loop or histone stem-loop structure.
**113.** Pharmaceutical composition of **any one of the preceding items,** wherein the at least one nucleic acid of **component A** and/or the at least one nucleic acid of **component B** is a DNA or an RNA.
**114.** Pharmaceutical composition of **any one of the preceding items,** wherein the at least one nucleic acid of **component A** and/or the at least one nucleic acid of **component B** is a coding RNA, *preferably* an mRNA, a self-replicating RNA, a circular RNA, or a replicon RNA.
**115.** Pharmaceutical composition of **any one of the preceding items,** wherein the at least one nucleic acid of **component A** and/or the at least one nucleic acid of **component B** is an mRNA, *preferably* a purified mRNA.
**116.** Pharmaceutical composition **of any one of the preceding items,** wherein the at least one nucleic acid of **component A** and/or the at least one nucleic acid of **component B** is an RNA, wherein the RNA has an RNA integrity of at least about 50%, preferably of at least about 60%, more preferably of at least about 70%, most preferably of at least about 80%.
**117.** Pharmaceutical composition of **any one of the preceding items,** wherein the at least one nucleic acid **of component A** and/or the at least one nucleic acid of **component B** comprises a 5'-cap structure, *preferably* m7G, cap0, cap1, cap2, a modified cap0 or a modified cap1 structure.
**118.** Pharmaceutical composition of **any one of the preceding items,** wherein the at least one nucleic acid **of component A** and/or the at least one nucleic acid of **component B** comprises at least one modified nucleotide.
**119.** Pharmaceutical composition of **any one of the preceding items,** wherein the at least one nucleic acid **of component A** and/or the at least one nucleic acid of **component B** comprises at least one modified nucleotide selected from pseudouridine (Ψ) and N1-methylpseudouridine (m1Ψ), *optionally* wherein all uracil nucleotides are replaced by pseudouridine (Ψ) nucleotides and/or N1-methylpseudouridine (m1Ψ) nucleotides.
**120.** Pharmaceutical composition of **items 1 to 117,** wherein the at least one nucleic acid of **component A** and/or the at least one nucleic acid of **component B** is an RNA that does not comprise chemically modified nucleotides.
**121.** Pharmaceutical composition of **items 1 to 117,** wherein the at least one nucleic acid of **component A** and/or the at least one nucleic acid of **component B** comprises a coding sequence that consists only of G, C, A and U nucleotides.
**122.** Pharmaceutical composition of **any one of the preceding items,** comprising at least one pharmaceutically acceptable carrier.
**123.** Pharmaceutical composition of **any one of the preceding items,** wherein the at least one nucleic acid of **component A** and/or the at least one nucleic acid of **component B** is complexed or associated with or at least partially complexed or partially associated with one or more cationic or polycationic compound, *preferably* cationic or polycationic polymer, cationic or polycationic polysaccharide, cationic or polycationic lipid, cationic or polycationic protein, cationic or polycationic peptide, or any combinations thereof.
**124.** Pharmaceutical composition of **any one of the preceding items,** wherein the at least one nucleic acid of **component A** and/or the at least one nucleic acid of **component B** is complexed or associated with one or more lipids, thereby forming liposomes, lipid nanoparticles (LNP), lipoplexes, and/or nanoliposomes.
**125.** Pharmaceutical composition of **any one of the preceding items,** wherein the at least one nucleic acid of **component A** and/or the at least one nucleic acid of **component B** is complexed or associated with one or more lipids thereby forming lipid nanoparticles (LNPs).
**126.** Pharmaceutical composition of **any one of the preceding items,** wherein the at least one nucleic acid of **component A** and/or the at least one nucleic acid of **component B** are formulated separately, *preferably* wherein the at least one nucleic acid **component A** and/or the at least one nucleic acid **component B** are formulated in separate liposomes, lipid nanoparticles (LNP), lipoplexes, and/or nanoliposomes.
**127.** Pharmaceutical composition of **any one of the preceding items,** wherein the at least one nucleic acid of **component A** and/or the at least one nucleic acid of **component B** are co-formulated, *preferably* wherein the at least one nucleic acid of **component A** and/or the at least one nucleic acid of **component B** are co-formulated in liposomes, lipid nanoparticles (LNP), lipoplexes, and/or nanoliposomes.
**128.** Pharmaceutical composition of **items 124 to 127,** wherein the liposomes, lipid nanoparticles (LNP), lipoplexes, and/or nanoliposomes comprises at least one cationic lipid, *preferably* a cationic lipid according to formula III-3: 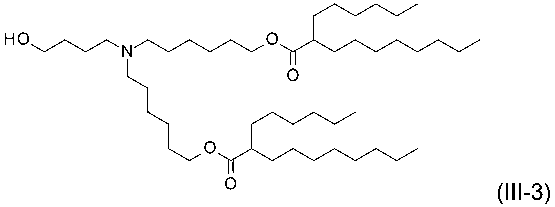
**129.** Pharmaceutical composition of **items 124 to 128,** wherein the liposomes, lipid nanoparticles (LNP), lipoplexes, and/or nanoliposomes comprises at least one polymer conjugated lipid, *preferably* a PEG lipid, *preferably* a PEG-lipid according to formula (IVa): 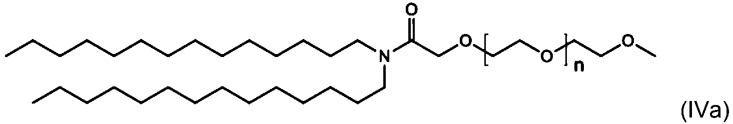 wherein n has a mean value ranging from 30 to 60, *preferably* wherein n has a mean value of about 45, 46, 47, 48, 49, 50, 51, 52, 53, 54, *most preferably* wherein n has a mean value of 49 or 45.
**130.** Pharmaceutical composition of **items 124 to 128,** wherein the liposomes, lipid nanoparticles (LNP), lipoplexes, and/or nanoliposomes comprises at least one polymer conjugated lipid, *preferably* a PEG lipid, *preferably* a PEG-lipid according to formula (IVa): 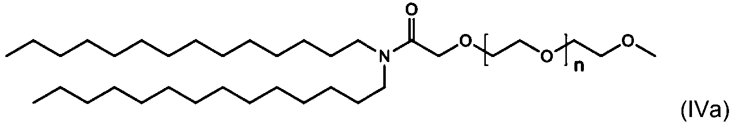 wherein n is an integer selected such that the average molecular weight of the PEG lipid is about 2000g/mol to about 3000g/mol or about 2300g/mol to about 2700g/mol, preferably about 2500g/mol.
**131.** Pharmaceutical composition of **items 124 to 130,** wherein the liposomes, lipid nanoparticles (LNP), lipoplexes, and/or nanoliposomes comprises one or more neutral lipids and/or one or more steroid or steroid analogues.
**132.** Pharmaceutical composition of **item 131,** wherein the neutral lipid is 1,2-distearoyl-sn-glycero-3-phosphocholine (DSPC), *preferably* wherein the molar ratio of the cationic lipid to DSPC is in the range from about 2:1 to about 8:1.
**133.** Pharmaceutical composition of **item 131 or 132,** wherein the steroid is cholesterol, *preferably* wherein the molar ratio of the cationic lipid to cholesterol is in the range from about 2:1 to about 1:1
**134.** Pharmaceutical composition of **items 124 to 133,** wherein the liposome, lipid nanoparticle (LNP), lipoplex, and/or nanoliposome, *preferably* the LNP comprises or consists of
   (i) at least one cationic lipid, *preferably* at least one cationic lipid as defined in **item 128;**
   (ii) a neutral lipid, *preferably* a neutral lipid as defined in **item 132;**
   (iii) a steroid or steroid analogue, *preferably* a steroid or steroid analogue as defined in **item 133;** and
   (iv) polymer conjugated lipid, *preferably* a PEG-lipid, e.g. PEG-DMG or PEG-cDMA, *preferably* a PEG-lipid as defined in **item 129 or 130.**
**135.** Pharmaceutical composition of **item 134,** wherein (i) to (iv) are in a molar ratio of about 20-60% cationic lipid, 5-25% neutral lipid, 25-55% sterol, and 0.5-15% polymer-conjugated lipid, *preferably* PEG-lipid.
**136.** Pharmaceutical composition of **item 134 or 135,** wherein (i) to (iv) are in a molar ratio of about 50:10:38.5:1.5, preferably 47.5:10:40.8:1.7 or more preferably 47.4:10:40.9:1.7.
**137.** Pharmaceutical composition **of items 124 to 136,** wherein the wt/wt ratio of lipid to nucleic acid is from about 10:1 to about 60:1, preferably from about 20:1 to about 30:1, for example about 25:1.
**138.** Pharmaceutical composition **of items 124 to 137,** wherein the n/p ratio of the liposome, lipid nanoparticle (LNP), lipoplex, and/or nanoliposome encapsulating the nucleic acid is in a range from about 1 to about 10, preferably in a range from about 5 to about 7, more preferably about 6.
**139.** Pharmaceutical composition **of items 124 to 138,** wherein the composition has a polydispersity index (PDI) value of less than about 0.4, preferably of less than about 0.3, more preferably of less than about 0.2, most preferably of less than about 0.1.
**140.** Pharmaceutical composition **of items 124 to 139,** wherein the LNPs have a Z-average size in a range of about 60nm to about 120nm, preferably less than about 120nm.
**141.** Pharmaceutical composition **of items 124 to 140,** wherein the composition comprises less than about 20% free (non complexed) RNA, preferably less than about 15% free (non complexed) RNA, more preferably less than about 10% free (non complexed) RNA.
**142.** Pharmaceutical composition of **any one of the preceding items,** wherein the composition is a lyophilized composition, a spray-dried composition, or a spray-freeze dried composition, *optionally* comprising at least one pharmaceutically acceptable lyoprotectant.
**143.** Pharmaceutical composition of **item 142,** wherein the composition the lyophilized or spray-dried composition has a water content of less than about 10%, *preferably* a water content of between about 0.5% and 5%.
**144.** Pharmaceutical composition of **any one of the preceding items,** wherein when administered to a subject, the at least one nucleic acid of **component A** and the at least one nucleic acid of **component B** are translated into an antigenic peptide or protein suitable for inducing an immune response.
**145.** Pharmaceutical composition of **any one of the preceding items,** wherein when administered to a subject, an antigen-specific humoral immune responses against the peptide or protein encoded by **component A** and an antigen-specific humoral immune responses against the peptide or protein encoded by **component B** is induced in said subject.
**146.** Pharmaceutical composition of **any one of the preceding items,** wherein when administered to a subject, an antigen-specific T-cell responses against the peptide or protein encoded by **component A** and an antigen-specific T-cell responses against the peptide or protein encoded by **component B** is induced in said subject.
**147.** Pharmaceutical composition of **any one of the preceding items,** wherein upon administration to a subject, an antigen-specific B-cell memory against the peptide or protein encoded by **component A** and an antigen-specific B-cell memory against the peptide or protein encoded by **component B** is established in said subject.
**148.** A combination vaccine comprising at least one **component A** and at least one **component B** as defined in **items 1 to 147.**
**149.** A combination vaccine **of item 148,** wherein the combination vaccine is against at least one coronavirus, *preferably* SARS-CoV-2, and at least one influenza virus and/or RSV virus.
**150.** A combination vaccine **of items 148 to 149,** wherein the combination vaccine is against at least one coronavirus, *preferably* SARS-CoV-2, at least one influenza virus, at least one RSV virus, and, optionally, against at least one PIV and/or at least one hMPV.
**151.** Combination vaccine **of items 148 to 150,** wherein the combination vaccine elicits antigen-specific immune responses comprising T-cell responses and/or B-cell responses against all encoded antigens provided by the at least one nucleic acid of **component A** and additionally elicits an antigen-specific immune responses comprising T-cell responses and/or B-cell responses against all encoded antigens provided by the at least one nucleic acid of **component B.**
**152.** Combination vaccine **of items 148 to 151,** wherein the vaccine elicits antigen-specific immune responses comprising T-cell responses and/or B-cell responses against all encoded antigens upon administration of a low dose of the vaccine, *preferably* wherein the low dose comprises less than about 20µg nucleic acid.
**153.** Combination vaccine **of items 148 to 152,** wherein the combination vaccine elicits antigen-specific immune responses in a subject that has an age of about 5 years old or younger.
**154.** Combination vaccine **of items 148 to 153,** wherein the combination vaccine elicits antigen-specific immune responses in a subject that has an age of about 60 years old or older.
**155.** A Kit or kit of parts, comprising at least one pharmaceutical composition of **items 1 to 147,** and/or at least one combination vaccine of **items 148 to 153,** *optionally* comprising at least one liquid vehicle for solubilising, and, *optionally,* technical instructions providing information on administration and dosage of the kit components.
**156.** Pharmaceutical composition of **items 1 to 147,** combination vaccine of **items 148 to 153,** kit or kit of parts of **item 155,** for use as a medicament.
**157.** Pharmaceutical composition of **items 1 to 147,** combination vaccine of **items 148 to 153,** kit or kit of parts of **item 155,** for use in the treatment or prophylaxis of an infection with a coronavirus, *preferably* SARS-CoV-2, and with at least one further virus, *preferably* wherein said further virus is selected from at least one different Coronavirus, at least one Influenza virus, at least one Pneumoviridae virus, and/or at least one Paramyxoviridae virus.
**158.** Pharmaceutical composition of **items 1 to 147,** combination vaccine of **items 148 to 153,** kit or kit of parts of **item 155,** for use in the treatment or prophylaxis of an infection with at least one coronavirus, *preferably* SARS-CoV-2, and at least one influenza virus, or of a disorder related to such an infection.
**159.** Pharmaceutical composition of **items 1 to 147,** combination vaccine of **items 148 to 153,** kit or kit of parts of **item 155,** for use in the treatment or prophylaxis of an infection with at least one coronavirus, *preferably* SARS-CoV-2, and at least one RSV, or of a disorder related to such an infection.
**160.** Pharmaceutical composition of **items 1 to 147,** combination vaccine of **items 148 to 153,** kit or kit of parts of **item 155,** for use in the treatment or prophylaxis of an infection with at least one coronavirus, *preferably* SARS-CoV-2, at least one RSV, and at least one influenza virus, or a disorder related to such an infection.
**161.** A method of treating or preventing a disorder, wherein the method comprises applying or administering to a subject in need thereof the pharmaceutical composition of **items 1 to 147,** the combination vaccine of **items 148 to 153,** or the kit or kit of parts of **item 155.**
**162.** Method of treating or preventing a disorder of **item 161,** wherein the disorder is an infection with a coronavirus, *preferably* SARS-CoV-2, and an infection with at least one further virus, *optionally* wherein said further virus is selected from at least one different Coronavirus, at least one Influenza virus, at least one Pneumoviridae virus, and/or at least one Paramyxoviridae virus, or of a disorder related to these infections.
**163.** Method of treating or preventing a disorder of **item 161 or 162,** wherein the subject in need is a mammalian subject, *preferably* a human subject.
**164.** Method of treating or preventing a disorder of **item 161 or 163,** wherein the subject in need is a human subject, *preferably* an elderly human subject.
**165.** Method of treating or preventing a disorder of **item 161 or 164,** wherein the method prevents moderate and severe COVID-19 disease in at least 80%, 85%, 90% or 95% of treated subjects from about 2 weeks to about 3 month, 6 months, 9 months, 1 year, 1.5 years, 2 years or 3 years after said administration and prevents at least one further disease associated with a Coronavirus infection, an Influenza virus infection, a Pneumoviridae virus infection, and/or a Paramyxoviridae virus infection.
**166.** Method of treating or preventing a disorder of **item 161 or 165,** wherein the subject is administered a single dose that comprises between about 5ug and about 50µg of RNA.
**167.** Method of treating or preventing a disorder of **item 161 or 165,** wherein the subject is administered a two doses that comprise between about 5µg and about 50µg of RNA.
**168. A method of stabilizing** a pharmaceutical composition **of items 1 to 147** or a combination vaccine of **items 148 to153** comprising lyophilizing the composition or vaccine to produce a stabilized composition or vaccine.
**169.** Method of stabilizing **of item 168,** wherein the stabilized composition or vaccine has a water content of less than about 10%, preferably a water content of between about 0.5% and 5.0%
**170. A stabilized, lyophilized composition or vaccine** produced by a method of **items 168 to 169.**

## Claims

1. A pharmaceutical composition comprising or consisting of
- at least one **component A** comprising at least one nucleic acid comprising at least one coding sequence encoding at least one antigenic peptide or protein selected or derived from a critical neutralisation domain (CND), a truncated receptor-binding domain (truncRBD) or a receptor-binding domain (RBD) of a SARS-CoV-2 Spike protein, or an immunogenic fragment or immunogenic variant thereof, and
- at least one **component B** comprising at least one nucleic acid comprising at least one coding sequence encoding at least one antigenic peptide or protein selected or derived from at least one hemagglutinin (HA) from at least one Influenza virus, or an immunogenic fragment or immunogenic variant thereof,
wherein the at least one nucleic acid of component A and the at least one nucleic acid of component B is an mRNA,
wherein the mRNAs are associated with one or more lipids, thereby forming lipid nanoparticles (LNPs).

2. Pharmaceutical composition of **claim 1,** wherein the at least one nucleic acid of **component A** additionally encodes at least one heterologous peptide or protein element selected or derived from a signal peptide; a linker; a helper epitope; an antigen clustering element, preferably selected or derived from a ferritin element, a lumazine synthase element, a surface antigen of Hepatitis B virus (HBsAg), or encapsuling; a trimerization element, preferably selected or derived from a foldon element, more preferably a fibritin foldon element, and/or wherein the at least one VLP-forming sequence is selected or derived from a Woodchuck hepatitis core antigen element (WhcAg); a transmembrane element; and/or a VLP-forming sequence.

3. Pharmaceutical composition of **claim 1 or 2,** wherein the at least one antigenic peptide or protein encoded by the at least one nucleic acid of **component A** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 13310-13376, 22917, 22923, 13243-13309, 22733-22736, 26938 or 26939,** or an immunogenic fragment or immunogenic variant of any of these.

4. Pharmaceutical composition of any of **claims 1 to 3,** wherein the at least one coding sequence of the at least one nucleic acid of **component A** is a codon modified coding sequence comprising or consisting of a nucleic acid sequence encoding a SARS-CoV-2 antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 22918-22922, 22924-22928, 13512, 13517, 22760, 22965, 22787, 22814, 23185, 23405, 23625, 23845, 24065, 24285, 24505, 24725, 24945, 25165, 25385, 25605, 25825, 26045, 26265, 26485, 26705, 22761, 22966, 22788, 22815, 23186, 23406, 23626, 23846, 24066, 24286, 24506, 24726, 24946, 25166, 25386, 25606, 25826, 26046, 26266, 26486, 26706, 22762, 22967, 22789, 22816, 23187, 23407, 23627, 23847, 24067, 24287, 24507, 24727, 24947, 25167, 25387, 25607, 25827, 26047, 26267, 26487, 26707, 22763, 22968, 23188, 23408, 23628, 23848, 24068, 24288, 24508, 24728, 24948, 25168, 25388, 25608, 25828, 26048, 26268, 26488, 26708, 26940, 26942, 26943, 26941, 26944, 26945** or a fragment or variant of any of these sequences.

5. Pharmaceutical composition of any of **claims 1 to 4,** wherein the at least one nucleic acid of component **A** comprises or consists of a nucleic acid sequence encoding a SARS-CoV-2 antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs: 22787, 22814, 23185, 23405, 23625, 23845, 24065, 24285, 24505, 24725, 24945, 25165, 25385, 25605, 25825, 26045, 26265, 26485, 26705, 22788, 22815, 23186, 23406, 23626, 23846, 24066, 24286, 24506, 24726, 24946, 25166, 25386, 25606, 25826, 26046, 26266, 26486, 26706, 22789, 22816, 23187, 23407, 23627, 23847, 24067, 24287, 24507, 24727, 24947, 25167, 25387, 25607, 25827, 26047, 26267, 26487, 26707, 23188, 23408, 23628, 23848, 24068, 24288, 24508, 24728, 24948, 25168, 25388, 25608, 25828, 26048, 26268, 26488, 26708, 26942, 26943, 26944, 26945or** a fragment or variant of any of these sequences.

6. Pharmaceutical composition of any of **claims 1 to 5,** wherein the spike protein is selected or derived from a pre-fusion stabilized spike protein (S_stab) comprising at least one pre-fusion stabilizing mutation, wherein the at least one pre-fusion stabilizing mutation preferably comprises at least one selected from at least one cavity filling mutation, at least one mutated protonation site, and/or at least one artificial intramolecular disulfide bond.

7. Pharmaceutical composition of any of **claims** 1 **to 6,** wherein at least one **Influenza virus** is selected from Influenza A, Influenza B or Influenza C, *preferably* wherein the at least one Influenza virus is selected from at least one Influenza A virus and/or at least one Influenza B virus.

8. Pharmaceutical composition of any of **claims 1 to 7,** wherein the at least one Influenza virus is selected from at least one Influenza A virus selected from at least one of the list comprising H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N7 and H10N8, *preferably from* at least one of H1N1, H3N2, H5N1, H5N8.

9. Pharmaceutical composition of any of **claims 1 to 8,** wherein the at least one antigenic peptide or protein selected or derived from at least one hemagglutinin (HA) from at least one Influenza virus encoded by the at least one nucleic acid of **component B** comprises or consists of at least one of the amino acid sequences being identical or at least 70%, 80%, 85%, 86%, 87%, 88%, 89%, 90%, 91%, 92%, 93%, 94%, 95%, 96%, 97%, 98%, or 99% identical to any one of **SEQ ID NOs: 14178-14209,** preferably to any one of SEQ ID NOs: 14178, 14181, 14182 or 14183, or an immunogenic fragment or immunogenic variant of any of these.

10. Pharmaceutical composition of any of **claims 1 to 9,** wherein the at least one coding sequence of the at least one nucleic acid of **component B** comprises or consists at least one nucleic acid sequence encoding an Influenza A HA antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14230-14233, 14236-14256, 14282-14285, 14288-14308, 14334-14337, 14340-14360, 14386-14389, 14392-14412, 14438-14441, 14444-14464, 14490-14493, 14496-14516, 26946-26950** or a fragment or variant of any of these sequences.

11. Pharmaceutical composition of any of **claims 1 to 10,** wherein the at least one coding sequence of the at least one nucleic acid of **component B** comprises or consists at least one nucleic acid sequence encoding an Influenza B antigen being identical or at least 70%, 80%, 85%, 86%, 87%, 88%, 89%, 90%, 91%, 92%, 93%, 94%, 95%, 96%, 97%, 98%, or 99% identical to any one of the nucleic acid sequences selected from **SEQ ID NOs: 14234, 14235, 14257-14261, 14286, 14287, 14309-14313, 14338, 14339, 14361-14365, 14390, 14391, 14413-14417, 14442, 14443, 14465-14469, 14494, 14495, 14517-14521, 26951, 26952,** or a fragment or variant of any of these sequences.

12. Pharmaceutical composition of any of **claims 1 to 11,** wherein the at least one nucleic acid of **component B** comprises or consists of a nucleic acid sequence encoding an Influenza virus antigen which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from **SEQ ID NOs:,** 14334-14365, 14386-14417, 14438-14469, 14490-14521, 26947-26952 or a fragment or variant of any of these sequences.

13. Pharmaceutical composition of any of **claims 1 to 12,** wherein **component B** comprises a plurality of nucleic acid sequences encoding an Influenza virus antigen, *preferably* 2, 3, 4, 5, 6, 7, 6, 9, 10, 11, 12, 13, 14, 15, 16, 17, 18, 19, 20, 25, 30, 35, 40, 45, 50, 60, 70, 80, or 100 of nucleic acid sequences encoding an Influenza virus antigen, *preferably* wherein each of the plurality is selected from nucleic acid sequences defined in any of **claims 1 to 12.**

14. Pharmaceutical composition of **claim 13,** wherein the plurality of nucleic acid sequences encodes at least one, *preferably* 2, 3, 4, 5, 6, antigenic peptide or protein selected or derived from HA and at least one, *preferably* 2, 3, 4, 5, 6, antigenic peptide or protein selected or derived from NA of at least one Influenza A virus.

15. Pharmaceutical composition of **claim 13 or 14,** wherein the plurality of nucleic acid sequences encodes at least one, *preferably* 2, 3, 4, 5, 6, antigenic peptide or protein selected or derived from HA and at least one, *preferably* 2, 3, 4, 5, 6, antigenic peptide or protein selected or derived from NA of at least one Influenza B virus.

16. Pharmaceutical composition of any of **claims 1 to 16,** wherein **component B** comprises a plurality of nucleic acid sequences encoding an Influenza virus antigen, wherein the Influenza virus is selected from the list comprising FLUAV/H1N1/A/California/7/2009, FLUAV/H1N1/A/Michigan/45/2015, FLUAV/H1N1/A/Netherlands/602/2009, FLUAV/H3N2/A/Hong Kong/4801/2014, FLUBV/H0N0/B/Brisbane/60/2008, FLUBV/H0N0/B/Phuket/3073/2013.

17. Pharmaceutical composition of any of **claims 1 to 16,** wherein **component B** comprises a plurality of nucleic acid sequences, *preferably* seven nucleic acid sequences, encoding a plurality of Influenza virus antigens, *preferably* seven Influenza virus antigens, wherein the influenza virus antigens are selected from
- HA protein of FLUAV/H3N2/A/Hong Kong/4801/2014,
- HA protein of FLUAV/H1N1/A/California/7/2009,
- HA protein of FLUBV/H0N0/B/Phuket/3073/2013,
- HA protein of FLUBV/H0N0/B/Brisbane/60/2008,
- NA protein of FLUAV/H3N2/A/Hong Kong/4801/2014,
- NA protein of FLUAV/H1N1/A/California/7/2009, and
- NA protein of FLUBV/H0N0/B/Brisbane/60/2008.

18. Pharmaceutical composition of **any one of the preceding claims,** wherein the at least one coding sequence of the nucleic acid of **component A** and/or the at least one coding sequence of the nucleic acid of **component B** is a codon modified coding sequence, *preferably* wherein the amino acid sequence encoded by the at least one codon modified coding sequence is not being modified compared to the amino acid sequence encoded by the corresponding wild type or reference coding sequence.

19. Pharmaceutical composition of **claim 18,** wherein the at least one codon modified coding sequence is selected from C maximized coding sequence, CAI maximized coding sequence, human codon usage adapted coding sequence, G/C content modified coding sequence, and G/C optimized coding sequence, or any combination thereof.

20. Pharmaceutical composition of **any one of the preceding claims,** wherein the at least one coding sequence **of component A** and/or the at least one nucleic acid of **component B** has a G/C content of at least about 50%, 55%, or 60%.

21. Pharmaceutical composition of **any one of the preceding claims,** wherein the at least one nucleic acid **of component A** and/or the at least one nucleic acid **of component B** comprises at least one heterologous untranslated region selected from at least one heterologous 5'-UTR and/or at least one heterologous 3'-UTR.

22. Pharmaceutical composition of **any one of the preceding claims,** wherein the at least one nucleic acid of **component A** and/or the at least one nucleic acid of **component B** comprises at least one poly(A) sequence, *preferably* comprising 30 to 200 adenosine nucleotides, *optionally* wherein the at least one nucleic acid of **component** A and/or the at least one nucleic acid of **component B** comprises at least one poly(C) sequence, *preferably* comprising 10 to 40 cytosine nucleotides.

23. Pharmaceutical composition of **any one of the preceding claims,** wherein the at least one nucleic acid of **component A** and/or the at least one nucleic acid of **component B** comprises at least one histone stem-loop or histone stem-loop structure.

24. Pharmaceutical composition **of any one of the preceding claims,** wherein the at least one nucleic acid of **component A** and/or the at least one nucleic acid of **component B** is purified mRNA, wherein the mRNA has an RNA integrity of at least about 50%, preferably of at least about 60%, more preferably of at least about 70%, most preferably of at least about 80%.

25. Pharmaceutical composition of **any one of the preceding claims,** wherein the at least one nucleic acid **of component A** and/or the at least one nucleic acid of **component B** comprises a 5'-cap structure, *preferably* m7G, cap0, cap1, cap2, a modified cap0 or a modified cap1 structure.

26. Pharmaceutical composition of **any one of the preceding claims,** wherein the at least one nucleic acid **of component A** and/or the at least one nucleic acid of **component B** comprises at least one modified nucleotide.

27. Pharmaceutical composition of **any one of the preceding claims,** wherein the at least one nucleic acid **of component A** and/or the at least one nucleic acid of **component B** comprises at least one modified nucleotide selected from pseudouridine (Ψ) and N1-methylpseudouridine (m1Ψ), *optionally* wherein all uracil nucleotides are replaced by pseudouridine (Ψ) nucleotides and/or N1-methylpseudouridine (m1Ψ) nucleotides.

28. Pharmaceutical composition of **any one of the preceding claims,** wherein the at least one nucleic acid of **component A** and/or the at least one nucleic acid of **component B** are formulated separately.

29. Pharmaceutical composition of **any one of the preceding claims,** wherein the at least one nucleic acid of **component A** and/or the at least one nucleic acid of **component B** are co-formulated.

30. Pharmaceutical composition of **any one of the preceding claims,** wherein the composition is a lyophilized composition, a spray-dried composition, or a spray-freeze dried composition, *optionally* comprising at least one pharmaceutically acceptable lyoprotectant.

31. A combination vaccine comprising at least one **component A** and at least one **component B** as defined in any of **claims 1 to 30.**

32. A combination vaccine **of claim 31,** wherein the combination vaccine is against at least one coronavirus, *preferably* SARS-CoV-2, and at least one influenza virus.

33. A Kit or kit of parts, comprising at least one pharmaceutical composition of any of **claims 1 to 30,** and/or at least one combination vaccine of **claim 31 or 32,** *optionally* comprising at least one liquid vehicle for solubilising, and, *optionally,* technical instructions providing information on administration and dosage of the kit components.

34. Pharmaceutical composition of any of **claims 1 to 30,** combination vaccine of **claim 31 or 32,** or kit or kit of parts of **claim 33,** for use as a medicament.

35. Pharmaceutical composition of any of **claims 1 to 30,** combination vaccine of **claim 31 or 32,** or kit or kit of parts of claim 33, for use in the treatment or prophylaxis of an infection with SARS-CoV-2 and at least one Influenza virus, or of a disorder related to such an infection.

36. A method of producing a pharmaceutical composition as defined in any one of claims 1 to 30, or a combination vaccine as defined in claim 31 or 32, comprising a step of complexing the at least one component A and the at least one component B with one or more lipids, thereby forming liposomes, lipid nanoparticles lipoplexes, and/or nanoliposomes.

37. Method of producing of claim 36, wherein the at least one nucleic acid of component A and the at least one nucleic acid of component B are formulated separately, and/or wherein the at least one nucleic acid of component A and the at least one nucleic acid of component B are co-formulated.

38. Method of producing of claim 36 or 37, wherein the at least one nucleic acid of component A and the at least one nucleic acid of component B are produced by a step of RNA in vitro transcription.

39. Method of producing of any one of claims 36 to 38, comprising a step of purifying the obtained RNA.

40. Method of producing of any one of claims 36 to 39, wherein the method comprises a step of lyophilizing the pharmaceutical composition or combination vaccine and/or filling the pharmaceutical composition or combination vaccine.
